# EUROPÄISCHE PATENTSCHRIFT

(11) **EP 2 225 223 B1**
(45) Veröffentlichungstag und Bekanntmachung des Hinweises auf die Patenterteilung: **11.01.2017**
(21) Anmeldenummer: 08851956.6
(22) Anmeldetag: 21.11.2008
(51) Int. Cl.: C07D 239/32, C07D 401/14, C07D 471/04, A61K 31/4188, A61K 31/4196, A61K 31/437, A61K 31/506, A61K 31/5513, A61P 3/10, A61P 9/00, A61P 17/00, A61P 19/00, A61P 25/06

(54) **ORGANISCHE VERBINDUNGEN**
ORGANIC COMPOUNDS
COMPOSÉS ORGANIQUES

(30) Priorität: 22.11.2007 EP 07121347
(43) Veröffentlichungstag der Anmeldung: 08.09.2010
(60) Teilanmeldung: 10172020.9
(73) Patentinhaber: Boehringer Ingelheim International GmbH, 55216 Ingelheim am Rhein (DE)
(72) Erfinder: GOTTSCHLING, Dirk, 55216 Ingelheim am Rhein (DE); DAHMANN, Georg, 55216 Ingelheim am Rhein (DE); DOODS, Henri, 55216 Ingelheim am Rhein (DE); HEIMANN, Annekatrin, 55216 Ingelheim am Rhein (DE); MUELLER, Stephan Georg, 55216 Ingelheim am Rhein (DE); RUDOLF, Klaus, 55216 Ingelheim am Rhein (DE); SCHAENZLE, Gerhard, 55216 Ingelheim am Rhein (DE); STENKAMP, Dirk, 55216 Ingelheim am Rhein (DE)
(74) Vertreter: Simon, Elke Anna Maria
(86) Internationale Anmeldenummer: PCT/EP2008/065960
(87) Internationale Veröffentlichungsnummer: WO 2009/065919

(56) Entgegenhaltungen:
- WO-A-2006/127588

## Beschreibung

Gegenstand der vorliegenden Erfindung sind neue CGRP-Antagonisten der allgemeinen Formel **I** 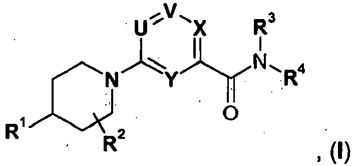 in der **U, V, X, Y, R¹, R², R³** und **R⁴** wie nachstehend erwähnt definiert sind, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

### DETAILLIERTE BESCHREIBUNG DER ERFINDUNG

In der obigen allgemeinen Formel I bedeuten in einer ersten Ausführungsform
- **R¹**: eine Gruppe ausgewählt aus

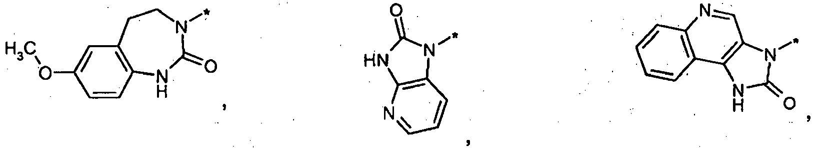

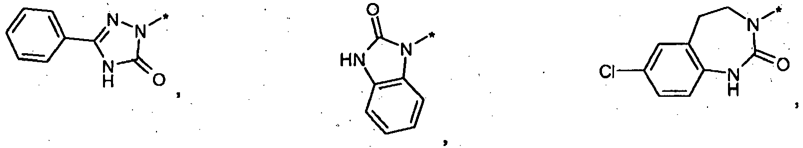

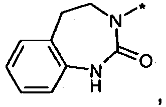

WO 2006/127588 offenbart eine Verbindung gemäß der Formel (I) zur Behandlung von Cystischer Fibrose oder COPD.

**R³** und **R⁴** zusammen mit dem Stickstoffatom, an das sie gebunden sind, eine Gruppe ausgewählt aus 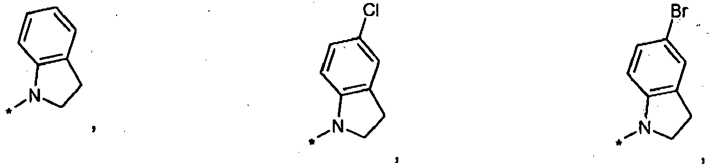 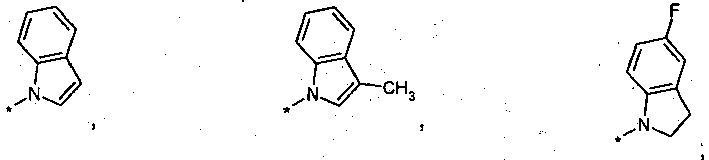 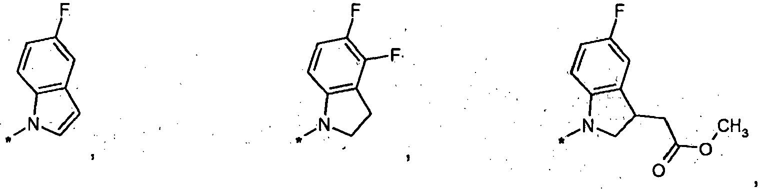 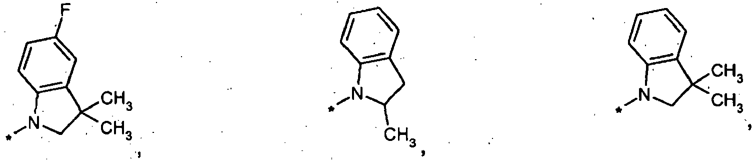 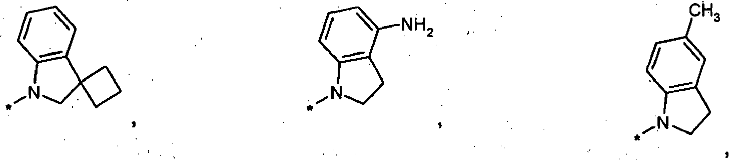 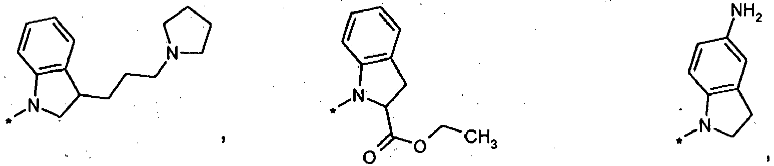 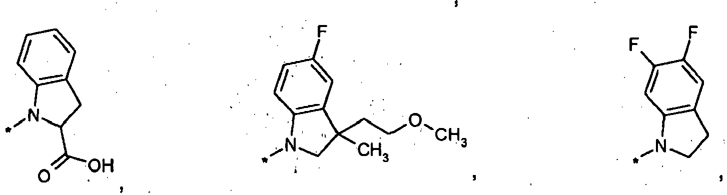 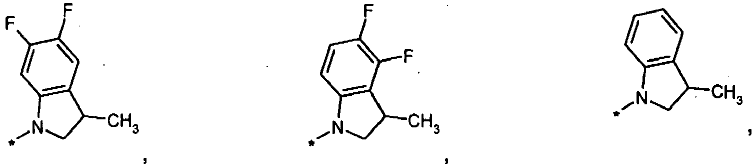 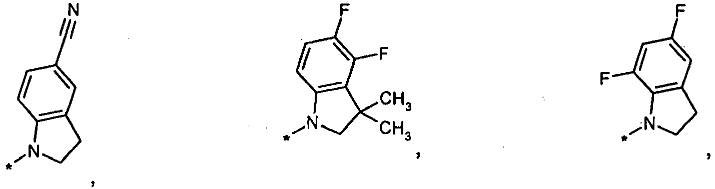 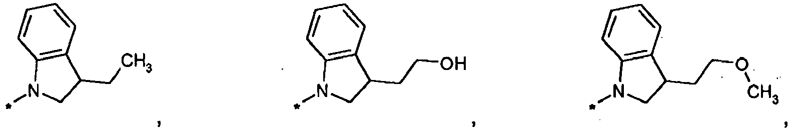 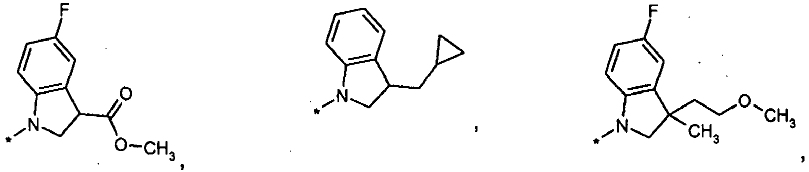 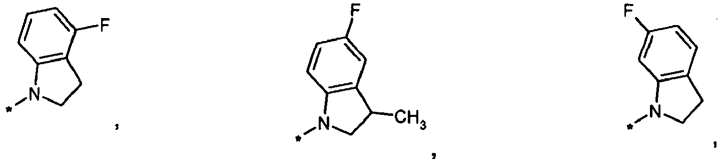 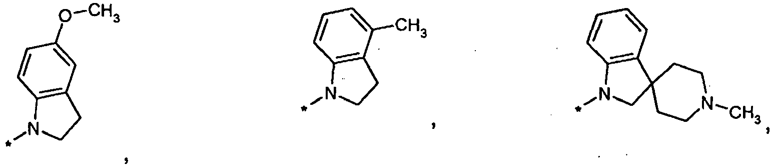 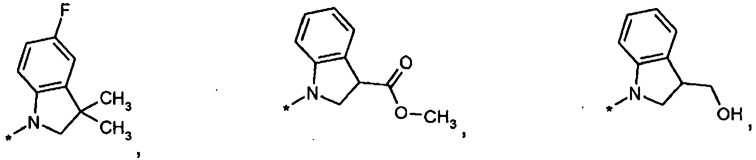 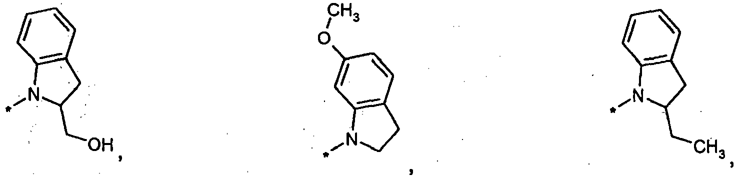 und der Ring 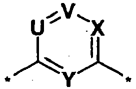 eine Gruppe ausgewählt aus 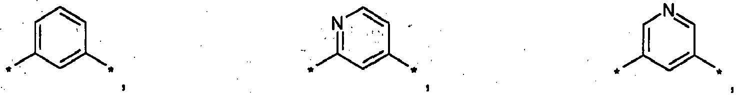 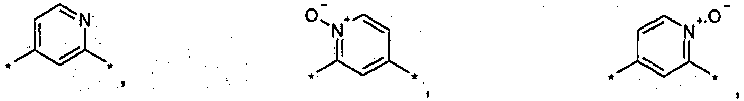 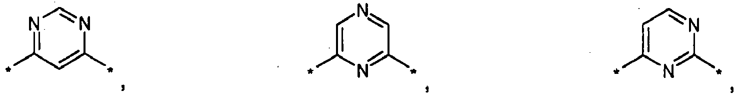 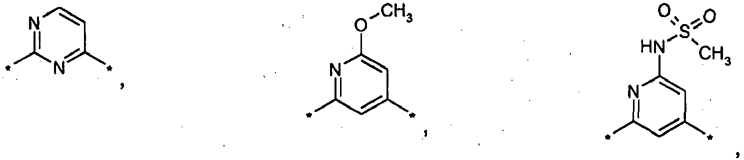 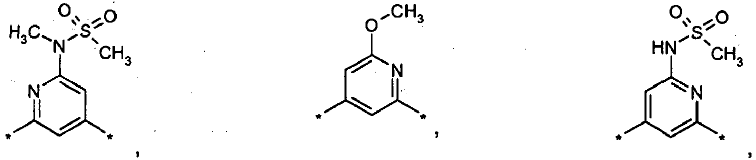 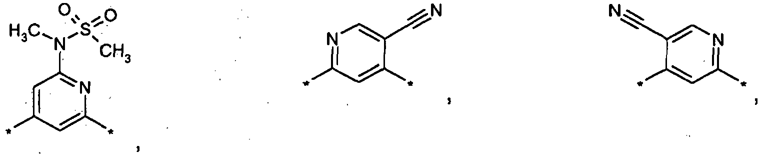 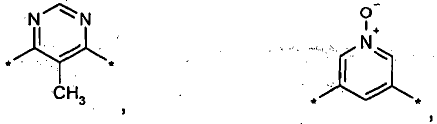 bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Als ganz besonders bevorzugte Verbindungen der obigen allgemeinen Formel I seien beispielsweise folgende Verbindungen genannt:

| **Nr.** | **Struktur** |
|---|---|
| (1) | 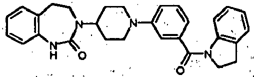 |
| (2) | 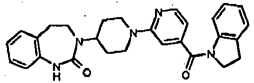 |
| (3) | 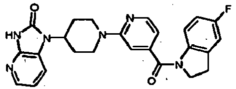 |
| (4) | 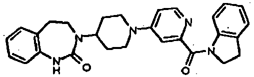 |
| (5) | 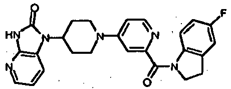 |
| (6) | 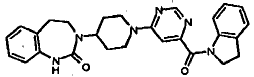 |
| (7) | 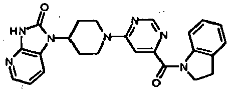 |
| (8) | 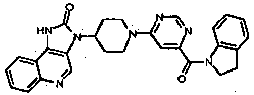 |
| (9) | 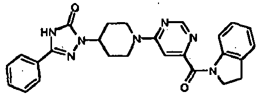 |
| (10) | 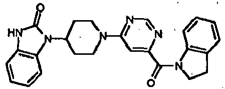 |
| (11) | 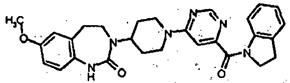 |
| (12) | 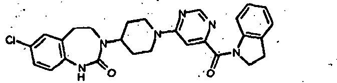 |
| (13) | 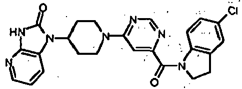 |
| (14) | 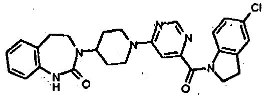 |
| (15) | 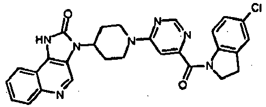 |
| (16) | 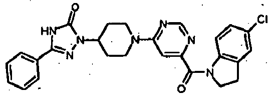 |
| (17) | 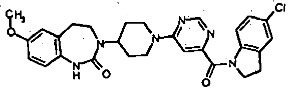 |
| (18) | 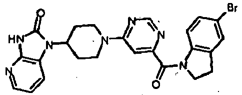 |
| (19) | 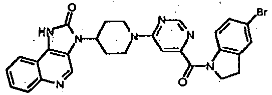 |
| (20) | 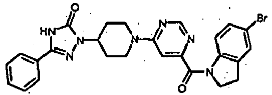 |
| (21) | 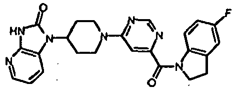 |
| (22) | 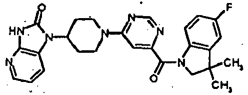 |
| (23) | 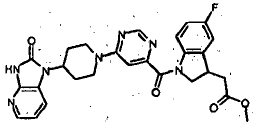 |
| (24) | 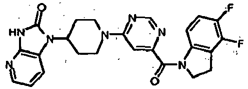 |
| (25) | 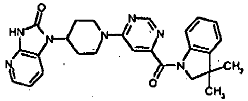 |
| (26) | 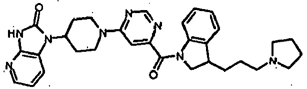 |
| (27) | 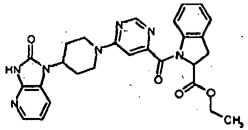 |
| (28) | 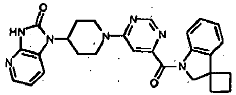 |
| (29) | 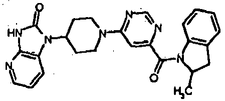 |
| (30) | 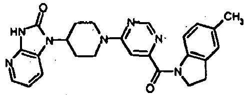 |
| (31) | 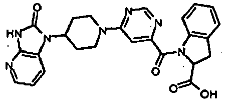 |
| (32) | 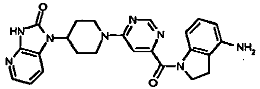 |
| (33) | 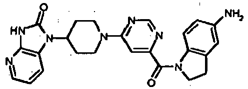 |
| (34) | 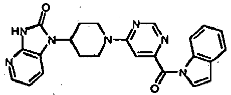 |
| (35) | 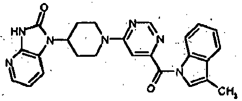 |
| (36) | 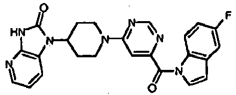 |
| (37) | 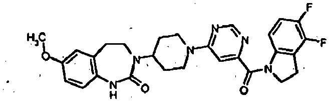 |
| (38) | 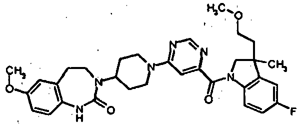 |
| (39) | 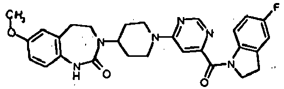 |
| (40) | 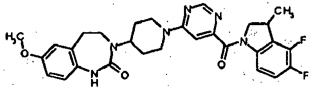 |
| (41) | 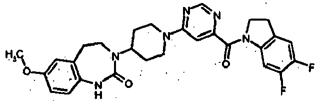 |
| (42) | 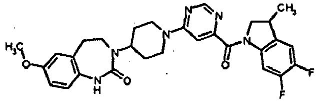 |
| (43) | 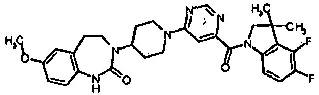 |
| (44) | 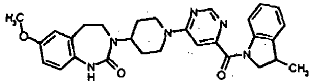 |
| (45) | 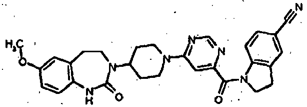 |
| (46) | 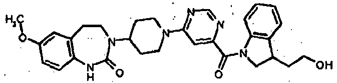 |
| (47) | 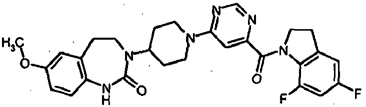 |
| (48) | 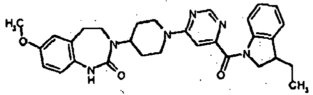 |
| (49) | 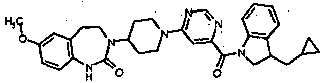 |
| (50) | 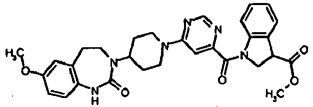 |
| (51) | 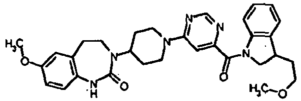 |
| (52) | 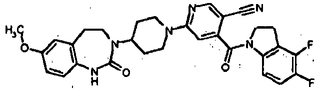 |
| (53) | 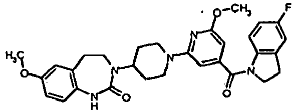 |
| (54) | 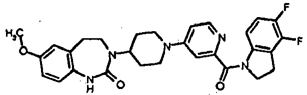 |
| (55) | 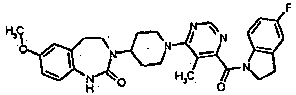 |
| (56) | 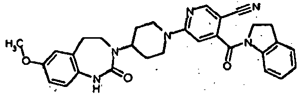 |
| (57) | 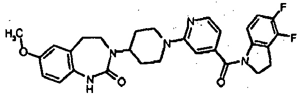 |
| (58) | 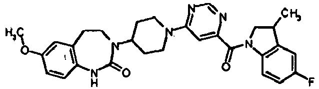 |
| (59) | 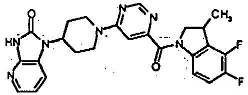 |
| (60) | 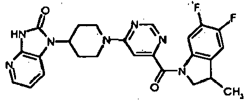 |
| (61) | 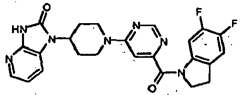 |
| (62) | 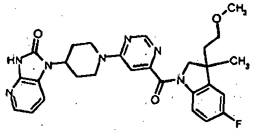 |
| (63) | 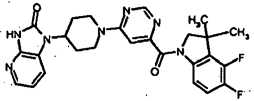 |
| (64) | 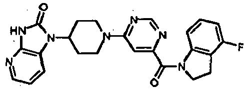 |
| (65) | 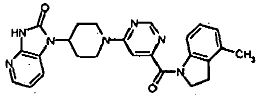 |
| (66) | 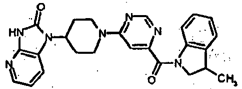 |
| (67) | 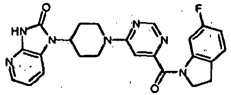 |
| (68) | 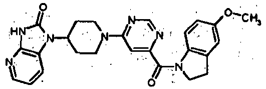 |
| (69) | 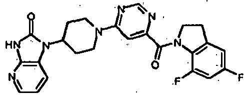 |
| (70) | 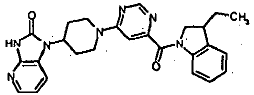 |
| (71) | 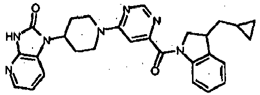 |
| (72) | 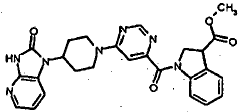 |
| (73) | 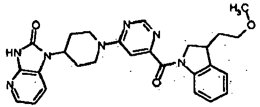 |
| (74) | 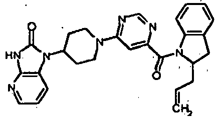 |
| (75) | 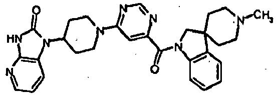 |
| (76) | 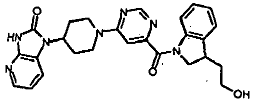 |
| (77) | 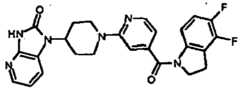 |
| (78) | 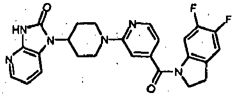 |
| (79) | 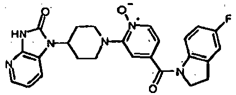 |
| (80) | 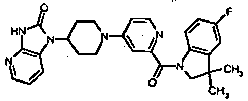 |
| (81) | 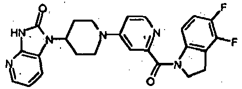 |
| (82) | 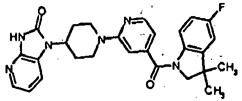 |
| (83) | 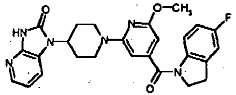 |
| (84) | 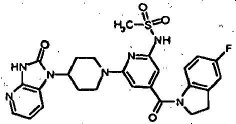 |
| (85) | 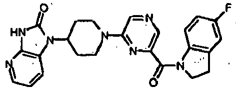 |
| (86) | 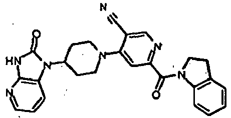 |
| (87) | 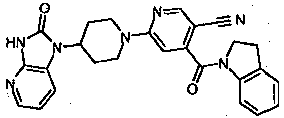 |
| (88) | 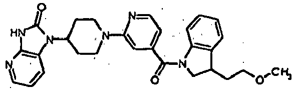 |
| (89) | 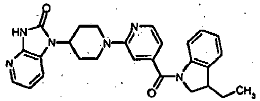 |
| (90) | 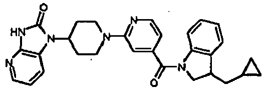 |
| (91) | 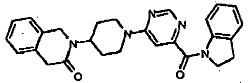 |
| (92) | 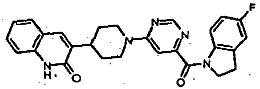 |
| (93) | 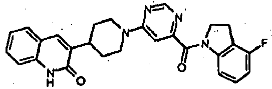 |
| (94) | 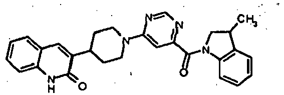 |
| (95) | 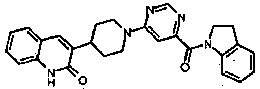 |
| (96) | 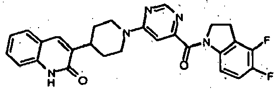 |
| (97) | 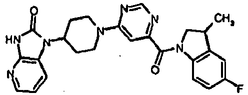 |
| (98) | 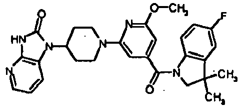 |
| (99) | 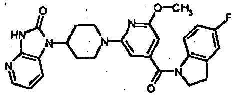 |
| (100) | 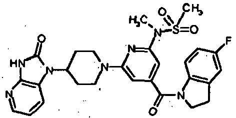 |
| (101) | 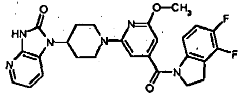 |
| (102) | 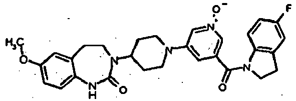 |
| (103) | 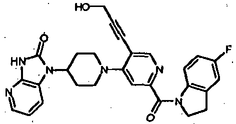 |
| (104) | 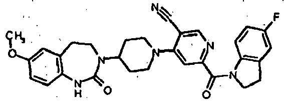 |
| (105) | 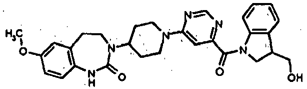 |
| (106) | 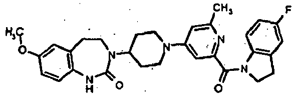 |
| (107) | 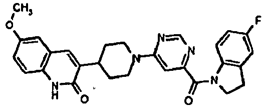 |
| (108) | 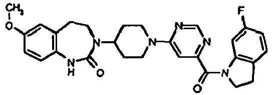 |
| (109) | 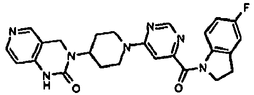 |
| (110) | 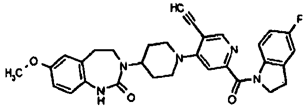 |
| (111) | 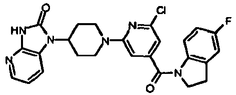 |
| (112) | 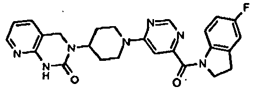 |
| (113) | 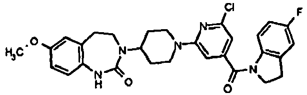 |
| (114) | 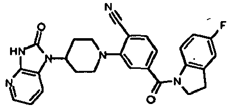 |
| (115) | 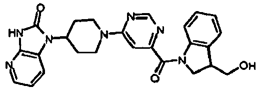 |
| (116) | 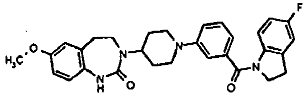 |
| (117) | 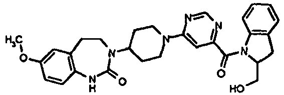 |
| (118) | 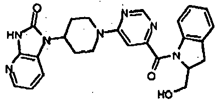 |
| (119) | 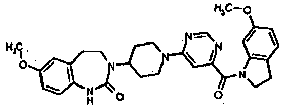 |
| (120) | 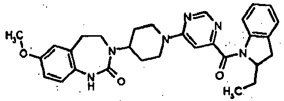 |

deren Enantiomere, deren Diastereomere, deren Hydrate, deren Gemische und deren Salze sowie die Hydrate der Salze, insbesondere deren physiologisch verträgliche Salze mit anorganischen oder organischen Säuren oder Basen.

### VERWENDETE BEGRIFFE UND DEFINITIONEN

Die vorliegende Beschreibung der Erfindung soll in Übereinstimmung mit den Gesetzmäßigkeiten und Regeln der chemischen Bindungen aufgefasst werden.
Die Verbindungen, die von dieser Erfindung umfasst sind, sind jene, die auch chemisch stabil sind.
Soweit nicht anders angegeben, sind alle Substituenten voneinander unabhängig. Sollten an einer Gruppe z. B. mehrere C₁₋₄-Alkylgruppen als Substituenten sein, so könnte, im Fall von drei Substituenten C₁₋₄-Alkyl unabhängig voneinander einmal Methyl, einmal Ethyl und einmal *n*-Propyl bedeuten.

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden. Beispielsweise wird ein Phenyl-Rest wie folgt dargestellt:

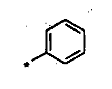

Weiterhin wird das auf den Verknüpfungspunkt folgende Atom des Substituenten als das Atom mit der Positionsnummer 1 verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "C₁₋₃-Alkyl" (auch soweit sie Bestandteil anderer Reste sind) werden verzweigte und unverzweigte Alkylgruppen mit 1 bis 3 Kohlenstoffatomen, unter dem Begriff "C₁₋₄-Alkyl" werden verzweigte und unverzweigte Alkylgruppen mit 1 bis 4 Kohlenstoffatomen und unter dem Begriff "C₁₋₆-Alkyl" werden verzweigte und unverzweigte Alkylgruppen mit 1 bis 6 Kohlenstoffatomen verstanden. Beispielsweise werden hierfür genannt: Methyl, Ethyl, *n*-Propyl, *iso*-Propyl, *n*-Butyl, *iso-*butyl, *sec*-Butyl, *tert*-Butyl, Pentyl, Neopentyl oder *n*-Hexyl. Gegebenenfalls werden für vorstehend genannten Gruppen auch die Abkürzungen Me, Et, *n*-Pr, *i*-Pr, *n*=Bu, *i*-Bu, *t*-Bu, etc. verwendet. Sofern nicht anders beschrieben, umfassen die Definitionen Propyl und Butyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₁₋₆-Alkylen" (auch soweit sie Bestandteil anderer Reste sind) werden verzweigte und unverzweigte Alkylengruppen mit 1 bis 6 Kohlenstoffatomen und unter dem Begriff "C₁₋₃-Alkylen" werden verzweigte und unverzweigte Alkylengruppen mit 1 bis 3 Kohlenstoffatomen verstanden Beispielsweise werden hierfür genannt: Methylen, Ethylen, Propylen, 1-Methylethylen, Butylen, 1-Methylpropylen, 1,1-Dimethylethylen, 1,2-Dimethylethylen, Pentylen, 1,1-Dimethylpropylen, 2,2-Dimethylpropylen, 1,2-Dimethylpropylen, 1,3-Dimethylpropylen oder Hexylen. Sofern nicht anders beschrieben, umfasst die Definition Propylen alle denkbaren isomeren Formen der jeweiligen Reste gleicher Kohlenstoffanzahl. So umfasst beispielsweise Propyl auch 1-Methylethylen und Butylen umfasst 1-Methylpropylen, 1,1-Dimethylethylen, 1,2-Dimethylethylen.
Die Definition für C₀-Alkylen bedeutet eine Bindung.

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂₋₄₋Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatomen. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc..

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindüng aufweisen. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc..

Unter dem Begriff "C₃₋₆-Cycloalkyl" (auch soweit sie Bestandteil anderer Reste sind) werden cyclische Alkylgruppen mit 3 bis 6 Kohlenstoffatomen, unter dem Begriff "C₅₋₆-Cycloalkyl" werden cyclische Alkylgruppen mit 5 bis 6 Kohlenstoffatomen und unter dem Begriff "C₅₋₇-Cycloalkyl" werden cyclische Alkylgruppen mit 5 bis 7 Kohlenstoffatomen verstanden. Beispielsweise werden hierfür genannt: Cyclopropyl, Cyclobutyl, Cyclopentyl, Cyclohexyl oder Cycloheptyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "C₅₋₆-Cycloalkenyl" (auch soweit sie Bestandteil anderer Reste sind) werden cyclische Alkenylgruppen mit 5 oder 6 Kohlenstoffatomen verstanden, die eine ungesättigte Bindung enthalten. Beispielsweise werden hierfür genannt: Cyclopentenyl oder Cyclohexenyl. Soweit nicht anders beschrieben, können die cyclischen Alkenylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Heterocyclyl" oder "Heterocyclus" werden, soweit in den Definitionen nicht anders beschrieben, stabile 5-, 6- oder 7-gliedrige monocyclische oder 8-, 9-, 10-oder 11-gliedrige bicyclische heterocyclische Ringsysteme verstanden, die in mindestens einem Ring kein aromatisches Ringsystem bilden und neben Kohlenstoffatomen ein bis vier Heteroatome tragen können, die ausgewählt sind aus der Gruppe bestehend aus Stickstoff, Sauerstoff und Schwefel. Dabei können sowohl Stickstoffatome als auch Schwefelatome optional oxidiert sein und Stickstoffatome können quaternisiert sein. Der heterocyclische Ring kann eine oder zwei Carbonyl-, Thiocarbonyl- oder Cyanimihogruppen benachbart zu einem Stickstoffatom enthalten. Die voranstehend genannten Heterocyclen können über ein Kohlenstdffatom oder über ein Stickstoffatom mit dem restlichen Molekül verknüpft sein.
Soweit nicht anders beschrieben, können die Heterocyclen substituiert sein mit einem oder mehreren Resten ausgewählt aus der Gruppe bestehend aus:
(a) OH, NO₂, CN, OCF₃, OCHF₂, OCH₂F, NH₂,
(b) Halogen, vorzugsweise Fluor oder Chlor,
(c) C₁₋₆-Alkyl, vorzugsweise C₁₋₃-Alkyl, besonders bevorzugt Ethyl, Methyl, *iso-*Propyl oder *tert*-Butyl,
(d) -SO₂-OC₁₋₃-Alkyl, vorzugsweise -O-Methyl,
(e) -O-C₁₋₃-Alkyl, vorzugsweise -O-Methyl oder-O-Ethyl,
(f) COOH, COO-C₁₋₃-Alkyl, vorzugsweise CO-O-Methyl oder CO-O-Ethyl,
wobei die Reste gleich oder verschieden sein können.
Als Beispiele seien folgende Verbindungen genannt, sollen jedoch nicht auf diese beschränkt sein: Azetidin, Oxetan, Thietan, Thietandioxid, Tetrahydrofuran, Dihydrofuran, Dioxalan, Imidazolidin, Imidazolin, Imidazolidinon, Dihydroimidazolon, Oxazolin, Oxazolidin, Oxazolidinon, Pyrrolidinon, Dihydropyrazol, Pyrrolidin, Pyrrolin, Morpholin, Tetrahydropyridin, Dihydropyran, Tetrahydropyran, Dioxan, Piperazin, Piperidin, Piperazinon, Piperidinon, Pyran, Thiomorpholinyl-S-oxid, Thiomorpholinyl-S-dioxid, Thiomorpholin, Dihydrooxazin, Morpholindion, Morpholinthion, Perhydrothiazindioxid, ε-Caprolactam, Oxazepanon, Diazepanon, Thiazepanon, Perhydroazepin, Dihydrochinazolinon, Dihydroindol, Dihydroisoindol, Benzoxazolon,-Bezimidazolon, Chromanon, Tetrahydrochinolin, Tetrahydrobenzoxazol, Tetrahydrobenzisoxazol, Tetrahydrobenzthiophen, Tetrahydrothieno-pyridin, Tetrahydrobenzofuran, Tetrahydrooxazolopyridin, Tetrahydro-isoxazolopyridin.

Bevorzugt im Sinne dieser Erfindung seien folgende Heterocyclen:

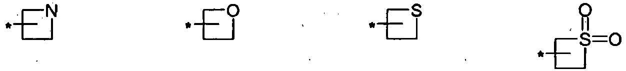

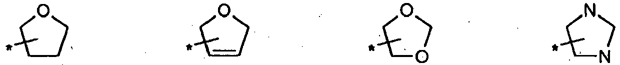

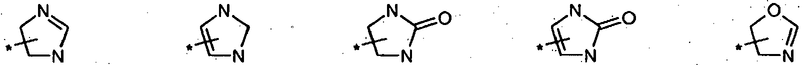

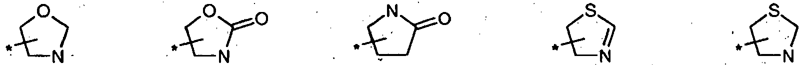

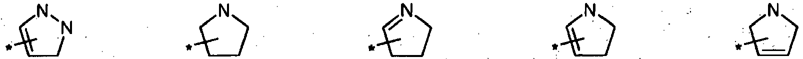

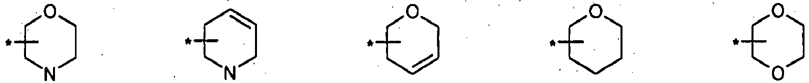

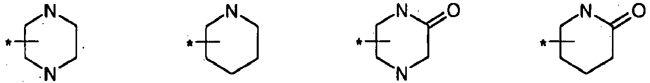

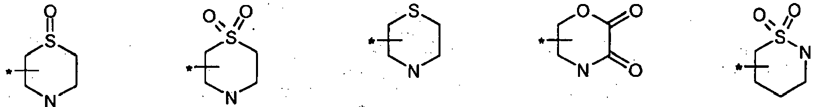

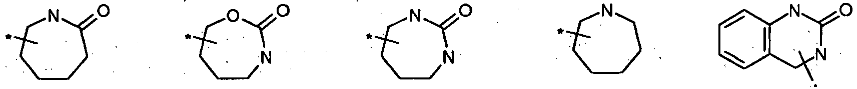

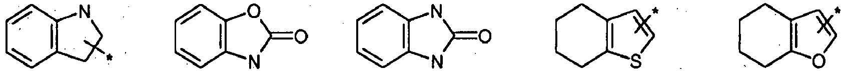

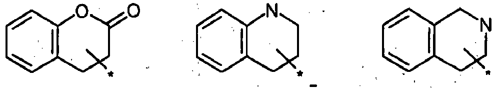

Unter dem Begriff "Aryl" (auch soweit sie Bestandteil anderer Reste sind) werden monocyclische aromatische Ringsysteme mit 6 Kohlenstoffatomen oder bicyclische aromatische Ringsysteme mit 10 Kohlenstoffatomen verstanden. Beispielsweise werden hierfür Phenyl, 1-Naphthyl oder 2-Naphthyl genannt; bevorzugter Arylrest ist Phenyl. Soweit nicht anders beschrieben, können die Aromaten substituiert sein mit einem oder mehreren Resten ausgewählt aus der Gruppe bestehend aus:
(a) OH, NO₂, CN, OCF₃, OCHF₂, OCH₂F, NH₂,
(b) Halogen, vorzugsweise Fluor oder Chlor,
(c) C₁₋₆-Alkyl, vorzugsweise C₁₋₃-Alkyl, besonders bevorzugt Ethyl, Methyl, *iso-*Propyl oder *tert*-Butyl,
(d) -SO₂-O-C₁₋₃-Alkyl, vorzugsweise -O-Methyl,
(e) -O-C₁₋₃-Alkyl, vorzugsweise -O-Methyl oder -O-Ethyl,
(f) COOH, CO-O-C₁₋₃-Alkyl, vorzugsweise CO-O-Methyl oder CO-O-Ethyl, wobei die Reste gleich oder verschieden sein können.

Unter dem Begriff "Heteroaryl" werden stabile fünf- oder sechsgliedrige heterocyclische Aromaten oder 8- bis 10-gliedrige bicyclische Heteroarylringe verstanden, die in jedem Ring ein, zwei oder drei Heteroatome, ausgewählt aus der Gruppe Sauerstoff, Schwefel und Stickstoff, enthalten können und zusätzlich so viele konjugierte Doppelbindungen enthalten, dass ein aromatisches System gebildet wird. Als Beispiele für fünf- oder sechsgliedrige heterocyclische Aromaten werden genannt, sollen aber nicht auf diese beschränkt sein:
Furan, Pyrrol, Thiophen, Pyrazol, Imidazol, Oxazol, Thiazol, Isothiazol, Isoxazol, Oxadiazol, Triazol, Tetrazol, Furazän, Thiadiazol, Pyridin, Pyrimidin, Pyrazin, Pyridazin, Triazin.

Bevorzugt im Sinne dieser Erfindung seien folgende fünfgliedrige heterocyclische Aromaten:

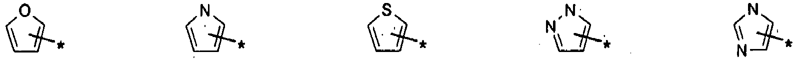

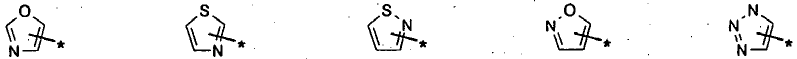

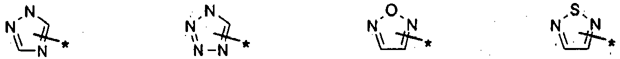

Bevorzugt im Sinne dieser Erfindung seien folgende sechsgliedrige heterocyclische Aromaten:

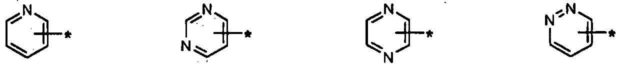

Als Beispiele für 9- oder 10-gliedrige bicyclische Heteroarylringe werden genannt, sollen aber nicht auf diese beschränkt sein:
Indol, Isoindol, Indazol, Indolizin, Benzofuran, Benzthiophen, Benzimidazol, Benzoxazol, Benzthiazol, Benztriazol, Benzisoxazol, Benzisothiazol, Chinolin, Isochinolin, Cinnolin, Phtalazin, Chinoxalin; Chinazolin, Pyridopyrimidin, Pyridopyrazin, Pyridopyridazin, Pyrimidopyrimidin, Pteridin, Purin, Chinolizin, Benzoxazolcarbonitril, Chinolin, Isochinolin, Chinolizin, Pteridin, Purin, Chinolizin, Benzoxazol-carbonitril.

Bevorzugt im Sinne dieser Erfindung seien folgende bicyclische Heteroarylringe:

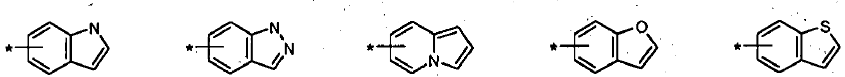

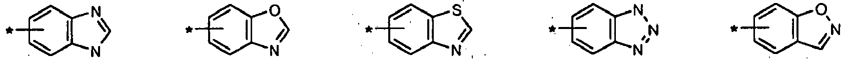

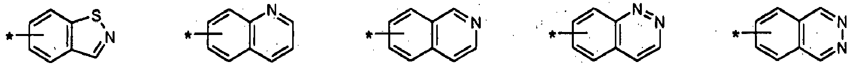

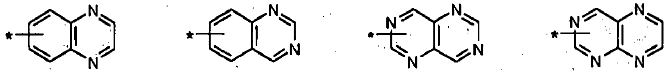

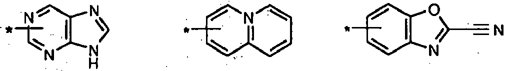

Soweit nicht anders beschrieben, können die voranstehend genannten Heteroaryle substituiert sein mit einem oder mehreren Resten ausgewählt aus der Gruppe bestehend aus:
(a) OH, NO₂, CN, OCF₃, OCHF₂, OCH₂F, NH₂,
(b) Halogen, vorzugsweise Fluor oder Chlor,
(c) C₁₋₆-Alkyl, vorzugsweise C₁₋₃-Alkyl, besonders bevorzugt Ethyl, Methyl, *iso-*Propyl oder *tert*-Butyl,
(d) -SO₂-O-C₁₋₃-Alkyl, vorzugsweise -O-Methyl,
(e) -O-C₁₋₃-Alkyl, vorzugsweise -O-Methyl oder -O-Ethyl,
(f) COOH, CO-O-C₁₋₃-Alkyl, vorzugsweise CO-O-Methyl oder CO-O-Ethyl, wobei die Reste gleich oder verschieden sein können.

Bicyclische Heteroarylringe können dabei vorzugsweise im Phenylrest substituiert sein.

Unter dem Begriff "Halogen" werden Fluor-, Chlor-, Brom- oder lodatome verstanden.

Verbindungen der allgemeinen Formel **I** können Säuregruppen besitzen, hauptsächlich Carboxylgruppen, und/oder basische Gruppen wie z.B. Aminofunktionen. Verbindungen der allgemeinen Formel **I** können deshalb als innere Salze, als Salze mit pharmazeutisch verwendbaren anorganischen Säuren wie beispielsweise Bromwasserstoffsäure, Phosphorsäure, Salpetersäure, Salzsäure, Schwefelsäure, Methansulfonsäure, Ethansulfonsäure, Benzolsulfonsäure, *p*-Toluolsulfonsäure-oder organischen Säuren wie beispielsweise Äpfelsäure, Bernsteinsäure, Essigsäure, Fumarsäure, Maleinsäure, Mandelsäure, Milchsäure, Weinsäure, Zitronensäure oder als Salze mit pharmazeutisch verwendbaren Basen wie Alkali- oder Erdalkalimetallhydroxiden, beispielsweise Natriumhydroxid oder Kaliumhydroxid, oder Carbonaten, Ammoniak, Zink- oder Ammoniumhydroxiden oder organischen Aminen wie z.B. Diethylamin, Triethylamin, Ethanolamin, Diethanolamin, Triethanolamin, Cyclohexylamin, Dicyclohexylamin u.a. vorliegen.

Die erfindungsgemäßen Verbindungen können als Racemate vorliegen, sofern sie nur ein Chiralitätselement besitzen, sie können aber auch als reine Enantiomere, d.h. in (R)- oder (S)-Form gewonnen werden.

Verbindungen mit einer Kohlenstoff-Doppelbindung können sowohl in der E- als auch Z-Form vorliegen.

Falls eine Verbindung in verschiedenen tautomeren Formen vorliegen kann, so ist die dargestellte Verbindung nicht auf eine tautomere-Form beschränkt, sondern umfasst alle tautomeren Formen. Dies gilt insbesondere auch bei stickstoffhaltigen Heteroarylen:

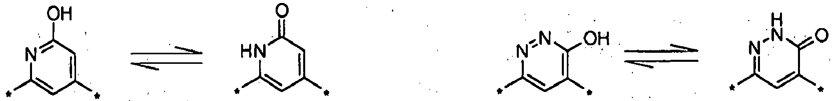

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enatiomeren, aus denen sich die erwähnten Racemate zusammensetzen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssälze mit pharmakologisch unbedenklichen.

Sogenannte Prodrugs von Verbindungen der allgemeinen Formel **I** sind ebenfalls Gegenstand dieser Erfindung. Als Prodrug wird jedes Molekül bezeichnet, das nach Applikation an Säugetieren das aktive Prinzip der allgemeinen Formel **I** in vivo freisetzt. Das Prodrug kann per se keine oder nur geringe pharmakologische Aktivität haben, es setzt allerdings nach Verabreichung in-vivo das aktive Prinzip der allgemeinen Formel **I** frei und dieses weist die beschriebene Aktivität auf. Prodrugs für Verbindungen der allgemeinen Formel **I** können hergestellt werden, indem geeignete funktionelle Gruppen in der Verbindung der allgemeinen Formel **I** so modifiziert werden, wie es einem Fachmann in diesem Gebiet bekannt ist. (H. Bundgaard (Editor), Design of Prodrugs. (1986), Elsevier)

Diese Erfindung umfasst auch jene Metaboliten, die sich aus der Verbindungen der allgemeinen Formel I ableiten. Unter Metaboliten versteht man in diesem Zusammenhang solche Verbindungen, die nach Applikation in-vivo aus der Verbindung der allgemeinen Formel **I** entstehen. Als Beispiel für Metaboliten werden genannt:
- Methylgruppen der Verbindung der allgemeinen Formel **I** können in die entsprechenden Hydroxymethyl-Gruppen überführt werden. (-CH₃ -> -CH₂OH)
- Alkoxy-Gruppen der Verbindung der allgemeinen Formel **I** können in die entsprechenden Hydroxyl-Gruppen überführt werden. (-OR -> -OH)
- Sekundäre Amine der Verbindung der allgemeinen Formel **I** können in die entsprechenden primären Amine überführt werden. (-NR₁R₂ -> -NHR₁ oder -NHR₂)
- Stickstoff-Atome der Verbindung der allgemeinen Formel **I** können in die entsprechenden Stickstoff-Oxide überführt werden. (=N- -> =N⁺-(O⁻)-)

### HERSTELLVERFAHREN

Gegenstand der Erfindung ist weiterhin ein Verfahren zur Herstellung der Verbindungen der allgemeinen Formel **I**, in der die Substituenten **U, V, X, Y, R¹, R², R³** und **R⁴** wie voranstehend erwähnt definiert sind.

Einige Methoden zur Herstellung der erfindungsgemäßen Verbindungen der allgemeinen Formel **I** 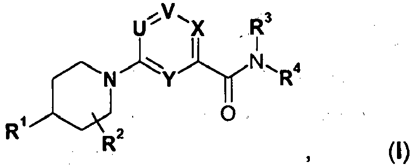 in der **U, V, X, Y, R¹, R², R³** und **R⁴** wie voranstehend erwähnt definiert sind, sind in den folgenden Syntheseschemata und Beispielen dargestellt.

In einigen Fällen kann die Reihenfolge bei der Durchführung der Reaktionsschemata variiert werden, um die Reaktionen zu vereinfachen oder um unerwünschte Nebenprodukte zu verhindern. Die nachfolgenden Beispiele sind genannt, um die Erfindung vollständig verständlich zu machen. Die Beispiele sollen der Anschaulichkeit der Erfindung dienen und sollen die Erfindung in keinster Weise einschränken.

Die erfindungsgemäßen Verbindungen können gemäß den dargestellten Schemata und spezifischen Beispielen oder entsprechenden Modifikationen davon hergestellt werden. Von diesen Reaktionen können auch Abwandlungen eingesetzt werden, die einem Fachmann bekannt sind, hier aber nicht detailliert beschrieben sind. Die allgemeinen Verfahren zur Herstellung der erfindungsgemäßen Verbindungen erschließen sich einem Fachmann beim Anblick der folgenden Schemata.

Ausgangsverbindungen sind kommerziell verfügbar oder werden gemäß Verfahren hergestellt, die in der Literatur beschrieben sind, in dem Fachgebiet dem Fachmann bekannt sind oder wie sie hierin beschrieben sind. Vor Durchführung der Reaktion können in den Verbindungen entsprechende funktionelle Gruppen durch übliche Schutzreste geschützt werden. Diese Schutzgruppen können auf einer geeigneten Stufe innerhalb der Reaktionssequenz nach für den Fachmann geläufigen Methoden wieder abgespalten werden.

Bei den nachstehend beschriebenen Umsetzungen können gegebenenfalls vorhandene reaktive Gruppen wie Hydroxy-, Carboxy-, Amino-, Alkylamino-, Amid- oder Iminogruppen während der Umsetzung durch übliche Schutzgruppen geschützt werden, welche nach der Umsetzung wieder abgespalten werden.

### Beispielsweise kommt

- als Schutzrest für eine Hydroxygruppe die Methoxy-, Benzyloxy-,Trimethylsilyl-, Acetyl-, Benzoyl-, tert.-Butyl-, Trityl-, Benzyl- oder Tetrahydropyranylgruppe,
- als Schutzreste für eine Carboxylgruppe die Trimethylsilyl-, Methyl-,Ethyl-, tert.-Butyl-, Benzyl- oder Tetrahydropyranylgruppe, und
- als Schutzreste für eine Amidgruppe die N=Methoxymethyl- (MOM), N-Benzyloxymethyl- (BOM), N-(Trimethylsilyl)ethoxymethyl (SEM), N-*tert*-Butyldimethylsiloxymethyl, N-tert-Butyldimethylsilyl- (TBDMS), N-Triisopropylsilyl- (TIPS), N-Benzyl-, N-4-Methoxybenzyl- (PMB), N-Triphenylmethyl- (Tr), N-tert-Butoxycarbonyl- (BOC), N-Benzyloxycarbonyl- (Cbz), N-Trimethylsilylethylsulforiyl- (SES)
- als Schutzrest für eine Amino-, Alkylamino- oder Iminogruppe die Acetyl-, Trifluor-acetol-, Benzoyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe,
in Betracht.

Weitere Schutzgruppen und deren Abspaltung sind in T.W. Greene, P.G.M. Wuts, "Protective Groups in Organic Synthesis", Wiley, 1991 und 1999 beschrieben.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von Iodtrimethylsilan, bei Temperaturen zwischen 0°C und 100°C, vorzugsweise bei Temperaturen zwischen 10°C und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0°C und 50°C, vorzugsweise jedoch bei Raumtemperatur, und bei einem Wasserstoffdruck von 1 bis 7 bar, vorzugsweise jedoch von 1 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0°C und 50°C, vorzugsweise jedoch bei Raumtemperatur, erfolgen.

Die Abspaltung einer Methoxygruppe erfolgt zweckmäßigerweise in Gegenwart von Bortribromid in einem Lösungsmittel wie Methylenchlorid bei Temperaturen zwischen -35°C und -25°C.

Die Abspaltung eines 2,4-Dimethoxybenzylrestes erfolgt vorzugsweise in Trifluoressigsäure in Gegenwart von Anisol.

Die Abspaltung eines tert.-Butyl- oder tert.-Butoxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder *n*-Butylamin in einem Lösungsmittel wie Methanol, Ethanol, Isopropanol, Toluol∼Wasser oder Dioxan bei Temperaturen zwischen 20°C und 50°C.

Die Abspaltung eines Methoxymethyl-Rests kann in Gegenwart einer Säure wie konzentrierter Salsäure in einem Lösungsmittel wie Dimethoxyethan durchgeführt werden. Alternativ kann eine Säure wie Trifluoressigsäure auch ohne Lösungsmittel eingesetzt werden.

Die Abspaltung eines N-(Trimethylsilyl)ethoxymethyl-Rests kann in Gegenwart von TBAF und 1,3-Dimethyl-3,4,5,6-Tetrahydro-2(1*H*)-Pyrimidon durchgeführt werden. Alternativ kann die SEM-Schutzgruppe auch mit einer Säure wie Chlorwasserstoff in einem organischen Lösungsmittel wie Dioxan oder Ethanol durchgeführt werden.

Die Abspaltung eines Allyloxycarbonylrestes erfolgt durch Behandlung mit einer katalytischen Menge Tetrakis-(triphenyl-phosphin)-palladium(0) vorzugsweise in einem Lösungsmittel wie Tetrahydrofuran und vorzugsweise in Gegenwart eines Überschusses von einer Base wie Morpholin bei Temperaturen zwischen 0°C und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo-[2.2.2]octan bei Temperaturen zwischen 20°C und 70°C.

Die folgenden Verfahren zur Herstellung der erfindüngsgemäßen Verbindungen der allgemeinen Formel I und ihrer Vorstufen haben sich besonders bewährt:

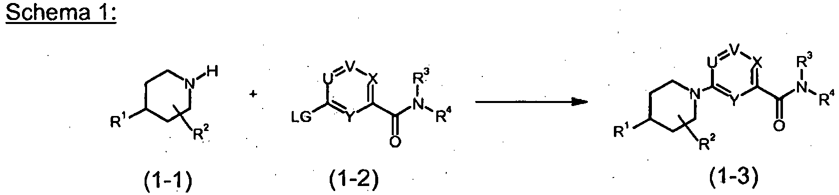

Die Herstellung einer Verbindung der allgemeinen Formel (1-3), in der **U, V, X, Y, R¹, R², R³** und **R⁴** wie voranstehend erwähnt definiert sind, kann durch Reaktion eines Amins oder Anilins der allgemeinen Formel (1-1), in der **R¹** und **R²** wie eingangs erwähnt definiert sind, mit einer elektronenarmen Verbindung der allgemeinen Formel (1-2), in der U, **V, X, Y, R³** und **R⁴** wie eingangs erwähnt definiert sind und **LG** eine Austrittsgruppe darstellt, erfolgen. Als Austrittsgruppe **LG** können Halogenide, vorzugsweise Chloride und Bromide, -SO₂CH₃, -OSO₂CH₃, -OSO₂C₆H₄-CH₃ oder -S-CH₃ (-S-CH₃ erfördert die weitere Umsetzung mit einem organischen Peroxid um in die eigentliche Austrittsgruppe überführt werden zu können) etc. fungieren, müssen aber nicht auf diese beschränkt sein. Ganz besonders bevorzugt ist die Verwendung von Chloriden.

Die Reaktion kann über eine nucleophile aromatische Substitution in einem inerten Lösungsmittel unter Verwendung einer Hilfsbase in einem Temperaturbereich von 0° bis zum Rückfluss des Lösungsmittels erfolgen. Nucleophile aromatische Substitutionen werden in einem geeigneten, inerten Lösungsmittel, wie Tetrahydrofuran, Toluol, Xylol, Dialkylformamide (besonders bevorzugt Dimethylformamid), cyclische Amide (besonders bevorzugt N-Methylpyrrolidon), 1,4-Dioxan, Acetonitril oder in Lösungsmittelgemischen durchgeführt. Als geeignete Hilfsbasen können tertiäre Amine wie Triethylamin oder Ethyldiisopropylamin, Alkalimetal Carbonate wie Kaliumcarbonat oder Natriumcarbonat Natriumhydrid (NaH) oder Lithium diisopropylamid (LDA) verwendet werden. Dabei muss das verwendete inerte Lösungsmittel kompatibel sein mit der verwendeten Base. Bevorzugt wird die Reaktion in Dimethylformamid, bei Temperaturen zwischen Raumtemperatur und Rückfluss des Lösungsmittels, in Gegenwart einer tertiären Aminbase durchgeführt.

Alternativ können Strukturen der allgemeinen Form (1-3), in der **U, V, X, Y, R¹, R², R³** und **R⁴** wie voranstehend erwähnt definiert sind, über Übergangsmetall-katalysierte Reaktionen synthetisiert werden. Dabei kann ein Amin oder Anilin der allgemeinen Formel (1-1), in der **R¹** und **R²** wie eingangs erwähnt definiert sind, mit einer Verbindung der allgemeinen Formel (1-2), in der **U, V, X, Y, R³** und **R⁴** wie eingangs erwähnt definiert sind und **LG** eine Austrittsgruppe darstellt, in einem inerten Lösungsmittel bei Anwesenheit eines Katalysators und einer Hilfsbase reagieren. Für den Katalysator kann zusätzlich ein geeigneter Ligand verwendet werden. Als Austrittsgruppe **LG** können Chloride, Bromide, lodide, Trifluoracetate, Trifluormethansulfonate, Methansulfonate und Toluolsulfonate fungieren, müssen aber nicht auf diese beschränkt sein. Als inerte Lösungsmittel können Xylol, Tetrahydrofuran, Dimethylformamid, Dimethoxyethan, Toluol, Benzol, *tert*-Butanol, 1,4-Dioxan, Acetonitril oder Lösungsmittelgemische verwendet werden. Bevorzugtes Lösungsmittel ist Xylol. Geeignete Basen sind besonders Amin-Basen wie z.B. Triethylamin oder Diisopropylethylamin oder auch anorganische Basen wie Cäsiumcarbonat, Cäsiumacetat, Kaliumcarbonat, Kalium-*tert*-Butylat, Natriumcarbonat, Natrium-*tert*-Butylat oder Kaliumphosphat. Bevorzugte Reaktionstemperaturen sind von RT bis Rückfluss des Lösungsmittels bei Normaldruck. Typische Katalysatoren sind z.B. Übergangsmetall-Katalysatoren, wie z.B. Palladium-Katalysatoren der Art Tris(dibenzylidenäceton)-dipalladium(0), Tetrakis-(triphenylphosphin)-palladium(0), Palladium-(II)-acetat, Pd(PPh₃)₂Cl₂, Pd(CH₃CN)₂Cl₂, Pd(dppf)Cl₂ or Palladium(II)-chlorid. Typische Liganden sind z.B. Triphenylphosphin, Triphenylarsen, BINAP, XPhos, XantPhos, oder 2-(Di-*tert-*butylphosphino)biphenyl.

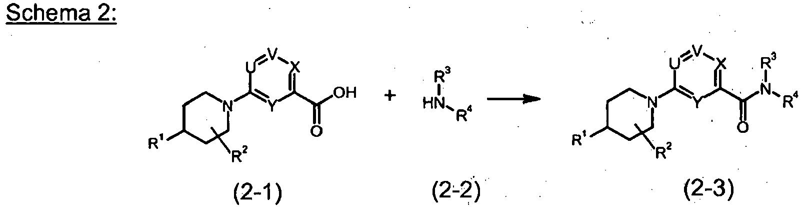

Die Herstellung einer Verbindung der allgemeinen Formel **(2-3),** in der **U, V, X, Y, R¹, R², R³** und **R⁴** wie voranstehend erwähnt definiert sind, kann wie in Schema 2 gezeigt durch Kupplung einer Verbindung der allgemeinen Formel (2-2), in der **R³** und **R⁴** wie eingangs erwähnt definiert sind, mit einer Carbonsäure der allgemeinen Formel (2-1), in der **U, V, X, Y, R¹** und **R²** wie voranstehend erwähnt definiert sind, unter Zuhilfenahme von Standard-Peptid-Kupplungsreagenzien und einer Base in einem inerten Lösungsmittel erfolgen (siehe z. B. Houben-Weyl, Methoden der Organischen Chemie, Bd. 15/2).

Als inerte Lösungsmittel können Dimethylformamid, N-Methylpyrrolidon, Dimethoxyethan, Dichlormethan, Acetonitril oder Lösungsmittelgemische verwendet werden. Bevorzugtes Lösungsmittel ist Dimethylformamid. Geeignete Basen sind besonders Amin-Basen wie z.B. Triethylamin oder Diisopropylethylamin. Als Kupplungsreagenzien können beispielsweise 1*H*-Benzotriazol-1-yl-oxy-tripyrrolidino-phosphonium-hexafluorophosphat (PyBOP), Dicyclohexylcarbodiimid (DCC), Diisopropylcarbodiimid (DIC), Ethyl-(3-dimethylaminopropyl)-carbodiimid, O-(1*H*-Benzotriazol-1-yl)-N,N-N,N-tetramethyluronium-hexafluorphosphat (HBTU) oder-tetrafluorborat (TBTU) oder 1*H*-Benzotriazol-1-yl-oxy-tris-(dimethylamino)-phosphonium-hexafluorphosphat (BOP) verwendet werden. Besonders bevorzugt ist die Verwendung von TBTU. Die Aktivierung der Carboxylgruppe kann alternativ auch über ein entsprechendes Säureanhydrid oder Säurechlorid erfolgen. Die Umsetzung erfolgt im Allgemeinen in einem Temperaturbereich von -20°C bis zum Rückfluss des Lösungsmittels bei Normaldruck. Bevorzugt sind Reaktionen bei Raumtemperatur. Durch Zugabe von 1-Hydroxybenzotriazol (HOBt) oder von 3-Hydroxy-4-oxo-3,4-dihydro-1,2,3-benzotriazin (HOOBT) kann die Reaktionsgeschwindigkeit gesteigert werden. Auch andere Standardkupplungsbedingungen können bei der Synthese solcher Amide verwendet werden.

Die Synthese von Verbindungen der allgemeinen Formel (3-4), in der **U, V, X, Y, R¹** und **R²** wie voranstehend erwähnt definiert sind, kann entweder durch Verfahren, die dem Fachmann bekannt sind, oder durch Reaktionen, die in Schema 3 beispielhaft dargestellt sind, hergestellt werden.

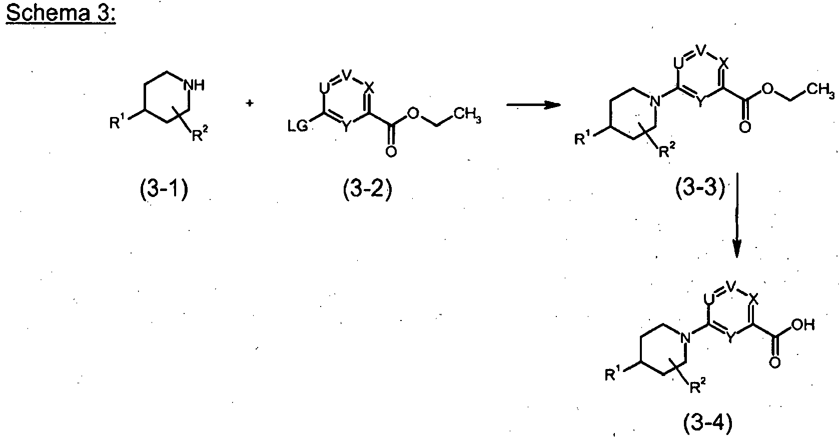

Eine Verbindung der allgemeinen Formel (3-1), in der **R¹** und **R²** wie eingangs erwähnt definiert sind, kann mit einer elektronenarmen Verbindung der allgemeinen Formel (3-2), in der **U, V, X** und **Y** wie eingangs erwähnt definiert sind und eine **LG** eine Austrittsgruppe darstellt, umgesetzt werden. Als Austrittsgruppe **LG** können Halogenide, vorzugsweise Chloride und Bromide, -SO₂CH₃ -OSO₂CH₃, -OSO₂C₆H₄-CH₃ oder -S-CH₃ (-S-CH₃ erfordert die weitere Umsetzung mit einem organischen Peroxid um in die eigentliche Austrittsgruppe -S(O)₂-CH₃ überführt werden zu können) fungieren, müssen aber nicht auf diese beschränkt sein. Ganz besonders bevorzugt ist die Verwendung von Chloriden. Die Reaktion kann in einem inerten Lösungsmittel unter Verwendung einer Hilfsbase in einem Temperaturbereich von 0°C bis Rückfluss des Lösungsmittels erfolgen. Als inertes Lösungsmittel können Tetrahydrofuran, Toluol, Xylol, Dialkylformamide (besonders bevorzugt ist Dimethylformamid), cyclische Amide (besonders bevorzugt ist N-Methylpyrrolidon), 1,4-Dioxan, Acetonitril oder Lösungsmittelgemische verwendet werden. Geeignete Hilfsbasen sind besonders tertiäre Amine wie Triethylamin oder Ethyldiisopropylamin und Alkalimetal Carbonate wie Kaliumcarbonat oder Natriumcarbonat. Bevorzugt wird die Reaktion in Dimethylformamid, bei Temperaturen zwischen Raumtemperatur und Rückfluss des Lösungsmittels, in Gegenwart einer tertiären Aminbase durchgeführt.
Ester der allgemeinen Formel (3-3), in der **U, V, X, Y, R¹** und **R²** wie voranstehend erwähnt definiert sind, können durch basische oder saure Hydrolyse (J. March, Advanced Organic Chemistry (New York: J. Wiley and Sons, 1985) oder durch Umsetzen mit Alkalimetal Salzen (bevorzugt Lil oder NaCN) in einem inerten Lösungsmittel in die Säure der allgemeinen Formel (3-4) überführt werden. Inerte Lösungsmittel können Dialkylformamide (besonders bevorzugt ist N,N-Dimethylformamid), Dialkylacetamide (besonders bevorzugt ist N,N-Dimethylacetamide), cyclische Amide (besonders bevorzugt ist N-Methylpyrrolidon) sein. Besonders bevorzugt ist eine alkalische Verseifung mit Alkalimetall Hydroxiden wie Natriumhydroxid oder Lithiumhydroxid in inerten Lösungsmitteln. Als inerte Lösungsmittel eignen sich Wasser und cyclische Ether wie 1,4-Dioxan oder Tetrahydrofuran sowie auch Lösungsmittelgemische.

Die Verbindungen der allgemeinen Formel (4-3), in der U, **V, X, Y, R³** und **R⁴** wie voranstehend erwähnt definiert sind und **LG** eine Austrittsgruppe darstellt, können analog Schema 4 synthetisiert werden.

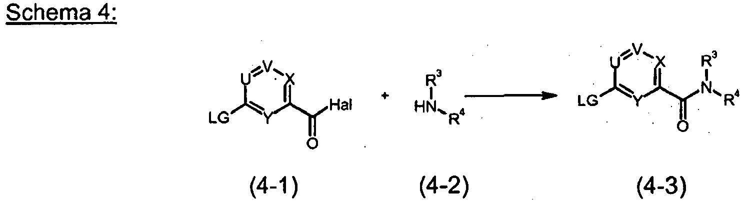

Carbonsäurehatogenide der allgemeinen Formel (4-1), in der **U, V, X** und **Y** wie eingangs erwähnt definiert sind, **LG** eine Austrittsgruppe, beispielsweise ein Halogenid, bedeutet und Hal ein Chlor oder Brom darstellt, können mit Verbindungen der allgemeinen Formel (4-2), in der **R³** und **R⁴** wie eingangs erwähnt definiet ist, umgesetzt werden. Dabei kann die Reaktion in einem inerten Lösungsmittel oder auch ohne Lösungsmittel durchgeführt werden. Ebenso kann die Reaktion mit oder ohne Base durchgeführt werden. Als inerte Lösungsmittel können halogenhaltige Kohlenwasserstoffe (besonders bevorzugt ist die Verwendung von Dichlormethan oder Dichlorethan), Dialkylether (bevorzugt ist Diethyleher), cyclische Ether (bevorzugt ist 1,4-Diöxan oder Tetrahydrofuran) und aromatische Kohlenwasserstoffe verwendet werden. Als Basen können tertiäre Amine (bevorzugt ist Triethylamin oder Düsopropylethylamin) und aromatische Amine (bevorzugt ist Pyridin) verwendet werden.

Die Verbindungen der allgemeinen Formel (5-3), in der **U, V, X,** Y, **R³** und **R⁴** wie voranstehend erwähnt definiert sind und **LG** eine Aüstrittgruppe darstellt, können analog Schema 5 synthetisiert werden.

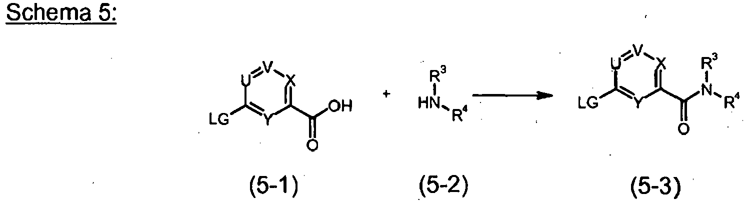

Carbonsäuren der allgemeinen Formel (5-1), in der **U, V, X** und **Y** wie eingangs erwähnt definiert sind und **LG** eine Austrittsgruppe darstellt, können mit Verbindungen der allgemeinen Formel (5-2), in der **R³** und **R⁴** wie eingangs erwähnt definiert sind, unter Zuhilfenahme von Standard-Peptid-Kupplungsreagenzien und einer Base in einem inerten Lösungsmittel zu Amiden der allgemeinen Formel (5-3), in der **U, V, X, Y, R³** und **R⁴** wie voranstehend erwähnt definiert sind und **LG** eine Austrittsgruppe darstellt, umgesetzt werden. (siehe z. B. Houben-Weyl, Methoden der Organischen Chemie, Bd. 15/2). Als Austrittsgruppe **LG** können Halogenide, vorzugsweise Chloride und Bromide, -SO₂CH₃, -OSO₂CH₃, -OSO₂C₆H₄-CH₃ oder -S-CH₃ verwendet werden (-S-CH₃ erfordert die weitere Umsetzung mit einem organischen Peroxid um in die eigentliche Austrittsgruppe -S(O)2-CH₃ überführt werden zu können), müssen aber nicht auf diese beschränkt sein. Ganz besonders bevorzugt ist die Verwendung von Chloriden. Als inerte Lösungsmittel können Dimethylformamid, N-Methylpyrrolidon, Dimethoxyethan, Dichlormethan, Acetonitril oder Lösungsmittelgemische verwendet werden. Bevorzugtes Lösungsmittel ist Dimethylformamid. Geeignete Basen sind besonders Amin-Basen wie z.B. Triethylamin oder Diisopropylethylamin. Als Kupplungsreagenzien können beispielsweise 1*H*-Benzotriazol-1-yl-oxy-tripyrrolidinö-phosphonium-hexafluorophosphat (PyBOP), Dicyclohexylcarbodiimid (DCC), Diisopropylcarbodiimid (DIC), Ethyl-(3-dimethylamino-propyl)-carbodiimid, O-(1*H*-Benzotriazöl-1-yl)-N,N-N,N-tetramethyl-uronium-hexafluorphosphat (HBTU) oder-tetrafluorborat (TBTU) oder 1*H*-Benzotriazol-1-yl-oxy-tris-(dimethylamino)-phosphonium-hexafluophosphat (BOP) verwendet werden. Besonders bevorzugt ist die Verwendung von TBTU. Die Aktivierung der Carboxylgruppe kann auch über ein entsprechendes Säureanhydrid oder Säurechlorid erfolgen. Die Umsetzung erfolgt im Allgemeinen in einem Temperaturbereich von -20°C bis zum Rückfluss des Lösungsmittels bei Normaldruck. Besonders bevorzugt ist die Verwendung von Diisopropylethylamin als Base und Dimethylformamid als Lösungsmittel.

Verbindungen der allgemeinen Formel (6-3), in der **U, V, X, Y, R¹** und **R²** wie voranstehend erwähnt definiert sind, lassen sich analog zu Schema 6 herstellen.

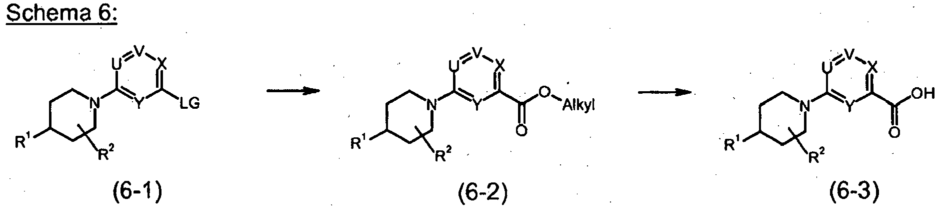

Dabei kann eine Verbindung der allgemeinen Formel (6-1), in der **U, V, X, Y, R¹** und **R²** wie eingangs erwähnt definiert sind und **LG** eine Austrittsgruppe darstellt, in Anwesenheit eines Katalysators und einer Hilfsbase mit einem Alkohol und Kohlenmonoxid reagieren. Für den Katalysator kann zusätzlich auch noch ein geeigneter Ligand verwendet werden. Als Austrittsgruppe **LG** können Chloride, Bromide, lodide, Trifluoracetate, Trifluormethansulfonate, Methansulfonate und Toluolsulfonate fungieren, müssen aber nicht auf diese beschränkt sein. Als Alkohole können bevorzugt Methanol und Ethanol verwendet werden, müssen aber nicht auf diese beschränkt sein. Geeignete Basen sind besonders Amin-Basen wie z.B. Triethylamin oder Diisopropylethylamin oder auch anorganische Basen wie Cäsiumcarbonat, Cäsiumacetat, Kaliumcarbonat, Kalium-*tert*-Butylat, Natriumcarbonat, Natriumacetat, Nätrium-*tert*-Butylat oder Kaliumphosphat. Typische Katalysatoren sind z.B.Übergangsmetall-Katalysatoren, wie z.B. Palladium-Katalysatoren wie Tris(dibenzylidenaceton)-dipalladium(O), Tetrakis-(triphenylphosphin)-palladium(0), Ralladium-(II)-acetat, Pd(PPh₃)₂Cl₂, Pd(CH₃CN)₂Cl₂, Pd(dppf)Cl₂ or Palladium(II)-chlorid. Typische Liganden sind z.B. Triphenylphosphin, Tricyclohexylphosphin, Tri-(*tert* Butyl)-phosphin, 1,4-Bis(diphenylphosphino)butan (dppb), 1,1'-Bis(diphenylphosphino)ferrocen (dppf), 1,3-Bis(diisopropylphosphino)-propan, 1,3-Bis(diphenylphosphino)propan (dppp), 1,4-bis(Dicyclohexylphosphino)butan, 1,1'-bis(Dicyclohexylphosphino)ferrocen. Der Druck von Kohlenmonoxid im Reaktionsgefäß beträgt von 1 bar bis 100 bar, bevorzugt sind erhöhte Kohlenmonoxid-Drücke von 10 bis 30 bar. Die Reaktionen können in einem Temperaturbereich von RT bis 200°C durchgeführt werden. Besonders bevorzugt ist ein Temperaturbereich von 100°C bis 150°C (M. Beller, W. Magerlein, A.F. Indolese, Ch. Fischer, Synthesis (2001) 7, 1098-1109 und darin zitierte Literatur).

Ester der allgemeinen Formel (6-2), in der **U, V, X,** Y, **R¹** und **R²** wie eingangs erwähnt definiert sind und Alkyl eine C₁₋₃-Alkylgruppe bedeutet, können durch basische oder saure Hydrolyse (J. March, Advanced Organic Chemistry (New York: J. Wiley and Sons,1985) oder durch Umsetzen mit Alkalimetal Salze (bevorzugt ist Lil oder NaCN) in einem inerten Lösungsmittel in die Säure der allgemeinen Formel (6-3) überführt werden. Inerte Lösungsmittel können Dialkylformamide (besonders bevorzugt N,N-Dimethylformamid), Dialkylacetamide (besonders bevorzugt ist N,N-Dimethylacetamide), cyclische Amide (besonders bevorzugt ist N-Methylpyrrolidon) sein. Besonders bevorzugt ist eine alkalische Verseifung mit Alkalimetal Hydroxiden wie Natriumhydroxid oder Lithiumhydroxid in inerten Lösungsmitteln. Als inerte Lösungsmittel eignen sich Wasser und cyclische Ether wie 1,4-Dioxan oder Tetrahydrofuran sowie auch Lösungsmittelgemische.

In einigen Fällen kann das Endprodukt weiter derivatisiert werden, z.B. durch Manipulation der Substituenten. Diese Manipulationen können unter anderem diejenigen sein, die dem Fachmann allgemein bekannt sind wie Oxidation, Reduktion, Alkylierung, Acylierung und Hydrolyse, müssen allerdings nicht auf diese beschränkt sein.

Die erfindungsgemäßen neuen Verbindungen der allgemeinen Formel I können ein oder mehrere Chiralitätszentren enthalten. Sind beispielsweise zwei Chiralitätszentren vorhanden, dann können die Verbindungen in Form zweier diastereomerer Antipodenpaare auftreten. Die Erfindung umfasst die einzelnen Isomere ebenso wie ihre Gemische.

Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel I fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)- oder (-)-Camphersulfonsäure, bzw. einer optisch aktiven Base, beispielsweise mit (R)-(+)-I-Phenylethylamin, (S)-(-)-1-Phenylethylamin oder (S)-Brucin, entstehen.

Nach einem üblichen Verfahren zur Isomerentrennung wird das Racemat einer Verbindung der allgemeinen Formel I mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemittel durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (R)- oder (S)-Enantiomer bzw. ein Gemisch zweier optisch aktiver, unter die allgemeine Formel **I** fallender, diastereomerer Verbindungen wird auch dadurch erhalten, dass man die oben beschriebenen Synthesen mit jeweils einer geeigneten *(R)-* bzw. (S)-konfigurierten Reaktionskomponente durchführt.

Die neuen Verbindungen der allgemeinen Formel **I** und deren physiologisch verträgliche Salze weisen wertvolle pharmakologische Eigenschaften auf, die auf ihre selektiven CGRP-antagonistischen Eigenschaften zurück gehen. Ein weiterer Gegenstand der Erfindung sind diese Verbindungen enthaltende Arzneimittel, deren Verwendung und deren Herstellung.

Die voranstehend genannten neuen Verbindungen und deren physiologisch verträgliche Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität der voranstehend genannten Verbindungen zu humanen CGRP-Rezeptoren und ihrer antagonistischen Eigenschaften wurden die folgenden Versuche durchgeführt:

### A. Bindungsstudien mit (den humanen CGRP-Rezeptor exprimierenden) SK-N-MC-Zellen

SK-N-MC Membranen (∼ 20 µg Proteinmenge) werden für 180 Minuten bei Raumtemperatur mit 50 pM ¹²⁵I-Iodotyrosyl-Calcitonin-Gene-Related Peptide und ansteigenden Konzentrationen der Testsubstanzen in einem Gesamtvolumen von 250 µl inkubiert (Assay Puffer: 10 mM Tris, 50 mM NaCl, 5 mM MgCl₂, 1mM EDTA, pH=7.4). Die Inkubation wird durch rasche Filtration durch mit Polyethylenimin (0.1%) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM BIBN4096BS während der Inkubation definiert.

Die Analyse der Konzentrations-Bindungskurven erfolgt mit Hilfe einer computergestützten nichtlinearen Kurvenanpassung.

Die eingangs erwähnten Verbindungen zeigen in dem beschriebenen Test Kᵢ-Werte ≤ 50 µM.

### B. CGRP-Antagonismus in SK-N-MC-Zellen

SK-N-MC Zellen (∼1000 Zellen pro well) werden in Anwesenheit von steigenden Konzentrationen von CGRP und verschiedenen Konzentrationen der Testsubstanz für 30 Minuten inkubiert.

Die cAMP-Gehalte der Proben werden mittels AlphaScreen cAMP assay kit (Perkin Elmer) bestimmt und die pA₂-Werte antagonistisch wirkender Substanzen graphisch ermittelt.

Die erfindungsgemäßen Verbindungen zeigen in dem beschriebenen *in vitro*-Testmodell CGRP-antagonistische Eigenschaften in einem Dosisbereich zwischen 10⁻¹² bis 10⁻⁴ M.

Um zu zeigen, dass die Verbindungen der allgemeinen Formel I mit unterschiedlichen Strukturelementen gute bis sehr gute CGRP-antagonistische Aktivitäten zeigen, werden in der folgenden Tabelle die Kᵢ-Werte angegeben, die gemäß dem voranstehenden Versuchsprotokoll erhalten wurden. Dazu wird angemerkt, dass die Verbindungen im Hinblick auf ihre unterschiedlichen strukturellen Elemente ausgewählt wurden und nicht, um spezifische Verbindungen hervorzuheben:

| **Beispiel** | **Kᵢ [nM]** |
|---|---|
| (9) | 21 |
| (21) | 5 |
| (22) | 2 |
| (26) | 21 |

### INDIKATIONSGEBIETE

Aufgrund ihrer pharmakologischen Eigenschaften eignen sich die erfindungsgemäßen Verbindungen und deren Salze mit physiologisch verträglichen Säuren somit zur akuten und prophylaktischen Behandlung von Kopfschmerzen, insbesondere Migräne-, Cluster-Kopfschmerz sowie Spannungskopfschmerzen. Weiterhin beeinflussen die erfindungsgemäßen Verbindungen auch die folgenden Erkrankungen positiv: Nicht-insulinabhängigen Diabetes mellitus ("NIDDM"), cardiovaskuläre Erkrankungen, Morphintoleranz, Clostridiumtoxin-bedingte Durchfallerkrankungen, Erkrankungen der Haut, insbesondere thermische und strahlenbedingte Hautschäden inklusive Sonnenbrand, Lichen, Prurigo, pruriginöse Toxidermien sowie schwerer Juckreiz, entzündliche Erkrankungen, z.B.entzündliche Gelenkerkrankungen (Osteoarthritis, rheumatoide Arthritis, neurogene Arthritis), generalisierter Weichteilrheumatismus (Fibromyalgie), neurogene Entzündungen der oralen Mucosa, entzündliche Lungenerkrankungen, allergische Rhinitis, Asthma, COPD, Erkrankungen, die mit einer überschießenden Gefäßerweiterung und dadurch bedingter verringerter Gewebedurchblutung einhergehen, z.B. Schock und Sepsis, chronische Schmerzerkrankungen, wie z.B. diabetische Neuropathien, durch Chemotherapien induzierte Neuropathien, HIV-induzierte Neuropathien, postherpetische Neuropathien durch Gewebetrauma induzierte Neuropathien, trigeminale Neuralgien, temporomandibuläre Dysfunktionen, CRPS (complex regional pain syndrome), Rückenschmerzen, und viszerale Erkrankungen, wie z.B. irritable bowel syndrome (IBS), inflammatory bowel syndrome Darüber hinaus zeigen die erfindungsgemäßen Verbindungen eine lindernde Wirkung auf Schmerzzustände im allgemeinen. Die Symptomatik menopausaler, durch Gefäßerweiterung und erhöhten Blutfluss verursachter Hitzewallungen östrogendefizienter Frauen sowie hormonbehandelter Prostatakarzinompatienten und Kastraten wird durch die CGRP-Antagonisten der vorliegenden Anwendung präventiv und akut-therapeutisch günstig beeinflusst, wobei sich dieser Therapieansatz vor der Hormonsubstitution durch Nebenwirkungsarmut auszeichnet.

Vorzugsweise eignen sich die erfindungsgemäßen Verbindungen zur akuten und prophylaktischen Behandlung von Migräne- und Cluster-Kopfschmerz, zur Behandlung des irritable bowel syndroms (IBS) und zur präventiven und akut-therapeutischen Behandlung von Hitzewallungen östrogendefizienter Frauen.

Die zur Erzielung einer entsprechenden Wirkung erforderliche Dosierung beträgt zweckmäßigerweise bei intravenöser oder subkutaner Gabe 0.0001 bis 3 mg/kg Körpergewicht, vorzugsweise 0.01 bis 1 mg/kg Körpergewicht, und bei oraler, nasaler oder inhalativer Gabe 0.01 bis 10 mg/kg Körpergewicht, vorzugsweise 0.1 bis 10 mg/kg Körpergewicht, jeweils ein- bis dreimal täglich.

Sofern die Behandlung mit CGRP-Antagonisten oder/und CGRP-Release-Hemmern in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann.

Ein weiterer Gegenstand der Erfindung ist die Verwendung der erfindungsgemäßen Verbindungen als wertvolle Hilfsmittel zur Erzeugung und Reinigung (Affinitätschromatographie) von Antikörpern sowie, nach geeigneter radioaktiver Markierung, beispielsweise durch Tritiierung geeigneter Vorstufen, beispielsweise durch katalytische Hydrierung mit Trithium oder Ersatz von Halogenatomen durch Tritium, in RIA- und ELISA-Assays und als diagnostische bzw. analytische Hilfsmittel in der Neurotransmitter-Forschung.

### KOMBINATIONEN

Als Kombinationspartner denkbare Wirkstoffklassen sind z.B. Antiemetica, Prokinetica, Neuroleptica, Antidepressiva, Neurokinin-Antägonisten, Anticonvulsiva, Histamin-H1-Rezeptorantagonisten, β-Blocker, α-Agonisten und α-Antagonisten, Ergotalkaloiden, schwachen Analgetica, nichtsteroidalen Antiphlogistica, Corticosteroiden, Calcium-Antagonisten, 5-HT_{1B/1D}-Agonisten oder andere Antimigräriemitteln, die zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/- Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Dosieraerosole oder Zäpfchen eingearbeitet werden können.

Für die oben erwähnten Kombinationen kommen somit als weitere Wirksubstanzen beispielsweise die nicht-steroidalen Antiphlogistika Aceclofenac, Acemetacin, Acetylsalicylsäure, Acetaminophen (Paracetamol), Azathioprin, Diclofenac, Diflunisal, Fenbufen, Fenoprofen, Flurbiprofen, Ibuprofen, Indometacin, Ketoprofen, Leflunomid, Lornoxicam, Mefenaminsäure, Naproxen, Phenylbutazon, Piroxicam, Sulfasalazin, Zomepirac oder deren pharmazeutisch verträgliche Salze sowie Meloxicam und andere selektive COX2-Inhibitoren, wie beispielsweise Rofecoxib, Valdecoxib, Parecoxib, Etoricoxib und Celecoxib, in Betracht sowie Substanzen, die frühere oder spätere Schritte in der Prostaglandin-Synthese inhibieren oder Prostaglandinrezeptor Antagonisten wie z.B. EP2-Rezeptor Antagonisten und IP-Rezeptor Antagonisten.

Weiterhin können Ergotamin, Dihydroergotamin, Metoclopramid, Domperidon, Diphenhydramin, Cyclizin, Promethazin, Chlorpromazin, Vigabatrin, Timolol, Isomethepten, Pizotifen, Botox, Gabapentin, Pregabalin, Duloxetin, Topiramat, Riboflavin, Montelukast, Lisinopril, Micardis, Prochlorperazin, Dexamethason, Flunarizin, Dextropropoxyphen, Meperidin, Metoprolol, Propranolol, Nadolol, Atenolol, Clonidin, Indoramin, Carbamazepin, Phenytoin, Valproat, Amitryptilin, Imipramine, Venlafaxine, Lidocain oder Diltiazem und andere 5-HT_{1B/1D}-Agonisten wie z.B. Almotriptan, Avitriptan, Eletriptan, Frovatriptan, Naratriptan, Rizatriptan, Sumatriptan und Zolmitriptan verwendet werden.

Außerdem können CGRP-Antagonisten mit Vanilloid-Rezeptor Antagonisten, wie z.B. VR-1 Antagonisten, Glutamatrezeptor Antagonisten, wie z.B. mGlu5-Rezeptor Antagonisten, mGlu1-Rezeptor Antagonisten, iGlu5-Rezeptor Antagonisten, AMPA-Rezeptor Antagonisten, Purinrezeptor Blockern, wie z.B. P2X3 Antagonisten, NO-Synthase Inhibitoren, wie z.B. iNOS Inhibitoren, Calciumkanal-Blockern, wie z.B. PQ-typ Blockern, N-typ Blockern, Kaliumkanalöffnern, wie z.B. KCNQ Kanalöffnern, Natriumkanal Blockern, wie z.B. PN3 Kanal Blockern, NMDA-Rezeptor Antagonisten, Acid-sensing Ionenkanal Antagonisten, wie z.B. ASIC3 Antagonisten, Bradykinin Rezeptor Antagonisten wie z.B. B1-Rezeptor Antagonisten, Cannabinoid-Rezeptor Agonisten, wie z.B. CB2 Agonisten, CB1 Agonisten, Somatostatin-Rezeptor Agonisten, wie z.B. sst2 Rezeptor Agonisten gegeben werden.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

### DARREICHUNGSFORMEN

Die erfindungsgemäß hergestellten Verbindungen können entweder alleine oder gegebenenfalls in Kombination mit anderen Wirksubstanzen zur Behandlung von Migräne intravenös, subkutan, intramuskulär, intraartikulär, intrarektal, intranasal, durch Inhalation, topisch, transdermal oder oral erfolgen, wobei zur Inhalation insbesondere Aerosolformulierungen geeignet sind. Die Kombinationen können entweder simultan oder sequentiell verabreicht werden.

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0.1 bis 90 Gew.-%, bevorzugt 0.5 bis 50 Gew.-% der Gesamtzusammensetzung liegen, d.h. in Mengen die ausreichend sind, um den voranstehend angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel I gemäß der obigen bevorzugten Ausführungsformen.

Besonders bevorzugt ist es, wenn die Verbindungen der Formel I oral verabreicht werden, ganz besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnussoder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Ebenfalls bevorzugt ist es, wenn die Verbindungen der Formel I inhalativ verabreicht werden, besonders bevorzugt ist es, wenn die Verabreichung ein oder zweimal täglich erfolgt. Hierzu müssen die Verbindungen der Formel I in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalätionslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### EXPERIMENTELLER TEIL

Für die hergestellten Verbindungen liegen in der Regel ¹H-NMR und/oder Massenspektren vor. Wenn nicht anders angegeben, werden R_{f}-Werte unter Verwendung von DC-Fertigplatten Kieselgel 60 F254 (E. Merck, Darmstadt, Artikel-Nr. 1.05714) ohne Kammersättigung bestimmt.
Die bei den Fliessmitteln angegebenen Verhältnisse beziehen sich auf Volumeneinheiten der jeweiligen Lösungsmittel. Die angegebenen Volumeneinheiten bei NH₃ beziehen sich auf eine konzentrierte Lösung von NH₃ in Wasser.
Verwendete Laufmittelsysteme für DC:
- Laufmittel A: DCM/Cyclohexan/MeOH/NH₄OH = 70/15/15/2
- Laufmittel B: Petrolether/Ethylacetat = 2/1

Soweit nicht anders vermerkt sind die bei den Aufarbeitungen der Reaktionslösungen verwendeten Säure-, Basen- und Salzlösungen wässrige Systeme der angegebenen Konzentrationen. Zu chromatographischen Reinigungen wird Kieselgel der Firma Millipore (MATREX™, 35-70 µm) verwendet.
Die angegebenen HPLC-Daten werden unter nachstehend angeführten Parametern und unter Verwendung der aufgeführten Säulen erhoben:

### Verwendete Säulen:

### (Säulentemperatur: 30°C; Injektionsvolumen: 5 µL; Detektion bei 254 nm)

| | |
|---|---|
| S1 | Zorbax-Säule (Agilent Technologies), SB (Stable Bond) C18; 3.5 µm; 4.6 x 75 mm |
| S2 | Waters Sunfire, SB (Stable Bond) C18; 3.5 µm; 4.6 x 75 mm |
| S3 | Agilent Bonus C18; 5 µm, 4.6 x 75 mm |
| S4 | Zorbax-Säule (Agilent Technologies), SB (Stable Bond) C18; 1.8 µm; 3.0 x 30 mm |
| S5 | Zorbax-Säule (Agilent Technologies), SB (Stable Bond) C18; 5 µm; 4.6 x 75 mm |
| S6 | Waters Symmetry C18; 3.5 µm; 4.6 x 75 mm |
| S7 | Waters XBridge C18; 3.5 µm; 4.6 x 75 mm (basische Säule) |
| S8 | WatersSunfire C18; 2.5 µm; 3.0 x 30 mm |

### Verwendete Lösungsmittel:

- Für die Säulen S1 bis S6 (saure Bedingungen) wurden als Lösungsmittel verwendet:
   Lösungsmittel A: Wasser (mit 0.1% Ameisensäure)
   Lösungsmittel B: Acetonitril (mit 0.1% Ameisensäure)
- Für die Säule S7 (basische Bedingungen) wurden folgende Lösungsmittel verwendet:
   Lösungsmittel A: Wasser (mit 0.1% NH₄OH)
   Lösungsmittel B: Acetonitril (mit 0.1% NH₄OH)
   (die prozentualen Angaben beziehen sich auf das Gesamtvolumen)

### Gradienten:

| Gradient (Fluss) | Zeit [min] | %A | %B |
|---|---|---|---|
| **G1** (0.8 mL/min) | 0.0 | 95 | 5 |
| | 8.0 | 50 | 50 |
| | 9.0 | 10 | 90 |
| | 10.0 | 10 | 90 |
| | 11.0 | 95 | 5 |

| Gradient | Zeit | %A | %B |
|---|---|---|---|
| | [min] | | |
| **G2** (1.6 mL/min) | 0.00 | 95 | 5 |
| | 0.10 | 95 | 5 |
| | 1.75 | 5 | 95 |
| | 1.90 | 5 | 95 |
| | 1.95 | 95 | 5 |
| | 2.00 | 95 | 5 |

| **Gradient** | Zeit [min] | %A | %B |
|---|---|---|---|
| **G3** (1.6 mL/min) | 0.00 | 95 | 5 |
| | 4.50 | 10 | 90 |
| | 5.00 | 10 | 90 |
| | 5.50 | 95 | 5 |

| Gradient | Zeit [min] | %A | %B |
|---|---|---|---|
| **G4** (1.6 mL/min) | 0.00 | 95 | 5 |
| | 4.00 | 50 | 50 |
| | 4.50 | 10 | 90 |
| | 5.00 | 10 | 90 |
| | 5.50 | 95 | 5 |

| Gradient | Zeit [min] | %A | %B |
|---|---|---|---|
| **G5** (1.6 mL/min) | 0.00 | 90 | 10 |
| | 4.50 | 10 | 90 |
| | 5.50 | 10 | 90 |

| Gradient (Fluss) | Zeit [min] | %A | %B |
|---|---|---|---|
| **G6** (0.8 mL/min) | 0.0 | 95 | 5 |
| | 9:0 | 10 | 90 |
| | 10.0 | 10 | 90 |
| | 11.0 | 95 | 5 |

| Gradient | Zeit [min] | %A | %B |
|---|---|---|---|
| **G7** (1.6 mL/min) | 0.00 | 95 | 5 |
| | 2.00 | 50 | 50 |
| | 2.25 | 10 | 90 |
| | 2.50 | 10 | 90 |
| | 2.75 | 95 | 5 |

### Methoden:

| | Säule | Gradient |
|---|---|---|
| Methode A | S1 | G4 |
| Methode B | S2 | G4 |
| Methode C | S4 | G2 |
| Methode D | S6 | G4 |
| Methode E | S1 | G3 |
| Methode F | S3 | G3 |
| Methode G | S5 | G4 |
| Methode H | S1 | G5 |
| Methode K | S2 | G3 |
| Methode L | S1 | G2 |
| Methode M | S7 | G3 |
| Methode N | S2 | G1 |
| Methode O | S2 | G6 |
| Methode Q | S5 | G5 |
| Methode R | S4 | G7 |
| Methode S | S8 | G7 |

Bei präparativen HPLC-Reinigungen erfolgt die Sammlung der Produkte entweder massengesteuert oder über UV-Detektion. Die Produkt enthaltenden Fraktionen werden vereinigt und gefriergetrocknet. Für präparative HPLC-Trennungen können folgende Säulen verwendet werden:

| | |
|---|---|
| S8 | Agilent Zorbax SB C18, 50 x 150 mm, 5 µm |
| S9 | Agilent Zorbax Stable Bond, 50 x 140 mm, 7 µm |
| S10 | Waters Sunfire C18, 30 x 100 mm, 5 µm |
| S11 | Waters Symmetry 50 x 140 mm, 7 µm |
| S12 | Agilent Zorbax Stable Bond C18, 30 x 100 mm, 5 µm, |

Für die präparative HPLC-Trennung können folgende Lösungsmittelsysteme verwendet werden:
- Lösungsmittel A: Wasser (mit 0.1% Ameisensäure)
   Lösungsmittel B: Acetonitril (mit 0.1% Ameisensäure)
- Lösungsmittel A: Wasser (mit 0.15% Ameisensäure)
   Lösungsmittel B: Acetonitril (mit 0.15% Ameisensäure)
- Lösungsmittel A: Wasser (mit 0.3% Ameisensäure)
   Lösungsmittel B: Acetonitril
- Lösungsmittel A: Wasser (mit 0.3% Ameisensäure)
   Lösungsmittel B: Acetonitril (mit 0.3% Ameisensäure)
- Lösungsmittel A: Wasser (mit 0.1% NH₄OH)
   Lösungsmittel B: Acetonitril (mit 0.1% NH₄OH)

Die prozentualen Angaben beziehen sich jeweils auf das Gesamtvolumen.

Falls nähere Angaben zur Konfiguration fehlen, bleibt offen, ob es sich um reine Enantiomere handelt oder ob partielle oder gar völlige Racemisierung eingetreten ist.

In den Versuchsbeschreibungen werden die folgenden Abkürzungen verwendet:
- AAV: Allgemeine Arbeitsvorschrift
- ACN: Acetonitril
- AcOH: Essigsäure
- BINAP: 2,2'-Bis-(diphenylphosphino)-1,1'-binaphtyl
- BOC: *tert*.-Butyloxycarbonyl
- CDI: 1,1'-Carbonyldiimidazol
- CO: Kohlenmonoxid
- Cyc: Cyclohexan
- DCM: Dichlormethan
- DIPE: Diisopropylether
- DIPEA: Diisopropylethylamin
- DMAP: 4-Dimethylaminopyridin
- DMF: *N,N*-Dimethylformamid
- dppf: 1,1'-Bis-(diphenylphosphino)ferrocen
- d.Th.: der Theorie
- EI: Elektronenstoß-Ionisation (bei MS)
- Eq: Äquivalent
- ESI: Elektrospray-Ionisation (bei MS)
- EtOAc: Essigsäureethylester
- EtOH: Ethanol
- E-Wasser: Entionisiertes Wasser
- HATU: [Dimethylamino-(1,2,3-triazolo[4,5-blpyridin-3-yloxy)-methylen]-dimethyl-ammonium-hexafluorphosphat
- HCl: Chlorwasserstoff
- HPLC: High Performance Liquid Chromatography
- HPLC-MS: HPLC gekoppelte Massenspektrometrie
- i.vac.: in vacuo (im Vakuum)
- konz.: Konzentriert
- MeOH: Methanol
- MS: Massenspektrometrie
- MW: Molekulargewicht [g/mol]
- NaOAc: Natriumacetat
- NaOH: Natriumhydroxid
- NH₄OH: Ammoniumhydroxid (wässrige Ammoniak-Lösung, 30%)
- NMP: N-Methyl-2-Pyrrolidin
- Pd/C: Palladium auf Kohle
- Pd₂dba₃: Bis(dibenzylidenaceton) palladium (0)
- PE: Petrolether
- R_{f}: Retentionsindex (bei DC)
- RT: Raumtemperatur
- R,: Retentionszeit (bei HPLC)
- TBME: tert.-Butyl-methylether
- TBTU: O-(Benzotriazol-1-yl)-*N,N,N',N'-*tetramethyluronium-tetrafluoroborat
- TEA: Triethylamin
- TFA: Trifluoressigsäure
- THF: Tetrahydrofuran
- TS: Trockenschrank
- ULTS: Umluft-Trockenschrank
- XantPhos: 4,5-Bis(Diphenylphosphino)-9,9-dimethylxanthen
- XPhos: 2-Dicyclohexylphosphino-2',4',6'-triisopropylbiphenyl

### Herstellung der Ausgangsverbindungen:

### Intermediat 1 a:

### 1-Piperidin-4-yl-1,3-dihydroimidazo[4.5-b]pyridin-2-on-Dihydrochlorid

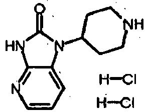

Diese Verbindung und deren Vorstufen wurden wie in der internationalen Anmeldung PCT/US2004/020209 beschrieben synthetisiert.

| | |
|---|---|
| ESI-MS: | m/z = 219 (M+H)⁺ |
| R_{f}: | 0.11 (Kieselgel, DCM/MeOH/NH₄OH = 80:20:2) |

### Intermediat 1 b:

### 1-Piperidin-4-yl-1,3-dihydroimidazo[4,5-b]pyridin-2-on

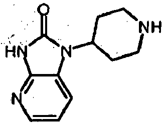

### Stufe 1: 4-(2-Chlor-pyridin-3-yl-amino)-piperidin-1-carbonsäurebenzylester

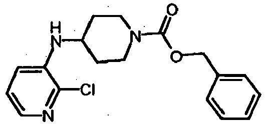

Zu 930 g (3.99 mol) *N*-Benzyloxycarbonyl-4-piperidon und 466 g (3.63 mol) 2-Chlor-3-aminopy idin in 9.5 L Isopropylacetat wurden bei ca. 15°C 560 mL (7.25 mol) TFA zugetropft. 922 g (4.35 mol) Natriumtriacetoxyborhydrid wurden portionsweise zugegeben. Es wurde bis zur Vollständigkeit der Reaktion nachgerührt. Bei RT wurde die Reaktionsmischung mit 860 mL Natronlauge (2 mol/L) versetzt. Die organische Phase wurde abgetrennt, mit 5 L Wasser gewaschen und eingeengt.

| | |
|---|---|
| Ausbeute: | 1250 g (roh, quant.) |
| ESI-MS: | m/z = 346 (M+H)⁺ |

### Stufe 2: 4-[1-(2-Chlor-pyridin-3-yl)-ureido]-piperidin-1-carbonsäurebenzylester

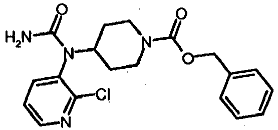

530 mL (6.1 mol) Chlorsulfonylisocyanat wurden in 6 L THF vorgelegt und auf -15°C abgekühlt. Zu diesem Gemisch würde anschließend eine Lösung von 1.25 kg (3.63 mol) 4-(2-Chlor-pyridin-3-yl-amino)-piperidin-1-carbonsäurebenzylester in 7 L THF innerhalb einer Stunde so zugetropft, dass die Temperatur der Reaktionsmischung -7°C nicht überstieg. Es wurde 90 Minuten bei ca. -8°C nachgerührt und anschließend 700 mL Wasser innerhalb von 30 Minuten zugetropft. Die Mischung wurde 30 Minuten bei ca. 10°C nachgerührt und anschließend mit 8.1 L Natronlauge (2 mol/L) langsam versetzt. Das Reaktionsgemisch wurde anschließend auf 50°C erwärmt und die Phasen getrennt. Die organische Phase wurde mit 2 L Wasser gewaschen. Danach wurden aus der organischen Phase 10 L Lösemittel abdestilliert, 15 L Butylacetat zum Rückstand zugegeben und hiervon nochmals 8 L abdestilliert. Durch langsames Abkühlen auf 0°C wurde das Produkt kristallisiert. Der Niederschlag wurde abgesaugt, mit 2L Butylacetat gewaschen und bei 40°C getrocknet.

| | |
|---|---|
| Ausbeute: | 1108 g (79% d.Th.) |
| ESI-MS: | m/z = 389/391 (M+H)⁺ |

### Stufe 3: 4-(2-Oxo-2,3-dihydro-imidazo[4,5-b]pyridin-1-yl)-piperidin-1-carbonsäure-Benzylester

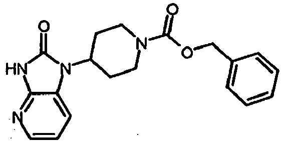

1108 g (2.85 mol) 4-[1-(2-Chlor-pyridin-3-yl)-ureido]-piperidin-1-carbonsäurebenzylester wurden mit 720 g (8.57 mol) Natriumhydrogencarbonat in 14.5 L tert-Amylalkohol unter Rückfluss erhitzt. Hierbei wurden 3 L Lösemittel abdestilliert. Das Reaktionsgemisch wurde auf 35°C abgekühlt und mit 11 mL Wasser versetzt. Anschließend wurden 13 g (0.058 mol) Palladiumacetat und 49 g (0.115 mol) 1,4-Bis-(diphenylphosphino)-butan (DPPB) zugegeben und auf Rückflusstemperatur erhitzt. Es wurde bei 100°C bis zur Vollständigkeit der Reaktion nachgerührt, auf RT abgekühlt und 7.5 L Wasser zugegeben. Die organische Phase wurde abgetrennt, mit 5 L Wasser gewaschen und anschließend eingeengt. Der ölige Rückstand wurde zweimal mit je 3 L Isopropylacetat versetzt und abdestilliert. Anschließend wurde der Rückstand in 7 L Isopropylacetat heiß gelöst und langsam auf Raumtemperatur abgekühlt. Der auskristallisierte Feststoff wurde abgesaugt, mit je 2 L Isopropylacetat und tert.-Butyl-methylether gewaschen und bei 50°C getrocknet.

| | |
|---|---|
| Ausbeute: | 690 g (69% d.Th.) |
| ESI-MS: | m/z = 353 (M+W)⁺ |

### Stufe 4: 1-Piperidin-4-yl-1,3-dihydro-imidazo[4,5-b]pyridin-2-on

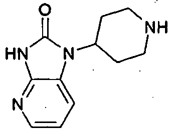

690 g (1.96 mol) 4-(2-Oxo-2,3-dihydro-imidazo[4,5-b]pyridin-1-yl)-piperidin-1-carbonsäure-benzylester wurden in 5.4 L Methanol gelöst und unter Zusatz von 46 g Pd/C (10%ig; 6.6 Gew.%) bei 60°C und einem Wasserstoffdruck von 60 psi bis zur vollständigen Wasserstoffaufnahme hydriert. Der Katalysator wurde abfiltriert. Vom Filtrat wurden 4 L Methanol abdestilliert. Es wurden 2 L Methylcyclohexan zugegeben und weitere 1.5 L Lösemittel abdestilliert. Die so erhaltene Suspension wurde abgesaugt, der Rückstand mit Methylcyclohexan gewaschen und bei 40°C getrocknet.

| | |
|---|---|
| Ausbeute: | 446 g (100% d.Th.) |
| ESI-MS: | m/z = 219 (M+H)⁺ |

### Intermediat 2:

### 3-Piperidin-4-yl-1,3,4,5-tetrahydro-1,3-benzodiazepin-2-on

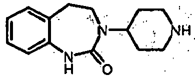

Diese Verbindung und deren Vorstufen wurden wie in der Europäischen Patentanmeldung

| | |
|---|---|
| Nr. EP 1 619 187 | beschrieben synthetisiert. |
| ESI-MS: | m/z = 246 (M+H)⁺ |

### Intermediat 3:

### 7-Chlor-3-piperidin-4-yl-1,3,4,5-tetrahydro-1,3-benzodiazepin-2-on

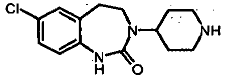

Zu 1.23 g (5.0 mmol) 3-Piperidin-4-yl-1,3,4,5-tetrahydro-1,3-benzodiazepin-2-on in 10 mL Tetrachlorkohlenstoff wurden 801 mg (6.0 mmol) N-Chlorsuccinimid zugegeben und der Reaktionsansatz wurde 3 Tage unter Rückfluss gekocht. Das Lösungsmittel wurde am Rotationsverdampfer eingeengt und der Rückstand wurde mittels flash-Chromatographie aufgereinigt. Die Produktfraktionen wurden vereinigt und das Lösungsmittel am Rotationsverdampfer entfernt. Zur weiteren Aufreinigung wurden die Produktfraktionen mittels präparativer HPLC aufgereinigt. Die Produktfraktionen wurden vereinigt und am Rotationsverdampfer bis zur Trockne eingeengt.

| | |
|---|---|
| Ausbeute: | 420 mg (30% d.Th.) |
| ESI-MS: | m/z = 280/282 (M+H)⁺ |
| Rₜ (HPLC-MS): | 2.04 min (Methode E) |

### Intermediat 4:

### 7-Methoxy-3-piperidin-4-yl-1,3,4,5-tetrahydro-1,3-benzodiazepin-2-on

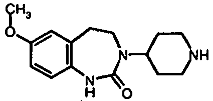

### Stufe 1: (5-Methoxy-2-nitrophenyl)-acetonitril

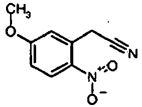

Zu einer Lösung aus 13.2 g (86.0 mmol) 4-Nitroanisol und 18.0 g (107 mmol) 4-Chlorphenoxyacetonitril in 50 mL DMF wurden 24.0 g (214 mmol) Kalium-*tert*-butylat in 100 mL DMF langsam zugetropft. Der Reaktionsansatz wurde 30 min bei -10°C gerührt und anschließend in 300 g einer 1:1 Mischung aus konz. Salzsäure und Eis gegossen. Nach Extraktion mit EtOAc wurde die organische Phase mit Wasser gewaschen, getrocknet und im Vakuum unter gelindem Erwärmen bis zur Trockne einrotiert. Der Rückstand wurde mit einer 1:1 Mischung Petrolether/EtOAc behandelt und das auskristallisierte Produkt abgesaugt. Nach dem Nachwaschen mit einer 1:1 Mischung Petrolether/EtOAc wurden die Kristalle an der Luft getrocknet.

| | |
|---|---|
| Ausbeute: | 6.5 g (39% d.Th.) |
| ESI-MS: | m/z = 210 (M+NH₄)⁺ |
| R_{f}: | 0.45 (Kieselgel; PE/EtOAc = 1:1) |

### Stufe 2: 2-(5-Methoxy-2-nitrophenyl)-ethylamin

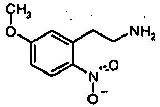

Unter einer Stickstoffatmosphäre wurden bei RT zu 12.6 g (65.7 mmol) (5-Methoxy-2-nitrophenyl)-acetonitril in 380 mL THF langsam 200 mL (200 mmol) einer 1 M Boran in THF Lösung zugetropft. Der Reaktionsansatz wurde 2 h unter Rückfluss gekocht. Nach dem Erkalten wurden innerhalb von 20 min 30 mL Methanol zugetropft. Dabei wurde mit einem Eisbad die Temperatur bei 10°C bis 20°C gehalten. Man ließ den Reaktionsansatz 30 min bei RT nachrühren und tropfte im Anschluss daran innerhalb von 30 min 45 mL einer 2M wässrigen Salzsäure-Lösung hinzu. Das Reaktionsgemisch wurde unter gelindem Erwärmen i.vac. einrotiert. Der Rückstand wurde mit Wasser auf ca. 200 mL verdünnt und mit 200 mL EtOAc extrahiert. Die wässrige Phase wurde mit einer 15%igen (w/v) wässrigen Kaliumcarbonat-Lösung alkalisch gestellt und über Nacht mit einem Rotations-Perforator nach Ludwig (Fa. Normag) mit Diethylether kontinuierlich extrahiert. Das organische Extrakt wurde bis zur Trockene einrotiert.

| | |
|---|---|
| Ausbeute: | 9.98 g (77% d.Th.) |
| ESI-MS: | m/z = 197 (M+H)⁺ |
| Rₜ(HPLC): | 2.1 min (Methode E) |

### Stufe 3: (1-Benzylpiperidin-4-yl)-[2-(5-methoxy-2-nitrophenyl)-ethyl]-amin

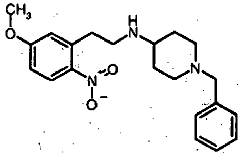

Unter einer Stickstoffatmosphäre wurde in einem Eisbad ein Gemisch aus-9.98 g (50.9 mmol) 2-(5-Methoxy-2-nitrophenyl)-ethylamin, 9.8 mL (54.9 mmol) *N*-Benzylpiperidon und 6.3 mL (114 mmol) Essigsäure in 270 mL Dichlormethan auf 0°C gekühlt. Bei dieser Temperatur wurden 14.2 g (67.0 mmol) Natriumtriacetoxyborhydrid portionsweise innerhalb von 20 min zugegeben. Man ließ den Reaktionsansatz weitere 4 h bei 0°C nachrühren und über Nacht auf RT aufwärmen. Anschließend wurde der Ansatz mit 400 mL einer 15%igen (w/v) wässrigen Kaliumcarbonat-Lösung versetzt und 1 h bei RT nachgerührt. Die organische Phase wurde abgetrennt, getrocknet und einrotiert.

| | |
|---|---|
| Ausbeute: | 18.8 g (quantitativ) |
| ESI-MS: | m/z = 370 (M+H)⁺ |
| Rₜ(HPLC): | 1.9 min (Methode E) |

### Stufe 4: [2-(2-Amino-5-methoxy-phenyl)-ethyl]-(1-benzylpiperidin-4-yl)-amin

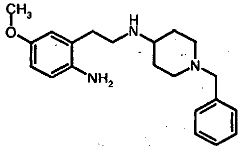

26.0 g (70.3 mmol) (1-Benzylpiperidin-4-yl)-[2-(5-methoxy-2-nitrophenyl)-ethyl]-amin wurden mit 5.0 g (2.45 mmol) Rhodiumkohle (5%, wasserfeucht) in 350 mL Methanol in einer 3 bar Wasserstoffatmosphäre 3 h bei RT hydriert. Der Katalysator wurde abgesaugt und die Lösung einrotiert. Der Rückstand wurde ohne weitere Aufreinigung sofort weiter umgesetzt.

| | |
|---|---|
| Ausbeute: | 23.9 g (quantitativ) |
| Rₜ(HPLC): | Rₜ = 0.99 min (Methode A) |

### Stufe 5: 3-(1-Benzylpiperidin-4-yl)-7-methoxy-1,3,4,5-tetrahydro-1,3-benzodiazepin-2-on

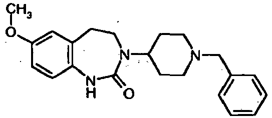

Zu 23.9 g (70.3 mmol) [2-(2-Amino-5-methoxyphenyl)-ethyl]-(1-benzylpiperidin-4-yl)-amin in 175 mL DMF wurden 35.0 g (216 mmöl) *N,N*'-Carbonyldiimidazol zugegeben und die Mischung 2 h bei 100°C gerührt. Der Reaktionsansatz wurde auf ca. 1 kg Eiswasser gegossen und über Nacht gerührt. Das ausgefallene Produkt wurde abgesaugt, mit 100 mL Wasser gewaschen und getrocknet. Der Rückstand Wurde mit 150 mL DIPE verrührt und abgesaugt. Das Festprodukt wurde mit 50 mL DIPE nachgewaschen und getrocknet.

| | |
|---|---|
| Ausbeute: | 21.6 g (84% d.Th.) |
| ESI-MS: | m/z = 366 (M+H)⁺ |
| Rₜ(HPLC): | 2.12 min (Methode E) |

### Stufe 6: 7-Methoxy-3-piperidin-4-yl-1,3,4,5-tetrahydro-1,3-benzodiazepin-2-on

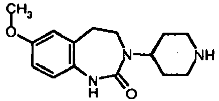

Eine Mischung aus 21.6 g (59.2 mmol) 3-(1-Benzylpiperidin-4-yl)-7-methoxy-1,3,4,5-tetrahydro-1,3-benzodiazepin-2-on und 2.5 g Palladium auf Kohle (10%) in 300 mL Methanol wurde in einer 3 bar Wasserstoffatmosphäre bei 50°C bis zur vollständigen Umsetzung hydriert. Der Katalysator wurde abgesaugt und die Mutterlauge einrotiert. Der Rückstand wurde mit 150 mL DIPE verrieben, abgesaugt, mit 100 mL DIPE nachgewaschen und getrocknet.

| | |
|---|---|
| Ausbeute: | 13.2 g (81% d.Th.) |
| ESI-MS: | m/z = 276 (M+H)⁺ |
| Rₜ(HPLC): | 0.73 min (Methode L) |

### Intermediat 5:

### 3-Piperidin-4-yl-1,3-dihydroimidazo[4,5-c]chinolin-2-on

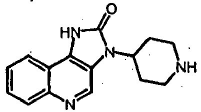

### Stufe1: 3-Bromchinolin-1-oxid

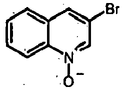

Zu einer auf 5°C gekühlten Lösung von 85.0 g (0.41 mol) 3-Bromchinolin in 100 mL DCM wurde eine Lösung aus 72%iger 3-Chlorperbenzoesäure (97.8 g (0.408 mol) gelöst in 1 L DCM, über Natriumsulfat getrocknet und abfiltriert) zugetropft. Dabei wurde darauf geachtet, däss die Temperatur der Reaktionsmischung nicht über 10°C anstieg. Nach Beendigung der Zugabe wurde 5 h gerührt, dann wurde nochmals eine Lösung von 72%iger 3-Chlorperbenzoesäure (25.0 g, 0.104 mol) gelöst in 200 mL DCM, über Natriumsulfat getrocknet und abfiltriert) zugetropft und über Nacht bei RT gerührt. Es wurde gesättigte wässrige Natriumcarbonat-Lösung zugegeben, die Phasen getrennt und die organische Phase über Natriumsulfat getrocknet. Die Lösung wurde über Aktivkohle filtriert und anschließend i.vac. eingeengt.

| | |
|---|---|
| Ausbeute: | 91 g (99% d.Th.) |
| MS: | m/z = 223/225 (M)⁺ |
| R_{f}: | 0.15 (Kieselgel, Laufmittel B) |

### Stufe2: 3-Brom-4-nitrochinolin-1-oxid

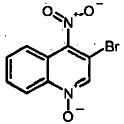

Eine Lösung aus 190 g (0.85 mol) 3-Bromchinolin-1-oxid in 500 mL konzentrierter Schwefelsäure wurde auf 90°C erhitzt. Dann wurden 120 g (1.19 mol) Kaliumnitrat in kleinen Portionen über einen Zeitraum von 100 min derart zugegeben, dass die Temperatur der Reaktion nicht über 95°C anstieg. Der Ansatz wurde 3 h bei 90°C gerührt; man ließ auf RT abkühlen und goss die Mischung auf Eis. Das ausgefallene Produkt wurde abfiltriert und der Filterkuchen mit Wasser gewaschen. Der Rückstand wurde in DCM gelöst und mit gesättigter, wässriger Natriumhydrogencarbonat-Lösung gewaschen, bis die Lösung alkalisch reagierte. Die Phasen wurden getrennt und die wässrige Phase nochmals mit DCM extrahiert. Die vereinigten organischen Phasen wurden über Natriumsulfat getrocknet und i.vac. eingeengt. Nach Zerkleinerung des Rückstandes und ausgiebiger Trocknung i.vac. wurde das Produkt erhalten.

| | |
|---|---|
| Ausbeute: | 104 g(46% d.Th.) |
| MS: | m/z = 268 / 270 (M)⁺ |
| R_{f}: | 0.77 (Kieselgel, EtOAc) |

### Stufe 3: (1-Benzylpiperidin-4-yl)-(4-nitro-1-oxychinolin-3-yl)-amin

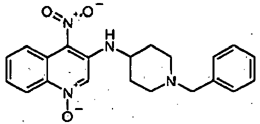

Zu 320 mL (1.54 mol) 4-Amino-1-benzylpiperidin wurden 104 g (0.387 mol) 3-Brom-4-nitrochinolin-1-oxid gegeben. Dann wurden 500 mL THF hinzugefügt und die Mischung bis zur vollständigen Lösung der Substanzen etwas erwärmt. Anschließend wurde 3 h bei 70°C gerührt und die Reaktionsmischung darauf i.vac. eingeengt. Der erhaltene Rückstand wurde in 2.5 L DCM gelöst und mit gesättigter wässriger Natriumhydrogencarbonat-Lösung gewaschen. Die wässrige Phase wurde nochmals mit 300 mL DCM extrahiert. Anschließend wurden die organischen Phasen vereinigt, über Natriumsulfat getrocknet und i.vac. eingeengt. Der Rückstand wurde in 250 mL Methanol gelöst. Das als Feststoff ausgefallene Produkt wurde abgesaugt und i.vac. getrocknet.

| | |
|---|---|
| Ausbeute: | 104 g (71% d.Th.) |
| ESI-MS: | m/z = 379 (M+H)⁺ |
| R_{f}: | 0.75 (Kieselgel, EtOAc) |

### Stufe 4: N³-(1-Benzylpiperidin-4-yl)chinolin-3,4-diamin

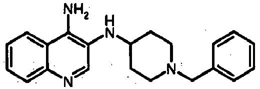

Zu einer Lösung von 76.0 g (0.20 mol) (1-Benzylpiperidin-4-yl)-(4-nitro-1-oxychinolin-3-yl)-amin in 1.0 L THF wurden 12.0 g Rhodiumkohle (5%, wasserfeucht) gegeben. Die Reaktion wurde 4.5 h unter einer Wasserstoffatmosphäre (50 psi) bei RT geschüttelt. Der Katalysator wurde abfiltriert und das Lösungsmittel i.vac. entfernt. Wegen seiner Oxidationsempfindlichkeit wurde das Rohprodukt sogleich für die nächste Stufe eingesetzt.

| | |
|---|---|
| Ausbeute: | 66.0 g (99% d.Th.) |
| R_{f}: | 0.30 (Kieselgel, Laufmittel A) |

### Stufe 5: 3-(1-Benzylpiperidin-4-yl)-1,3-dihydroimidazo[4.5-c]chinolin-2-on

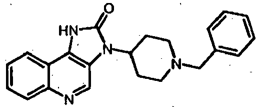

Zu einer Lösung von 9.0 g (27.1 mmol) *N*³-(1-Benzylpiperidin-4-yl)-chinolin-3,4-diamin in 100 mL DMF wurden 22.6 g (139 mmol) 1,1'-Carbonyldiimidazol gegeben. Die Mischung wurde auf 100°C erhitzt und 1.5 h bei dieser Temperatur gerührt. Nach Abkühlen der Reaktionsmischung wurde diese auf 300 mL Wasser gegossen. Der ausgefallene Feststoff wurde abfiltriert, mit Wasser gewaschen und bei 30°C i.vac. getrocknet. Der Rückstand wurde mit Diethylether verrieben, abgesaugt und das Festprodukt i.vac. getrocknet.

| | |
|---|---|
| Ausbeute: | 7.42 g (77% d.Th.) |
| ESI-MS: | m/z = 359 (M+H)⁺ |
| Rₜ(HPLC): | 1.6 min (Methode E) |

### Stufe 6: 3-Piperidin-4-yl-1,3-dihydroimidazo[4,5-c]chinolin-2-on

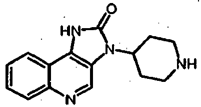

Eine Mischung aus 44.0 g (0.123 mol) 3-(1-Benzylpiperidin-4-yl)-1,3-dihydroimidazo-[4,5-c]chinolin-2-on und 10.0 g Palladium (Pd/C 10%) in 500 mL Methanol wurde 16 h bei 50 C in einer Wasserstoff-Atmosphäre von 50 psi hydriert. Nach Filtration des Reaktionsgemisches wurde das Lösungsmittel im Vakuum entfernt. Durch Zugabe von Isopropanol fiel das Produkt als Niederschlag aus. Dieser wurde abfiltriert und getrocknet.

| | |
|---|---|
| Ausbeute: | 31.2 g (95% d.Th.) |
| ESI-MS: | m/z = 269 (M+H)⁺ |
| R_{f}: | 0.20 (Kieselgel, Laufmittel A) |

### Intermediat 6:

### 6-Chlorpyrimidin-4-carbonsäurechlorid

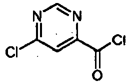

### Stufe 1: 6-Hydroxypyrimidin-4-carbonsäure

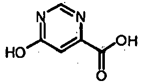

Zu 24.1 g (0.6 mol,) NaOH in 3.6 L Wasser wurden 63.5 g (0.29 mol) Natriumdiethyloxalacetat und 30.2 g (0.29 mol) Formamidin-acetat zugegeben. Der Ansatz wurde über Nacht bei RT gerührt. Anschließend fügte man Aktivkohle hinzu und kochte den Ansatz für 1h unter Rückfluss. Es wurde heiß abfiltriert und nach dem Erkalten mit wässriger Salzsäure angesäuert. Die Lösung wurde bis zur Trockne einrotiert. Der Rückstand enthielt das gewünschte Produkt und wurde ohne weitere Aufreinigung in die nächste Stufe eingesetzt.

| | |
|---|---|
| Ausbeute: | 83.0 g |

### Stufe 2: 6-Chlorpyrimidin-4-carbonsäurechlorid

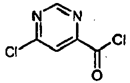

50.0 g (0.35 mol) 6-Hydroxypyrimidin-4-carbonsäure wurde vorgelegt und 500 mL Phosphoroxychlorid zugegeben. Anschließend wurde unter Rühren 150 g (0.720 mol) Phosphorpentachlorid portionsweise zugegeben. Der Reaktionsansatz wurde 5 h unter Rückfluss gekocht. Das Phosphoroxychlorid wurde abdestilliert und der Rückstand durch Vakuumdestillation über eine Kolonne aufgereinigt.

| | |
|---|---|
| Ausbeute: | 51.9 g (83% d.Th.) |
| MS: | m/z = 176 / 178 / 180 (M)⁺ |

### Intermediat 7:

### 6-Chlorpyrimidin-4-carbonsäureethylester

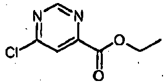

1.0 g (5.65 mmol) 6-Chlorpyrimidin-4-carbonsäurechlorid und 0.4 mL (6.94 mmol) Ethanol wurden in 30 mL Dichlormethan zusammengegeben und über Nacht bei RT gerührt. Das Lösungsmittel wurde i.vac. entfernt.

| | |
|---|---|
| Ausbeute: | 1.0 g (95% d.Th.) |
| ESI-MS: | m/z = 187 / 189 (M+H)⁺ |
| R_{f}: | 0.85 (Kieselgel, EtOAc) |

### Intermediat 8:

### 3-[4-(2-Oxo-1,2,4,5-tetrahydro-1,3-benzodiazepin-3-yl)-piperidin-1-yl]benzoesäureethylester

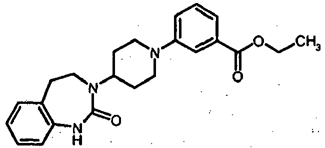

Unter einer Stickstoff-Atmosphäre wurden 1.06 g (4.32 mmol) 3-Piperidin-4-yl-1,3,4,5-tetrahydro-1,3-benzodiazepin-2-on, 2.15 g (6.60 mmol) Cäsiumcarbonat, 100 mg (0.45 mmol) Palladium-(III)-acetat und 280 mg (0.45 mmol) BINAP in 40 mL Xylol für 10 min bei RT gerührt. Es wurden 850 µL (5.20 mmol) 3-Brombenzoesäure-ethylester zugegeben und über Nacht bei 120°C gerührt. Anschließend wurde der unlösliche Feststoff abgesaugt und das Filtrat i.vac. eingeengt. Der Rückstand wurde mittels Flash-Chromatographie aufgereinigt. Die vereinigten Produktfraktionen wurden i.vac. eingeengt. Der Rückstand wurde mit Diisopropylether verrührt und abgesaugt. Der Feststoff wurde bei 50°C im ULTS getrocknet.

| | |
|---|---|
| Ausbeute: | 650 mg (38% d.Th.) |
| ESI-MS: | m/z = 394 (M+H)⁺ |
| R_{f}: | 0.81 (Kieselgel, Laufmittel A) |

### Intermediat 9:

### 3-[4-(2-Oxo-1,2,4,5-tetrahydro-1,3-benzodiazepin-3-yl]-piperidin-1-yl]-benzoesäure

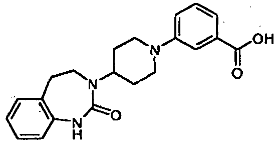

650 mg (1.65 mmol) 3-[4-(2-Oxo-1,2,4,5-tetrahydro-1,3-benzodiazepin-3-yl)-piperidin-1-yl]-benzoesäureethylester, 10 mL THF, 2 mL Wasser und 2 mL (8.00 mmol) einer wässrigen 4M Natriumhydroxid-Lösung wurden bis zur vollständigen Umsetzung bei RT gerührt. Anschließend wurde das THF i.vac. entfernt. Der wässrige Rückstand wurde mit einer 4M Salzsäure-Lösung angesäuert. Nach mehrstündigem Rühren bei RT wurde der Niederschlag abgesaugt und getrocknet.

| | |
|---|---|
| Ausbeute: | 540 mg (90% d.Th.) |
| ESI-MS: | m/z = 366 (M+H)⁺ |
| R_{f}: | 0.20 (Kieselgel, Laufmittel A) |

### Intermediat 10:

### 6-[4-(2-Oxo-1,2,4,5-tetrahydro-1,3-benzodiazepin-3-yl)-piperidin-1-yl]-pyrimidin-4-carbonsäureethylester

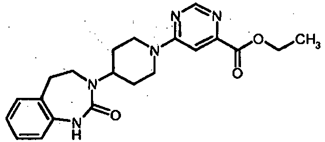

Zu 500 mg (2.68 mmol) 6-Chlorpyrimidin-4-carbonsäureethylester in 10 mL DMF wurden 700 mg (2.85 mmol) 3-Piperidin-4-yl-1,3,4,5-tetrahydro-1,3-benzodiazepin-2-on und 470 µL (2.73 mmol) DIPEA hinzugefügt und der Reaktionsansatz 1 h bei RT gerührt. Das Reaktionsgemisch wurde mit Wasser verdünnt und 30 min gerührt. Der Niederschlag wurde abgesaugt, mit Wasser gewaschen und im ULTS bei 50°C getrocknet.

| | |
|---|---|
| Ausbeute: | 620 mg (59% d.Th.) |
| ESI-MS: | m/z = 396 (M+H)⁺ |
| R_{f}: | 0.48 (Kieselgel, Laufmittel A) |

### Intermediat 11:

### 6-[4-(2-Oxo-1,2,4,5-tetrahydro-1,3-benzodiazepin-3-yl)-piperidin-1-yl]-pyrimidin-4-carbonsäure

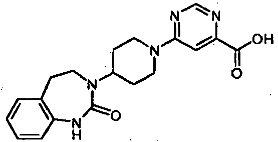

590 mg (1.49 mmol) 6-[4-(2-Oxo-1,2,4,5-tetrahydro-1,3-benzodiazepin-3-yl)-piperidin-1-yl]-pyrimidin-4-carbonsäureethylester, 20 mL Ethanol, 1 mL Wasser und 1 mL (4.0 mmol) einer 4M NaOH-Lösung wurden 2 h bei RT gerührt. Der Reaktionsansatz wurde mit Wasser verdünnt bis sich der Niederschlag aufgelöst hatte. Es wurden 1 mL einer 4M Salzsäure-Lösung hinzugefügt und das Ethanol i.vac. entfernt. Der Reaktionsansatz wurde 30 min bei RT gerührt, der Niederschlag abgesaugt und bei 50°C im ULTS getrocknet.

| | |
|---|---|
| Ausbeute: | 500 mg (91% d.Th.) |
| MS: | m/z = 367 (M)⁺ |
| R_{f}: | 0.13 (Kieselgel, Laufmittel A) |

### Intermediat 12:

### 6-[4-(7-Methoxy-2-oxo-1,2,4,5-tetrahydro-1,3-benzodiazepin-3-yl)-piperidin-1-yl]-pyrimidin-4-carbonsäureethylester

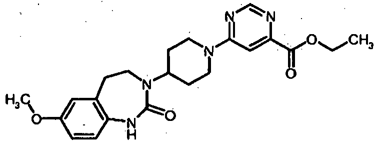

Zu 1.90 g (10.2 mmol) 6-Chlorpyrimidin-4-carbonsäureethylester in 60 mL DMF wurden 2.80 g (10.2 mmol) 7-Methoxy-3-piperidin-4-yl-1,3,4,5-tetrahydro-1,3-benzodiazepin-2-on und 6.00 mL (34.9 mmol) DIPEA hinzugefügt und der Reaktionsansatz wurde 3 h bei RT gerührt. Nach dem Entfernen des Lösungsmittels i.vac. wurde der Rückstand mit 70 mL Wasser versetzt und 10 min gerührt. Man fügte 5 mL Ethylacetat hinzu. Nach kräftigem Rühren wurde der Feststoff abgesaugt und im ULTS getrocknet.

| | |
|---|---|
| Ausbeute: | 2.55 g (59% d. Th.) |
| ESI-MS: | m/z = 426 (M+H)⁺ |
| R_{f}: | 0.63 (Kieselgel, Laufmittel A) |

### Intermediat 13:

### 6-[4-(7-Methoxy-2-oxo-1,2,4,5-tetrahydro-1,3-benzodiazepin-3-yl)-piperidin-1-yl]-pyrimidin-4-carbonsäure

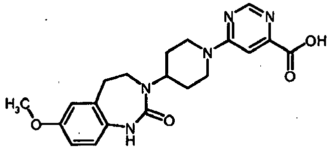

2.55 g (5.99 mmol) 6-[4-(7-Methoxy-2-oxo-1,2,4,5-tetrahydro-1,3-benzodiazepin-3-yl)-piperidin-1-yl]-pyrimidin-4-carbonsäureethylester, 50 mL THF und5 mL (20.0 mmol) einer 4M NaOH-Lösung wurden über Nacht bei RT gerührt: Nach dem Entfernen von THF i.vac. wurde der wässrige Rückstand mit 5 mL einer 4M Salzsäure-Lösung versetzt. Die überstehende Lösung wurde abdekantiert, der ölige Rückstand abermals mit 50 mL Wasser versetzt und über Nacht bei RT gerührt. Der Niederschlag wurde abgesaugt und bei 50°C im ULTS getrocknet.

| | |
|---|---|
| Ausbeute: | 2.05 g (86% d.Th.) |
| MS: | m/z = 397 (M)⁺ |
| R_{f}: | 0.23 (Kieselgel, Laufmittel A) |

### Intermediat 14:

### 6-[4-(2-Oxo-2,3-dihydro-imidazo[4,5-b]pyridin-1-yl)-piperidin-1-yl]-pyrimidin-4-carbonsäure ethylester

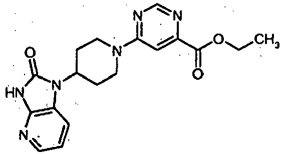

Zu 3.40 g (18.2 mmol) 6-Chlorpyrimidin-4-carbonsäureethylester in 80 mL DMF wurden 5.40 g (18.6 mmol) 1-Piperidin-4-yl-1,3-dihydro-imidazo[4,5-*b*]pyridin-2-on Dihydrochlorid und 11 mL (63.93 mmol) DIPEA hinzugefügt. Nach 3 h Rühren bei RT wurde das Lösungsmittel i.vac. entfernt. Der Rückstand wurde mit 70 mL Wasser versetzt und 10 min nachgerührt. Es wurden 5 mL Ethylacetat zugegeben und kräftig gerührt. Der ausgefallene Feststoff wurde abgesaugt und bei 40°C im Umluftschrank getrocknet.

| | |
|---|---|
| Ausbeute: | 5.50 g (82% d.Th.) |
| ESI-MS: | m/z = 369 (M+H)⁺ |
| R_{f}: | 0.48 (Kieselgel; Laufmittel A) |

### Intermediat 15:

### 6-[4-(2-Oxo-2,3-dihydroimidazo[4.5-b]pyridin-1-yl)-piperidin-1-yl]-pyrimidin-4-carbonsäure

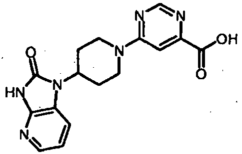

Zu 5.50 g (14.9 mmol) 6-[4-(2-Oxo-2,3-dihydro-imidazo[4,5-*b*]pyridin-1-yl)-piperidin-1-yl]-pyrimidin-4-carbonsäureethylester, 25 mL Wasser in 150 mL THF wurden 9.0 mL (36.0 mmol) einer 4M NaOH-Lösung hinzugefügt. Nach 3 h Rühren bei RT wurde das THF i.vac. entfernt. Der wässrige Rückstand wurde mit 9 mL einer 4M Salzsäure-Lösung versetzt. Nach 3 h Rühren bei RT wurde der Niederschlag abgesaugt und im ULTS bei 60 C getrocknet.

| | |
|---|---|
| Ausbeute: | 4.5 g (89% d.Th.) |
| ESI-MS: | m/z = 341 (M+H)⁺ |
| R_{f}: | 0.07 (Kieselgel; Laufmittel A) |

### Intermediat 16:

### (2-Chlorpyridin-4-yl)-(2,3-dihydroindol-1-yl)-methanon

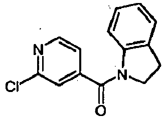

Zu 500 mg (3.17 mmol) 2-Chlorisonicotinsäure, 600 µL (3.49 mmol) DIPEA und 1.10 g (3.43 mmol) TBTU in 20 mL THF wurden 400 µL (3.53 mmol) 2,3-Dihydro-1*H*-indol zugegeben. Der Reaktionsansatz wurde über Nacht gerührt, mit Ethylacetat verdünnt und mit 15%-iger Kaliumcarbonat-Lösung (1 x), Wasser (1 x) und 1M Salzsäure (1 x) gewaschen. Die organische Phase wurde über Magnesiumsulfat getrocknet, filtriert und i.vac. eingeengt. Der Rückstand wurde mit Diisopropylether verrieben und abgesaugt. Der Feststoff wurde im Umluftschrank bei 40°C getrocknet.

| | |
|---|---|
| Ausbeute: | 700 mg (85% d.Th.) |
| ESI-MS: | 259/261 (M+H)⁺ |
| R_{f}: | 0.38 (Kieselgel, Laufmittel B) |

### Intermediat 17:

### (2-Chlorpyridin-4-yl)-(5-fluor-2,3-dihydroindol-1-yl)-methanon

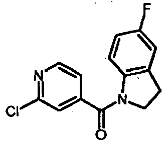

Zu 198 mg (1.26 mmol) 2-Chlorisonicotinsäure, 351 µL (2.50 mmol) Triethylamin und 434 mg (1.35 mmol) TBTU in 3 mL DMF wurden 174 mg (1.27 mmol) 5-Fluor-2,3-dihydro-1*H*-indol zugegeben. Der Reaktionsansatz wurde über Nacht bei RT gerührt. Die Aufreinigung erfolgte mittels präparativer HPLC. Die Produktfraktionen wurden vereinigt und i.vac. eingeengt.

| | |
|---|---|
| Ausbeute: | 230 mg (66% d.Th.) |
| ESI-MS: | 277/279 (M+H)⁺ |
| Rₜ (HPLC-MS): | 4.0 min (Methode E) |

### Intermediat 18:

### (4-Chlorpyridin-2-yl)-(2,3-dihydroindol-1-yl)-methanon

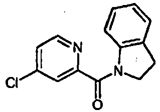

Zu 1.0 g (6.35 mmol) 4-Chlorpyridin-2-carbonsäure, 1.0 mL (7.12 mmol) Triethylamin und 2.20 g (6.85 mmol) TBTU in 100 mL THF wurden 800 µL (7.07 mmol) 2,3-Dihydro-1H-indol zugegeben. Der Reaktionsansatz wurde 3 h bei RT gerührt, mit Ethylacetat (100 mL) verdünnt und mit 15%-iger Kaliumcarbonat-Lösung (2 x 50 mL), gesättigter Natriumchlorid-Lösung (1 x 50 mL) und 1M Salzsäure (2 x 30 mL) gewaschen. Die organische Phase wurde über Magnesiumsulfat getrocknet, filtriert und i.vac. eingeengt.

| | |
|---|---|
| Ausbeute: | 850 mg (52% d.Th.) |
| ESI-MS: | 259/261 (M+H)⁺ |
| R_{f}: | 0.88 (Kieselgel, EtOAc) |

### Intermediat 19:

### (4-Chlorpyridin-2-yl)-(5-fluor-2,3-dihydroindol-1-yl)-methanon

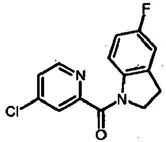

Zu 200.0 mg (1.27 mmol) 4-Chlorpyridin-2-carbonsäure, 351 µL (2.50 mmol) Triethylamin und 434.0 mg (1.35 mmol) TBTU in 3 mL DMF wurden 174 mg (1.27 mmol) 5-Fluor-2,3-dihydro-1*H*-indol zugegeben. Der Reaktionsansatz wurde über Nacht bei RT gerührt. Die Aufreinigung erfolgte mittels präparativer HPLC. Die Produktfraktionen wurden vereinigt und i.vac. eingeengt.

| | |
|---|---|
| Ausbeute: | 300 mg (85% d.Th.) |
| ESI-MS: | 277/279 (M+H)⁺ |
| Rₜ (HPLC-MS): | 4.1 min (Methode E) |

### Intermediat 20:

### (6-Chlorpyrimidin-4-yl)-(octahydroindol-1-yl)-methanon

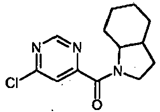

0.517 g (2.92 mmol) 6-Chlorpyrimidin-4-carbonsäurechlorid in 10 mL Dichlormethan wurden mit 1.07 mL (6.00 mmol) DIPEA versetzt. Es wurde 0.73 g (2.92 mmol) Octahydroindol in 10 mL DCM langsam zugetropft. Nach 2 h Rühren bei RT wurde der Ansatz mit Dichlormethan verdünnt und mit Wasser ausgeschüttelt. Die organische Phase wurde abgetrennt, über Natriumsulfat getrocknet, filtriert, i.vac. eingeengt und getrocknet. Das so erhaltene Produkt wurde ohne weitere Aufreinigung umgesetzt.

| | |
|---|---|
| Ausbeute: | 800 mg (quant.) |
| ESI-MS: | 266/268 (M+H)⁺ |
| Rₜ (HPLC-MS): | 3.62 min (Methode E) |

### Intermediat 21:

### (6-Chlorpyrimidin-4-yl)-(2,3-dihydroindol-1-yl)-methanon

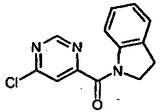

500 mg (2.83 mmol) 6-Chlorpyrimidin-4-carbonsäurechlorid in 20 mL Dichlormethan wurden mit einem Eis/Ethanol-Bad gekühlt und mit 0.300 mL (2.68 mmol) 2,3-Dihydro-1*H*-indol versetzt. Es wurden 2.70 mL (2.70 mmol) einer 1 M Natronlauge zugetropft. Der Reaktionsansatz wurde 2 h unter Kühlung und 1 h bei RT gerührt. Anschließend gab man 50 mL einer wässrigen gesättigten Natriumhydrogencarbonat-Lösung hinzu. Nach 10 min Rühren würde die organische Phase abgetrennt und mit Wasser (1 x 30 mL) und mit 1 M Salzsäure (1 x 50 mL) extrahiert. Die organische Phase wurde über Magnesiumsulfat getrocknet, filtriert und i.vac. eingeengt.

| | |
|---|---|
| Ausbeute: | 570 mg (78% d.Th.) |
| ESI-MS: | m/z = 260/262 (M+H)⁺ |
| R_{f}: | 0.59 (Kieselgel, Laufmittel B) |

### Allgemeines Verfahren zur Umsetzung von 6-Chlorpyrimidin-4-carbonsäurechlorid mit stickstoffhaltigen Heterocyclen:

1.50 g (8.48 mmol) 6-Chlorpyrimidin-4-carbonsäurechlorid in 50 mL Dichlormethan wurden mit einem Eis/Ethanol-Bad gekühlt und mit der jeweils angegebenen Menge eines stickstoffhaltigen Heterocyclus versetzt. Es wurden 7.90 mL (7.90 mmol) einer 1 M Natronlauge zugetropft. Der Reaktionsansatz wurde 2 h unter Kühlung und 1 h bei RT gerührt. Anschließend gab man 50 mL einer gesättigten Natriumhydrogencarbonat-Lösung hinzu. Nach 10 min Rühren wurde die organische Phase abgetrennt und mit Wasser (1 x 30 mL) und anschließend mit 1 M Salzsäure (1 x 50 mL) extrahiert. Die organische Phase wurde über Magnesiumsulfat getrocknet, filtriert und i.vac. eingeengt. Das Produkt wurde ohne weitere Aufreinigung umgesetzt.

| Intermediat | Struktur | N-Heterocyclus [Menge N-Heterocyclus] Ausbeute | Analytische Daten |
|---|---|---|---|
| 22 | 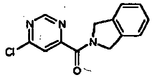 | 2,3-Dihydro-1*H-*isoindol | ESI-MS: m/z = 260/262 (M+H)⁺ |
| | (6-Chlorpyrimidin-4-yl)-(1,3-dihydroisoindol-2-yl)-methanon | 0.89 mL (7.84 mmol) | R_{f} = 0.57 |
| | | 1.8 g (82% d.Th.) | Laufmittel B |
| 23 | 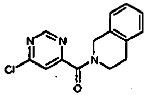 | 1,2,3,4-Tetrahydroisochinolin | ESI-MS: m/z = 274/276 (M+H)⁺ |
| | (6-Chlorpyrimidin-4-yl)-(3,4-dihydro-1*H*-isochinolin-2-yl)-methanon | 0.978 mL (7.81 mmol) | |
| | | 1.7g (73% d.Th.) | R_{f} = 0.46 Laufmittel B |
| 24 | 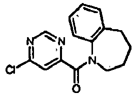 | 2,3,4,5-Tetrahydro-1*H*-1-benzazepin | ESI-MS: m/z = 288/290 (M+H)⁺ |
| | (6-Chlorpyrimidin-4-yl)-(3,4-dihydro-2H-chinolin-1-yl)-methanon | 1.15g (7.81 mmol) | |
| | | 2.0 g (82% d.Th.) | R_{f}=0.61 Laufmittel A |

### Intermediat 25:

### (5-Chlor-2,3-dihydroindol-1-yl)-(6-chlorpyimidin-4-yl)-methanon

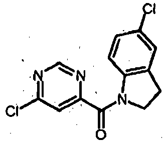

1.50 g (8.48 mmol) 6-Chlorpyrimidin-4-carbonsäurechlorid in 50 mL Dichlormethan wurden mit einem Eis/Ethanol-Bad gekühlt und mit 1.20 g (7.81 mmol) 5-Chlor-2,3-dihydro-1H-indol versetzt. Es wurden 7.90 mL (7.90 mmol) einer 1 M Natronlauge zugetropft. Der Reaktionsansatz wurde 2 h unter Kühlung und 1 h bei RT gerührt. Anschließend gab man 50 mol einer wässrigen gesättigten Natriumhydrogencarbonat-Lösung hinzu. Nach 10 min Rühren wurde die organische Phase abgetrennt und mit Wasser (1 x 30 mL) und mit 1 M Salzsäure (1 x 50 mL) extrahiert. Die organische Phase wurde über Magnesiumsulfat getrocknet, filtriert und i.vac. eingeengt.

| | |
|---|---|
| Ausbeute: | 2.00 g (80% d.Th. ) |
| ESI-MS: | m/z = 294/296/298 (M+H)⁺ |
| R_{f}: | 0.65 (Kieselgel, Laufmittel B) |

### Intermediat 26:

### (5-Brom-2,3-dihydroindol-1-yl)-(6-chlorpyrimidin-4-yl)-methanon

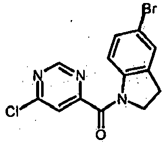

2.0 g (11.3 mmol) 6-Chlorpyrimidin-4-carbonsäurechlorid in 50 mL Dichlormethan wurden mit einem Eis/Ethanol-Bad gekühlt und mit 2.2 g (10.9 mmol) 5-Brom-2,3-dihydro-1*H*-indol versetzt. Es wurden 10.9 mL (10.9 mmol) einer 1M Natronlauge zugetropft. Der Reaktionsansatz wurde 2 h unter Kühlung gerührt. Nachdem sich der Ansatz auf RT erwärmt hatte, gab man anschließend 50 mL einer wässrigen gesättigten Natriumhydrogencarbonat-Lösung hinzu. Die organische Phase wurde abgetrennt und mit Wasser (1x) und mit 1 M Salzsäure (1x) extrahiert. Die organische Phase wurde über Magnesiumsulfat getrocknet, filtriert und i.vac. eingeengt. Der Rückstand wurde mit Diisopropylether verrieben, abgesaugt und bei 50°C im ULTS getrocknet.

| | |
|---|---|
| Ausbeute: | 620 mg (16% d.Th.) |
| MS: | m/z = 337/339/341 (M)⁺ |
| R_{f}: | 0.89 (Kieselgel, EtOAc) |

### Intermediat 27:

### (5-Fluor-2,3-dihydro-1H-indol-3-yl)-essigsäuremethylester

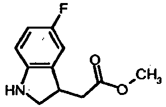

### Stufe 1: (5-Fluor-1H-indol-3-yl)-essigsäuremethylester

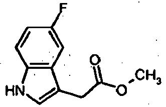

1.0 g (5.2 mmol) 5-Fluorindol-3-essigsäure wurde in 60 mL methanolischer HCl bei RT für 2h gerührt. Das Lösungsmittel wurde am Rotationsverdampfer entfernt. Das Produkt wurde ohne weitere Aufreinigung umgesetzt.

| | |
|---|---|
| Ausbeute: | 1.0 g (93% d.Th.) |
| ESI-MS: | m/z = 208 (M+H)⁺ |
| Rₜ (HPLC): | 3.18 min (Methode E) |

### Stufe 2: (5-Fluor-2,3-dihydro-1H-indol-3-yl)-essigsäuremethylester

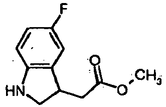

Unter einer Stickstoffatmosphäre wurde zu 1.0 g (4.83 mmol) (5-Fluor-1*H*-indol-3-yl)-essigsäuremethylester in 12.5 g Essigsäure unter leichter Kühlung 910 mg (14.5 mmol) Natriumcyanoborhydrid portionsweise zugegeben. Nach 2 h Rühren bei RT wurde abermals unter leichter Kühlung 910 mg (14.5 mmol) Natriumcyanoborhydrid portionsweise zugegeben. Nach 3 h Rühren bei RT wurde das Lösungsmittel am Roationsverdampfer eingeengt. Der Rückstand wurde in 4M Salzsäure aufgenommen und 30 min nachgerührt. Anschließend wurde die Reaktionslösung mit festem Kaliumcarbonat alkalisch gestellt und mit Dichlormethan extrahiert (3 x). Die vereinigten organischen Phasen wurden über Natriumsulfat getrocknet, filtriert und am Rotationsverdampfer eingeengt. Das so erhaltene Produkt wurde direkt umgesetzt.

| | |
|---|---|
| Ausbeute: | 300 mg (30% d.Th.) |
| ESI-MS: | m/z = 210 (M+H)⁺ |

### Intermediat 28:

### 5-Fluor-3,3-dimethyl-2,3-dihydro-1H-indol

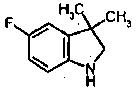

### Stufe 1: 1-Aceyl-5-fluor-1,3-dihydro-indol-2-on

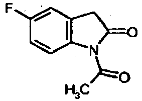

Bei 170°C wurden 3.0 g (20 mmol) 5-Fluorindolinon in 10 mL (98 mmol) Acetanhydrid für 3 h gerührt. Nach dem Abkühlen auf RT wurde der Ansatz auf 200 mL Eiswasser gegossen, die ausgefallene Substanz wurde abgesaugt und mit 100 mL Wasser nachgewaschen. Der Feststoff wurde aus 100 mL Wasser und 50 mL Ethanol in der Siedehitze umkristallisiert. Das ausgefallene Produkt wurde abgesaugt, mit 30 mL Wasser nachgewaschen und im ULTS getrocknet.

| | |
|---|---|
| Ausbeute: | 2.4 g (63% d.Th.) |
| ESI-MS: | m/z = 192 (M+H)⁺ |
| Rₜ (HPLC): | 1.2 min (Methode C) |

### Stufe 2: 1-Acetyl-5-fluor-3,3-dimethyl-1,3-dihydro-indol-2-on

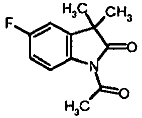

Bei 0°C bis 5°C wurde unter einer Argonatmosphäre zu 2.40 g (12.4 mmol) 1-Acetyl-5-fluor-1,3-dihydroindol-2-on in 30 mL DMF portionsweise 1.14 g (26.0 mmol) 55%-iges Natriumhydrid in Öl zugegeben und 1 h nachgerührt. Anschließend wurden 1.91 mL (31.0 mmol) Methyliodid zugetropft und über Nacht bei RT gerührt. Der Reaktionsansatz wurde auf 200 mL Wasser gegossen und die ausgefallene Substanz abgesaugt. Der Feststoff wurde mit 50 mL Wasser nachgewaschen und im ULTS getrocknet.

| | |
|---|---|
| Ausbeute: | 2.1 g (76% d.Th.) |
| ESI-MS: | m/z = 222 (M+H)⁺ |
| Rₜ (HPLC): | 1.48 min (Methode C) |

### Stufe 3: 5-Fluor-3.3-dimethyl-1,3-dihydroindol-2-on

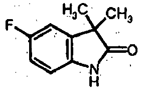

2.10 g (9.49 mmol) 1-Acetyl-5-fluor-3,3-dimethyl-1,3-dihydro-indol-2-on in 20 mL Isopropanol wurden mit 50 mL einer wässrigen 6N Salzsäure-Lösung für 1h unter Rückfluß gekocht. Nach dem Abkühlen wurde das Isopropanol i.vac. entfernt. Der Rückstand wurde mit 100 mL Wasser verdünnt und mit Eis gekühlt. Die ausgefallene Substanz wurde abgesaugt und mit 30 mL Wasser nachgewaschen. Der Feststoff wurde im ULTS getrocknet.

| | |
|---|---|
| Ausbeute: | 1.40 g (82% d.Th.) |
| ESI-MS: | m/z = 180 (M+H)⁺ |
| Rₜ (HPLC): | 1.14 min (Methode C) |

### Stufe 4: 5-Fluor-3.3-dimethyl-2,3-dihydro-1H-indol

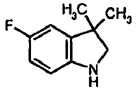

Unter einer Argonatmosphäre wurde zu 1.40 g (7.81 mmol) 5-Fluor-3,3-dimethyl-1,3-dihydroindol-2-on in 50 mL THF eine Lösung aus 9.30 mL (9.30 mmol) einer 1 M Lösung Lithiumaluminiumhydrid in THF und 10 mL THF langsam zugetropft. Anschließend wurde der Reaktionsansatz für 1 h auf 70°C erwärmt. Nach dem Erkalten wurden 2 mL Wasser hinzugefügt. Die Lösung wurde über Natriumsulfat getrocknet und abfiltriert. Das Lösungsmittel wurde i.vac. entfernt.

| | |
|---|---|
| Ausbeute: | 1.30 g (quant) |
| ESI-MS: | m/z = 166 (M+H)⁺ |
| Rₜ (HPLC): | 0.75 min (Methode C) |

### Intermediat 29:

### 3,3-Dimethyl-2,3-dihydro-1H-indol

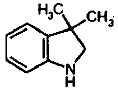

### Stufe 1: 1-Acetyl-1,3-dihydroindol-2-on

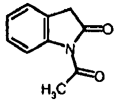

Die Verbindung wurde wie in der Patentanmeldung US 2003/0069299 synthetisiert.

### Stufe 2: 1-Acetyl-3,3-dimethyl-1,3-dihydroindol-2-on

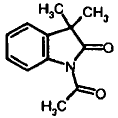

Bei 0°C bis 5°C wurde unter einer Argon-Atmosphäre zu 5.30 g (30.0 mmol) 1-Acetyl-1,3-dihydroindol-2-on in 50 mL DMF portionsweise 2.75 g (63.0 mmol) 55%-iges Natriumhydrid in Öl zugegeben und 1 h nachgerührt. Anschließend wurden 4.70 mL (75 mmol) Methyliodid zugetropft und über Nacht bei RT gerührt. Der Reaktionsansatz wurde auf Wasser gegossen und mehrmals mit *tert*-Butylmethylether extrahiert. Die vereinigten organischen Phasen wurden getrocknet und i.vac. eingeengt. Der Rückstand wurde über eine Kieselgel-Säule aufgereinigt. Die Produktfraktionen wurden vereinigt und bis zur Trockne ein rotiert.

| | |
|---|---|
| Ausbeute: | 3.60 g (59% d.Th.) |
| ESI-MS: | m/z = 204 (M+H)⁺ |
| R_{f}: | 0.9 ((Kieselgel, Petrolether/Dichlormethan/Ethylacetat = 5/4/1) |

### Stufe 3: 3,3-Dimethyl-1,3-dihydroindol-2-on

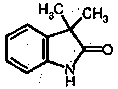

3.50 g (17.2 mmol) 1-Acetyl-3,3-dimethyl-1,3-dihydro-indol-2-on wurden in 50 mL einer 6N Salzsäure-Lösung für 1 h unter Rückfluss gekocht. Nach dem Abkühlen wurde der Reaktionsansatz zwischen *tert*-Butylmethylether und Wasser verteilt. Die organische Phase wurde mit Wasser gewaschen, getrocknet und eingedampft. Der Rückstand wurde aus PE kristallisiert. Der Feststoff wurde abgesaugt und im ULTS bei 80°C getrocknet.

| | |
|---|---|
| Ausbeute: | 2.40 g (87% d.Th.) |
| ESI-MS: | m/z = 162 (M+H)⁺ |
| R_{f} : | 0.3 (Kieselgel, Petrolether/Dichlormethan/Ethylacetat = 5/4/1) |

### Stufe 4: 3,3-Dimethyl-2,3-dihydro-1H-indol

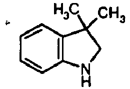

Unter einer Stickstoff-Atmosphäre wurde zu 1.00 g (6.20 mmol) 3,3-Dimethyl-1,3-dihydroindol-2-on in 50 mL THF eine Lösung aus 6.20 mL (6.20 mmol) einer 1 M Lösung Lithiumaluminiumhydrid in THF und 10 mL THF langsam zugetropft. Anschließend wurde der Reaktionsansatz für 1 h auf 60°C erwärmt. Nach dem Abkühlen auf 0°C wurden 3 mL Eiswasser langsam zugetropft. Es wurden 20 g Natriumsulfat zugegeben und abgesaugt. Die Lösung wird i.vac. eingeengt.

| | |
|---|---|
| Ausbeute: | 0.80 g (88% d.Th.) |
| ESI-MS: | m/z = 148 (M+H)⁺ |
| Rₜ (HPLC): | 0.7 min (Methode C) |

### Intermediat 30:

### Spiro[cyclobutan-1,3'-indolin]

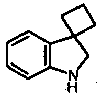

### Stufe 1: Cyclobutancarbonsäure N'-phenylhydrazid

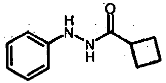

Zu 3.00 mL (30.2 mmol) Phenylhydrazin und 4.75 mL (60.0 mmol) Pyridin in 30 mL DMF wurden bei RT 3.54 mL (31.0 mmol) Cyclobutancarbonsäurechlorid zugetropft. Der Ansatz wurde 1 h bei RT nachgerührt und auf 200 mL einer 1 M Salzsäure-Lösung gegossen. Der ausgefallene Feststoff wurde abgesaugt, mit 50 mL Wasser gewaschen und i.vac. getrocknet. Das Produkt wurde mit 50 mL Ether ausgerührt und abgesaugt. Der Feststoff wurde mit 20 mL Ether gewaschen und an der Luft getrocknet.

| | |
|---|---|
| Ausbeute: | 3.00 g (52% d.Th.) |
| ESI-MS: | m/z = 191 (M+H)⁺ |
| Rₜ (HPLC-MS): | 1.05 min (Methode C) |

### Stufe 2: Spiro[cyclobutan-1,3'-indolin]-2'-on

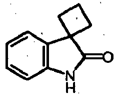

Unter einer Stickstoff-Atmosphäre wurden 1.50 g (7.89 mmol) Cyclobutancarbonsäure *N*'-phenylhydrazid und 530 mg (12.6 mmol) Calciumhydrid gut vermischt und auf 230°C erwärmt. Es wurde 30 min bei 230°C nachgerührt und anschließend wieder auf RT abgekühlt. Der Reaktionsansatz wurde vorsichtig mit einer Lösung aus 7 mL Wasser und 16 mL Methanol versetzt. Es wurde 1 h nachgerührt bis kein Wasserstoff mehr freigesetzt wurde. Anschließend wurde mit konzentrierter Salzsäure-Lösung der pH Wert auf 1 eingestellt und 1 h bei 100°C gerührt. Mit 4M Natronlauge wurde der pH-Wert auf 3 eingestellt und über Nacht bei RT nachgerührt. Die ausgefallene Substanz wurde abgesaugt und mit 10 mL Wasser nachgewaschen. Die Mutterlauge wurde i.vac. eingeengt und der Rückstand mittels präparativer HPLC aufgereinigt. Die Produktfraktionen wurden vereinigt, i.vac. eingeengt und getrocknet.

| | |
|---|---|
| Ausbeute: | 100 mg (7% d.Th.) |
| ESI-MS: | m/z = 174 (M+H)⁺ |
| Rₜ (HPLC-MS): | 1.18 min (Methode C) |

### Stufe 3: Spiro[cyclobutan-1,3'-indolin]

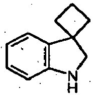

Unter einer Stickstoff-Atmosphäre wurde zu 100 mg (0.58 mmol) Spiro[cyclobutan-1,3'-indolin]-2'-ön in 20 mL THF 0.60 mL (0.60 mmol) einer 1M Lösung Lithiumaluminiumhydrid in THF zugetropft. Anschließend wurde der Reaktionsansatz 1 h bei 65°C gerührt. Nach dem Abkühlen wurde 1 mL Wasser zugegeben und 10 min nachgerührt. Die organische Phase wurde mit Natriumsulfat getrocknet und eingedampft. Das Produkt wurde ohne weitere Aufreinigung umgesetzt.

| | |
|---|---|
| Ausbeute: | 100 mg (quant) |
| ESI-MS: | m/z = 160 (M+H)⁺ |
| Rₜ (HPLC-MS): | 0.77 min (Methode C) |

### Intermediat 31:

### 3-(3-Pyrrolidin-1-yl-propyl)-2.3-dihydro-1H-indol

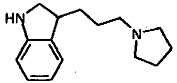

### Stufe 1: 3-(1H-Indol-3-yl)-1-pyrrolidin-1-yl-propan-1-on

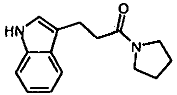

681 mg (3.60 mmol) 3-(1*H*-Indol-3-yl)-propionsäure, 0.3 mL (3.60 mmol) Pyrrolidin, 1.13 g (3.50 mmol) TBTU und 0.98 mL (7.00 mmol) Triethylamin in 5.0 mL DMF wurden über Nacht bei RT gerührt. Der Ansatz wurde mittels präparativer HPLC aufgereinigt. Die Produktfraktionen wurden vereinigt und i.vac. eingeengt.

| | |
|---|---|
| Ausbeute: | 670 mg (77% d.Th.) |
| ESI-MS: | m/z = 243 (M+H)⁺ |
| Rₜ (HPLC-MS): | 3.33 min (Methode E) |

### Stufe 2: 3-(Pyrrolidin-1-yl-propyl)-1H-indol

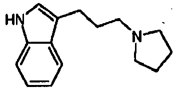

Unter einer Stickstoff-Atmosphäre wurde zu 670 mg (2.77 mmol) 3-(1*H*-Indol-3-yl)-1-pyrrolidin-1-yl-propan-1-on in 30 mL THF langsam 3.40 mL (3.40 mmol) einer 1M Lithiumaluminiumhydrid-Lösung in THF zugetropft. Der Reaktionsansatz wurde 1h bei 65 C gerührt und nach dem Erkalten mit 1 mL Wasser versetzt. Nach 10 min Rühren bei RT wurde die organische Phase mit Natriumsulfat getrocknet und i.vac. eingeengt. Das Produkt wurde ohne weitere Aufreinigung weiter umgesetzt.

| | |
|---|---|
| Ausbeute: | 600 mg (95% d.Th.) |
| ESI-MS: | m/z = 229 (M+H)⁺ |
| Rₜ (HPLC-MS): | 0.9 min (Methode C) |

### Stufe 3: 3-(3-Pyrrolidin-1-yl-propyl)-2,3-dihydro-1H-indol

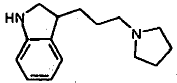

Bei 15°C wurde zu 600 mg (2.63 mmol) 3-(3-Pyrrolidin-1-yl-propyl)-1*H-*indol in 7.50 g Essigsäure 508 mg (8.10 mmol) Natriumcyanoborhydrid zugegeben und 1 h bei 15°C gerührt. Nach dieser Zeit wurden abermals 600 mg Natriumcyanoborhydrid zugegeben und bei 15°C für 3 h gerührt. Der Reaktionsansatz wurde am Rotationsverdampfer eingeengt und mit 40 mL einer 4M Salzsäure-Lösung versetzt. Der Reaktionsansatz wurde 1 h bei RT gerührt. Anschließend wurde der Ansatz mit Kaliumcarbonat basisch gestellt und mit Ethylacetat (2 x 50 mL) extrahiert. Die vereinigten organischen Phasen wurden getrocknet und i.vac. eingeengt. Das Produkt wurde ohne weitere Aufreinigung weiter umgesetzt.

| | |
|---|---|
| Ausbeute: | 600 mg (99% d.Th.) |
| ESI-MS: | m/z = 231 (M+H)⁺ |
| Rₜ (HPLC-MS): | 0.25 min (Methode C) |

### Intermediat 32:

### 2,3-Dihydro-1H-indol-2-carbonsäureethylester Hydrochlorid

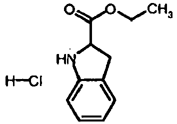

1.50 g (9.19 mmol) 2,3-Dihydro-1*H*-indol-2-carbonsäure in 50 mL ethanolischer Salzsäure wurden über Nacht bei RT gerührt. Das Lösungsmittel wurde am Rotationsverdampfer entfernt.

| | |
|---|---|
| Ausbeute: | 2.10 g (quant) |
| ESI-M5: | m/z = 192 (M+H)⁺ |

### Intermediat 33:

### 7,7-Dimethyl-4.5.6.7-tetrahydro-1H-pyrazol[4,3-c]pyridin-dihydrochlorid

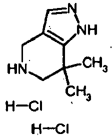

Bei 0°C wurde zu 1.60 g (6.05 mmol) 7,7-Dimethyl-1,4,6,7-tetrahydro-pyrazolo[4,3-*c*]-pyridin-5-carbonsäure-*tert*-butylester (wurde analog zu WO2005/065779 synthetisiert) in 15 mL Dichlormethan 5 mL Trifluoressigsäure hinzugefügt und der Reaktionsansatz 2 h bei RT gerührt. Anschließend wurde das Lösungsmittel i.vac. entfernt. Der Rückstand wurde mit Ethanol versetzt und abermals i.vac. eingeengt. Der Rückstand wurde in Ethanol gelöst und 12 mL einer 1.25 M ethanolischen HCl-Lösung wurden hinzugefügt. Der Ansatz wurde wieder i.vac. eingeengt. Der Rückstand wurde mit Ethanol verrieben. Der Feststoff wurde abgesaugt und getrocknet.

| | |
|---|---|
| Ausbeute: | 1.24 g (92% d.Th.) |
| ESI-MS: | m/z = 152 (M+H)⁺ |
| Rₜ (HPLC): | 0.65 min (Methode N) |

### Intermediat 34:

### 1,1-Dimethyl-2-m-tolyl-ethylamin

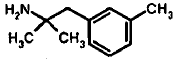

### Stufe 1: m-Tolyl-essigsäureethylester

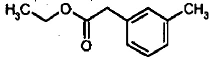

Zu 25.5 g (169 mmol) m-Tolylessigsäure in 200 mL Ethanol wurden 50 mL ethanolische Salzsäure hinzugegeben und der Ansatz über Nacht bei RT gerührt. Nach dem Entfernen des Lösungsmittels am Rotationsverdampfer wurde der Rückstand mit 250 mL EtOAc versetzt und mit 150 mL einer 15%-igen Kaliumcarbonat Lösung extrahiert. Die organische Phase wurde über Magnesiumsulfat getrocknet, filtriert und am Rotationsverdampfer bis zur Trockne eingeengt.

| | |
|---|---|
| Ausbeute: | 25.6 g (85% d.Th.) |
| MS: | m/z =178 (M)⁺ |
| R_{f}: | 0.76 (Kieselgel, PE/ EtOAc = 8/2) |

### Stufe 2: 2-Methyl-1-m-tolylpropan-2-ol

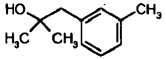

Bei 10°C wurde zu 100 mL (300.0 mmol) einer 3M Lösung von Methylmagnesiumbromid in THF 17.8 g (99.87 mmol) m-Tolyl-essigsäureethylester in 400 mL THF über eine Stunde hinweg langsam zugetropft. Der Reaktionsansatz wurde 30 min bei RT und 3 h unter Rückfluss gekocht. Nach dem Abkühlen wurden langsam 250 mL einer gesättigten Ammoniumchlorid-Lösung zugetröpft. Man ließ den Ansatz über Nacht stehen. Es wurden 300 mL einer 0.5 M Salzsäure-Lösung zugegeben und kurz gerührt. Die Phasen wurden getrennt und die wässrige Phase wurde mit EtOAc extrahiert. Die vereinigten organischen Phasen wurden mit gesättigter Natriumchlorid-Lösung gewaschen, über Magnesiumsulfat getrocknet, filtriert und i.vac. eingeengt.

| | |
|---|---|
| Ausbeute: | 14.7 g (90% d.Th.) |
| R_{f}: | 0.39 (Kieselgel, PE/ EtOAc = 8/2) |

### Stufe 3: N-(1,1-Dimethyl-2-m-tolylethyl-formamid

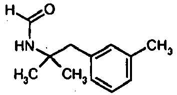

Unter Eiskühlung wurden zu 60 mL Eisessig 5.20 g (106 mmol) Natriumcyanid zugegeben. Nach kurzem Rühren wurde bei 0°C 40 mL konz. Schwefelsäure so zugetropft, dass die Reaktionstemperatur nicht über 20°C stieg. Nach kurzem Rühren wurden bei 0°C 14.5 g (88.3 mmol) 2-Methyl-1-m-tolylpropan-2-ol in 40 mL Eisessig so zugetropft, dass die Reaktionstemperatur 20°C nicht überstieg. Der Reaktionsansatz Wurde 10 min bei 0°C und über Nacht bei RT gerührt. Der Reaktionsansatz wurde auf Eis gegossen und mit 40%-iger Natronlauge neutralisiert. Die wässrige Phase wurde mit EtOAc (3 x 150 mL) extrahiert. Die vereinigten organischen Phasen wurden mit 15%-iger Kaliumcarbonat-Lösung gewaschen, getrocknet, fitriert und i.vac. eingeengt.

| | |
|---|---|
| Ausbeute: | 14.4 g (85% d.Th.) |
| ESI-MS: | m/z = 192 (M+H)⁺ |
| R_{f}: | 0.31 (Kieselgel, PE/EtOAc = 8/2) |

### Stufe 4: 1,1-Dimethyl-2-m-tolyl-ethylamin

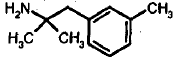

1.80 g (9.41 mmol) *N*-(1,1-Dimethyl-2-*m*-tolylethyl)-formamid, 20 mL Wasser und 20 mL konz. Salzsäure wurden 2 h unter Rückfluss gekocht. Das Reaktionsgemisch wurde mit 20 mL Eiswasser verdünnt und mit gesättigter Kaliumcarbonat-Lösung alkalisch gestellt. Die wässrige Phase wurde mit DCM (2 x 20 mL) extrahiert. Die organische Phase wurde mit Wasser gewaschen, mit Natriumsulfat getrocknet und i.vac. eingeengt. Der Rückstand wurde mit Toluol coevaporiert (2 x).

| | |
|---|---|
| Ausbeute: | 1.30 g (85% d.Th.) |
| ESI-MS: | m/z = 164 (M+H)⁺ |

### Intermediat 35:

### 3.3-Dimethylpyrrolidin Hydrochlorid

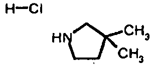

### Stufe 1: 1-Benzyl-3.3-dimethylpyrrolidin-2,5-dion

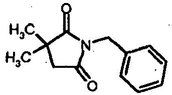

25.0 g (171 mmol) 2,2-Dimethylbernsteinsäure und 20.6 mL (188 mmol) Benzylamin wurden in einer Apparatur mit Wasserabscheider 1.5 h unter Rückfluss gerührt. Das Xylol wurde abrotiert und der Rückstand zwischen 300 mL EtOAc und 150 mL einer 5%-igen Natriumhydrogencarbonat-Lösung verteilt. Die organische Phase wurde über Natriumsulfat getrocknet, abgesaugt und einrotiert. Der Rückstand wurde in einem Trockeneisbad in Isopropanol auskristallisiert.

| | |
|---|---|
| Ausbeute: | 34.6 g (93% d.Th.) |
| ESI-MS: | m/z = 218 (M⁺H)⁺ |
| R_{f}: | 0.58 (Kieselgel, PE/EtOAc =4/1) |

### Stufe 2: 1-Benzyl-3,3-dimethylpyrrolidin

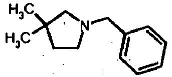

Zu 400 mL (400 mmol) einer 1M Lithiumaluminiumhydrid-Lösung in THF wurde unter Trockeneis/Isopropanol-Kühlung 34.5 g (158.8 mmol) 1-Benzyl-3,3-dimethyl-pyrrolidin-2,5-dion in 200 mL THF langsam bei 10°C bis 20°C innerhalb von 1.5 h zugetropft. Der Reaktionsansatz wurde über Nacht bei RT nachgerührt. Unter Isopropanol/Trockeneis-Kühlung wurden bei 10°C bis 20°C tropfenweise 120 mL einer 3:1 Mischung THF:Wasser zugetropft. Der Reaktionsansatz wurde mit 600 mL THF verdünnt. Nach der vollständigen Zersetzung wurde der Niederschlag abgesaugt und mit THF nachgewaschen. Das Filtrat wurde am Rotationsverdampfer eingeengt, mit 100 mL einer 5%-igen Natriumhydrogencarbonat-Lösung versetzt und mit 300 mL EtOAc extrahiert. Die organische Phase wurde über Natriumsulfat getrocknet, abgesaugt und einrotiert.

| | |
|---|---|
| Ausbeute: | 28.7 g (96% d.Th.) |
| ESI-MS: | m/z = 190 (M+H)⁺ |
| R_{f}: | 0.16 (Kieselgel, DCM/EtOH = 50/1) |

### Stufe 3: 3,3-Dimethylpyrrolidin Hydrochlorid

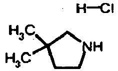

28.7 g (151 mmol) 1-Benzyl-3,3-dimethylpyrrolidin und 2.0 g Palladium auf Kohle (10%) in 100 mL Methanol wurden in einer Wasserstoff-Atmosphäre für 3 Tage bei 3 bar Wasserstoff-Druck hydriert. Während dieser Zeit wurde 3 x je 300 mg Palladium auf Kohle (20%) der Reaktion zugesetzt. Anschließend wurde der Katalysator abgesaugt und das Filtrat mit 100 mL etherischer 2N HCl-Lösung versetzt und einrotiert. Das Produkt wurde im Exsikkator über Phosphorpentoxid im Vakkum getrocknet.

| | |
|---|---|
| Ausbeute: | 20.9 g (quant.) |
| ESI-MS: | m/z = 100 (M+H)⁺ |
| R_{f} : | 0.34 (Kieselgel, DCM/MeOH/NH₄OH = 8/2/0.2) |

### Intermediat 36:

### 2,3-Dihydro-1H-pyrrolo[3,2-c]pyridin

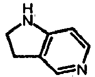

1.50 g (12.7 mmol) 5-Azaindol und 0.75 g Raney-Nickel in 70 mL Ethanol wurden in einer Wasserstoff-Atmosphäre bei 3 bar Wasserstoff-Druck für 3 Tage bei 70°C hydriert. Anschließend wurde der Katalysator abgesaugt und die Lösung i.vac. eingeengt. Der Rückstand wurde über eine Kieselgel-Säule aufgereinigt. Die Produktfraktionen wurden vereinigt und am Rotationsverdampfer eingeengt.

| | |
|---|---|
| Ausbeute: | 620 mg (41 % d.Th.) |
| ESI-MS: | m/z = 121 (M+H)⁺ |
| R_{f}: | 0.12 (Kieselgel, DCM/MeOH/NH₄OH = 80/20/2) |

### Intermediat 37:

### (6-Chlorpyrimidin-4-yl-indol-1-yl-methanon

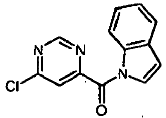

Unter einer Stickstoffatmosphäre wurden zu 350 mg (2.96 mmol) Indol in 15 mL THF 120 mg (3.00 mmol) Natriumhydrid (60% in Mineralöl) portionsweise zugegeben und 30 min bei RT nachgerührt. 500 mg (2.83 mmol) 6-Chlorpyrimidin-4-carbonsäurechlorid wurde portionsweise zugegeben und anschließend wurde der Reaktionsansatz 2 h bei RT gerührt. Dann wurden 50 mL EtOAc hinzugefügt und mit 50 mL gesättigter Natriumhydrogencärbonät-Lösung (1 x), 30 mL Wasser und mit 50 mL 1 M Salzsäure gewaschen. Die organische Phase wurde über Magnesiumsulfat getrocknet, filtriert und anschließend i.vac. eingeengt. Der Rückstand wurde über Kieselgel aufgereinigt. Die Produktfraktionen wurden vereinigt und i.vac. eingeengt.

| | |
|---|---|
| Ausbeute: | 200 mg (28% d.Th.) |
| MS: | m/z = 257/259 (M)⁺ |
| R_{f}: | 0.80 (Kieselgel, PE/EtOAc = 7/3) |

### Intermediat 38:

### (6-Chlorpyrimidin-4-yl)-(3-methylindol-1-yl)-methanon

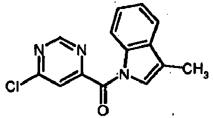

Unter einer Stickstoffatmosphäre wurden zu 380 mg (2.90 mmol) 3-Methylindol in 15 mL THF 120 mg (3.00 mmol) Natriumhydrid (60% in Mineralöl) portionsweise zugegeben und 30 min bei RT nachgerührt. 500 mg (2.83 mmol) 6-Chlorpyrimidin-4-carbonsäurechlorid wurden portionsweise zugegeben und anschließend wurde der Reaktionsansatz 2 h bei RT gerührt. Anschließend wurden 50 mL EtOAc hinzugefügt und mit 50 mL gesättigter Natriumhydrogencarbonat-Lösung (1x), 30 mL Wasser und mit 50 mL 1 M Salzsäure gewaschen. Die organische Phase wurde über Natriumsulfat getrocknet, filtriert und anschließend i.vac. eingeengt. Ohne weitere Aufreinigung wurde das Produkt in die nächste Stufe eingesetzt.

| | |
|---|---|
| Ausbeute: | 300 mg (39% d.Th.) |

### Intermediat 39:

### (6-Chlorpyrimidin-4-yl)-(5-fluorindol-1-yl)-methanon

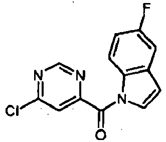

Unter einer Stickstoffatmosphäre wurden zu 400 mg (2.90 mmol) 5-Fluorindol in 15 mL THF 120 mg (3.00 mmol) Natriumhydrid (60% in Mineralöl) portionsweise zugegeben und 30 min bei RT nachgerührt. 500 mg (2.83 mmol) 6-Chlorpyrimidin-4-carbonsäurechlorid wurden portionsweise zugegeben und anschließend wurde der Reaktionsansatz 2 h bei RT gerührt. Anschließend wurden 50 mL EtOAc hinzugefügt und mit 50 mL gesättigter Natriumhydrogencarbonat-Lösung (1 x), 30 mL Wasser und mit 50 mL 1 M Salzsäure gewaschen. Die organische Phase wurde über Magnesiumsulfat getrocknet, filtriert und anschließend i.vac. eingeengt. Der Rückstand wurde mit wenig EtOAc versetzt, das ausgefallene Produkt abgesaugt und das Filtrat über Kieselgel aufgereinigt. Die Produktfraktionen wurden vereinigt und i.vac. eingeengt.

| | |
|---|---|
| Ausbeute: | 70 mg (9% d.Th.) |
| R_{f}: | 0.39 (Kieselgel, PE/EtOAc = 4/1) |

### Intermediat 40:

### 6-Chlor-Pyrimidin-4-carbonsäure-benzyl-(2,2,2-trifluorethyl)-imid

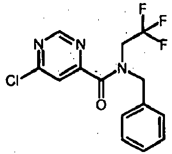

### Stufe 1: Benzyl-(2,2,2-trifluorethyl)-amin

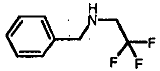

Zu 2.00 g (18.7 mmol) Benzylamin in 50 mL Xylol wurden 2.17 g (9.4 mmol) 2,2,2-Trifluorethyltrifluormethansulfonat hinzugefügt und der Reaktionsansatz über Nacht unter Rückfluss gekocht. Nach dem Abkülen wurde das Reaktionsgemisch abgesaugt, mit DIPE nachgewaschen und das Filtrat am Rotationsverdampfer eingeengt. Der Rückstand wurde mittels Flash-Chromatographie aufgereinigt. Die Produktfraktionen wurden vereinigt und am Rotationsverdampfer eingeengt.

| | |
|---|---|
| Ausbeute: | 2.30 g (65% d.Th.) |
| ESI-MS: | m/z = 190.0 (M+H)⁺ |

### Stufe 2: 6-Chlorpyrimidin-4-carbonsäure-benzyl-(2,2,2-trifluor-ethyl)-amid

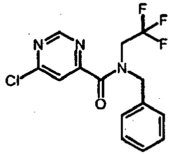

Zu 1.00 g (5.65 mmol) 6-Chlorpyrimidin-4-carbonsäurechlorid in 20 mL Dichlormethan wurden unter Eis/Ethanol-Bad-Kühlung 1.04 g (5.50 mmol) Benzyl-(2,2,2-trifluorethyl)-amin und 5.50 mL (5.50 mmol) einer 1 M Natronlauge zugetropft. Der Ansatz wurde zunächst 2h unter Kühlung und anschließend 1 h bei RT nachgerührt. Es wurde 50 mL einer gesättigten Natriumhydrogencarbonat-Lösung zugegeben und 10 min gerührt. Die organische Phase wurde abgetrennt, mit Wasser (1 x 30 mL) und 1 M Salzsäure (1 x 50 mL) gewaschen, über Natriumsulfat getrocknet, filtriert und i.vac. eingeengt. Das Produkt wurde ohne weitere Aufreinigung weiter umgesetzt.

| | |
|---|---|
| Ausbeute: | 1.20 g (64% d.Th.) |
| ESI-MS: | m/z = 330/332 (M+H)⁺ |
| Rₜ (HPLC-MS): | 1.56 min (Methode C) |

### Intermediat 41:

### 1-{1-[6-(4-Nitro-2.3-dihydroindol-1-carbonyl)-pyrimidin-4-yl]-piperidin-4-yl}-1,3-dihydro-imidazo[4,5-b]pyridin-2-on

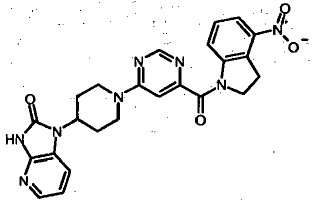

Zu 150 mg (0.44 mmol) 6-[4-(2-Oxo-2,3-dihydro-imidazo[4,5-*b*]pyridin-1-yl)-piperidin-1-yl]-pyrimidin-4-carbonsäure, 75 mg (0.46 mmol) 4-Nitro-2,3-dihydro-1*H*-indol, 100 µL (0.712 mmol) Triethylamin in 1.5 mL DMF wurden 150 mg (0.470 mmol) TBTU hinzugefügt und über Nacht bei RT gerührt. Der Reaktionsansatz wurde auf 30 mL Wasser gegossen. Die wässrige Phase wurde mit DCM extrahiert (3 x 20 mL). Die vereinigten organischen Phasen wurden über Magnesiumsulfat getrocknet, filtriert und i.vac. eingeengt. Der Rückstand wurde in 3 mL DMF gelöst, über einen Spritzenfilter filtriert und mittels präparativer HPLC aufgereinigt. Die Produktfraktionen wurden vereinigt und lyophilisiert.

| | |
|---|---|
| Ausbeute: | 30 mg (14% d.Th.) |
| ESI-MS: | m/z = 487 (M+H)⁺ |
| R_{f}: | 0.54 (Kieselgel, Laufmittel A) |

### Intermediat 42:

### 1-{1-[6-(5-Nitro-2,3-dihydro-indol-1-carbonyl)-pyrimidin-4-yl]-piperidin-4-yl}-1,3-dihydro-imidazo[4.5-b]pyridin-2-on

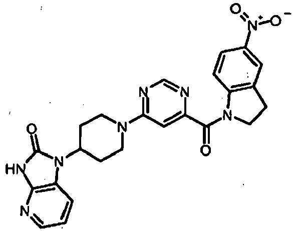

Zu 150 mg (0.44 mmol) 6-[4-(2-Oxo-2,3-dihydro-imidazo[4,5-*b*]pyridin-1-yl)-piperidin-1-yl]-pyrimidin-4-carbonsäure, 75 mg (0.46 mmol) 5-Nitro-2,3-dihydro-1*H*-indol, 100 µL und (0.712 mmol) Triethylamin in 1.5 mL DMF wurden 150 mg (0.47 mmol) TBTU hinzugefügt und über Nacht bei RT gerührt. Der Reaktionsansatz wurde auf 30 mL Wasser gegossen. Das ausgefallene Produkt wurde abgesaugt und bei 50°C im ULTS getrocknet.

| | |
|---|---|
| Ausbeute: | 130 mg (61% d.Th.) |
| ESI-MS: | m/z = 487 (M+H)⁺ |
| R_{f}: | 0.61 (Kieselgel, Laufmittel A) |

### Intermediat 43:

### 3-{1-[6-(3-Brom-7,8-dihydro-5H-1,6-naphthyridin-6-carbonyl)-pyrimidin-4-yl]-piperidin-4-yl}-7-methoxy-1,3,4,5-tetrahydro-1,3-benzodiazeoin-2-on

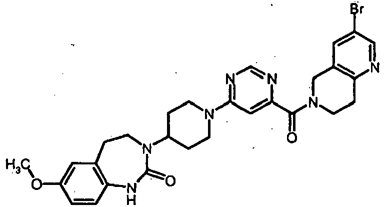

Zu 80 mg (0.20 mmol) 6-[4-(7-Methoxy-2-oxo-1,2,4,5-tetrahydro-1,3-benzodiazepin-3-yl)-piperidin-1-yl]-pyrimidin-4-carbonsäure, 70 mg (0.28 mmol) 3-Brom-5,6,7,8-tetrahydro-1,6-naphthyridin-Hydrochlorid und 0.120 mL (0.86 mmol) Triethylamin in 0.9 mL DMF wurden 90 mg (0.24 mmol) TBTU hinzugefügt und über Nacht bei RT gerührt. Der Reaktionsansatz wurde mit 1.0 mL Methanol, 1.0 mL gesättigter Natriumhydrogencarbonat-Lösung und 8.0 mL Eiswasser versetzt. Der Niederschlag wurde abgesaugt, mit Wasser und Diethylether nachgewaschen und getrocknet.

| | |
|---|---|
| Ausbeute: | 94 mg (75% d.Th.) |
| ESI-MS: | m/z = 592/594 (M+H)⁺ |
| Rₜ (HPLC-MS): | 3.06 min (Methode E) |

### Intermediat 44:

### 3-Ethyl-2,3-dihydro-1H-indol

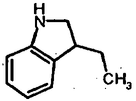

### Stufe 1: 3-Ethyliden-1,3-dihydro-indol-2-on

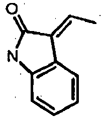

Zu 2.0 g (15 mmol) Indolin-2-on und 0.20 mL Piperidin in 20 mL Methanol wurden 0.85 mL (15 mmol) Acetaldehyd zugetropft. Der Reaktionsansatz wurde 3 h unter Rückfluss gekocht und anschließend eingeengt. Der Rückstand wurde in Diisopropylether verrieben und abgesaugt.

| | |
|---|---|
| Ausbeute: | 2.2 g (92% d.Th.) |
| ESI-MS: | m/z = 158 (M-H)⁻ |
| Rₜ(HPLC): | 1.19 min (Methode C) |

### Stufe 2: 3-Ethyl-2,3-dihydro-1H-indol

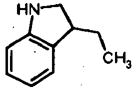

Unter einer Stickstoff-Atmosphäre wurde zu 2.2 g (14 mmol) 3-Ethyliden-1,3-dihydro-indol-2-on in 50 mL THF 41 mL (41 mmol) einer 1M Boran in THF-Lösung zugetropft. Der Reaktionsansatz wurde 3h unter Rückfluss gekocht und anschließend bei 0°C mit 10 mL Methanol gefolgt von 15 mL halbkonzentrierter Salzsäure-Lösung versetzt. Der Reaktionsansatz wurde 3 h unter Rückfluss gerührt, abgekühlt und mit Ethylacetat zweimal gewaschen. Die wässrige Phase wurde mit wässriger 4M Natronlauge alkalisch gestellt und dreimal mit Ethylacetat extrahiert. Die organischen Phasen wurden vereinigt, mit Natriumsulfat getrocknet und eingeengt.

| | |
|---|---|
| Ausbeute: | 1.7g (84% d.Th.) |
| ESI-MS: | m/z = 148 (M+H)⁺ |
| Rₜ(HPLC): | 2.21 min (Methode E) |

### Intermediat 45

### 3-Cyclopropylmethyl-2,3-dihydro-1H-indol

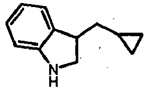

### Stufe 1 3-Cyclopropylmethylen-1,3-dihydro-indol-2-on

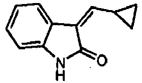

2.0 g (15 mmol) 1,3-Dihydro-indol-2-on und 0.20 mL Piperidin wurden in 20 mL Methanol vorgelegt. 1.1 mL (15 mmol) Cyclopropancarboxaldehyd wurden zugetropft, das Reaktionsgemisch 3 h refluxiert und anschließend zur Trockene einrotiert. Der Rückstand wurde mit Diisopropylether verrieben und der verbliebene Feststoff wurde abgesaugt und getrocknet.

| | |
|---|---|
| Ausbeute: | 2.7 g (97% d.Th.) |
| ESI-MS: | m/z = 186 (M+H)⁺ |
| Rₜ (HPLC-MS) | 1.27 min (Methode C) |

### Stufe 2 3-Cyclopropylmethyl-2,3-dihydro-1H-indol

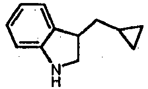

1.0 g (5.4 mmol) 3-Cyclopropylmethylen-1,3-dihydro-indol-2-on wurden in 50 mL THF vorgelegt. 12 mL (12 mmol) 1 M Boran in THF wurde langsam zugetropft. Das Reaktionsgemisch wurde 3 h refluxiert. Nach dem Abkühlen auf RT wurden nacheinander 10 mL Methanol und 15 mL halbkonzonzentrierte wässrige Salzsäure-Lösung zugetropft und anschließend 3 h refluxiert. Nach dem Abkühlen auf RT wurde mit EtOAc extrahiert. Die wässrige Phase wurde mit wässriger 4M Natriumhydroxid-Lösung alkalisch gestellt und ein weiteres Mal mit EtOAc extrahiert. Die organische Phase wurde getrocknet, filtriert und eingeengt.

| | |
|---|---|
| Ausbeute: | 0.23 g (25% d.Th.) |
| ESI-MS: | m/z = 174 (M+H)⁺ |
| Rₜ (H PLC-MS): | 1.01 min (Methode C) |

### Intermediat 46

### 7,7-Dimethyl-4,5,6,7-tetrahydro-thieno[3,4-c]pyridin

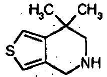

### Stufe 1 Methylen-(2-methyl-2-thiophen-3-yl-propyl)-amin

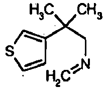

5.75 g (37.0 mmol) 2-Methyl-2-thiophen-3-yl-propylamin und 3.61 mL (44.4 mmol) Formaldehyd wurden zusammen mit 2.0 g Molsieb (4Å Pulver) über Nacht bei RT gerührt. Das Reaktionsgemisch wurde filtriert und das Filtrat wurde zur Trockene einrotiert.

| | |
|---|---|
| Ausbeute: | 6.00 g (97% d.Th.) |
| ESI-MS: | m/z = 168 (M+H)⁺ |

### Stufe 2 7,7-Dimethyl-4,5,6,7-tetrahydro-thieno[3,4-c]pyridin

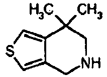

6.00 g (35.9 mmol) Methylen-(2-methyl-2-thiophen-3-yl-propyl)-amin, 11.3 mL (45.3 mmol) 4M HCl und 11.8 mL (142 mmol) konz. HCl wurden über das Wochenende bei RT gerührt. Das Reaktionsgemisch wurde mit 4M NaOH Lösung alkalisch gestellt. Der ausgefallene Niederschlag wurde abgesaugt, mit Wasser gewaschen und getrocknet. Die Substanz wurde über Alox gereinigt. Die Produkt enthaltenden Fraktionen wurden vereinigt und zur Trockene einrotiert.

| | |
|---|---|
| Ausbeute: | 740 mg (12% d.Th.) |
| Rₜ (HPLC-MS): | 1.24 min (Methode K) |

### Intermediat 47:

### 1,7,7-Trimethyl-4,5,6,7-tetrahydro-1H-pyrazolo[4,3-c]pyridiniumtrifluoracetat

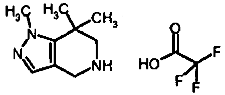

### Stufe 1 1,7,7-Trimethyl-1,4,6,7-tetrahydro-pyrazolo[4,3-c]piridin-5-carboxylsäure-tert-butylester

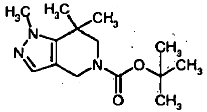

2.10 g (8.36 mmol) 7,7-Dimethyl-1,4,6,7-tetrahydro-pyrazolo[4,3-c]pyridin-5-carboxylsäure-*tert*-butylesfer wurden in 25 mL DMF vorgelegt. Unter Eiskühlung wurden 350 mg (8.75 mmol) Natriumhydrid (55%) zugegeben. Das Reaktionsgemisch wurde 30 min gerührt, anschließend wurden 0.540 mL (8.67 mmol) lodmethan zugegeben und 1 h bei 0°C gerührt. Das Reaktionsgemisch wurde zur Trockene einrotiert und der Rückstand wurde mit Wasser versetzt und mit EtOAc extrahiert. Die organische Phase wurde getrocknet, gefiltert und zur Trockene einrotiert. Der Rückstand wurde mittels HPLC gereinigt. Die Produkt enthaltenden Fraktionen wurden vereinigt und organische Lösungsmittel abrotiert. Der wässrige Rückstand wurde mit DCM extrahiert. Die organische Phase wurde getrocknet, filtriert und zur Trockene einrotiert.

| | |
|---|---|
| Ausbeute: | 100 mg (4% d.Th.) |
| Rₜ (HPLC-MS): | 3.64 min (Methode E) |

### Stufe 2 1,7,7-Trimethyl-4,5,6,7-tetrahydro-1H-pyrazolo[4,3-c]pyridinium-trifluoracetat

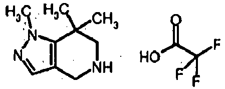

0.10 g (0.34 mmol) 1,7,7-Trimethyl-1,4,6,7-tetrahydro-pyrazolo[4,3-c]pyridin-5-5-carboxylsäure-*tert-*butylester und 1.0 mL TFA wurden in 2.0 mL DCM 2 h bei RT gerührt. Das Reaktionsgemisch wurde zur Trockene einrotiert.

| | |
|---|---|
| Ausbeute: | 125 mg (99% d.Th.) |
| ESI-MS: | m/z = 166 (M+H)⁺ |

### Intermediat 48:

### (1,2,3,4-Tetrahydro-isochinolin-4-yl)-methanol

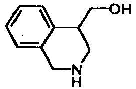

0.50 mg (3.1 mmol) Isochinolin-4-yl-methanol, 75 mg Platindioxid und 3.2 mL 1 N Salzsäure-Lösung wurden in 50 mL Methanol 4 h bei RT unter einer Wasserstoff-Atmosphäre bei 50 psi hydriert. Das Reaktionsgemisch wurde filtriert und das Filtrat zur Trockne einrotiert.

| | |
|---|---|
| Ausbeute: | 0.51 g (quantitativ) |
| Rₜ (HPLC-MS): | 1.12 min (Methode O) |

### Intermediat 49:

### 4,5-Difluor-3,3-dimethyl-2,3-dihydro-1H-indol

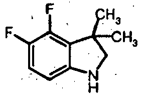

### Stufe 1 1-Acetyl-4,5-difluor-1,3-dihydro-indol-2-on

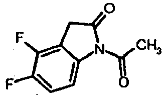

2.00 g (11.8 mmol) 4,5-Difluor-1,3-dihydro-indol-2-on I wurden in 6.62 mL (55.0 mmol) Essigsäureanhydrid 2 h bei 150°C gerührt. Das Reaktionsgemisch wurde auf Eiswasser gegeben, der ausgefallene Feststoff abgesaugt und mit Wasser gewaschen. Das Produkt wurde aus einem Gemisch aus Wasser und Ethanol umkristallisiert, abgesaugt und getrocknet.

| | |
|---|---|
| Ausbeute: | 1.00 g (40% d.Th.) |
| ESI-MS: | m/z = 210 (M-H)⁻ |
| Rₜ (HPLC-MS): | 1.40 min (Methode C) |

### Stufe 2 4,5-Difluor-3,3-dimethyl-1,3-dihydro-indol-2-on

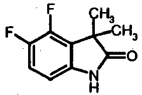

0.50 g (2.4 mmol) 1-Acetyl-4,5-difluor-1,3-dihydro-indol-2-on wurden unter Argon in 10 mL DMF vorgelegt. 0.22 g (5.1 mmol) Natriumhydrid (55%) wurden bei 0°C zugegeben und das Reaktionsgemisch 1 h gerührt. Anschließend wurden 0.32 mL (5.1 mmol) lodmethan zugetropft. Das Reaktionsgemisch wurde über Nacht bei RT gerührt, auf Wasser gegossen und mit EtOAc extrahiert. Die organische Phase wurde getrocknet, gefiltert und zur Trockne einrotiert. Der Rückstand wurde mittels HPLC gereinigt. Die Produkt enthaltenden Fraktionen wurden vereinigt und zur Trockne einrotiert.

| | |
|---|---|
| Ausbeute: | 0.15 g (32% d.Th.) |
| ESI-MS: | m/z = 198 (M+H)⁺ |
| Rₜ (HPLC-MS): | 1.26 min (Methode C) |

### Stufe 3 4,5-Difluor-3,3-dimethyl-2,3-dihydro-1H-indol

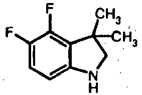

0.15 mg (0.76 mmol) 4,5-Difluor-3,3-dimethyl-1,3-dihydro-indol-2-on wurden in 20 mol THF unter Argon vorgelegt. 0.91 mL (0.91 mmol) einer 1 M Lithiumaluminiumhydrid-Lösung in 10 mL THF wurden zugetropft. Das Reaktionsgemisch wurde 1 h bei 70°C gerührt, abgekühlt und mit Wasser versetzt. Das Reaktionsgemisch wurde getrocknet, filtriert und zur Trockene einrotiert.

| | |
|---|---|
| Ausbeute: | 160 mg (quantitativ) |
| Rₜ (HPLC-MS): | 1.24 min (Methode C) |

### Intermediat 50:

### 5,6-Difluor-3-methyl-2,3-dihydro-1H-indol

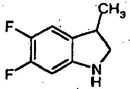

### Stufe 1 5,6-Difluor-3-methyl-1,3-dihydro-indol-2-on

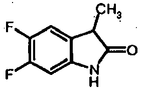

1.5 g (9.0 mmol) 5,6-Difluor-1,3-dihydro-indol-2-on wurden mit 1.0 g Raney-Nickel in 50 mL Methanol 3 h bei 200°C in einem Autoklaven gerührt. Der Katalysator wurde abfiltriert und das Filtrat wurde zur Trockne einrotiert.

| | |
|---|---|
| Ausbeute: | 1.6 g (99% d.Th.) |
| ESI-MS: | m/z = 184 (M+H)⁺ |
| Rₜ (HPLC-MS): | 3.2 min (Methode E) |

### Stufe 2 5,6-Difluor-3-methyl-2,3-dihydro-1H-indol

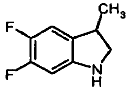

Unter einer Argon-Atmosphäre wurden 1.6 g (8.7 mmol) 5,6-Difluor-3-methyl-1,3-dihydro-indol-2-on in 50 mL THF vorgelegt. 18 mL (18 mmol) 1 M Boran in THF wurden langsam zugetropft. Das Reaktionsgemisch wurde 2 h bei 70°C gerührt. Nach dem Abkühlen auf RT wurden nacheinander 10 mL Methanol und 30 mL halbkonz. HCl zugetropft und anschließend 1 h refluxiert. Nach dem Abkühlen auf RT wurde mit EtOAc extrahiert. Die wässrige Phase wurde mit 4M NaOH Lösung alkalisch gestellt und mit DCM extrahiert. Die organische Phase wurde getrocknet, gefiltert und eingeengt.

| | |
|---|---|
| Ausbeute: | 0.7 mg (47% d.Th.) |
| ESI-MS: | m/z = 170 (M+H)⁺ |
| Rₜ (HPLC-MS): | 2.7 min (Methode E) |

### Intermediat 51:

### 4,5-Difluor-3-methyl-2,3-dihydro-1H-indol

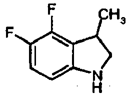

Analog zu 5;6-Difluor-3-methyl-2,3-dihydro-1H-indol wurde 4,5-Difluor-3-methyl-2,3-dihydro-1H-indol aus 1.50 g (8.87 mmol) 4,5-Difluor-1,3-dihydro-indol-2-on hergestellt.

| | |
|---|---|
| Ausbeute: | 320 mg (41% d.Th.) |
| ESI-MS: | m/z = 170 (M+H)⁺ |
| Rₜ (HPLC-MS): | 2.97 min (Methode E) |

### Intermediat 52:

### 5-Fluor-3-(2-methoxy-ethyl)-3-methyl-2,3-dihydro-1H-indol

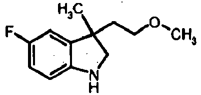

### Stufe 1 5-Fluor-3-methyl-1,3-dihydro-indol-2-on

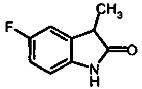

3.0 g (20 mmol) 5-Fluor1,3-dihydro-indol-2-onl wurden mit 2.0 g Raney-Nickel in 50 mL Methanol 1.5 h bei 200°C in einem Autoklaven gerührt. Der Katalysator wurde abgefiltert und das Filtrat wurde zur Trockene einrotiert. Der Rückstand wurde aus Methanol umkristallisiert, abgesaugt und getrocknet.

| | |
|---|---|
| Ausbeute: | 2.8 g (85% d.Th.) |
| ESI-MS: | m/z = 166 (M+H)⁺ |
| Rₜ (HPLC-MS): | 1.1 min (Methode C) |

### Stufe 2 5-Fluor-3-(2-methoxy-ethyl)-3-methyl-1,3-dihydro-indol-2-on

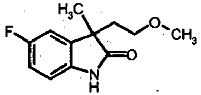

0.50 mg (3.0 mmol) 5-Fluor-3-methyl-1,3-dihydro-indol-2-on wurden unter einer Stickstoff-Atmosphäre 10 mL DMF vorgelegt. 0.13 g (3.0 mmol) Natriumhydrid (55%) wurden bei 0°C zugegeben und das Reaktionsgemisch wurde 30 min gerührt. Anschließend wurden 0.28 mL (3 mmol) 2-(Brommethyl)-methylether in 1.0 mL DMF zugetropft. Das Reaktionsgemisch wurde über Nacht bei RT gerührt. Die Substanz wurde mittels HPLC gereinigt und die Produkt enthaltenden Fraktionen wurden vereinigt und zur Trockne einrotiert.

| | |
|---|---|
| Ausbeute: | 130 mg (19% d.Th.) |
| ESI-MS: | m/z = 224 (M+H)⁺ |
| Rₜ (HPLC-MS): | 1. 24 min (Methode C) |

### Stufe 3 5-Fluor-3-(2-methoxy-ethyl)-3-methyl-2,3-dihydro-1H-indol

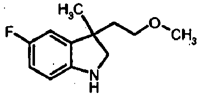

Unter einer Argon-Atmosphäre wurden 0.12 g (0.54 mmol) 5-Fluor-3-(2-methoxy-ethyl)-3-methyl-1,3-dihydro-indol-2-on in 20 mL THF vorgelegt. 0.56 mL (0.56 mmol) einer 1 M Lithiumaluminiumhydrid-Lösung in 10 mL THF wurden zugetropft. Das Reaktionsgemisch wurde 1 h bei 70°C gerührt, anschließend mit Wasser versetzt und getrocknet, gefiltert und zur Trockene einrotiert.

| | |
|---|---|
| Ausbeute: | 160 mg (quantitativ) |
| ESI-MS: | m/z = 210 (M+H)⁺ |
| Rₜ (HPLC-MS): | 0.94 min (Methode C) |

### Intermediat 53:

### 5'-Cyano-4-(7-methoxy-2-oxo-1,2,4,5-tetrahydro-benzo[d][1,3]diazepin-3-yl)-3,4,5,6-tetrahydro-2H-[1,2']-bipyridinyl-4'-carbonsäure

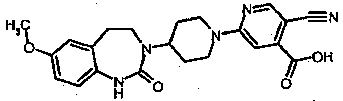

### Stufe 1 4'-Chlor-4-(7-methoxy-2-oxo-1,2,4,5-tetrahydro-benzo[d][1,3]diazepin-3-yl)-3,4,5,6-tetrahydro-2H-[1,2']bipyridinyl-5'-carbonitril

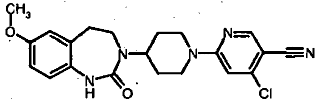

3.00 g (10.9 mmol) 7-Methoxy-3-piperidin-4-yl-1,3,4,5-tetrahydro-1,3-benzodiazepin-2-on und 7.59 mL (43.6 mmol) DIPEA wurden in 90 mL Ethanol vorgelegt. 1.89 g (10.9 mmol) 4,6-Dichlor-nicotinonitril und 3 Spatelspitzen DMAP wurden zugegeben und das Reaktionsgemisch wurde 4 h bei RT gerührt. Der ausgefallene Feststoff wurde abgesaugt und getrocknet.

| | |
|---|---|
| Ausbeute: | 3.70 g (82% d.Th.) |
| Rₜ (HPLC-MS): | 1.44 min (Methode C) |

### Stufe 2 5'-Cyano-4-(7-methoxy-2-oxo-1,2,4,5-tetrahydro-benzo[d][1,3]diazepin-3-yl)-3,4,5,6-tetrahydro-2H-[1,2']bipyridinyl-4'-carbonsäuremethylester

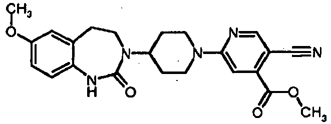

3.70 g (8.98 mmol) 6'-Chlor-4-(7-methoxy-2-oxo-1,2,4,5-tetrahydro-1,3-benzodiazepin-3-yl)-3,4,5,6-tetrahydro-2H-[1,4']bipyridinyl-3'-carbonitril, 345 mg (0.900 mmol) PdCl₂(PhCN)₂, 498 mg (0.900 mmol) dppf und 1.52 mL (10.8 mmol) TEA wurden in 100 mL Methanol unter einer Kohlenmonoxid-Atmosphäre 4 h bei 130°C und 25 bar gerührt. Der Katalysator wurde abgesaugt und das Filtrat wurde zur Trockne einrotiert.

| | |
|---|---|
| Ausbeute: | 3.4 g (87% d.Th.) |
| Rₜ (HPLC-MS): | 1.28 min (Methode C) |

### Stufe 3 5'-Cyano-4-(7-methoxy-2-oxo-1,2,4,5-tetrahydro-benzo[d][1,3]diazepin-3-yl)-3,4,5,6-tetrahydro-2H-[1,2']bipyridinyl-4'-carbonsäure

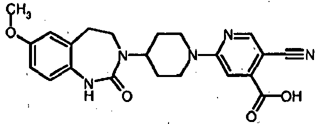

3.40 g (7.81 mmol) 5'-Cyano-4-(7-methoxy-2-oxo-1,2,4,5-tetrahydro-1,3-benzodiazepin-3-yl)-3,4,5,6-tetrahydro-2H-[1,4']bipyridinyl-2'-carbonsäuremethylester und 6 mL einer 4M NaOH Lösung wurden in 50 mL THF und 6 mL Wasser über Nacht bei RT gerührt. Das organische Lösungsmittel wurde abrotiert und der ausgefallene Feststoff wurde abgesaugt. Das Filtrat wurde mit einer 4M HCl Lösung sauer gestellt und der ausgefallene Feststoff wurde abgesaugt und getrocknet.

| | |
|---|---|
| Ausbeute: | 560 mg (17% d.Th.) |
| Rₜ (HPLC-MS): | 1.27 min (Methode C) |

### Intermediat 54:

### (4-Chlor-pyridin-2-yl)-(4,5-difluor-2,3-dihydro-indol-1-yl)-methanon

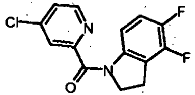

0.50 g (3.2 mmol) 4-Chlorpicolinsäure, 1.1 g (3.4 mmol) TBTU und 0.91 mL (6.5 mmol) TEA wurden in 10 mL DMF vorgelegt. 0.63 g (3.3 mmol) 4,5-Difluorindolin Hydrochlorid wurden zugegeben. Das Reaktionsgemisch wurde über Nacht bei RT gerührt und dann nacheinander mit einer 15%-igen Kaliumcarbonat-Lösung, Wasser, einer 1 M HCl-Lösung und EtOAc extrahiert. Die organische Phase wurde getrocknet, filtriert und zur Trockene einrotiert. Der Rückstand wurde mit Diisopropylether verrührt und der ungelöste Feststoff wurde abgesaugt und getrocknet.

| | |
|---|---|
| Ausbeute: | 850 mg (91% d.Th.) |
| ESI-MS: | m/z = 295/297 (Cl) (M+H)⁺ |
| Rₜ (HPLC-MS): | 1.56 min (Methode C) |

### Intermediat 55:

### (2-Chlor-pyridin-4-yl)-(4,5-difluor-2.3-dihydro-indol-1-yl)-methanon

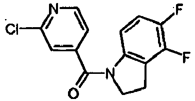

Analog zu (4-Chlor-pyridin-2-yl)-(4,5-difluor-2,3-dihydro-indol-1-yl)-methanon wurde diese Verbindung aus 500 mg (3.17 mmol) 2-Chlorisonicotinsäure und 1.0 eq 4,5-Difluorindolin Hydrochlorid hergestellt.

| | |
|---|---|
| Ausbeute: | 900 mg (96% d.Th.) |
| ESI-MS: | m/z = 295/297 (Cl) (M+H)⁺ |
| Rₜ (HPLC-MS): | 1.44 min (Methode C) |

### Intermediat 56:

### (2-Chlor-6-methoxy-pyridin-4-yl)-(5-fluor-2,3-dihydro-indol-1-yl)-methanon

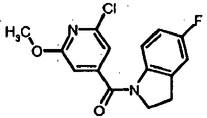

0.50 g (2.7 mmol) 2-Chlor-6-methoxylsonicotinsäure, 0.37 g (2.7 mmol) 5-Fluorindolin und 0.42 mL (3 mmol) TEA wurden in 10 mL DMF vorgelegt. 0.97 g (3.0 mmol) TBTU wurden zugegeben und das Reaktionsgemisch wurde 2 h bei RT gerührt. Die Substanz wurde mittels HPLC gereinigt. Die Produkt enthaltenden Fraktionen wurden vereinigt und gefriergetrocknet.

| | |
|---|---|
| Ausbeute: | 700 mg (86% d.Th.) |
| ESI-MS: | m/z = 307/309 (Cl) (M+H)⁺ |
| Rₜ (HPLC-MS): | 1.6 min (Methode C) |

### Intermediat 57:

### (2-Chlor-6-methoxy-pyridin-4-yl)-(4,5-difluor-2,3-dihydro-indol-1-yl)-methanon

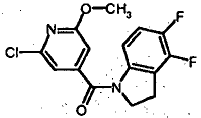

Zu 0.69 g (3.7 mmol) 2-Chlor-6-methoxyisonicotinsäure, 0.70 g (3.7 mmol) 4,5-Fluorindolin-Dihydrochlorid und 1.1 mL (8.0 mmol) Triethylamin in 10 mL DMF wurden bei RT 1.2 g (3.8 mmol) TBTU hinzugefügt. Der Ansatz wurde 2 h bei RT gerührt und anschließend auf 200 mL einer 15%-igen wässrigen Kaliumcarbonat-Lösung gegossen. Der ausgefallene Niederschlag wurde abgesaugt, mit Wasser gewaschen und getrocknet.

| | |
|---|---|
| Ausbeute: | 1.05 g (89% d.Th.) |
| ESI-MS: | m/z = 325 / 327 (M+H)⁺ (Cl) |
| Rₜ(HPLC): | 1.66 min (Methode C) |

### Intermediat 58:

### 4-[4-(7-Methoxy-2-oxo-1,2,4,5-tetrahydro-1,3-benzodiazepin-3-yl)-piperidin-1-yl]-[1,3,5]triazin-2-carbonsäure

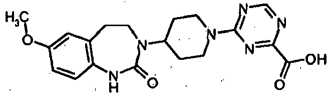

### Stufe 1 3-[1-(4-Chlor-[1,3,5]triazin-2-yl)-piperidin-4-yl]-7-methoxy-1,3,4,5-tetrahydro-1,3-benzodiazepin-2-on

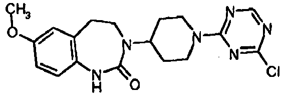

1.84 g (6.67 mmol) 7-Methoxy-3-piperidin-4-yl-1,3,4,5-tetrahydro-1,3-benzodiazepin-2-on und 4.54 mL (26.7 mmol) DIPEA wurden in 50 mL Ethanol vorgelegt. 1.00 g (6.67 mmol) 2,4-Dichlor-[1,3,5]triazin wurden zugegeben und das Reaktionsgemisch über Nacht bei RT gerührt. Der ausgefallene Feststoff wurde abgesaugt und getrocknet,

| | |
|---|---|
| Ausbeute: | 1.76 g (68% d.Th.) |
| ESI-MS: | m/z = 389 (M+H)⁺ |
| Rₜ (HPLC-MS): | 1.35min (Methode C) |

### Stufe 2 4-[4-(7-Methoxy-2-oxo-1,2,4,5-tetrahydro-1,3-benzodiazepin-3-yl)-piperidin-1-yl]-[1,3,5]triazin-2-carbonsäuremethylester

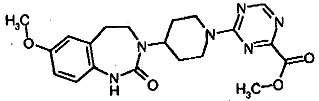

In einer CO-Atmosphäre wurden 400 mg (1.03 mmol) 3-[1-(4-Chlor-[1,3,5]triazin-2-yl)-piperidin-4-yl]-7-methoxy-1,3,4,5-tetrahydro-1,3-benzodiazepin-2-on, 39.5 mg (0.1 mmol) PdCl₂(PhCN)₂, 57.0 mg (0.1 mmol) dppf und 0.173 mL (1.23 mmol) TEA in 30 mL Methanol 16 h bei 130°C carbonyliert. Der Katatlysator wurde abgesaugt und das Filtrat wurde zur Trockene einrotiert. Der Rückstand wurde mit Isopropanol versetzt und der ausgefallene Feststoff wurde abgesaugt und getrocknet.

| | |
|---|---|
| Ausbeute: | 265 mg (63% d.Th.) |
| ESI-MS: | m/z = 413 (M+H)⁺ |
| Rₜ (HPLC-MS): | 1.22min (Methode C) |

### Stufe 3 4-[4-(7-Methoxy-2-oxo-1,2,4,5-tetrahydro-1,3-benzodiazepin-3-yl)-piperidin-1-yl][1,3,5]triazin-2-carbonsäure

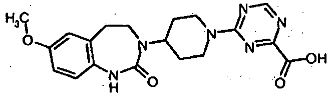

0.27 g (0.64 mmol) 4-[4-(7-Methoxy-2-oxo-1,2,4,5-tetrahydro-1,3-benzodiazepin-3-yl)-piperidin-1-yl]-[1,3,5]triazin-2-carbonsäuremethylester und 0.5 mL (2 mmol) einer 4M NaOH Lösung wurden in 0.5 mL Wasser und 4.0 mL THF 3 Tage bei RT gerührt. Das organische Lösungsmittel wurde abrotiert und das Reaktionsgemisch mit 0.5 mL einer 4M HCl Lösung versetzt. Der ausgefallene Feststoff wurde abgesaugt und getrocknet.

| | |
|---|---|
| Ausbeute: | 210 mg (82% d.Th.) |
| Rₜ (HPLC-MS): | 0.94 min (Methode C) |

### Intermediat 59:

### 6-[4-(7-Methoxy-2-oxo-1,2,4,5-tetrahydro-1,3-benzodiazepin-3-yl)-piperidin-1-yl]-5-methyl-pyrimidin-4-carbonsäure

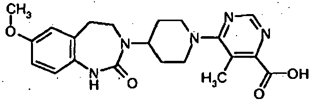

### Stufe 1 6-[4-(7-Methoxy-2-oxo-1,2,4,5-tetrahydro-1,3-benzodiazepin-3-yl)-piperidin-1-yl]-5-methyl-pyrimidin-4-carbonsäure-tert-butylester

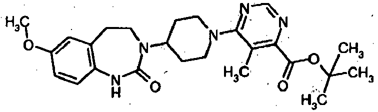

0.20 g (0.73 mmol) 7-Methoxy-3-piperidin-4-yl-1,3,4,5-tetrahydro-1,3-benzodiazepin-2-on und 0.14 mL (0.80 mmol) DIPEA wurden in 3.0 mL DMF vorgelegt. 0.17 g (0.74 mmol) 6-Chlor-5-methyl-pyrimidin-4-carbonsäure-*tert*-butylester wurden zugegeben und das Reaktionsgemisch wurde 3 h bei RT gerührt. Das Reaktionsgemsich wurde mit Wasser versetzt und mit DCM extrahiert. Die organische Phase wurde getrocknet, filtriert und zur Trockene einrotiert. Der Rückstand wurde mit Diisopropylether versetzt und der ausgefallene Feststoff wurde abgesaugt und getrocknet.

| | |
|---|---|
| Ausbeute: | 140 mg (41% d.Th.) |
| ESI-MS: | m/z = 468 (M+H)⁺ |
| Rₜ (HPLC-MS): | 1.95 min (Methode C) |

### Stufe 2 6-[4-(7-Methoxy-2-oxo-1,2,4,5-tetrahydro-1,3-benzodiazepin-3-yl)-piperidin-1-yl]-5-methyl-pyrimidin-4-carbonsäure

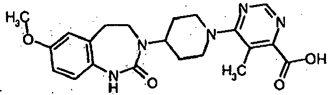

70 mg (0.17 mmol) 6-[4-(7-Methoxy-2-oxo-1,2,4,5-tetrahydro-1,3-benzodiazepin-3-yl)-piperidin-1-yl]-5-methyl-pyrimidin-4-carbonsäure-*tert-*butylester, 26 mg (0.19 mmol) 5-Fluorindolin, 61 mg (0.19 mmol) TBTU und 27 µL (0.19 mmol) TEA wurden in 1.0 mL DMF 3 h bei RT gerührt. Der Reaktionsansatz wurde mittels HPLC gereinigt. Die Produkt enthaltenden Fraktionen wurden vereinigt und gefriergetrocknet.

| | |
|---|---|
| Ausbeute: | 58mg (64% d.Th.) |
| ESI-MS: | m/z = 531 (M+H)⁺ |
| Rₜ (HPLC-MS): | 1.45 min (Methode C) |

### Intermediat 60:

Isomerengemisch aus 5'-Cyano-4-(2-oxo-2,3-dihydro-imidazo[4,5-b]pyridin-1-yl)-3,4,5,6-tetrahydro-2H-[1,2']bipyridinyl-4'-carbonsäure und 5'-Cyano-4-(2-oxo-2,3-dihydro-imidazo[4,5-b]pyridin-1-yl)-3,4,5,6-tetrahydro-2H-[1,4']bipyridinyl-2'-carbonsäüre

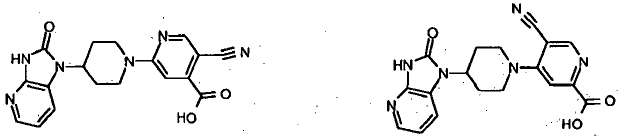

### Stufe 1: Isomerengemisch aus 4'-Chlor-4-(2-oxo-2,3-dihydro-imidazo[4,5-b]pyridin-1-yl)-3,4,5,6-tetrahydro-2H-[1,2']bipyridinyl-5'-carbonitril und 6'-Chlor-4-(2-oxo-2,3-dihydro-imidazo[4,5-b]pyridin-1-yl)-3,4,5,6-tetrahydro-2H-[1,4']bipyridinyl-3'-carbonitril

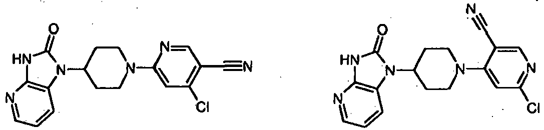

1.50 g (5.15 mmol) 1-Piperidin-4-yl-1,3-dihydroimidazo[4,5-b]pyridin-2-on-Dihydrochlorid und 3.59 mL (20.6 mmol) DIPEA wurden in 45 mL Ethanol vorgelegt. 891 mg (5.00 mmol) 4,6-Dichlor-nicotinnitril und 3 Spateispitzen DMAP wurden zugegeben und das Reaktionsgemisch wurde 4 h bei RT gerührt. Der ausgefallene Feststoff wurde abgesaugt, mit Ethanol gewaschen und getrocknet.

| | |
|---|---|
| Ausbeute: | 1.41 g (77% d.Th.) |
| ESI-MS: | m/z = 355/357 (Cl) (M+H)⁺ |
| Rₜ (HPLC-MS): | 1.15 min (Methode C) |

### Stufe 2 isomerengemisch aus 5'-Cyano-4-(2-oxo-2,3-dihydro-imidazo[4,5-b]pyridin-1-yl)-3,4,5,6-tetrahydro-2H-[1,2']bipyridinyl-4'-carbonsäuremethylester und 5'-Cyano-4-(2-oxo-2,3-dihydro-imidazo[4,5-b]pyridin-1-yl)-3,4,5,6-tetrahydro-2H-[1,4']bipyridinyl-2'-carbonsäuremethylester

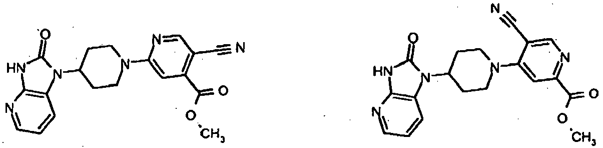

In einer CO-Atmosphäre wurden 717 mg (2.02 mmol) eines Isomerengemischs aus 4'-Chlor-4-(2-oxo-2,3-dihydro-imidazo[4,5-b]pyridin-1-yl)-3,4,5,6-tetrahydro-2H-[1,2']bipyridinyl-5'-carbonitril und 6'-Chlor-4-(2-oxo-2,3-dihydro-imidazo[4,5-b]pyridin-1-yl)-3,4,5,6-tetrahydro-2H-[1,4']bipyridinyl-3'-carbonitril, 78 mg (0.20 mmol) PdCl₂(PhCN)₂, 112 mg (0.20 mmol) dppf und 0.34 mL (2.4 mmol) TEA in 30 mL Methanol 4 h bei 130°C und 25 bar carbonyliert. Der Katalysator wurde abgesaugt und das Filtrat wurde zur Trockene einrotiert. Der Rückstand wurde mit Isopropanol versetzt und der ausgefallene Feststoff wurde abgesaugt und getrocknet.

| | |
|---|---|
| Ausbeute: | 112 mg (15% d.Th.) |
| Rₜ (HPLC-MS): | 1.05 min (Methode C) |

### Stufe 3 Isomerengemisch aus 5'-Cyano-4-(2-oxo-2,3-dihydro-imidazo[4,5-b]pyridin-1-yl)-3,4,5,6-tetrahydro-2H-[1,2']bipiridinyl-4'-carbonsäure und 5'-Cyano-4-(2-oxo-2,3-dihydro-imidazo[4,5-b]pyridin-1-yl)-3,4,5,6-tetrahydro-2H-[1,4']bipyridinyl-2'-carbonsäure

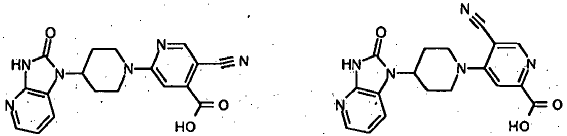

272 mg (0.720 mmol) eines Isomerengemischs aus 5'-Cyano-4-(2-oxo-2,3-dihydroimidazo[4,5-b]pyridin-1-yl)-3,4,5,6-tetrahydro-2H-[1,2']bipyridinyl-4'-carbonsäure-methylester und 5'-Cyano-4-(2-oxo-2,3-dihydro-imidazo[4,5-b]pyridin-1-yl)-3,4,5,6-tetrahydro-2H-[1,4']bipyridinyl-2'-carbonsäure methyl ester wurden in 4.0 mL THF, 0.54 mL (2.1 mmol) einer 4M NaOH Lösung und 0.54 mL Wasser über Nacht bei RT gerührt. Das organische Lösungsmittel wurde abrotiert und die wässrige Phase wurde mit 50 mL Wasser und 25 mL einer 4M HCl Lösung versetzt. Das Reaktionsgemisch wurde eine Stunde bei RT gerührt, anschließend wurde der ausgefallene Feststoff abgesaugt und getrocknet.

| | |
|---|---|
| Ausbeute: | 210 mg (80% d.Th.) |
| ESI-MS: | m/z = 365 (M+H)⁺ |
| Rₜ (HPLC-MS): | 3.65 min (Methode C) |

### Intermediat 61:

### 4-(2-Oxo-2,3-dihydro-imidazo[4_{,}5-b]pyridin-1-yl)-3,4,5,6-tetrahydro-2H-[1,2']bipyridinyl-4'-carbonsäure

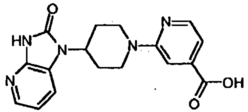

0.50 g (2.4 mmol) 2-Brompyridin-4-carbonsäure und 1.1 g (5.0 mmol) 1-Piperidin-4-yl-1,3-dihydro-imidazo[4,5-b]pyridin-2-on wurden gemischt und mit dem Heissluftfön 10 min geschmolzen. Das Reaktionsgemisch wurde abgekühlt, mit Wasser versetzt und mit Ammoniak basisch gestellt. Es wurde mit EtOAc extrahiert. Die wässrige Phase wurde aufkonzentriert und mittels HPLC gereinigt. Die Produkt enthaltenden Fraktionen wurden vereinigt und zur Trockene einrotiert.

| | |
|---|---|
| Ausbeute: | 200 mg (25% d.Th.) |
| ESI-MS: | m/z = 340 (M+H)⁺ |
| Rₜ(HPLC-MS): | 0.74 min (Methode C) |

### Intermediat 62:

### 6-[4-(2-Oxo-1,2-dihydro-chinolin-3-yl)-piperidin-1-yl]-pyrimidin-4-carbonsäure

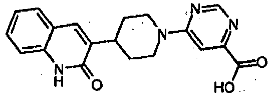

### Stufe 1: 1-Benzyl-4-(2-chlor-chinolin-3-yl)-piperidin-4-ol

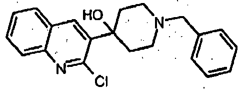

Unter einer Argon-Atmosphäre wurde bei -78°C zu 68.0 mL (136 mmol) einer 2M Lithiumdiisopropylamid (in THF) Lösung in 280 mL THF 22.3 g (136 mmol) 2-Chlorchinolin in 60 mL THF langsam zugetropft. Das Reaktionsgemisch wurde 1 h bei -78°C gerührt und anschließend wurden 24.3 mL (136 mmol) N-Benzylpiperidon in 50 mL THF zugetropft. Das Reaktionsgemisch wurde 40 min bei -70°Cund 3h bei RT gerührt Der Reaktionsansatz wurde auf -20°C abgekühlt, und 200 mL Wasser wurden zugetropft. Der Reaktionsansatz wurde auf RT kommen gelassen und mit EtOAc extrahiert. Die organische Phase wurde getrocknet, gefiltert und zur Trockene einrotiert. Das Produkt wurde mittels Alox-Säule gereinigt. Die Produkt enthaltenden Fraktionen wurden vereinigt und zur Trockne einrotiert.

| | |
|---|---|
| Ausbeute: | 15.5 g (32% d.Th.) |
| ESI-MS: | m/z = 353 (M+H)⁺ |
| Rₜ (HPLC-MS): | 1.05 min (Methode C) |

### Stufe 2: 3-(1-Benzyl-1,2,3,6-tetrahydro-pyridin-4-yl-chinolin-2-ol

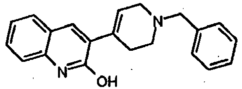

15.5 g (43.9 mmol) 1-Benzyl-4-(2-chlor-chinolin-3-yl)-piperidin-4-ol wurden in 150 mL einer 6M wässrigen Salzsäure-Lösung 8 h refluxiert. Zum Reaktionsgemisch wurden 100 mL Wasser getropft und der ausgefallene Feststoff wurde abgesaugt, getrocknet und anschließend in 150 mL einer 15%igen, wässrigen Kaliumcarbonat-Lösung verrührt. Nach Absaugen des Niederschlags wurde das Produkt als freie Base erhalten und getrocknet.

| | |
|---|---|
| Ausbeute: | 6.20 g (45% d.Th.) |
| ESI-MS: | m/z = 317 (M+H)⁺ |
| Rₜ(HPLC-MS): | 1.00 min (Methode C) |

### Stufe 3: 3-Piperidin-4-yl-1H-chinolin-2-on

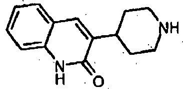

5.70 g (18.0 mmol) 3-(1-Benzyl-1,2,3,6-tetrahydro-pyridin-4-yl)-chinolin-2-ol wurden in 200 mL Methanol vorgelegt. 1.00 g Palladium auf Kohle (10%) wurden hinzugefügt und der Reaktionsansatz wurde 3 h bei 50°C unter einer Wasserstoff-Atmosphäre hydriert. Der Katalysator wurde abgesaugt und die Mutterlauge wurde zur Trockene einrotiert. Der Rückstand wurde mit Diethylether verrieben und der ungelöste Feststoff wurde abgesaugt und getrocknet.

| | |
|---|---|
| Ausbeute: | 3.7 g (90% d.Th.) |
| ESI-MS: | m/z = 229 (M+H)⁺ |
| Rₜ (HPLC-MS): | 0.77 min (Methode C) |

### Stufe 4: 6-[4-(2-Oxo-1,2-dihydro-chinolin-3-yl)-piperidin-1-yl]-pyrimidin-4-carbonsäureethylester

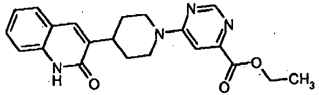

730 mg (3.91 mmol) 6-Chlorpyrimidin-4-carbonsäureethylester wurden in 10 mL DMF vorgelegt. 900 mg (3.94 mmol) 3-Piperidin-4-yl-1,2-dihydro-chinolin-2-ol und 2.30 mL (13.4 mmol) DIPEA wurden zugegeben und das Reaktionsgemisch über Nacht bei RT gerührt. Das Reaktionsgemisch wurde mit 60 mL Wasser versetzt und 30 min gerührt. Der ausgefallene Feststoff wurde abgesaugt und getrocknet.

| | |
|---|---|
| Ausbeute: | 1.15 g (78% d.Th.) |
| ESI-MS: | m/z = 379 (M+H)⁺ |
| Rₜ (HPLC-MS): | 2.93 min (Methode E) |

### Stufe 5: 6-[4-(2-Oxo-1,2-dihydro-chinolin-3-yl)-piperidin-1-yl]-pyrimidin-4-carbonsäure

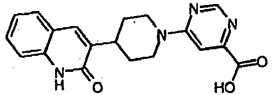

1.10 g (2.91 mmol) 6-[4-(2-Hydroxy-1,2-dihydro-chinolin-3-yl)-piperidin-1-yl]-pyrimidin-4-carbonsäure-ethylester wurden in 20 mL THF, 1.5 mL Wasser und 1.5 mL einer 4 M NaOH Lösung über Nacht bei RT gerührt. Das organische Lösungsmittel wurde abrotiert und der ausgefallene Feststoff abgesaugt. Das Filtrat wurde mit einer 4 M HCl Lösung sauer gestellt und der ausgefallene Feststoff wurde abgesaugt und getrocknet.

| | |
|---|---|
| Ausbeute: | 1 g (98% d.Th.) |
| ESI-MS: | m/z = 351(M+H)⁺ |
| Rₜ (HPLC-MS): | 0.98 min (Methode C) |

### Intermediat 63:

### 6-Methoxy-3-piperidin-4-yl-1H-chinolin-2-on

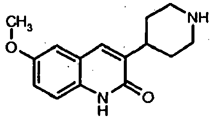

### Stufe 1: 1-Benzyl-4-(2-chlor-6-methoxy-chinolin-3-yl)-piperidin-4-ol

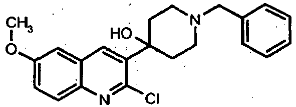

Unter einer Argon-Atmosphäre wurden bei -78°C zu 14 mL (28 mmol) einer 2 M Lithiumdiisopropylamid-Lösung (in THF) in 50 mL THF 5.0 g (25 mmol) 2-Chlor-6-methoxychinolin in einer geringen Menge THF zugetropft. Im Anschluss wurde 1.5 h bei -78°C nachgerührt und 4.5 mL (25 mmol) N-Benzylpiperidon zugetropft. Es wurden weitere 15 min unter Kühlen nachgerührt bevor langsam auf RT erwärmt wurde. Der Reaktionsansatz wurde über Nacht nachgerührt, eingeengt und mittels flash-Chromatographie über Aluminiumoxid gereinigt. Die Produkt enthaltenden Fraktionen wurden vereinigt und eingeengt.

| | |
|---|---|
| Ausbeute: | 2.1 g (13% d.Th.) |
| ESI-MS: | m/z = 383 (M+H)⁺ |
| Rₜ(HPLC): | 1.14 min (Methode C) |

### Stufe 2: 3-(1-Benzyl-1,2,3,6-tetrahydro-pyridin-4-yl)-6-methoxy-chinolin-2-ol

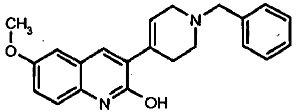

1.90 g (4.96 mmol) 1-Benzyl-4-(2-chlor-6-methoxy-chinolin-3-yl)-piperidin-4-ol wurden min 25 mL einer 4N wässrigen Salzsäure-Lösung über Nacht bei 100°C gerührt. Anschließend wurden 15 mL einer konzentrierten wässrigen Salzsäure-Lösung langsam zugetropft und nochmals über Nacht gerührt. Nach Einengen des Reaktionsansatzes auf die Hälfte wurde dieser mit Wasser verdünnt und mit EtOAc extrahiert. Die organische Phase wurde über Natriumsulfat getrocknet, filtriert und eingeengt. Der Rückstand wurde mit PE/EtOAc verrieben.

| | |
|---|---|
| Ausbeute: | 165 mg (8% d.Th.) |
| ESI-MS: | m/z = 347 (M+H)⁺ |

### Stufe 3: 6-Methoxy-3-piperidin₋4-yl-1H-chinolin-2-on

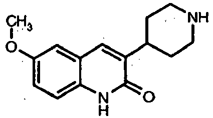

In einer Wasserstoff-Atmophäre wurden 160 mg (0.46 mmol) 3-(1-Benzyl-1,2,3,6-tetra-hydro-pyridin-4-yl)-6-methoxy-chinolin-2-ol und 20 mg Palladium auf Kohle (10%) in 30 mL MeOH 17.5 h bei 50°C bei 50 psi hydriert. Anschließend wurden nochmals 10 mL THF und Katalysator zugefügt und weitere 2 h hydriert. Es wurde abermals Katalysator zugegeben und über Nacht bei 50°C in einer Wasserstoff-Atmosphäre von 60 psi hydriert. Nach Filtration des Reaktionsgemisches wurde der Filterkuchen mit DMF nachgewaschen und das Filtrat eingeengt. Der Rückstand wurde in EtOAc gegeben, mit PE verrieben und filtriert. Der Niederschlag wurde mit Diisopropylether nachgewaschen und getrocknet.

| | |
|---|---|
| Ausbeute: | 56 mg (35% d.Th.) |
| ESI-MS: | m/z = 259 (M+H)⁺ |
| Rₜ(HPLC): | 0.90 min (Methode C) |

### Intermediat 64:

### 7.7-Dimethyl-3-trifluormethyl-4.5,6,7-tetrahvdro-1H-pyazolo[4.3-c]pyridin

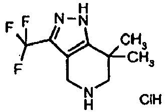

### Stufe 1: 3.3-Dimethyl-4-oxo-piperidin-1-carbonsäure-tert-butylester

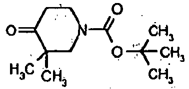

20 g (0.10 mol) 4-Oxo-piperidin-1-carbonsäure-tert-butylester in 500 mL THF wurden auf 0°C abgekühlt und portionsweise mit 8.2 g (0.21 mol) Natriumhydrid (55%) versetzt. Der Reaktionsansatz wurde 15 min nachgerührt bevor 15 mL (0.24 mol) lodmethan zugetropft wurden und über Nacht bei RT nachgerührt wurde. Anschließend wurde der Reaktionsansatz eingeengt und der Rückstand in Diethylether aufgenommen. Die organische Phase wurde mit Wasser und gesättigter Natriumchlorid-Lösung gewaschen, getrocknet und eingeengt. Der Rückstand wurde aus PE umkristallisiert.

| | |
|---|---|
| Ausbeute: | 10.05 g (37% d.Th.) |
| ESI-MS: | m/z = 172 (M-tert.Butyl+H)⁺ |
| Rₜ (HPLC-MS): | 1.44 min (Methode C) |

### Stufe 2: 3.3-Dimethvl-4-oxo-5-(2,2,2-trifluor-acetyl)-piperidin-1-carbonsäure-tert-butylester

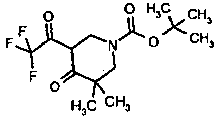

Unter einer Stickstoff-Atmosphäre wurden zu 1.00 g (3.96 mmol) 3,3-Dimethyl-4-oxo-piperidin-1-carbonsäure-tert-butylester in 10 mL Toluol bei 0°C 4.20 mL (4.2 mmol) einer 1 M Lithiumbis(trimethylsilyl)amid-Lösung zugegeben, 1 min nachgerührt und anschließend 0.56 mL (4.00 mmol) Trifluoressigsäureanhydrid hinzugefügt. Das Kältebad wurde entfernt, 2 min nachgerührt und mit 10 mL Wasser und 1.2 mL Essigsäure versetzt. Der Reaktionsansatz wurde 15 min gerührt. Nach dem Extrahieren mit Diethylether wurde die organische Phase getrocknet und eingeengt. Der Rückstand wurde mittels flash-Chromatographie gereinigt.

| | |
|---|---|
| Ausbeute: | 300 mg (23% d.Th.) |
| ESI-MS: | m/z = 322 (M-H)⁻ |
| Rₜ (HPLC-MS): | 1.84 min (Methode C) |

### Stufe 3: 7,7-Dimethvl-3-trifluormethyl-1.4.6.7-tetrahydro-pyrazolo[4,3-c]pyridin-5-carbonsäure-tert-butylester

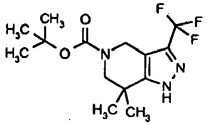

480 mg (1.41 mmol) 3,3-Dimethyl-4-oxo-5-(2,2,2-trifluor-acetyl)-piperidin-1-carbonsäuretert-butylester in 5.00 mL EtOH wurden mit 0.15 mL Hydrazinhydrat, 0.18mL Essigsäure und 1.00 g Molsieb (3A) versetzt und 48 h bei RT stehen gelassen. Anschließend wurde der Reaktionsansatz 3 h unter Rückfluss gekocht. und über Nacht abgekühlt. Zusätzlich wurde noch eine Spatelspitze p-Toluölsulfonsäure zugefügt und zunächst 1 h und dann noch 3 h unter Rückfluss gekocht. Nach dem Abkühlen wurde der Reäktiönsansatz filtriert und eingeengt. Der Rückstand wurde mittels flash-Chromatographie gereinigt. Es entstand ein Gemisch aus 7,7-Dimethyl-3-trifluormethyl-1,4,6,7-tetrahydro-pyrazol[4,3-c]-pyridin-5-carbonsäure-tert-butylester und 4-Hydrazon-3,3-dimethyl-5-(2,2,2-trifluor-acetyl)-piperidin-1-carbonsäure-tert-butylester im Verhältnis 3:2.

| | |
|---|---|
| Ausbeute: | 370 mg (37% d.Th.) |
| ESI-MS: | m/z = 318 (M-H)⁻ |

### Stufe 4: 7,7-Dimethyl-3-trifluormethyl-4,5,6,7-tetrahydro-1H-pyrazolo[4.3-c]-pyridin-Hydrochlorid

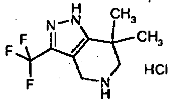

0.37 g (0.52 mmol) eines Gemischs aus 7,7-Dimethyl-3-trifluormethyl-1,4,6,7-tetrahydro-pyrazolo[4,3-c]pyridin-5-carbonsäure-tert-butylester und 4-Hydrazon-3,3-dimethyl-5-(2,2,2-trifluor-acetyl)-piperidin-1-carbonsäure-tert-butylester in 8.0 mL DCM wurde mit 4.0 mL Trifluoressigsäure versetzt und 2 h bei RT gerührt. Anschließend wurde der Reaktionsansatz eingeengt, der Rückstand in EtOH gelöst, mit 0.90 mL (1.1 mmol) einer 1.25 molaren ethanolischen Salzsäure versetzt und nochmals cooevaporiert. Der Rückstand wurde mit Diethylether verrieben, abgesaugt und getrocknet.

| | |
|---|---|
| Ausbeute: | 270 mg (77% d.Th.) |
| ESI-MS: | m/z = 220 (M+H)⁺ |
| Rₜ (HPLC-MS): | 0.43 min (Methode R) |

### Intermediat 65:

### 4.4-Dimethyl-4,5,6,7-tetrahydro-thieno[2.3-c]pyridin

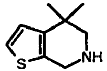

### Stufe 1: Methylen-2-methyl-2-thiophen-3-yl-propyl)-amin

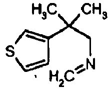

5.8 g (37 mmol) 2-Methyl-2-thiophen-3-yl-propylamin und 3.6 mL (44 mmol) Formaldehyd wurden zusammen mit 2.0 g Molsieb (4Ǻ Pulver) über Nacht bei RT gerührt. Das Reaktionsgemisch wurde filtriert und das Filtrat wurde zur Trockne einrotiert.

| | |
|---|---|
| Ausbeute: | 6.0 g (97% d.Th.) |
| ESI-MS: | m/z = 168 (M+H)⁺ |

### Stufe 2: 4,4-Dimethyl-4,5,6,7-tetrahydro-thieno[2,3-c]pyridin

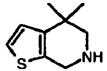

6.0 g (36 mmol) Methylen-(2-methyl-2-thiophen-3-yl-propyl)-amin, 11 mL (45 mmol) einer 4M HCl Lösung und 12 mL (0.14 mol) konz. HCl wurden über das Wochenende bei RT gerührt. Der Reaktionsansatz wurde mit einer 4M Natriumhydroxid-Lösung alkalisch gestellt. Der ausgefallene Niederschlag wurde abgesaugt, mit Wasser gewaschen und getrocknet. Die Substanz wurde über Alox gereinigt. Die Produkt enthaltenden Fraktionen wurden vereint und zur Trockene einrotiert.

| | |
|---|---|
| Ausbeute: | 0.74 g (12% d.Th.) |
| Rₜ (HPLC-MS): | 1.24 min (Methode K) |

### Intermediat 66:

### 5,6-Difluor-2.3-dihydro-1H-indol

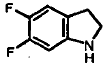

Unter einer Argonatmosphäre wurden 0.30 g (1.8 mmol) 5,6-Difluor-1,3-dihydro-indol-2-on in 10 mL THF gelöst und 3.0 mL einer 1 M Boran in THF Lösung zugetropft. Anschließend wurde der Reaktionsansatz 2 h auf 70°C erwärmt und dann abgekühlt. Nach dem Versetzen mit 3 mL MeOH wurden noch 5 mL einer 4N wässrigen Salzsäure-Lösung zugefügt und 1 h unter Rückfluss gekocht. Die organische Phase wurde eingeengt, die wässrige Phase wurde mit DCM gewaschen und anschließend mit einer 4N wässrigen Natronlauge-Lösung alkalisch gestellt und mehrmals mit EtOAc extrahiert. Die vereinigten organischen Phasen wurden mit Natriumsulfat getrocknet, filtriert und eingeengt.

| | |
|---|---|
| Ausbeute: | 160 mg (47% d.Th.) |
| ESI-MS: | m/z = 156 (M+H)⁺ |
| Rₜ (HPLC-MS): | 0.73 min (Methode C) |

### Intermediat 67:

### (2.3-Dihydro-1H-indo)-3-yl)-methanol

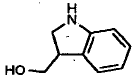

### Stufe 1: 2,3-Dihydro-1H-indol-3-carbonsäureethylester Hydrochlorid

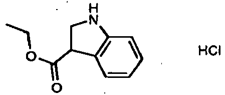

Diese Verbindung wurde analog der WO 2007/054453 synthetisiert.

### Stufe 2: (2.3-Dihydro-1H-indol-3-yl)-methanol

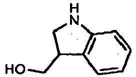

Zu 7.8 mL (7.8 mmol) einer 1 M Lithiumaluminiumhydrid-Lösung (in THF) in 40 mL THF wurden 0.79 g (3.5 mmol) 2,3-Dihydro-1H-indol-3-carbonsäureethylester portionsweise bei RT zugegeben und 1 h unter Rückfluss gekocht. Anschließend wurde der Reaktionsansatz unter Kühlen mit Wasser zersetzt, der entstandene Niederschlag abfiltriert und das Filtrat eingeengt.

| | |
|---|---|
| Ausbeute: | 52 mg (95% d.Th.) |
| ESI-MS: | m/z = 150 (M+H)⁺ |
| Rₜ (HPLC-MS): | 0.31 min (Methode R) |

### Intermediat 68:

### 6-Fluor-2,3-dihydro-1H-indol

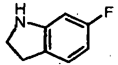

Unter einer Stickstoff-Atmosphäre wurden zu-0.54 g (4.0 mmol) 6-Fluorindol in 5,0 mL Eisessig 0.29 g (4.6 mmol) Natriumcyanoborhydrid portionsweise zugegeben und 30 min bei RT gerührt. Anschließend wurde der Reaktionsansatz auf eine 4N wässrige Natriumhydroxid-Lösung gegossen und mit EtOAc extrahiert. Die vereinigten organischen Phasen wurden mehrmals mit gesättigter Natriumhydrogencarbonat-Lösung gewaschen, über Natriumsulfat getrocknet und eingeengt.

| | |
|---|---|
| Ausbeute: | 0.56, g (97% d.Th.) |
| ESI=MS: | m/z = 138 (M+H)⁺ |
| Rₜ (HPLC-MS): | 0.74 min (Methode C) |

### Intermediat 69:

### 4-Methyl-1,2,3,4-tetrahydro-isochinolin Hydrochlorid

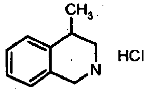

In einer Wasserstoff-Atmosphäre wurden 0.50 g (3.5 mmol) 4-Methyl-isochinolin, 50 mg Platindioxid in 50 mL Methanol und 3.5 mL 1 M wässrige Salzsäure-Lösung bei RT und 3 bar für 4 h hydriert. Nach dem Absaugen des Katalysators wurde der Reaktionsansatz eingeengt. Man erhielt ein Gemisch aus Edukt und Produkt, das ohne weitere Aufreinigung weiter umgesetzt wurde.

| | |
|---|---|
| Ausbeute: | 0.60 g (94% d.Th.) |
| ESI-MS: | m/z = 148 (M+H)⁺ |
| Rₜ (HPLC-MS): | 0.70 min (Methode C) |

### Intermediat 70:

### 3-Methyl-decahydro-chinolin Hydrochlorid,

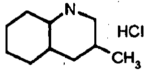

In einer Wasserstoff-Atmosphäre wurden 500 mg (3.5 mmol) 3-Methyl-decahydro-chinolin, 75 mg Platindioxid in 50 mL Methanol und 3.5 mL 1 M wässriger Salzsäure-Lösung bei RT und 50 psi für 4h hydriert. Nach dem Absaugen des Katalysators wurde der Reaktionsansatz eingeengt.

| | |
|---|---|
| Ausbeute: | 0.60 g (94% d.Th.) |
| ESI-MS: | m/z = 148 (M+H)⁺ |
| Rₜ (H PLC-MS): | 0.70 min (Methode C) |

### Intermediat 71:

### 1,2,2a,5-Tetrahydro-3H-pyrrolo[4,3,2-de]chinolin-4-on

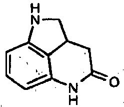

### Stufe 1: Oxo-pyrrolidin-1-yl-essigsäuremethylester

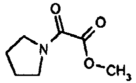

Zu 33 mL (0.40 mmol) Pyrrolidin und 55 mL (0.40 mol) TEA in 500 mL Diethylether wurden 30 mL (0.33 mol) Methyloxalylchlorid in 220 mL Diethylether unter Eisbadkühlung zugetropft. Nach dem Erwärmen auf RT wurde noch 2h bei RT nachgerührt. Der entstandene Niederschlag wurde abgesaugt und das Filtrat eingeengt. Der Rückstand wurde im Hochvakuum fraktioniert destilliert.

| | |
|---|---|
| Ausbeute: | 41.8 g (82% d.Th.) |
| MS: | m/z = 180 (M+Na)⁺ |
| Rₜ: | 0.3 (Kieselgel, PE/EtOAc 1/1) |

### Stufe 2: (4-Nitro-1H-indol-3-yl)-oxo-essigsäuremethylester

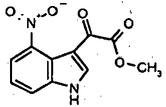

Zu 10 g (62 mmol) 4-Nitroindol und 11 g (68 mmol) Oxo-pyrrolidin-1-yl-essigsäuremethyl-ester wurden unter Rühren und Eiskühlung 9.4 mL (68 mmol) Diphosphorylchlorid langsam zugetropft. Der Reaktionsansatz wurde auf RT erwärmt und 3 h bei RT gerührt. Anschließend wurden zunächst 10 mL MeOH bei 0°C zugetropft und dann gesättigte Natriumhydrogencarbonat-Lösung tropfenweise bei 0°C zugegeben. Nach dem mehrmaligen Extrahieren mit DCM wurde die organische Phase getrocknet und bis auf 100 mL eingeengt. Dieser Rückstand wurde bei RT stehen gelassen und der entstandene Niederschlag wurde abgesaugt, gewaschen und an der Luft getrocknet.

| | |
|---|---|
| Ausbeute: | 2.30 g (15% d.Th.) |
| ESI-MS: | m/z = 249 (M+H)⁺ |
| Rₜ (HPLC-MS): | 1.23 min (Methode C) |

### Stufe 3: (4-Nitro-2,3-dihydro-1H-indol-3-yl)-essigsäuremethylester

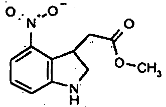

Unter einer Argon-Atmosphäre wurden zu 2.3 g (9.3 mmol) (4-Nitro-1 H-indol-3-yl)-oxo-essigsäuremethylester in 18 mL Trifluoressigsäure unter Eiskühlung 16 mL (0.10 mmol) Triethylsilan langsam zugetropft. Anschließend wurde der Ansatz 3 h bei RT nachgerührt und eingeengt. Der Rückstand wurde getrocknet und anschließend mit Diisopropylether verrieben, abgesaugt und an der Luft getrocknet.

| | |
|---|---|
| Ausbeute: | 2.0 g (91% d.Th.) |
| ESI-MS: | m/z = 237 (M+H)⁺ |
| Rₜ (HPLC-MS): | 1.34 min (Methode C) |

### Stufe 4: (4-Amino-2.3-dihydro-1H-indol-3-yl)essigsäuremethylester

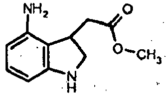

2.0 g (8.5 mmol) (4-Nitro-2,3-dihydro-1H-indol-3-yl)-essigsäuremethylester in 70 mL MeOH wurden mit 0.30 g Raney-Nickel versetzt und 2h in einer Wasserstoffatmosphäre hydriert. Der Katalysator wurde abgesaugt und die Lösung einrotiert. Der Rückstand wurde ohne weitere Aufreinigung sofort weiter umgesetzt.

| | |
|---|---|
| Ausbeute: | 1.80 g (quantitativ) |
| ESI-MS: | m/z = 237 (M+H)⁺ |
| Rₜ (HPLC-MS): | 0.40 min (Methode C) |

### Stufe 5: 1,2,2a,5-Tetrahydro-3H-Pyrrolo[4,3,2-de]chinolin-4-on

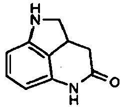

1.80 g (8.73 mmol) (4-Amino-2,3-dihydro-1H-indol-3-yl)essigsäuremethylester in 100 mL Xylol wurden 30 h unter Rückfluss gekocht. Der Reaktionsansatz wurde eingeengt und mittels flash-Chromatographie gereinigt. Die Produkt enthaltenden Fraktionen wurden vereinigt und eingeengt.

| | |
|---|---|
| Ausbeute: | 0.21 g (14% d.Th,) |
| ESI-MS: | m/z = 175 (M+H)⁺ |
| Rₜ (HPLC-MS): | 0.03 min (Methode C) |

### Intermediat 72:

### 5,6,7,8-Tetrahydro-4H-thiazol[4,5-d]azepin Hydrochlorid

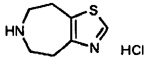

### Stufe 1: 3-Benzvlamino-propionsäureethylester

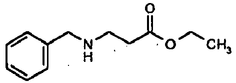

25 g (0.23 mol) Benzylamin und 21 g (0.21 mol) Ethylacrylat in 125 mL EtOH wurden 15 h bei RT gerührt. Anschließend wurde das Lösungsmittel eingeengt und das Rohprodukt ohne weitere Reinigung in die nächste Stufe eingesetzt.

| | |
|---|---|
| Ausbeute: | 30 g (62% d.Th.) |
| ESI-MS: | m/z = 208 (M+H)⁺ |
| R_{f}: | 0.5 (Kieselgel, EtOAc/PE 50%) |

### Stufe 2: 4-[Benzyl-(2-ethoxycarbonyl-ethyl)-amino]-propancarbonsäure ethylester

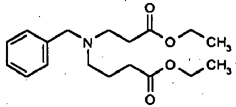

Zu 50 g (0.24 mol) 3-Benzylamino-propionsäureethylester und 83 g (0.60 mol) Kaliumcarbonat in 1.0 L Acetonitril wurden langsam 71 g (0.36 mol) 4-Brombuttersäureethylester bei RT zugetropft. Anschließend wurde der Reaktionsansatz bei 90°C 12 h gerührt. Nach dem Abkühlen wurde der Reaktionsansatz mit EtOAc verdünnt und die organische Phase abgetrennt. Diese wurde mit Wasser und gesättigter Natriumchlorid-Lösung gewaschen und anschließend über Natriumsulfat getrocknet. Nach dem Filtrieren wurde das Filtrat eingeengt und der Rückstand mittels Flash-Chromatographie (über Aluminiumoxid) gereinigt.

| | |
|---|---|
| Ausbeute: | 55 g (68%d.Th.) |
| R_{f}: | 0.7 (Kieselgel, EtOAc/PE 2%) |

### Stufe 3: 1-Benzyl-azepan-4-on

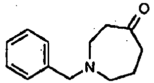

Unter einer Argonatmosphäre wurden 1.0 L Xylol mit einer Dean-Stark-Apparatur bei 145°C 1 bis 2 h erhitzt. Das Lösungsmittel wurde auf 65°C abgekühlt, mit 21 g (0.19 mol) Kalium-tert-butylat versetzt und für weitere 1 bis 2 h auf 145°C erhitzt. Anschließend wurden 30 g (93 mmol) 4-[Benzyl-(2-ethoxycarbonyl-ethyl)-amino]-buttersäureethylester in Xylol tropfenweise über einen Zeitraum von 1 h zu dem Reaktionsansatz gegeben und bei 145°C 2 bis 3 h gerührt. Nach dem Abkühlen auf 0°C wurde der Reaktionsansatz mit 0.45 L einer 6 N wässrigen Salzsäure-Lösung versetzt, die wässrige Phase abgetrennt und für 2 h unter Rückfluss gekocht. Anschließend wurde wieder auf 0°C abgekühlt, der Reaktionsansatz mit wässriger Natriumhydroxid-Lösung alkalisch gestellt und mit EtOAc extrahiert. Die vereinigten organischen Phasen wurden über Natriumsulfat getrocknet, filtriert und eingeengt. Der Rückstand wurde mittels Flash-Chromatographie (Aluminiumoxid) gereinigt.

| | |
|---|---|
| Ausbeute: | 5.5 g (29% d.Th.) |
| ESI-MS: | m/z = 204 (M+H)⁺ |
| R_{f}: | 0.4 (Kieselgel, EtOAc/PE 30%) |

### Stufe 4: 1-Benzyl-5-brom-azepan-4-on-Hydrobromid

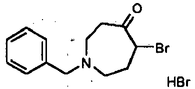

Zu 10 g (49 mmol) 1-Benzyl-azepan-4-on in 28 mL Essigsäure wurden 5.7 mL HBr in Essigsäure (33%) bei RT zugetropft. Anschließend wurden noch 9.5 g (60 mmol) Brom bei RT zugegeben und 1.5 h bei RT gerührt. Nach dem Einengen des Reaktionsansatzes unter 35°C wurde der Rückstand in EtOAc gegeben und ca. 1 h unter Rückfluss gekocht. Es wurde die überstehende organische Phase von dem ausgefallenen Feststoff abdekantiert, anschließend nochmals mit EtOAc versetzt und ca. 1 h unter Rückfluss gekocht. Der ausgefallene Feststoff wurde filtriert, mit EtOAc gewaschen und getrocknet.

| | |
|---|---|
| Ausbeute: | 6.0 g (34% d.Th.) |
| R_{f}: | 0.6 (Kieselgel, EtOAc/PE 30%) |

### Stufe 5: 6-Benzyl-5,6,7,8-tetrahydro-4H-thiazolo[4.5-d]azepin-Hydrochlorid

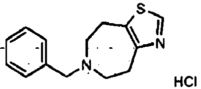

2.1 g (9.7 mmol) Phosphorpentasulfid und 1.9 g (41 mmol) Formamid in Dioxan wurden insgesamt 2.5 h bei 100°C gerührt. Nach dem Abkühlen auf RT wurden 10 g (28 mmol) 1-Benzyl-5-brom-azepan-4-on Hydrobromid zugefügt und 5 h bei 100°C gerührt. Anschließend wurde das Lösungsmittel eingeengt, der Rückstand in gesättigte Natriumbicarbonat-Lösung gegeben und mit EtOAc extrahiert. Die vereinigten organischen Phasen wurden mit Wasser, wässriger Natriumbicarbonat-Lösung und gesättigter Natriumchlorid-Lösung gewaschen. Die organische Phase würde über Natriumsulfat getrocknet, filtriert und eingeengt. Der Rückstand wurde mittels flash-Chromatographie gereinigt. Die Produkt enthaltenden Fraktionen wurden vereinigt und eingeengt. Die freie Base wurde mit methanolischer Salzsäurelösung versetzt. Der entstandene Niederschlag wurde abfiltriert.

| | |
|---|---|
| Ausbeute: | 3.50 g (45% d.Th.) |
| ESI-MS: | m/z = 245 (M+H)⁺ |
| R_{f}: | 0.5 (Kieselgel, MeOH/Chloroform 10%) |

### Stufe 6: 4,5,7,8-Tetrahydro-thiazolo[4,5-d]azepin-6-carbonsäureethylester

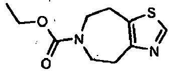

Zu 2.0 g (8.2 mmol) 6-Benzyl-5,6,7,8-tetrahydro-4H-thiazolo[4,5-d]azepin in 100 mL DCM wurden 1.4 g (9.8 mmol) 1-Chlorethylchlöroformiat bei -20°C zugetropft und 30 min nachgerührt. Das organische Lösungsmittel wurde eingeengt und der Rückstand wurde ohne weitere Reinigung weiter umgesetzt.

| | |
|---|---|
| Ausbeute: | 1.5 g (81% d.Th.) |
| R_{f}: | 0.6 (Kieselgel, EtOAc/PE 20%) |

### Stufe 7: 5,6,7,8-Tetrahydro-4H-thiazolo[4,5-d]azepin Hydrochlorid

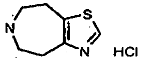

1.5 g (6.6 mmol) 4,5,7,8-Tetrahydro-thiazolo[4,5-d]azepin-6-carbonsäureethyl ester in 50 mL MeOH wurden 3 h unter Rückfluss gekocht. Nach dem Einengen des organischen Lösungsmittels wurde der Rückstand mittels flash-Chromatographie gereinigt. Die Produkt enthaltenden Fraktionen wurden vereinigt und eingeengt. Die freie Base wurde mit 5.0 mL (12.5 mmol)) einer 2.5 molaren methänölischen Salzsäurelösung versetzt und das überschüssige Lösungsmittel eingeengt.

| | |
|---|---|
| Ausbeute: | 0.70 g (55% d.Th.) |
| ESI-MS: | m/z = 155 (M+H)⁺ |
| R_{f}: | 0.2 (Kieselgel, MeOH/Chloroform 20%) |

### Intermediat 73:

### 6-Fluor-4,4-dimethyl-1,2,3,4-tetrahydro-isochinolin Hydrochlorid

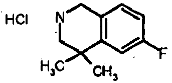

### Stufe 1: 2-(3-Fluor-phenyl)-2-methyl-propionitril

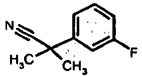

Unter einer Argonatmosphäre wurde bei -70°C zu 5.0 g (37 mmol) (3-Fluor-phenyl)-acetonitril in 150 mL THF 77 mL (77 mmol) einer 1 M Lithium bis(trimethylsilyl)amid-Lösung zugetropft. Man ließ den Ansatz auf -50°C erwärmen und rührte bei dieser Temperatur für 1 h. Anschließend wurden bei -50°C 4.8 mL (78 mmol) Methyljodid zugegeben. Der Reaktionsansatz wurde über Nacht auf RT erwärmt. Der Reaktionsansatz wurde langsam mit gesättigter wässriger Ammoniumchlorid=Lösung versetzt und mit Ethylacetat extrahiert. Die organische Phase wurde mit Natriumsulfat getrocknet und eingeengt.

| | |
|---|---|
| Ausbeute: | 5.6 g (93% d.Th.) |
| ESI-MS: | m/z = 163 (M+H)⁺ |
| Rₜ(HPLC): | 1.54 min (Methode C) |

### Stufe 2: 2-(3-Fluor-phenyl)-2-methyl-propylamin

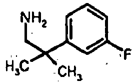

Unter einer Argonatmosphäre wurden bei 0°C zu 5.6 g (34 mmol) 2-(3-Fluor-phenyl)-2-methyl-propionitril in 40 mL THF langsam 69 mL (69 mmol) einer 1 M Lithiumaluminium-hydrid-Lösung in THF zugetropft. Der Reaktionsansatz wurde 30 min bei 0°C und über Nacht bei RT gerührt. Bei 0°C wurde Ethylacetat und anschließend Wasser zugetropft. Der Reaktionsansatz wurde über Celite^{®} filtriert, die organische Phase abgetrennt und eingeengt. Der Rückstand wurde mittel flash-Chromatographie aufgereinigt.

| | |
|---|---|
| Ausbeute: | 1.7 g (30% d.Th.) |
| ESI-MS: | m/z = 168 (M+H)⁺ |
| Rₜ(HPLC): | 0.86 min (Methode C) |

### Stufe 3: 2,2,2-Trifluor-N-[2-(3-fluor-phenyl)-2-methyl-propyl]-acetamid

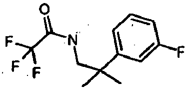

Bei 0°C wurden zu 1.7 g (10 mmol) 2-(3-Fluor-phenyl)-2-methyl-propylamin und 5.4 mL (31 mmol) DIPEA in 35 mL Dichlormethan 1.7 mL (12 mmol) Triflüoressigsäureanhydrid zugetropft. Der Reaktionsansatz wurde 3 h bei RT gerührt, mit Wasser versetzt und mit Dichlormethan extrahiert. Die organische Phase wurde getrocknet und eingeengt.

| | |
|---|---|
| Ausbeute: | 2.7 g (97% d.Th.) |
| ESI-MS: | m/z = 262 (M-H)⁻ |
| Rₜ(HPLC): | 1.54 min (Methode C) |

### Stufe 4: 6-Fluor-4.4-dimethyl-1,2,3,4-tetrahydro-isochinolin Hydrochlorid

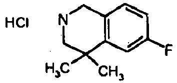

1.0 g (3.8 mmol) 2,2,2-Trifluor-N-[2-(3-fluor-phenyl)-2-methyl-propyl]-acetamid und 0.18 g (6.1 mmol) Formaldehyd in 5.0 mL Essigsäure und 3.5 mL konzentrierte Schwefelsäure wurden 16 h bei RT gerührt. Der Reaktionsanstz wurde auf Wasser gegossen und mit Dichlormethan extrahiert. Die organische Phase wurde mit einer wässrigen Natriumhydrogencarbonat-Lösung extrahiert. Die organische Phase wurde getrocknet und eingeengt. Der Rückstand wurde mit wässriger 0.1 M Salzsäure versetzt und mit Ethylacetat extrahiert. Die wässrige Phase wurde eingeengt. Das Produkt wurde ohne weitere Aufreinigung umgesetzt

| | |
|---|---|
| Ausbeute: | 0.14 g (17% d.Th.) |
| Rₜ(HPLC): | 0.86 min (Methode C) |

### Intermediat 74:

### 2',3'-Dihydro-1'H-spiro[cyclopropan-1,4'-isochinolin]

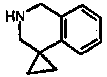

### Stufe 1: 1'H-Spiro[cyclopropan-1,4'-isochinolin]-1',3'(2'H)-dion

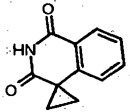

3.0 g (19 mmol) Isochinolin-1,3(2H,4H)-dion, 15.4 mL (0.19 mol) 1-Brom-2-chlorethan und 5.1 g (37 mmol) Kaliumcarbonat, in 35 mL DMF wurden über das Wochenende bei RT gerührt. Anschließend wurden 200 mL Wasser hinzugefügt und mit Ethylacetat extrahiert. Die organische Phase wurde getrocknet und eingeengt.

| | |
|---|---|
| Ausbeute: | 3.0 g (86% d.Th.) |
| ESI-MS: | m/z = 188 (M+H)⁺ |
| Rₜ(HPLC): | 2.95 min (Methode E) |

### Stufe 2: 2',3'-Dihydro-1'H-spiro[cyclopropan-1.4'-isochinolin]

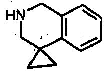

0.5 g (2.7 mmol) 1'H-Spiro[cyclopropan-1,4'-isochinolin]-1',3'(2'H)-dion in 50 mL THF wurden bis zum Rückfluss des Lösungsmittels erhitzt. Es wurden 11 mL (11 mmol) einer 1 M Boran in THF Lösung zugetropft und 3 h unter Rückfluss erhitzt. Der Reaktionsansatz wurde auf 0°C abgekühlt und mit 50 mL Methanol versetzt. Der Reaktionsansatz wurde eingeengt, der Rückstand mit 15 mL einer 4M Salzsäure-Lösung versetzt und 30 min unter Rückfluss gekocht. Nach dem Neutralisieren mit 15 mL einer wässrigen 4M Natronlauge wurde zweimal mit Ethylacetat extrahiert. Die vereinigten organischen Phasen wurden getrocknet und eingeengt. Das Produkt wurde mittels HPLC aufgereinigt. Die Produkt enthaltenden Fraktionen wurden vereinigt und eingeengt.

| | |
|---|---|
| Ausbeute: | 20 mg (5% d.Th.) |
| ESI-MS: | m/z = 160 (M+H)⁺ |
| Rₜ(HPLC): | 0.74 min (Methode C) |

### Intermediat 75:

### 3-(2-Methoxy-ethyl)-2,3-dihydro-1H-indol

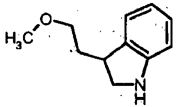

### Stufe 1: 3-(2-Methoxy-ethyl)-1.3-dihydro-indol-2-on

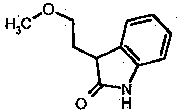

2.0 g (15 mmol) Indolin-2-on, 5 mL (63 mmol) 2-Methoxyethanol und 1.5 g Raney Nickel in 20 mL THF wurden 4 h bei 200°C in einem Autoklaven gerührt. Nach dem Abfiltrieren des Katalysators wurde die Mutterlauge eingeengt, der Rückstand mit Diisopropylether verrieben, abgesaugt und getrocknet

| | |
|---|---|
| Ausbeute: | 1.2 g (42% d.Th.) |
| ESI-MS: | m/z = 190 (M-H)⁻ |
| Rₜ(HPLC): | 2.87 min (Methode E) |

### Stufe 2: 3-(2-Methoxy-ethyl)2,3-dihydro-1H-indol

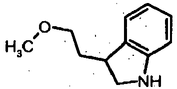

Unter einer Stickstoff-Atmosphäre wurde zu 1.2 g (6.3 mmol) 3-(2-Methoxy-ethyl)-1,3-dihydro-indol-2-on in 50 mL THF 12.6 mL (12.6 mmol) einer 1M Boran in THF-Lösung zugetropft. Der Reaktionsansatz wurde 3 h unter Rückfluss gekocht, auf 0°C abgekühlt und mit 10 mL Methanol und im Folgenden mit 15 mL halbkonzentrierter Salzsäure-Lösung versetzt. Der Reaktionsansatz wurde 3 h unter Rückfluss gerührt, abgekühlt und zweimal mit Ethylacetat gewaschen. Die wässrige Phase wurde mit einer wässrigen 4M Natronlauge alkalisch gestellt und dreimal mit Essigester extrahiert. Die organischen Phasen wurden vereinigt, über Natriumsulfat getrocknet und eingeengt.

| | |
|---|---|
| Ausbeute: | 0.95 g (85% d.Th.) |
| ESI-MS: | m/z = 178 (M+H)⁺ |
| R₁(HPLC): | 0.76 min (Methode C) |

### Intermediat 76:

### 5-Fluor-3-methyl-2,3-dihydro-1H-indol

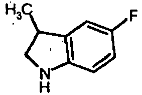

### Stufe 1: 5-Fluor-3-methyl-1.3-dihydro-indol-2-on

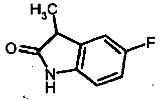

3.0 g (20 mmol) 5-Fluor-1,3-dihydro-indol-2-on, 50 mL Methanol und 2.0 g Raney Nickel wurden 1.5 h bei 200°C in einem Autoklaven gerührt. Nach dem Abfiltrieren des Katalysators wurde die Mutterlauge eingeengt und der Rückstand aus Methanol umkristallisiert.

| | |
|---|---|
| Ausbeute: | 2.8 g (85% d.Th.) |
| ESI-MS: | m/z = 166 (M+H)⁺ |
| Rₜ(HPLC): | 1.1 min (Methode C) |

### Stufe 2: 5-Fluor-3-methyl-2,3-dihydro-1H-indol

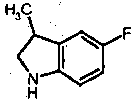

Unter einer Argon-Atmosphäre wurde zu 1.5 g (9.1 mmol) 5-Fluor-3-methyl-1,3-dihydro-indol-2-on in 50 mL THF 18 mL (18 mmol) einer 1M Boran in THF-Lösung zugetropft. Der Reaktionsansatz wurde 2 h bei 70°C gerührt, auf 0°C abgekühlt und mit 10 mL Methanol und im Folgenden mit 30 mL halbkonzentrierter Salzsäure-Lösung versetzt. Der Reaktionsansatz wurde 1 h unter Rückfluss gerührt, abgekühlt und die organische Phase am Rotationsverdampfer entfernt. Die wässrige Phase wurde mit Ethylacetat extrahiert. Die wässrige Phase wurde mit einer wässrigen 4M Natronlauge alkalisch gestellt und mit. Dichlormethan extrahiert. Die organische Phase wurde über Natriumsulfat getrocknet, abfiltriert und eingeengt.

| | |
|---|---|
| Ausbeute: | 0.50 g (36%.d.Th.) |
| ESI-MS: | m/z = 152 (M+H)⁺ |
| Rₜ(HPLC): | 1.85 min (Methode E) |

### Intermediat 77:

### (2-Chlor-pyridin-4-yl)-(5,6-difluor-2,3-dihydro-indol-1-yl)-methanon

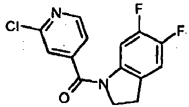

0.35 g (2.2 mmol) 2-Chlorisonicotinsäure, 0.34 g (2.2 mmol) 5,6-Difluor-2,3-dihydro-1H-indol, 0.70 mL (5.0 mmol) TEA und 10 mL DMF wurden mit 0.77 g (2.4 mmol) TBTU versetzt und 2 h bei RT gerührt. Der Reaktionsansatz wurde mittels HPLC aufgereinigt. Die Produktfraktionen wurden vereinigt und eingeengt.

| | |
|---|---|
| Ausbeute: | 0.45 g (69% d.Th.) |
| ESI-MS: | m/z = 295/297 (M+H)⁺Cl |
| Rₜ(HPLC-MS): | 1.5 min (Methode C) |

### Intermediat 78:

### (2-Chlor-1-oxy-pyridin-4-yl)-5-fluor-2,3-dihydro-indol-1-yl)-methanon

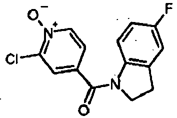

0.12 g (0.43 mmol) (2-Chlor-pyridin-4-yl)-(5-fluor-2,3-dihydro-indol-1-yl)-methanon und 0.22 g (0.90 mmol) 3-Chlorperoxybenzoesäure in 5.0 mL Chloroform wurden 48 h bei 40°C gerührt. Der Ansatz wurde mit 50 mL Dichlormethan verdünnt und mit 50 mL 15%-iger wässriger Kaliumcarbonat-Lösung zweimal gewaschen. Die organische Phase wurde getrocknet und eingeengt.

| | |
|---|---|
| Ausbeute: | 0.14 g (quantitativ) |
| Rₜ (HPLC-MS): | 1.11 min (Methode C) |

### Intermediat 79:

### (2-Chlor-pyridin-4-yl)-(5-fluor-3,3-dimethyl-2,3-dihydro-indol-1-yl)-methanon

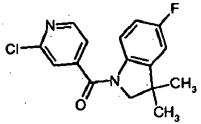

0.17 g (1.1 mmol) 2-Chlorisonicotinsäure, 0.18 g (1.1 mmol) 5-Fluor-3,3-dimethyl-2,3-dihydro-1H-indol, 0.28 mL (2.0 mmol) TEA und 3.0 mL DMF wurden mit 0.39 g (1.2 mmol) TBTU versetzt und über Nacht bei RT gerührt. Der Reaktionsansatz wurde mittels HPLC. aufgereinigt. Die Produktfraktionen wurden vereinigt und eingeengt.

| | |
|---|---|
| Ausbeute: | 0.12 g (36% d.Th.) |
| ESI-MS: | m/z = 305 / 307 (M+H)⁺Cl |
| Rₜ (HPLC-MS): | 1.55 min (Methode C) |

### Intermediat 80:

### (4-Chlor-pyridin-2-yl)-(5-fluor-3,3-dimethyl-2,3-dihydro-indol-1-yl)-methanon

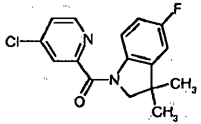

0.17 g (1.1 mmol) 4-Chlor-pyridin-2-carbonsäure, 0.18 g (1.1 mmol) 5-Fluor-3,3-dimethyl-2,3-dihydro-1H-indol, 0.28 mL (2.0 mmol) TEA und 3.0 mL DMF wurden mit 0.39 g (1.2 mmol) TBTU versetzt und über Nacht bei RT gerührt. Der Reaktionsansatz wurde mittels HPLC aufgereinigt. Die Produktfraktionen wurden vereinigt und eingeengt.

| | |
|---|---|
| Ausbeute: | 120 mg (36% d.Th.) |
| ESI-MS: | m/z = 305/307 (M+H)⁺Cl |
| Rₜ (HPLC-MS): | 1.66 min (Methode C) |

### Intermediat 81:

### N-[6-Chlor-4-(5-fluor-2,3-dihydro-indol-1-carbonyl)-pyridin-2-yl]-methansulfonamid

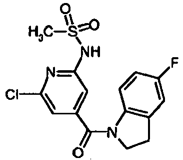

### Stufe 1: 2-Chlor-6-methansulfonylamino-isonicotinsäuremethylester

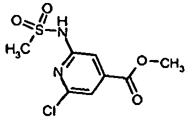

Unter einer Stickstoff-Atmosphäre wurden 10 g (49 mmol)2,6-Dichlor-isonicötinsäure-methylester, 5.6 g (59 mmol) Methansulfonamid, 14 g (68 mmol) Kaliumphosphat, 1.7 g (2.9 mmol) Xantphos und 0.90 g Tris(dibenzylidenaceton)dipalladium in 300 mL Dioxan, 5 h bei 100°C gerührt. Der Ansatz wurde über Kieselguhr abgesaugt und eingeengt. Der Rückstand wurde mit Ethanol ausgerührt und der Feststoff abgesaugt.

| | |
|---|---|
| Ausbeute: | 4.4 g (34% d.Th.) |
| ESI-MS: | m/z = 265 / 266 (M+H)⁺ (Cl) |

### Stufe 2: 2-Chlor-6-methanesulfonylamino-isonicotinsäure

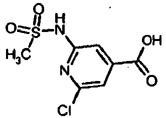

0.29 g (1.1 mmol) 2-Chlor-6-methansulfonylamino-isonicotinsäure methyl ester in 5.0 mL Tetrahydrofuran und 1 mL Wasser wurden mit 79 mg (3.3 mmol) Lithiumhydroxid versetzt und über Nacht bei RT gerührt. Der Reaktionsansatz wurde mit 1 M wässriger Salzsäure angesäuert und eingeengt. Das Produkt wurde ohne weitere Aufreinigung umgesetzt.

| | |
|---|---|
| Ausbeute: | 300 mg (quantitativ) |
| Rₜ (HPLC-MS): | 0.93 min (Methode C) |

### Stufe 3: N-[6-Chlor-4-(5-fluor-2.3-dihydro-indol-1-carbonyl)-pyridin-2-yl]-methansulfonamid

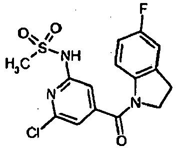

0.28 g (1.1 mmol) 2-Chlor-6-methansulfonylamino-isonicotinsäure, 0.16.g (1.1 mmol) 5-Fluor-2,3-dihydro-1H-indol, 0.31 mL (2.2 mmol) TEA in 4.0 mL DMF wurden mit 0.39 g (1.2 mmol) TBTU versetzt und 2 h bei RT gerührt. Der Reaktionsansatz wurde mittels HPLC aufgereinigt. Die Produktfraktionen wurden vereinigt und eingeengt.

| | |
|---|---|
| Ausbeute: | 340 mg (81 % d.Th.) |
| ESI-MS: | m/z = 370/372 (M+H)⁺ (Cl) |
| Rₜ (HPLC-MS): | 1.4 min (Methode C) |

### Intermediat 82:

### N-[6-Chlor-4-(5-fluor-2,3-dihydro-indol-1-carbonyl)-pyridin-2-yl]-N-methyl-methansulfonamid

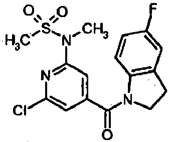

0.30 g (1.1 mmol) 2-Chlor-6-(methansulfonyl-methyl-amino)-isonicotinsäure, 0.16 g (1.1 mmol) 5-Fluor-2,3-dihydro-1H-indol, 0.31 mL (2.2 mmol) TEA in 4.0 mL DMF wurden mit 0.39 g (1.2 mmol) TBTU versetzt und 2 h bei RT gerührt. Der Reaktionsansatz wurde mittels HPLC aufgereinigt. Die Produktfraktionen wurden vereinigt und eingeengt.

| | |
|---|---|
| Ausbeute: | 360 mg (83% d.Th.) |
| ESI-MS: | m/z = 384 / 386 (M+H)⁺ (Cl) |
| Rₜ (HPLC-MS): | 1.55 min (Methode C) |

### Intermediat 83:

### (6-Chlor-pyrazin-2-yl)-(5-fluor-2,3-dihydro-indol-1-yl)-methanon

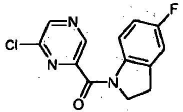

0.18 g (1.1 mmol) 6-Chlor-pyrazin-2-carbonsäure, 0.15 g (1.1 mmol) 5-Fluor-2,3-dihydro-1H-indol, 0.31 mL (2.2 mmol) TEA in 3.0 mL DMF wurden mit 0.39 g (1.2 mmol) TBTU versetzt und 1 h bei RT gerührt. Der Reaktionsansatz wurde mit Wasser versetzt und 5 min gerührt. Der ausgefallene Feststoff wurde filtriert, mit Wasser gewaschen und getrocknet.

| | |
|---|---|
| Ausbeute: | 235 mg (66% d.Th.) |
| EI-MS: | m/z = 277 (M+H)⁺ (Cl) |
| Rₜ (HPLC-MS): | 1.48 min (Methode C) |

### Intermediat 84:

### (2-Chlor-6-methoxypyridin-4-yl)-(5-fluor-3,3-dimethyl-2,3-dihydroindol-1-yl)-methanon

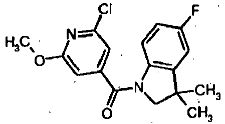

Diese Verbindung wurde analog zu (2-Chlor-6-methoxypyridin-4-yl)-(5-fluor-2,3-dihydro-indol-1-yl)-methanon aus 0.50 g (2.7 mmol) 2-Chlor-6-methoxyisonicotinsäure, 0.44 g (2.7 mmol) 5-Fluor-3,3-dimethyl-2,3-dihydro-1H-indol und 0.42 mL (3.0 mmol) Triethylamin in 10 mL DMF erhalten.

| | |
|---|---|
| Ausbeute: | 0.60 g (67% d.Th.) |
| ESI-MS: | m/z = 335/337 (M+H)⁺ (Cl) |
| Rₜ(HPLC): | 1.73 min (Methode C) |

### Intermediat 85:

### (6-Chlor-pyrimidin-4-yl)-(2-ethyl-2,3-dihydro-indol-1-yl)-methanon

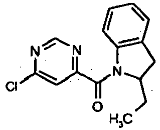

### Stufe 1: 1-Benzolsulfonyl-1H-indol

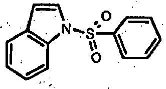

Zu 2.0 g (17 mmol) Indol in 30 mL THF wurden unter Eisbadkühlung 0.89 g (22 mmol) Natriumhydrid (60%ig) zugefügt und 15 min bei dieser Temperatur gerührt. Anschließend wurden 2.2 mL (17 mmol) Benzolsulfonsäurechlorid zugegeben und bei RT über Nacht gerührt. Das Reaktionsgemisch wurde mit Wasser und EtOAc versetzt und mehrmals mit EtOAc extrahiert. Die vereinten organischen Phasen wurden über Natriumsulfat getrocknet und eingeengt.

| | |
|---|---|
| Ausbeute: | 4.6 g (quantitativ) |
| ESI-MS: | m/z = 275 (M+H)⁺ |

### Stufe 2: 1-Benzolsulfonyl-2-ethyl-1H-indol

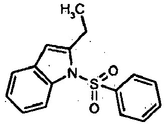

Unter einer Argonatmosphäre wurden zu 2.8 g (11 mmol) 1-Benzolsulfonyl-1H-indol in 25 mL THF bei -78°C langsam 6.7 mL (12 mmol) einer 1.8 molaren Lithiumdiisopropylamid-Lösung in THF zugetropft. Danach wurde die Kühlung entfernt, der Reaktionsansatz auf RT erwärmt und eine weitere Stunde bei RT nachgerührt. Der Reaktionsansatz wurde wieder auf -78°C abgekühlt und mit 1.0 mL (12 mmol) lodethan versetzt. Anschließend wurde der Reaktionsansatz wieder auf RT erwärmt und über Nacht nachgerührt. Da die Umsetzung unvollständig war wurde der Reaktionsansatz nochmals auf -78°C abgekühlt, mit 3.3 mL (6.0 mmol) einer 1.8 molaren Lithiumdiisopropylamid-Lösung in THF versetzt und nach beendeter Zugabe auf RT erwärmt. Anschließend wurde der Reaktionsansatz auf Eiswasser gegossen und mit EtOAc extrahiert. Die organische Phase wurde über Natriumsulfat getrocknet und eingeengt. Der Rückstand wurde mittels flash-Chromatographie gereinigt. Die Produkt enthaltenden Fraktionen wurden vereinigt, eingeengt und im HV getrocknet.

| | |
|---|---|
| Ausbeute: | 0.75 g (24% d.Th.) |
| R_{f}: | 0.61 (Kieselgel, PE/EtOAc 3/1) |

### Stufe 3: 2-Ethyl-1H-indol

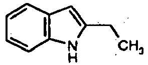

1.2 g (4.2 mmol) 1-Benzolsulfonyl-2-ethyl-1 H-indol in 10 mL EtOH wurden mit 5 mL einer (20 mmol) 4 N wässrigen Natronlauge versetzt und 8h unter Rückfluss gekocht. Anschließend wurde das Lösungsmittel am Rotationsverdampfer entfernt und der Rückstand mit Eiswasser verdünnt. Nach dem Ansäuern mit halbkonzentrierter wässriger Salzsäure wurde die ausgefallene Schmiere mit Ethylacetat extrahiert. Die organische Phase wurde über Natriumsulfat getrocknet, abfiltriert, eingeengt und getrocknet.

| | |
|---|---|
| Ausbeute: | 0.66 g (quantitativ) |
| ESI-MS: | m/z = 146 (M+H)⁺ |

### Stufe 4: 2-Ethyl-2,3-dihydro-1H-indol

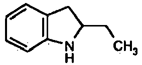

0.66 g (4.2 mmol) 2-Ethyl-1 H-indol in 10 mL Essigsäure wurden mit 1.3 g (20 mmol) Natriumcyanoborhydrid versetzt und einen Tag bei RT gerührt. Der Reaktionsansatz wurde am Rotationsverdampfer eingeengt, mit 20 mL wässriger 4N Salzsäure versetzt und 1 h bei RT gerührt. Unter Eiskühlung wurden anschließend 45 mL einer wässrigen 4N Natriumhydroxid-Lösung hinzu gefügt und mit Ethylacetat extrahiert. Die organische Phase wurde über Natriumsulfat getrocknet, filtriert, eingeengt und der Rückstand im Vakuum getrocknet.

| | |
|---|---|
| Ausbeute: | 0.80 g (quantitativ) |

### Stufe 5: (6-Chlor-pyrimidin-4-yl)-(2-ethyl-2,3-dihydro-indol-1-yl)-methanon

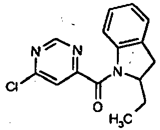

0.80 g (4.5 mmol) 6-Chlorpyrimidin-4-carbonsäurechlorid in 30 mL DCM wurden in einem Eis/Ethanol-Bad abgekühlt und mit 0.62 g (4.2 mmol) 2-Ethyl-2,3-dihydro-1 H-indol in DCM und 4.7 mL (4.7 mmol) einer 1M wässrigen Natronlauge-Lösung versetzt. Anschließend wurde 30 min unter Kühlen und 1h bei RT nachgerührt. Nach Zugabe von 50 mL einer gesättigten Natriumhydrogencarbonat-Lösung wurden weitere 10 min gerührt. Die organische Phase wurde abgetrennt, mit Wasser gewaschen und eingeengt. Der Rückstand wurde mittels flash-Chromatographie gereinigt. Die Produkt enthaltenden Fraktionen wurden vereinigt, eingeengt und getrocknet.

| | |
|---|---|
| Ausbeute: | 0.25 g (19% d.Th.) |
| R_{f}: | 0.54 (Kieselgel, PE/EtOAc 4/1 ) |

### Intermediat 86:

### 3-Brom-5-(5-fluorindolin-1-carbonyl)pyridin 1-oxid

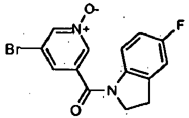

### Stufe 1: (5-Brom-pyridin-3-yl)-(5-fluor-2,3-dihydro-indol-1-yl-methanon

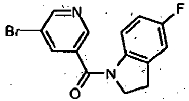

0.44 g (2.2 mmol) 5-Bromnicotinsäure, 0.30 g (2.2 mmol) 5-Fluorindolin, 0.75 g (2.3 mmol) TBTU und 0.60 mL (4.3 mmol) TEA wurden in 3.0 mL DMF zusammen gegeben und über Nacht bei RT gerührt. ANschließend wurde der Reaktionsansatz auf Wasser gegeben, der entstandene Niederschlag abgesaugt und getrocknet.

| | |
|---|---|
| Ausbeute: | 700 mg (quantitativ) |
| ESI-MS: | m/z = 321/ 323 (Br) (M+H)⁺ |
| Rₜ (HPLC-MS): | 0.43 min (Methode C) |

### Stufe 2: 3-Brom-5-(5-fluorindolin-1-carbon)pyridin1-oxid

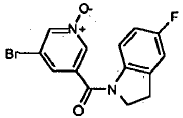

Zu 0.32 g (0.98 mmol) (5-Brom-pyridin-3-yl)-(5-fluor-2,3-dihydro-indol-1-yl)-methanon in 5.0 mL DCM wurden 0.19 g (1.1 mmol) m-Chlorperbenzoesäure zugefügt und 4 h bei RT gerührt. Zusätzlich wurden zu dem Reaktionsansatz nochmal 95 mg (0.55 mmol) m-Chlorperbenzoesäure zugefügt und 48 h bei RT nachgerührt. ANschließend wurde der Reaktionsansatz mit DCM verdünnt und mit 1 N wässriger Natronlauge extrahiert. Die organische Phase wurde über Natriumsulfat getrocknet, eingeengt und im HV getrocknet.

| | |
|---|---|
| Ausbeute: | 330 mg (quantitativ) |
| ESI-MS: | m/z = 337 (M+H)⁺ |
| Rₜ (HPLC-MS): | 1.22 min (Methode C) |

### Intermediat 87:

### (4-Chlor-5-iod-pyridin-2-yl)-(5-fluor-2,3-dihydro-indol-1-yl)-methanon

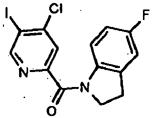

### Stufe 1: 5-Brom-2-methylpyridin1-oxid

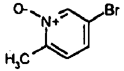

Zu 25 g (0.15 mol) 5-Brom-2-methylpyridin in 50 mL DCM wurden 39 g (0.16 mol) 3-Chlorperbenzoesäure in 450 mL DCM (über Natriumsulfat getrocknet) innerhalb von 2 h zugetropft. Anschließend wurde der Reaktionsansatz 20 h bei RT gerührt und mit 15%iger Kaliumcarbonat-Lösung extrahiert. Die organische Phase wurden über Natriumsulfat getrocknet und eingeengt.

| | |
|---|---|
| Ausbeute: | 21 g (76% d.Th.) |
| ESI-MS: | m/z= 337 (M+H)⁺ |
| Rₜ (HPLC-MS): | 0.75 min (Methode C) |

### Stufe 2: 5-Brom-2-methyl-4-nitropyridin 1-oxid

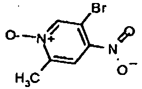

Zu 6.0 mL konz. Schwefelsäure wurden unter Eiskühlung und Rühren 6.0 mL Sälpetersäure zugetropft. Anschließend wurden portionsweise 3.6 g (21 mmol) 5-Brom-2-methylpyridin1-oxid zugefügt und der Reaktionsansatz 18 h bei 60°C gerührt. Nach dem Abkühlen auf RT wurde der Reaktionsansatz auf Eiswasser gegeben und mit 4N wässriger Natronlauge neutralisiert. Der ausgefallene Feststoff wurde abgesaugt und bei 50°C getrocknet.

| | |
|---|---|
| Ausbeute: | 3.60 g (73% d.Th.) |
| ESI-MS: | m/z = 233 (M+H)⁺ |
| Rₜ (HPLC-MS): | 1.09 min (Methode C) |

### Stufe 3: 5-Brom-4-chlor-2-methylpyridin1-oxid

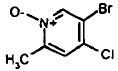

Zu 3.6 g (15 mmol) 5-Brom-2-methyl-4-nitropyridin1-oxid in 30 mL DCM wurden 4.2 mL (46 mmol) Phosphoroxichlorid in 20 mL DCM bei 10°C zugetropft. Der Reaktionsansatz wurde anschließend 5 h unter Rückfluss gekocht, dann auf Eiswasser gegeben und mit 4N wässriger Natronlauge Lösung ein pH-Wert von 10 eingestellt. Die organische Phase wurde abgetrennt und die wässrige Phase wurde noch zwei Mal mit DCM extrahiert. Die vereinigten organischen Phasen wurden über Natriumsulfat getrocknet und eingeengt. Der Rückstand wurde in Petrolether verrührt, der entstandene Niederschlag abgesaugt und getrocknet.

| | |
|---|---|
| Ausbeute: | 2.60 g (76% d.Th.) |
| Rₜ (HPLC-MS): | 1.08 min (Methode C) |

### Stufe 4: (5-Brom-4-chlor-pyridin-2-yl)-methanol

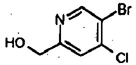

Zu 2.6 g (12 mmol) 5-Brom-4-chlor-2-methylpyridin1-oxid in 30 mL DCM wurden bei 10°C 3.0 mL Trifluoressigsäureanhydrid zugetropft. Der Reaktionsansatz wurde 5 Tage bei RT gerührt. Nach Zugabe von MeOH wurde der Reaktionsansatz eingeengt, der Rückstand mit 15%iger Kaliumcarbonat-Lösung versetzt und mehrmals mit EtOAC extrahiert. Die vereinigten organischen Phasen wurden über Natriumsulfat getrocknet und eingeengt.

| | |
|---|---|
| Ausbeute: | 2.15g (83% d.Th.) |
| ESI-MS: | m/z = 222 (M+H)⁺ |
| Rₜ (HPLC-MS): | 1.10 min (Methode C) |

### Stufe 5: 5-Brom-4-chlor-pyridin-2-carbonsäure

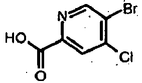

Zu 0.71 g (4.5 mmol) Kaliumpermanganat in 10 mL Aceton wurden 0.50 g (2.3 mmol) (5-Brom-4-chlor-pyridin-2-yl)-methanol in 8 mL Aceton bei RT zugetropft und anschließend 17 h bei RT gerührt. Anschließend wurden 10 mL Isopropanol zugefügt und weitere 5 h bei RT gerührt. Der ausgefallene Braunstein wurde abgesaugt und mit Wasser gewaschen. Das Filtrat wurde teilweise eingeengt und die wässrige Phase wurde mit 1 N wässriger Salzsäure-Lösung auf einen pH-Wert von 3 eingestellt. Der ausgefallene Niederschlag wurde abgesaugt.

| | |
|---|---|
| Ausbeute: | 330 mg (62% d.Th.) |
| ESI-MS: | m/z = 234 (M-H)⁻ |
| Rₜ (HPLC-MS): | 1.09 min (Methode C) |

### Stufe 6: (5-Brom-4-chlor-pyridin-2-yl)-(5-fluor-2,3-dihydro-indol-1-yl)-methanon

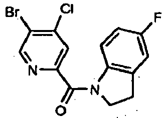

Zu 0.50 g (2.1 mmol) 5-Brom-4-chlor-pyridin-2-carbonsäure, 0.29 g (2.1 mmol) 5₋Fluor-2,3-dihydro-1H-indol und 0.62 mL (4.4 mmol) TEA in 11 mL DMF wurden 0.75 mg *(2.3 mmol) TBTU zugegeben und über Nacht bei RT gerührt. Dann wurde der Reaktionsansatz mit Wasser versetzt, der ausgefallene Niederschlag abgesaugt und getrocknet.

| | |
|---|---|
| Ausbeute: | 356 mg (85% d.Th.) |
| ESI-MS: | m/z = 355 (M+H)⁺ |

### Stufe 7: (4-Chlor-5-iod-pyridin-2-yl)-(5-fluor-2,3-dihydro-indol-1-yl)-methanon

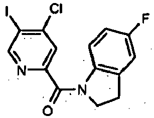

Unter einer Stickstoffatmosphäre wurden zu 1.5 g (4.2 mmol) (5-Brom-4-chlor-pyridin-2-yl)-(5-fluor-2,3-dihydro-indol-1-yl)-methanon in 15 mL 1,4-Dioxan 1.3 g (8.4 mmol) Natriumiodid, 80 mg (0.42 mmol) Kupferiodid und 90 µL (0.84 mmol) N,N-Dimethylen-ethylendiamin zugefügt und 5 Tage bei 110°C gerührt. Der Reaktionsansatz wurde mit Wasser verdünnt, der ausgefallene Niederschlag abgesaugt und getrocknet.

| | |
|---|---|
| Ausbeute: | 1.57 g (92% d.Th.) |
| ESI-MS: | m/z = 403 (M+H)⁺ |
| Rₜ (HPLC-MS): | 1.80 min (Methode C) |

### (Intermediat 88:

### (4-Chlor-6-methoxy-pyridin-2-yl)-(5-fluor-2,3-dihydro-indol-1-yl)-methanon

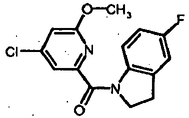

0.55 g (2.9 mmol) 4-Chlor-6-methoxy-pyridin-2-carbonsäure, 0.41 g (3.0 mmol) 5-Fluorindolin, 1.1 g (3.3 mmol) TBTU und 0.93 mL (6.6 mmol) Triethylamin in 5.0 mL DMF wurden 3 h bei RT gerührt. Der Reaktionsansatz wurde mittels HPLC aufgereinigt. Die produktenthaltenden Fraktionen wurden vereinigt und am Rotationsverdampfer eingeengt.

| | |
|---|---|
| Ausbeute: | 450 mg (50% d.Th.) |
| ESI=MS: | m/z = 307/309 (M+H)⁺ (Cl) |
| Rₜ(APLC): | 1.7 min (Methode C) |

### Intermediat 89:

### 4-Chlor-6-(5-fluor-2,3-dihydro-indol-1-carbonyl)-nicotinonitril

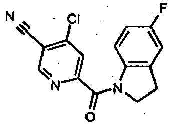

Unter einer Stickstoffatmosphäre wurden zu 0.10 g (0.25 mmol) (4-Chlor-5-iod-pyridin-2-yl)-(5-fluor-2,3-dihydro-indol-1-yl)-methanon in 1.5 mL DMF 45 mg (0.50 mmol) Kupfercyanid zugegeben und 2 h bei RT gerührt. Der Reaktionsansatz wurde bei 100°C über Nacht gerührt und anschließend mit E-Wasser versetzt. Der ausgefallene Niederschlag wurde abgesaugt und getrocknet.

| | |
|---|---|
| Ausbeute: | 75 mg (quantitativ) |
| MS: | m/z = 301 (M⁺) |
| Rₜ (HPLC-MS): | 1.65 min (Methode C) |

### Intermediat 90:

### (4-Chlor-6-methyl-pyridin-2-yl)-(5-fluor-2,3-dihydro-indol-1-yl)-methanon

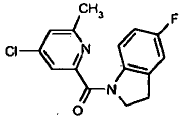

0.10 g (0.58 mmol) 4-Chlor-6-methyl-pyridin-2-carbonsäure und 80 mg (0.58 mmol) 5-Fluor-2,3-dihydro-1 H-indol in 0.17 mL (1.2 mmol) TEA und 2.0 mL DMF wurden mit 0.19 g (0.58 mmol) TBTU versetzt und über Nacht bei RT gerührt. Der Reaktionsansatz wurde mit Wasser verdünnt, der ausgefallene Feststoff abgesaugt und getrocknet.

| | |
|---|---|
| Ausbeute: | 125 mg (74% d.Th.) |
| ESI-MS: | m/z = 291 (M+H)⁺ |
| Rₜ (HPLC-MS): | 0.31 min (Methode C) |

### Intermediat 91:

### (6-Chlor-pyrimidin-4-yl)-(5-fluor-2,3-dihydro-indol-1-yl)-methanon

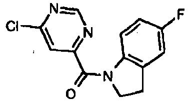

0.92 g (4.9 mmol) 6-Chlor-pyrimidin-4-carbonsäurechlorid in 40 mL DCM wurden in einem Eis/Aceton-Bad abgekühlt und mit 0.67 g (4.9 mmol) 5-Fluor-2,3-dihydro-1H-indol versetzt. Weiterhin wurden noch 5.0 mL (5.0 mmol) einer 1 N wässrigen Natronlauge-Lösung zugetropft und 1 h unter Kühlen gerührt. Es wurden 50 mL einer gesättigten Natriumhydrogencarbonat-Lösung zugegeben und 10 min weitergerührt. Die organische Phase wurde abgetrennt, mit einer 1 N wässriger Salzsäure-Lösung und mit Wasser ausgeschüttelt, getrocknet und eingeengt.

| | |
|---|---|
| Ausbeute: | 0.81 g (60% d.Th.) |
| ESI-MS: | m/z = 278 (M+H)⁺ |
| Rₜ (HPLC-MS): | 1.50 min (Methode C) |

### Intermediat 92:

### (2,6-Dichlor-pyridin-4-yl)-(5-fluor-2,3-dihydro-indol-1-yl)-methanon

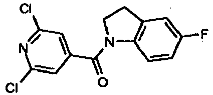

2.0 g (9.5 mmol) 2,6-Dichlor-pyridin-4-carbonsäurechlorid in 50 mL DCM wurden im Eis/EtOH-Bad abgekühlt und mit 1.3 g (9.6 mmol) 5-Fluor-2,3-dihydro-1 H-indol versetzt. Weiterhin wurden noch 9.6 mL (9.6 mmol) einer 1 N wässrigen Natronlauge-Lösung zugetropft und 2 h unter Kühlung und 1 h bei RT gerührt. Anschließend wurden 50 mL einer gesättigten Natriumhydrogencarbonat-Lösung zugegeben und 10 min weitergerührt. Die organische Phase wurde abgetrennt, mit 1 N wässriger Salzsäure-Lösung und mit Wasser ausgeschüttelt, über Natriumsulfat getrocknet und eingeengt.

| | |
|---|---|
| Ausbeute: | 2.85 g (96% d.Th.) |
| ESI-MS: | m/z = 311 (M+H)⁺ |
| Rₜ (HPLC-MS): | 4.57 min (Methode E) |

### Intermediat 93:

### (3-Brom-phenyl)-(5-fluor-2,3-dihydro-indol-1-yl)-methanon

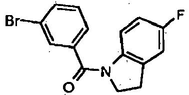

0.40 g (2.0 mmol) 3-Brombenzoesäure und 0.28 g (2.0 mmol) 5-Fluor-2,3-dihydro-1H-indol in 0.55 mL (4.0 mmol) TEA und 10 mL DCM wurden mit 0.64 mg (2.0 mmol) TBTU versetzt und über Nacht bei RT gerührt. Der Reaktionsansatz mit gesättigter Natriumhydrogencarbonat-Lösung und DCM ausgeschüttelt. Die vereinigten organischen Phasen wurden über Natriumsulfat getrocknet, filtriert und eingeengt. Der Rückstand wurde in MeOH suspendiert, abgesaugt und getrocknet.

| | |
|---|---|
| Ausbeute: | 475 mg (71% d.Th.) |
| ESI-MS: | m/z = 320 (M+H)⁺ |
| Rₜ (HPLC-MS): | 1.64 min (Methode C) |

### Intermediat 94:

### 4-(7-Methoxy-2-oxo-1,2,4,5-tetrahydro-benzo[d][1,3]diazepin-3-yl)-3,4,5,6-tetrahydro-2H-[1,4']bipyridinyl-2'-carbonsäure

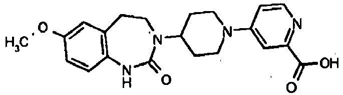

7.0 g (25 mmol) 7-Methoxy-3-piperidin-4-yl-1,3,4,5-tetrahydro-1,3-benzodiazepin-2-on und 2.5 g (12 mmol) 4-Brompicolinsäure in 25 mL NMP wurden 2 h bei 110°C gerührt. Der Reaktionsansatz wurde mit etwas Ameisensäure versetzt und mittels präparativer HPLC-MS gereinigt. Die Produkt enthaltenden Fraktionen wurden vereinigt und eingeengt. Der Rückstand wurde mit MeOH verührt, abgesaugt und getrocknet.

| | |
|---|---|
| Ausbeute: | 0.95 g (19% d.Th.) |
| ESI-MS: | m/z = 397 (M+H)⁺ |
| Rₜ (HPLC-MS): | 1.25 min (Methode S) |

### Intermediat 95:

### 4-(7-Methoxy-2-oxo-1,2,4,5-tetrahydro-benzo[d][1,3]diazeain-3-yl)-3,4,5,6-tetrahydro-2H-[1,2']bipyridinyl-4'-carbonsäure

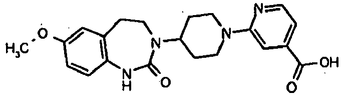

6.0 g (22 mmol) 7-Methoxy-3-piperidin-4-yl-1,3,4,5-tetrahydro-1,3-benzodiazepin-2-on und 1.5 g (11 mmol) 2-Fluorpyridin-4-carbonsäure in 20 mL NMP wurden über Nacht bei 110°C gerührt. Der Reaktionsansatz wurde abgekühlt und der entstandene Niederschlag abgesaugt. Dieser wurde mit Wasser verrührt, zusätzlich mit 15%iger Kaliumcarbonat-Lösung versetzt und mehrmals mit DCM extrahiert. Die wässrige Phase wurde sauer gestellt, der ausgefallene Niederschlag wurde abgesaugt und getrocknet.

| | |
|---|---|
| Ausbeute: | 1.30 g (31% d.Th.) |
| ESI-MS: | m/z = 397 (M+H)⁺ |
| Rₜ (HPLC-MS): | 1.19 min (Methode S) |

### Intermediat.96:

### 4-Cyano-3-[4-(2-oxo-2,3-dihydro-imidazo[4,5-b]pyridin-1-yl)-piperidin-1-yl]-benzoesäure

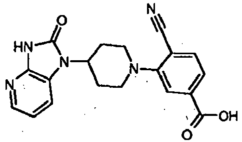

0.20 g (1.2 mmol) 4-Cyano-3-fluorbenzoesäure und 0.53 g (2.4 mmol) 1-Piperidin-4-yl-1,3-dihydroimidazo[4,5-*b*]pyridin-2-on wurden zunächst gut durchmischt bevor sie mit dem Heissluftfön 10 min lang geschmolzen wurden. Nach dem Abkühlen des Reaktionsansatzes wurde dieser in Wasser aufgenommen, mit wässriger Ammoniak-Lösung basisch gestellt und mehrmals mit EtOAc extrahiert. Die wässrige Phase wurde eingeengt und mittels präparativer HPLC-MS gereinigt.

| | |
|---|---|
| Ausbeute: | 0.20 g (41% d.Th.) |
| ESI-MS: | m/z = 364 (M+H)⁺ |
| Rₜ (HPLC-MS): | 1.28 min (Methode C) |

### Intermediat 97:

### 1-[2'-(5-Fluor-2,3-dihydro-indol-1-carbonyl)-5'-iod-3,4,5,6-tetrahydro-2H-[1,4']bipyridinyl-4-yl]-1,3-dihydro-imidazo[4,5-b]pyridin-2-on

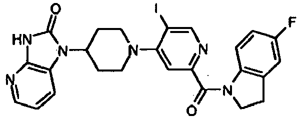

0.50 g (1.2 mmol) (4-Chlor-5-iod-pyridin-2-yl)-(5-fluor-2,3-dihydro-indol-1-yl)-methanon, 0.27 g (1.2 mmol) 1-Piperidin-4-yl-1,3-dihydroimidazo[4,5-*b*]pyridin-2-on, 0.26 g (1.9 mmol) Kaliumcarbonat und 3.5 mL NMP wurden zusammengegeben und bei 130°C 10 h gerührt. Nach dem Zufügen von Wasser wurde der ausgefallene Niederschlag abgesaugt und getrocknet.

| | |
|---|---|
| Ausbeute: | 450 mg (62% d.Th.) |
| Rₜ (HPLC-MS): | 1.70 min (Methode C) |

### Intermediat 98:

### 3-[2'-(5-Fluor-2,3-dihydro-indol-1-carbonyl)-5'-trimethylsilanylethynyl-3,4,5,6-tetrahydro-2H-[1,4']bipyridinyl-4-yl]-7-methoxy-1,3,4,5-tetrahydro-benzo[d][1,3]diazepin-2-on

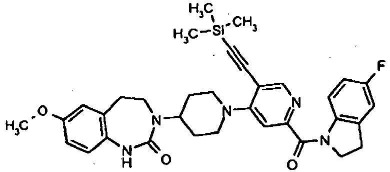

### Stufe 1: 3-[5'-Brom-2'-(5-fluor-2,3-dihydro-indol-1-carbonyl)-3,4,5,6-tetrahydro-2H-[1,4']bipyridinyl-4-yl]-7-methoxy-1,3,4,5-tetrahydro-benzo[d][1,3]-diazepin-2-on

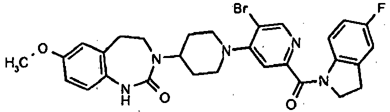

0.38 g (1.1 mmol) (5-Brom-4-chlor-pyridin-2-yl)-(5-fluor-2,3-dihydro-indol-1-yl)-methanon, 0.29 g (1.1 mmol) 7-Methoxy-3-piperidin-4-yl-1,3,4,5-tetrahydro-1,3-benzodiazepin-2-on, 0.22 g (1.6 mmol) Kaliumcarbonat und 3.0 mL NMP wurden zusammen gegeben und 10 h bei 130°C gerührt. Anschließend wurde der Reaktionsanstz mit Wasser versetzt und der ausgefallene Niederschlag abgesaugt und getrocknet.

| | |
|---|---|
| Ausbeute: | 0.47 g (74% d.Th.) |
| ESI-MS: | m/z = 594 (M+H)⁺ |
| Rₜ(HPLC): | 1.72 min (Methode C) |

### Stufe 2: 3-[2'-(5-Fluor-2,3-dihydro-indol-1-carbonyl)-5'-iod-3,4,5,6-tetrahydro-2H-[1,4']bipyridinyl-4-yl]-7-methoxy-1,3,4,5-tetrahydro-benzo[d][1,3]-diazepin-2-on

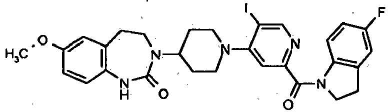

Unter einer Stickstoffatmosphäre wurden zu 0.18 g (0.30 mmol) 3-[5'-Brom-2'-(5-fluor-2,3-dihydro-indol-1-carbonyl)-3,4,5,6-tetrahydro-2H-[1,4']bipyridinyl-4-yl]-7-methoxy-1,3,4,5-tetrahydro-benzo[d][1,3]diazepin-2-on in 1.0 mL 1,4-Dioxan 91 mg (0.61 mmol) Natriumjodid, 6 mg (0.03 mmol) Kupferjodid und 0.09 mL (0.01 mmol) N,N-Dimethylenethylen-diamin zugefügt und über Nacht bei 110°C gerührt. Danach wurde noch zusätzlich 0.3 mL DMF zugefügt und der Reaktionsansatz weitere 10 Tage bei 110°C gerührt. Nach dem Verdünnen mit Wasser wurde der ausgefallene Niederschlag abgesaugt und getrocknet.

| | |
|---|---|
| Ausbeute: | 194 mg (75% d.Th.) |
| Reinheit: | 75% |
| Rₜ (HPLC-MS): | 1.79 min (Methode C) |

### Stufe 3: 3-[2'-(5-Fluor-2,3-dihydro-indol-1-carbonyl)-5'-trimethylsilanylethynyl-3,4,5,6-tetrahydro-2H-[1,4']bipyridinyl-4-yl]-7-methoxy-1,3,4,5-tetra-hydro-benzo[d][1,3]diazepin-2-on

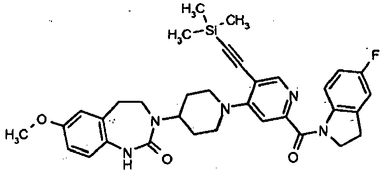

Unter einer Argonatmosphäre wurden zu 0.19 g (0.23 mmol) 3-[2'-(5-Fluor-2,3-dihydro-indol-1-carbonyl)-5'-iod-3,4,5,6-tetrahydro-2H-[1,4']bipyridinyl-4-yl]-7-methoxy-1,3,4,5-tetrahydro-benzo[d][1,3]diazepin-2-on in 0.13 mL (0.91 mmol) TEA und 4.0 mL Dioxan, 22 mg (0.03 mmol) 1,1-Bis(diphenylphosphino)ferrocen-dichlor-palladium (II) und 12 mg (0.06 mmol) Kupferjodid zugegeben. Anschließend wurden noch 0.42 mL (3.0 mmol) Trimethyl-prop-1-ynyl-silan zugefügt und der Reaktionsansatz über Nacht bei RT gerührt. Nach dem Versetzen mit MeOH wurde der entstandene Niederschlag abgesaugt und das Filtrat eingeengt. Der Rückstand wurde in DMF und Acetonitril gelöst und mittels präparativer HPLC-MS gereinigt. Die Produkt enthaltenden Fraktionen wurden vereinigt und eingeengt.

| | |
|---|---|
| Ausbeute: | 35 mg (25% d.Th.) |
| Rₜ (HPLC-MS): | 1.88 min (Methode C) |

### Intermediat 99:

### 3-{1-[6-(5-Benzyloxy-pyrrolo[3,2-b]pyridin-1-carbonyl)-pyrimidin-4-yl]-piperidin-4-yl}-7-methoxy-1,3,4,5-tetrahydro-benzo[d][1,3]diazepin-2-on

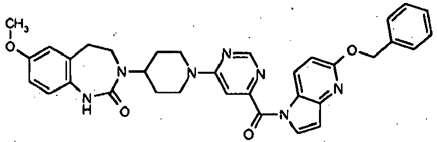

### Stufe 1: 2-Benzyloxy-5-nitro-pyridin

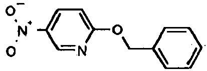

32 g (0.20 mol) 2-Chlor-5-nitropyridin in 120 mL Toluol wurden mit 200 mL (0.20 mmol) einer 1 M Natriumbenzylat-Lösung in Benzylalkohol versetzt und über Nacht bei RT gerührt. Die organische Phase wurde mit Wasser und gesättigter Natriumchlorid Lösung gewaschen, mit Natriumsulfat getrocknet und Toluol destillativ entfernt. Der Rückstand wurde mit Eis abgekühlt, der ausgefallene Niederschlag abgesaugt und mehrmals mit tert.-Butylmethylether gewaschen.

| | |
|---|---|
| Ausbeute: | 33.1 g (72% d.Th.) |

### Stufe 2: (6-Benzyloxy-3-nitro-pyridin-2-yl)-acetonitril

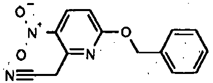

Unter einer Stickstoffatmosphäre wurden 11.5 g (50 mmol) 2-Benzyloxy-5-nitro-pyridin und 9.2 g (55 mmol) 4-Chlorphenoxyacetonitril in 100 mL DMF bei -10°C zu 13.7 g (120 mmol) Kalium-tert.butylat in 50 mL DMF zugetropft. Nach einer Stunde Rühren bei -10°C wurden zum Reaktionsansatz 120 mL einer 1 N wässrigen Salzsäure-Lösung zugetropft und anschließend 30 min bei 0°C nachgerührt. Der entstandene Niederschlag wurde abgesaugt und mittels flash-Chromatographie gereinigt. Die Produkt enthaltenden Fraktionen wurden eingeengt und der Rückstand mit Diisopropylether/PE (1/1) versetzt und gerührt. Der entstandene Niederschlag wurde abgesaugt, mit Diisopropylether gewaschen und getrocknet.

| | |
|---|---|
| Ausbeute: | 10.9 g (81% d.Th.) |
| ESI-MS: | m/z = 268 (M-H)⁻ |
| R_{f}: | 0.75 (Kieselgel, DCM) |

### Stufe 3: (3-Amino-6-benzyloxy-pyridin-2-yl)-acetonitril

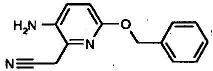

11 g (40 mmol) (6-Benzyloxy-3-nitro-pyridin-2-yl)-acetonitril und 1.7 g Raney-Nickel (gewaschen mit abs. EtOH) in 120 mL EtOH und 50 mL Essigsäure wurden bei RT in einer Wasserstoffatmosphäre von 3 bar hydriert. Der Katalysator wurde abfiltriert und das Filtrat eingeengt. Der Rückstand wurde mit 30 mL Wasser versetzt und mit festem Natriumcarbonat auf pH=10 gestellt. Die wässrige Phase wurde mehrmals mit EtOAc extrahiert. Die vereinigten organischen Phasen wurden über Magnesumsulfat getrocknet, filtriert und eingeengt. Der Rückstand wurde mittels flash-Chromatographie gereinigt. Die Produkt enthaltenden Fraktionen wurden vereinigt und eingeengt.

| | |
|---|---|
| Ausbeute: | 1.80 g (9% d.Th.) |
| ESI-MS: | m/z = 225 (M+H)+⁻ |
| Rₜ (H PLC-MS): | 1.10 min (Methode C) |

### Stufe 4: (5-Benzyloxy-pyrrolo[3,2-b]pyridin-1-yl)-(6-chlor-pyrimidin-4-yl)-methanon

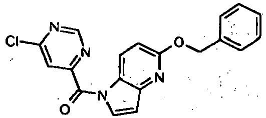

Unter einer Stickstoffatmosphäre wurden zu 0.60 g (2.7 mmol) (3-Amino-6-benzyloxy-pyridin-2-yl)-acetonitril in 15 mL THF 0.11 g (2.8 mmol) Natriumhydrid (60%) portionsweise zugegeben und 30 min bei RT nachgerührt. Es wurden 0.45 g (2.5 mmol) 6-Chlorpyrimidin-4-carbonsäurechlorid portionsweise zugegeben und 2 h bei RT nachgerührt. Der Reaktionsansatz wurde anschließend mit 50 mL EtOAc verdünnt und mit gesättigter Natriumhydrogencarbonat-Lösung, Wasser und 1 N wässriger Salzsäure-Lösung extrahiert. Die organische Phase wurde über Magnesiumsulfat getrocknet, filtriert und eingeengt. Der Rückstand wurde mittels flash-Chromatographie gereinigt. Die Produkt enthaltenden Fraktionen wurden vereinigt und eingeengt.

| | |
|---|---|
| Ausbeute: | 420 mg (45% d.Th.) |
| ESI-MS: | m/z = 364 (M+H)+⁻ |
| Rₜ (HPLC-MS): | 1.79 min (Methode C) |

### Stufe 5: 3-{1-[6-(5-Benzyloxy-pyrrolo[3,2-b]pyridin-1-carbonyl)-pyrimidin-4-yl]-piperidin-4-yl}-7-methoxy-1,3,4,5-tetrahydro-benzo[d][1,3]diazepin-2-on

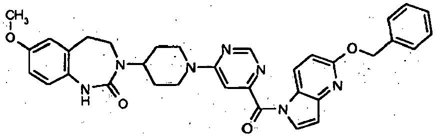

0.11 g (0.40 mmol) 7-Methoxy-3-piperidin-4-yl-1,3,4,5-tetrahydro-1,3-benzodiazepin-2-on, 0.14 g (0.38 mmol) (5-Benzyloxy-pyrrolo[3,2-b]pyridin-1-yl)-(6-chlor-pyrimidin-4-yl)-methanon und 0.10 mL (0.58 mmol) DIPEA in 5 mL DMF wurden über Nacht bei RT gerührt. Der Reaktionsansatz wurde mit Wasser verdünnt und 30 min nachgerührt. Der entstandene Niederschlag wurde abgesaugt, mit Wasser und MeOH gewaschen und getrocknet.

| | |
|---|---|
| Ausbeute: | 210 mg (91% d.Th.) |
| ESI-MS: | m/z = 604 (M+H)⁺ |
| Rₜ (HPLC-MS): | 1.75 min (Methode C |

### Beispiel 100:

### 4-[4-(2-Oxo-2,3-dihydro-imidazo[4,5-b]pyridin-1-yl)-piperidin-1-yl]-pyrimidin-2-carbonsäure

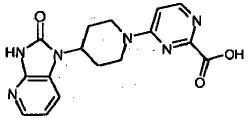

### Stufe 1: 1-[1-(2-Brom-pyrimidin-4-yl)-piperidin-4-yl]-1,3-dihydro-imidazo[4,5-b]-pyridin-2-on

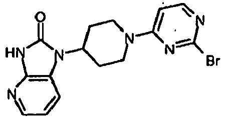

1.4 g (4.8 mmol) 1-Piperidin-4-yl-1,3-dihydro-imidazo[4,5-b]pyridin-2-on-Dihydrochlorid, 1.1 g (4.8 mmol) 2,4-Dibrom-pyrimidin, eine Spatelspitze DMAP und 3.3 mL (19.1 mmol) DIPEA in 35 mL Ethanol wurden bei RT gerührt. Nach erfolgter Umsetzung wurde d Niederschlag abgesaugt, mit wenig Ethanol gewaschen und getrocknet.

| | |
|---|---|
| Ausbeute: | 1.6 g (90% d.Th.) |
| ESI-MS: | m/z = 375 / 377 (Br) (M+H)⁺ |
| Rₜ (HPLC-MS): | 1.08 min (Methode C) |

### Stufe 2: 4-[4-(2-Oxo-2,3-dihydro-imidazo[4,5-b]pyridin-1-yl)-piperidin-1-yl]-pyrimidin-2-carbonsäuremethylester

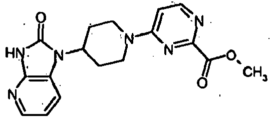

In einer Kohlenmonoxid-Atmosphäre wurden 1.0 g (2.7 mmol) 1-[1-(2-Brom-pyrimidin-4-yl)-piperidin-4-yl]-1,3-dihydro-imidazo[4,5-b]pyridin-2-on, 0.10 g (0.27 mmol) Bis-(benzo-nitril)-palladium (II) chlorid, 0.15 g (0.27 mmol) dppf und 0.45 mL Triethylamin in 30 mL Methanol 13 h bei 130°C und 25 bar CO-Druck carbonyliert. Der Reaktionsansatz wurde eingeengt und der Rückstand mit Methanol verrührt. Der Feststoff wurde abgesaugt und getrocknet.

| | |
|---|---|
| Ausbeute: | 0. 50 g (53% d.Th.) |
| ESI-MS: | m/z = 355 (M+H)⁺ |
| Rₜ (HPLC-MS): | 0.83 min (Methode C) |

### Stufe 3: 4-[4-(2-Oxo-2,3-dihydro-imidazo[4,5-b]pyridin-1-yl)piperidin-1-yl]-pyrimidin-2-carbonsäure

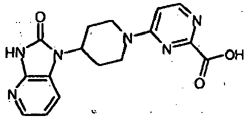

0.50 g (1.4 mmol) 4-[4-(2-Oxo-2,3-dihydro-imidazo[4,5-b]pyridin-1-yl)-piperidin-1-yl]-pyrimidin-2-carbonsäure methyl ester, 1.1 mL E-Wasser und 1.1 mL 4 M Natriumhydroxid-Lösung in 9.0 mL Tetrahydrofuran würde über Nacht bei RT gerührt. Das organische Lösungsmittel wurde entfernt und der Rückstand mit 250 mL Wasser verdünnt. Nach Zugabe von 25 mL 4M Salzsäure-Lösung wurde 1 h gerührt. Der ausgefallene Feststoff wurde abgesaugt, mit Wasser gewaschen und getrocknet.

| | |
|---|---|
| Ausbeute: | 0. 37 g (53% d.Th.) |
| ESI-MS: | m/z = 341 (M+H)⁺ |
| Rₜ (HPLC-MS): | 0.63 min (Methode C) |

### Intermediat 101:

### (S)-2-(3,5-Difluorphenyl)-5,5-dimethylpiperidin

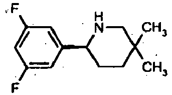

Zu 0.48 g (2.0 mmol) (S)-6-(3,5-Difluorphenyl)-3,3-dimethylpiperidin-2-on in 10 mL THF wurden unter Eiskühlung 7.0 mL (7.0 mmol) einer 1 molaren Di-isobutyl-aluminiumhdyrid-Lösung in Toluol zugefügt und 20 h bei RT nachgerührt. Anschließend wurde der Reaktionsansatz 8 h unter Rückfluss gekocht. Es wurden noch zweimal eine 1 M Diisobutylaluminiumhdyrid-Lösung in Toluol zugefügt und jeweils 8 h und 24 h unter Rückfluss gekocht. Nach Hydrolyse des Reaktionsansatzes wurde der entstandene Niederschlag abgesaugt und mit THF nachgewaschen. Das Filtrat wurde eingeengt und der Rückstand mittels flash-Chromatographie (Aluminiumoxid) gereinigt.

| | |
|---|---|
| Ausbeute: | 0.40 g (53% d.Th.) |
| ESI-MS: | m/z = 226 (M+H)^{+ +} |
| Rₜ (HPLC-MS): | 1.55 min (Methode C) |

### Intermediat 102:

### 6-Chlor-5-methyl-pyrimidin-4-carbonsäure-tert-butylester

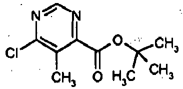

### Stufe 1: 6-Hydroxy-5-methyl-pyrimidin-4-carbonsäureethylester

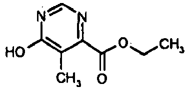

Unter einer Stickstroff-Atmosphäre wurde bei -10°C zu 6.2 g (0.27 mol) Natrium in 150 mL Ethanol 28.4 g (0.27 mol) Formamidin-Acetat in 200 mL Ethanol zugetropft. Der Reaktionsansaatz wurde 5 min nachgerührt und die Suspension wurde abgesaugt. Zu dem Filtrat tropfte man bei -10°C 50 mL Diethyloxalpropionat hinzu. Der Reaktionsansatz wurde über Nacht im Eisbad gerührt. Anschließend wurde der Reaktionsansatz für 5 h unter Rückfluss erhitzt. Der Suspension wurden 300 mL tert.-Butylmethylether hinzugefügt und auf 3°C abgekühlt. Die Suspension wurde abgesaugt und mit TBME gewaschen. Das Filtrat wurde einrotiert und der Rückstand mit 300 mL Ethylacetat und Celite^{®} versetzt und zu Rückfluss erhitzt. Bei dieser Temperatur wurde Celite^{®} abgesaugt und mit 150 mL kochendem Ethylacetat nachgewaschen. Das Filtrat wurde unter Rühren abgekühlt und im Reagenzglas Impfkristalle hergestellt. Die Suspension wurde abgesaugt, mit Ethylacetat gewaschen und getrocknet.

| | |
|---|---|
| Ausbeute: | 7.0 g (14% d.Th.) |
| ESI-MS: | m/z = 181.(M-H)⁻ |

### Stufe 2: 6-Hydroxy-5-methyl-pyrimidin-4-carbonsäure

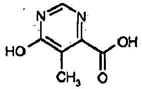

5.0 g (27 mmol) 6-Hydroxy-5-methyl-pyrimidin-4-carbonsäüre ethyl esterin 20 mL konzentrierter Salzsäure gelöst und über Nacht bei RT gerührt. Anschließend wurde der Reaktionsansatz auf 75°C erhitzt und 6 h gerührt. Der Reaktionsanstz wurde auf 5°C abgekühlt, abgesaugt, mit 2 ml konzentrierter Salzsäure nachgewaschen und getrocknet.

| | |
|---|---|
| Ausbeute: | 2.8 g (66% d.Th.) |
| ESI-MS: | m/z = 153 (M-H)⁻ |

### Stufe 3: 6-Chlor-5-methyl-pyrimidin-4-carbonsäurechlorid

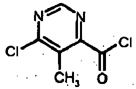

2.6 g (17 mmol) 6-Hydroxy-5-methyl-pyrimidin-4-carbonsäure, 6.7 mL Thionylchlorid, 0.10 mL DMF in 16 mL Acetonitril wurden über Nacht unter Rückfluss gekocht. Der Reaktionsansatz wurde eingeengt und mit Toluol coevaporiert. Der Rückstand wurde mit Petrolether versetzt, der Feststoff abgesaugt und getrocknet.

| | |
|---|---|
| Ausbeute: | 1.0 g (31% d.Th.) |

### Stufe 4: 6-Chlor-5-methyl-pyrimidin-4-carbonsäure-tert-butylester

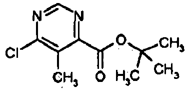

Bei 0°C wurde zu 6.0 g 6-Chlor-5-methyl-pyrimidin-4-carbonsäurechlorid in 10 mL Dichlormethan eine Lösung aus 5.8 mL Pyridin und 15 mL tert.-Butanol in 10 mL Dichlormethan zugetropft. Der Reaktionsanstz wurde auf RT erwärmt und für 30 min gerührt. Der Reaktionsanstz wurde mit Dichlormethan verdünnt und nacheinander mit einer 2M Natriumhydroxid-Lösung, zweimal mit einer 10%-igen wässrigen ZitronensäureLösung und einer Natriumchlorid-Lösung gewaschen. Die organische Pase wurde getrocknet und eingeengt. Der Rückstand wurde in Dichlormethan aufgenommen und mittels flash-Chromatograhie aufgereinigt. Die Produkt enthaltenden Fraktionen wurden eingeengt und getrocknet.

| | |
|---|---|
| Ausbeute: | 5.7 g (79% d.Th.) |

### Herstellung der Endverbindungen:

### Beispiel 1:

### 3-{1-[3-(2,3-Dihydro-indol-1-carbonyl)-phenyl]-piperidin-4-yl}-1,3,4,5-tetrahydro-1,3-benzodiazepin-2-on

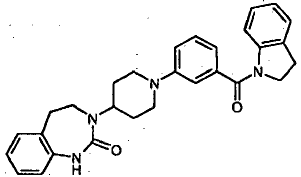

110 mg (0.30 mmol) 3-[4-(2-Oxo-1,2,4,5-tetrahydro-1,3-benzodiazepin-3-yl)-piperidin-1-yl]-benzoesäure, 40 µL (0.35 mmol) 2,3-Dihydro-1*H*-indol, 50 µL (0.36 mmol) Triethylamin und 100 mg (0.31 mmol) TBTU in 2 mL DMF wurden über Nacht bei RT gerührt. Der Reaktionsansatz wurde auf 25 mL Wasser gegossen. Das ausgefallene Produkt wurde abgesaugt, mit Wasser gewaschen, getrocknet und mittels einer Kieselgel-Säule aufgereinigt. Die Produktfraktionen wurden vereinigt und i.vac. eingeengt. Der Rückstand wurde mit Methanol verrieben, abgesaugt und bei 40°C im ULTS getrocknet.

| | |
|---|---|
| Ausbeute: | 83 mg (59% d.Th.) |
| ESI-MS: | m/z = 467 (M+H)⁺ |
| R_{f}: | 0.66 (Kieselgel, DCM/MeOH/NH₄OH = 75/25/5) |

### Vergleichsbeispiel:

### 3-{1-[3-(7,7-Dimethyl-1,4,6,7-tetrahydropyrazolo[4,3-c]pyridin-5-carbonyl)-phenyl]-piperidin-4-yl}-1,3,4,5-tetrahydro-1,3-benzodiazepin-2-on

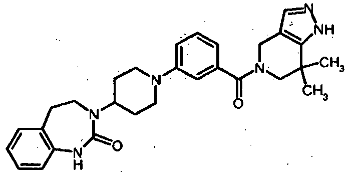

70.0 mg (0.19 mmol) 3-[4-(2-Oxo-1,2,4,5-tetrahydro-1,3-benzodiazepin-3-yl)-piperidin-1-yl]-benzoesäure, 60 mg (0.27 mmol) 7,7-Dimethyl-4,5,6,7-tetrahydro-1*H*-pyrazolo[4,3-*c*]-pyridin-Dihydrochlorid, 100 µL (0.71 mmol) Triethylamin und 70.0 mg (0.22 mmol) TBTU in 1 mL DMF wurden über Nacht bei RT gerührt. Der Reaktionsansatz wurde mit 1mL Methanol und 10 mL Eiswasser versetzt. Das ausgefallene Produkt wurde abgesaugt, mit Wasser und Diethylether nachgewaschen und über präparative HPLC gereinigt. Die Produktfraktionen wurden vereinigt und i.vac. eingeengt.

| | |
|---|---|
| Ausbeute: | 36 mg (38% d.Th.) |
| ESI-MS: | m/z = 499 (M+H)⁺ |

### Beispiel 2:

### 3-[4'-(2,3-Dihydroindol-1-carbonyl)-3,4,5,6-tetrahydro-2H-1,2'-bipyridinyl-4-yl]-1,3,4,5-tetrahydro-1,3-benzodiazepin-2-on

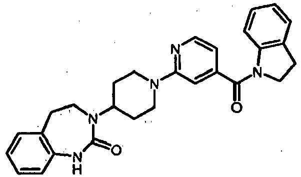

Zu 110 mg (0.43 mmol) (2-Chlorpyridin-4-yl)-(2,3-dihydro-indol-1-yl)-methanon und 3.0 mL (17.4 mmol) DIPEAwurden 100 mg (0.41 mmol) 3-Piperidin-4-yl-1,3,4,5-tetrahydro-1,3-benzodiazepin-2-on zugegeben. Der Reaktionsansatz wurde 5 h unter Rückfluss gerührt. Anschließend wurden 1 mL DMF zugegeben und über Nacht bei 130 C gerührt. Nach dem Erkalten wurde Wasser zugesetzt, der ausgefallene Niederschlag abgesaugt und über präparative HPLC gereinigt. Die Produktfraktionen wurden vereinigt und i.vac. eingeengt.

| | |
|---|---|
| Ausbeute: | 10 mg (5% d.Th.) |
| ESI-MS: | m/z = 468 (M+H)⁺ |
| R_{f}: | 0.74 (Kieselgel, Laufmittel A) |

### Beispiel 3:

### 1-[4'-(5-Fluor-2,3-dihydroindol-1-carbonyl)-3,4,5,6-tetrahydro-2H-[1,2']bipyridinyl-4-yl]-1,3-dihydroimidazo[4,5-b]pyridin-2-on

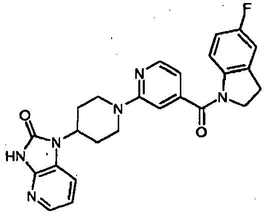

Zu 220 mg (0.80 mmol) (2-Chlorpyridin-4-yl)-(5-fluor-2,3-dihydro-indol-1-yl)-methanon und 0.52 mL (3.00 mmol) DIPEA in 3.0 mL *N*-Methylpyrrolidon wurden 232 mg (0.8 mmol) 1-Piperidin-4-yl-1,3-dihydro-imidazo[4,5-*b*]pyridin-2-on Dihydrochlorid zugegeben. Der Reaktionsansatz wurde 6 h bei 130°C gerührt. Nach 4h fügte man 400 mg Kaliumcarbonat hinzu und ließ den Reaktionsansatz weitere 24 h bei 130°C rühren. Anschließend wurde der Ansatz auf 100 mL Wasser gegossen und mit EtOAc (3 x 100 mL) extrahiert. Die vereinigten organischen Phasen wurden getrocknet und am Rotationsverdampfer eingeengt. Der Rückstand wurde mittels präparativer HPLC aufgereinigt. Die Produktfraktionen wurden vereinigt und i.vac. eingeengt. Der Rückstand wurde mit 30 mL Diethylether verrieben. Der ausgefallene Feststoff wurde abgesaugt und an der Luft getrocknet.

| | |
|---|---|
| Ausbeute: | 20 mg (6% d.Th.) |
| ESI-MS: | m/z = 459 (M+H)⁺ |
| Rₜ (HPLC-MS): | 1.14 min (Methode C) |

### Beispiel 4:

### 3-[2'-(2,3-Dihydroindol-1-carbonyl)-3,4,5,6-tetrahydro-2H-1,4'-bipyridinyl-4-yl]-1,3,4,5-tetrahydro-1,3-benzodiazepin-2-on

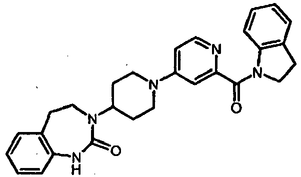

110 mg (0.43 mmol) (4-Chlor-pyridin-2-yl)-(2,3-dihydro-indol-1-yl)-methanon, 150 mg (1.0 mmol) Kaliumcarbonat und 100 mg (0.41 mmol) 3-Piperidin-4-yl-1,3,4,5-tetrahydro-1,3-benzodiazepin-2-on in 10 mL THF wurden 3 Tage unter Rückfluss gekocht. Anschließend wurde das Reaktionsgemisch am Rotationsverdampfer eingeengt, mit 20 mL Xylol aufgenommen und weitere 3 Tage unter Rückfluss gekocht. Der Feststoff wurde abfiltriert und das Filtrat i.vac. eingeengt. Der Rückstand wurde in DMF gelöst und mittels präparativer HPLC aufgereinigt. Die Produktfraktionen wurden vereinigt und lyophilisiert.

| | |
|---|---|
| Ausbeute: | 45 mg (24% d.Th.) |
| ESI-MS: | m/z = 468 (M+H)⁺ |
| R_{f}: | 0.57 (Kieselgel, Laufmittel A) |

### Beispiel 5:

### 1-[2'-(5-Fluor-2,3-dihydroindol-1-carbonyl)-3,4,5,6-tetrahydro-2H-[1,4']bipyridinyl-4-yl]-1,3-dihydroimidazo[4,5-b]pyridin-2-on

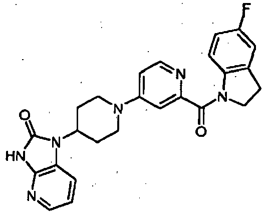

Zu 300 mg (1.08 mmol) (4-Chlorpyridin-2-yl)-(5-fluor-2,3-dihydro-indol-1-yl)-methanon und 373 mg (2.70 mmol) Kaliumcarbonat in 5.0 mL *N-*Methylpyrrolidon wurden 314 mg (1.08 mmol) 1-Piperidin-4-yl-1,3-dihydroimidazo[4,5-*b*]pyridin-2-on Dihydrochlorid zugegeben. Der Reaktionsansatz wurde 30 h bei 130°C gerührt. Anschließend wurde der Ansatz auf 100 mL Wasser gegossen und mit EtOAc (3 x 100 mL) extrahiert. Die vereinigten organischen Phasen wurden getrocknet und am Rotationsverdampfer eingeengt. Der Rückstand wurde mittels präparativer HPLC aufgereinigt. Die Produktfraktionen wurden vereinigt und i.vac. eingeengt. Der Rückstand wurde mit 30 mL Diethylether verrieben. Der ausgefallene Feststoff wurde abgesaugt und an der Luft getrocknet.

| | |
|---|---|
| Ausbeute: | 190 mg (38% d.Th.) |
| ESI-MS: | m/z = 459 (M+H)⁺ |
| Rₜ (HPLC-MS): | 1.0 min (Methode C) |

### Vergleichsbeispiel:

### 6-[4-(7-Methoxy-2-oxo-1,2,4,5-tetrahydro-1,3-benzodiazepin-3-yl)-piperidin-1-yl]-pyrimidin-4-carbonsäure benzyl-(2,2,2-trifluorethyl)-amid

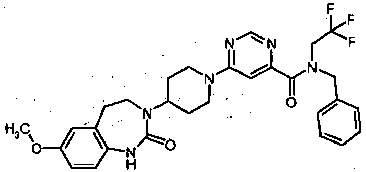

Zu 50 mg (0.15 mmol) 6-Chlorpyrimidin-4-carbonsäure-benzyl-(2,2,2-trifluorethyl)-amid und 34 µL (0.20 mmol) DIPEA in 5 mL DMF wurden 44 mg (0.16 mmol) 7-Methoxy-3-piperidin-4-yl-1,3,4,5-tetrahydro-1,3-benzodiazepin-2-on zugegeben. Der Reaktionsansatz wurde 2 h bei RT gerührt. Der Reaktionsansatz wurde mittels präparativer HPLC-MS gereinigt. Die Produktfraktionen wurden vereinigt und i.vac. eingeengt.

| | |
|---|---|
| Ausbeute: | 62 mg (72% d.Th.) |
| ESI-MS: | m/z = 569 (M+H)⁺ |
| Rₜ (HPLC-MS): | 1.52 min (Methode C) |

### Vergleichsbeispiel:

### 3-{1-[6-(7,7-Dimethyl-1,4,6,7-tetrahydro-pyrazolo[4,3-c]pyridin-5-carbonyl)-pyrimidin-4-yl]-piperidin-4-yl}-7-methoxy-1,3,4,5-tetrahydro-1,3-benzodiazepin-2-on

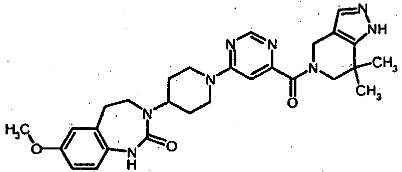

Zu 100 mg (0.25 mmol) 6-[4-(7-Methoxy-2-oxo-1,2,4,5-tetrahydro-1,3-benzodiazepin-3-yl)-piperidin-1-yl]-pyrimidin-4-carbonsäure, 80.0 mg (0.36 mmol) 7,7-Dimethyl-4,5,6,7-tetrahydro-1*H*-pyrazolo[4,3-*c*]pyridin-Dihydrochlorid, 120.0 µl (0.86 mmol) Triethylamin in 1.0 mL DMF wurden 105 mg (0.28 mmol) TBTU hinzugefügt und über Nacht bei RT gerührt. Der Reaktionsansatz wurde mittels präparativer HPLC aufgereinigt, die Produktfraktionen vereinigt und anschließend lyophilisiert.

| | |
|---|---|
| Ausbeute: | 22 mg (16% d.Th.) |
| ESI-MS: | m/z = 531 (M+H)⁺ |
| Rₜ (HPLC): | 2.51 min (Methode E) |

### Vergleichsbeispiel:

### 1-{1-[6-(7,7-Dimethyl-1,4,6,7-tetrahydropyrazolo[4,3-c]pyridin-5-carbonyl)-pyrimidin-4-yl]-piperidin-4-yl}-1,3-dihydroimidazo[4,5-b]pyridin-2-on

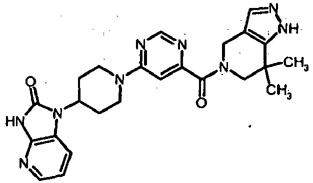

Zu 80 mg (0.24 mmol) 6-[4-(2-Oxo-2,3-dihydroimidazo[4,5-*b*]pyridin-1-yl)-piperidin-1-yl]-pyrimidin-4-carbonsäure, 70.0 mg (0.31 mmol) 7,7-Dimethyl-4,5,6,7-tetrahydro-1*H-*pyrazolo[4,3-*c*]pyridin Dihydrochlorid, 120.0 µL (0.86 mmol) Triethylamin in 1.0 mL DMF wurden 100 mg (0.26 mmol) TBTU hinzugefügt und über Nacht bei RT gerührt. Der Reaktionsansatz wurde mittels präparativer HPLC aufgereinigt, die Produktfraktionen vereinigt und anschließend lyophilisiert.

| | |
|---|---|
| Ausbeute: | 34 mg (16% d.Th.) |
| ESI-MS: | m/z = 474 (M+H)⁺ |
| Rₜ (HPLC): | 2.03 min (Methode E) |

### Vergleichsbeispiel:

### 7-Methoxy-3-{1-[6-(3-phenyl-1,4,6,7-tetrahydropyrazolo[4,3-c]pyridin-5-carbonyl)-pyrimidin-4-yl]-piperidin-4-yl}-1,3,4,5-tetrahydro-1,3-benzodiazepin-2-on

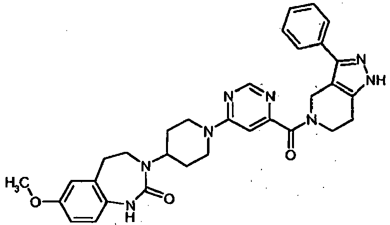

Zu 80 mg (0.20 mmol) 6-[4-(7-Methoxy-2-oxo-1,2,4,5-tetrahydro-1,3-benzodiazepin-3-yl)-piperidin-1-yl]-pyrimidin-4-carbonsäure, 75 mg (0.28 mmol) 3-Phenyl-4,5,6,7-tetrahydro-1*H*-pyrazolo[4,3-*c*]pyridin-Dihydrochlorid, 120.0 µL (0.86 mmol) Triethylamin in 0.9 mL DMF wurden 90 mg (0.24. mmol) TBTU hinzugefügt und über Nacht bei RT gerührt. Der Reaktionsansatz wurde mit 1 mL Methanol, 1mL gesättigter Natriumhydrogencarbonat-Lösung und 8 mL Eiswasser versetzt. Der Niederschlag wurde abgesaugt, mit Wasser und Diethylether nachgewaschen und getrocknet.

| | |
|---|---|
| Ausbeute: | 79 mg (68% d.Th.) |
| ESI-MS: | m/z = 579 (M+H)⁺ |
| Rₜ (HPLC): | 2.88 min (Methode E) |

### Vergleichsbeispiel:

### 1-{1-[6-(Octahydroindol-1-carbonyl)-pyrimidin-4-yl]-piperidin-4-yl}-1,3-dihydroimidazo[4,5-b]pyridin-2-on

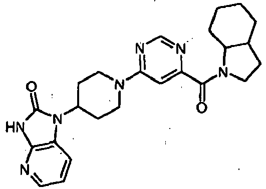

Zu 184 mg (0.69 mmol) (6-Chlorpyrimidin-4-yl)-(octahydroindol-1-yl)-methanon und 488 µL (2.8 mmol) DIPEA in 3 mL DMF wurden 201 mg (0.69 mmol) 1-Piperidin-4-yl-1,3-dihydroimidazo[4,5-*b*]pyridin-2-on-dihydrochlorid zugegeben. Der Reaktionsansatz wurde über das Wochenende bei RT gerührt und anschließend mittels präparativer HPLC-MS gereinigt. Die Produktfraktionen wurden vereinigt und am Rotationsverdampfer das organische Lösungsmittel entfernt. Die wässrige Lösung wurde mit wässriger 1 N NaOH-Lösung neutralisiert und der ausgefallene Niederschlag abgesaugt. Der Niederschlag wurde mit Wasser gewaschen und getrocknet.

| | |
|---|---|
| Ausbeute: | 50 mg (16% d.Th.) |
| ESI-MS: | m/z = 448 (M+H)⁺ |
| Rₜ (HPLC-MSI): | 2.69 min (Methode E) |

### Beispiel 6:

### 3-{1-[6-(2,3-Dihydroindol-1-carbonyl)-pyrimidin-4-yl]-piperidin-4-yl}-1,3,4,5-tetrahydro-1,3-benzodiazepin-2-on

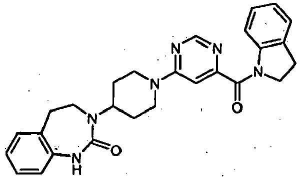

Zu 100 mg (0.27 mmol) 6-[4-(2-Oxo-1,2,4,5-tetrahydro-1,3-benzodiazepin-3-yl)-piperidin-1-yl]-pyrimidin-4-carbonsäure und 42 µL (0.30 mmol) Triethylamin in 4.0 mL DMF wurde 34 µL (0:30 mmol) 2,3-Dihydro-1*H*-indol und 90.0 mg (0.28 mmol) TBTU zugegeben. Der Ansatz wurde 1 h bei RT gerührt und anschließend auf 40 mL Wasser gegossen. Das ausgefallene Produkt wurde abgesaugt. Der Feststoff wurde mit Methanol verrührt, abgesaugt und getrocknet.

| | |
|---|---|
| Ausbeute: | 65 mg (51% d.Th.) |
| ESI-MS: | m/z = 469 (M+H)⁺ |
| R_{f}: | 0.48 (Laufmittel A) |

### Beispiel 7:

### 1-{1-[6-(2,3-Dihydroindol-1-carbonyl)-pyrimidin-4-yl]-piperidin-4-yl}-1,3-dihydro-imidazo-[4,5-b]pyridin-2-on

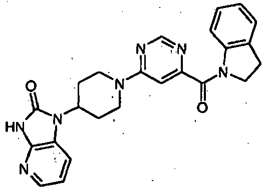

Zu 300 mg (1.16 mmol) (6-Chlorpyrimidin-4-yl)-(2,3-dihydro-indol-1-yl)-methanon und 750 µL (4.36 mmol) DIPEA in 10 mL DMF wurden 350 mg (1.20 mmol) 1-Piperidin-4-yl-1,3-dihydroimidazo[4,5-*b*]pyridin-2-on Dihydrochlorid zugegeben. Der Reaktionsansatz wurde über Nacht bei RT gerührt und anschließend i.vac. eingeengt. Der Rückstand wurde mittels präparativer HPLC aufgereinigt. Die Produktfraktionen wurden vereinigt und lyophilisiert.

| | |
|---|---|
| Ausbeute: | 330 mg (65% d.Th.) |
| ESI-MS: | m/z = 442 (M+H)⁺ |
| R_{f}: | 0.52 (Laufmittel A) |

### Allgemeine Arbeitsvorschrift 1 (AAV1) zur Umsetzung von (6-Chlorpyrimidin-4-yl)-2,3-di-hydroindol-1-yl)-methanon mit einem Amin-Derivat:

Zu 100 mg (0.39 mmol) (6-Chlorpyrimidin-4-yl)-(2,3-dihydro-indol-1-yl)-methanon und 100 µL (0.58 mmol) DIPEA in 10 mL DMF wurde die in der Tabelle angegebenen Menge eines Amin-Derivats zugegeben. Der Reaktionsansatz wurde für 2 h bei RT gerührt. Die Aufarbeitung konnte auf verschiedene Weisen erfolgen:
[A]: Der Reaktionsansatz wurde am Rotationsverdampfer eingeengt und der Rückstand mit 20 mL Wasser versetzt und 30 min bei RT gerührt. Das ausgefallene Produkt wurde abgesaugt, mit Methanol verrührt und abermals abgesaugt. Die Trocknung des Produkts erfolgte im ULTS bei 40°C.
[B] Der Reaktionsansatz wurde am Rotationsverdampfer eingeengt und der Rückstand mit 20 mL Wasser versetzt und 30 min bei RT gerührt. Das ausgefallene Produkt wurde abgesaugt, mit Diisopropylether und Isopropanol verrührt und abermals abgesaugt. Die Trocknung des Produkts erfolgte im ULTS bei 40°C.

| Beispiel Methode | Struktur | Amin-Derivat [Menge Amin-Derivat] Ausbeute | Analytische Daten |
|---|---|---|---|
| Beispiel 8: | 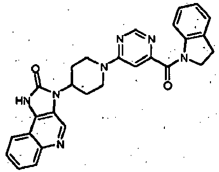 | 3-Piperidin-4-yl-1,3-dihydro-imidazo[4,5-*c*]chinolin-2-on | ESI-MS: m/z = 492 [M+H]⁺ |
| AAV1[B] | 3-{1-[6-(2,3-Dihydro-indol-1-carbonyl)-pyrimidin-4-yl]-piperidin-4-yl}-1,3-dihydro-imidazo[4,5-*c*]chinolin-2-on | 110 mg (0.41 mmol) 125 mg (66% d.Th.) | R_{f} = 0.50 Laufmittel A |
| Beispiel 9: | 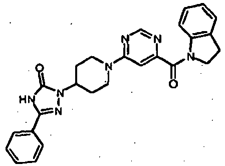 | 5-Phenyl-2-piperidin-4-yl-2,4-dihydro-1,2,4-triazol-3-on | ESI-MS: m/z = 448 [M+,H]⁺ |
| AAV1[B] | 2-{1-[6-(2,3-Dihydroindol-1-carbonyl)-pyrimidin-4-yl]-piperidin-4-yl}-5-phenyl-2,4-dihydro-1,2,4-triazol-3-on | 100 mg (0.41 mmol) | R_{f} = 0.53 Laufmittel A |
| | | 115 mg (64% d.Th.) | |
| Beispiel 10: | 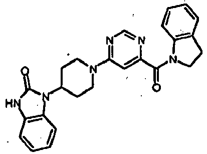 | 1-Piperidin-4-yl-1,3-dihydro-benzimidazol-2-on | ESI-MS: m/z = 441 |
| AAV1[B] | 1-{1-[6-(2,3-Dihydroindol-1-carbonyl)-pyrimidin-4-yl]-piperidin-4-yl}-1,3-dihydro-benzimidazol-2-on | 90 mg (0.41 mmol) | [M+H]⁺ |
| | | 130 mg (77% d.Th.) | R_{f} = 0.54 Laufmittel A |

### Allgemeine Arbeitsvorschrift 2 (AAV2) zur Umsetzung von 1-Piperidin-4-yl-1,3-dihydro-imidazo[4,5-b]pyridin-2-on Dihydrochlorid mit (6-Chlorpyrimidin-4-yl)-methanon-Derivaten:

Zu einem (6-Chlorpyrimidin-4-yl)-methanon-Derivat und 100 µL (0.58 mmol) DIPEA in 10 mL DMF wurden 90 mg (0.31 mmol) 1-Piperidin-4-yl-1,3-dihydro-imidazo[4,5-*b*]pyridin-2-on Dihydrochlorid zugegeben. Der Reaktionsansatz wurde für 2 h bei RT gerührt, dann eingeengt und der Rückstand mit 20 mL Wasser versetzt und 30 min bei RT gerührt. Das ausgefallene Produkt wurde abgesaugt und mittels Flash-Chromatographie aufgereinigt. Die Produktfraktionen wurden vereinigt und am Rotationsverdampfer eingeengt. Der Rückstand wurde mit Diisopropylether verrieben und abgesaugt. Das Produkt wurde im ULTS bei 40°C getrocknet.

| Beispiel | Struktur | 6-Chlorpyrimidin-4-yl-Derivat [Menge 6-Chlor-pyrimidin-4-yl-Derivat] Ausbeute | Analytische Daten |
|---|---|---|---|
| Vergleichsbeispiel: | 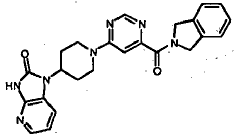 | (6-Chlorpyrimidin-4-yl)-(1,3-dihydro-isoindol-2-yl)-methanon | ESI=MS: m/z = 442 [M+H]⁺ |
| | 1-{1-[6-(1,3-Dihydro-isoindol-2-carbonyl)-pyrimidin-4-yl]-piperidin-4-yl}-1,3-dihydro-imidazo[4,5-*b*]pyridin-2-on | 100 mg (0.39 mmol) | R_{f} = 0.66 Laufmittel A |
| | | 30 mg (22% d.Th.) | |
| Vergleichsbeispiel: | 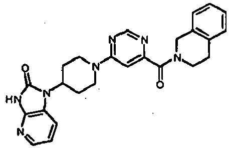 | (6-Chlorpyrimidin-4-yl)-(3,4-dihydro-1*H*-isochinolin-2-yl)-methanon | ESI-MS: m/z = 456 [M+H]⁺ |
| | 1-{1-[6-(3,4-Dihydro-1*H*-isochinolin-2-carbonyl)-pyrimidin-4-yl]-piperidin-4-yl}-1,3-dihydroimidazo[4,5-*b*]pyridin-2-on | 100 mg (0.37 mmol) | R_{f} = 0.56 Laufmittel A |
| | | 35 mg (25% d.Th.) | |
| Vergleichsbeispiel: | 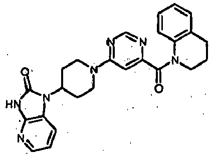 | (6-Chlorpyrimidin-4-yl)-(3,4-dihydro-2*H*-chinolin-1-yl)-methanon | ESI-MS: m/z = 456 [M+H]⁺ |
| | 1-{1-[6-(3,4-Dihydro-2*H-*chinolin-1-carbonyl)-pyrimidin-4-yl]-piperidin-4-yl}-1,3-dihydro-imidazo[4,5-*b*]pyridin-2-on | 100 mg (0.37 mmol) | R_{f} = 0.56 Laufmittel A |
| | | 25 mg (18% d.Th.) | |

### Vergleichsbeispiel:

### 1-{1-[6-(1,2,4,5-Tetrahydro-3-benzazepin-3-carbonyl)-pyrimidin-4-yl]-piperidin-4-yl}-1,3-dihydroimidazo[4,5-b]pyridin-2-on

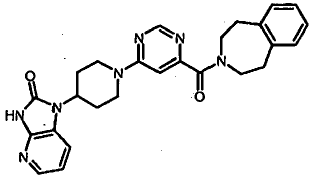

100.0 mg (0.29 mmol) 6-[4-(2-Oxo-2,3-dihydro-imidazo[4,5-*b*]pyridin-1-yl)-piperidin-1-yl]-pyrimidin-4-carbonsäure, 50.0 mg (0.34 mmol) 2,3,4,5-Tetrahydro-1*H*-3-benzazepin, 100 µL (0.71 mmol) Triethylamin und 100.0 mg (0.31 mmol) TBTU in 1.5 mL DMF wurden über Nacht bei RT gerührt. Der Reaktionsansatz wurde über einen Spritzenfilter filtriert und die Lösung mittels präparativer HPLC aufgereinigt. Die Produktfraktionen wurden vereinigt und lyophilisiert.

| | |
|---|---|
| Ausbeute: | 60 mg (44% d.Th.) |
| ESI-MS: | m/z = 470 (M+H)⁺ |
| R_{f} : | 0.50 (Kieselgel, Laufmittel A) |

### Allgemeine Arbeitsvorschrift 3 (AAV3) zur Umsetzung von 5-Phenyl-2-piperidin-4-yl-2,4-dihydro-1,2,4-triazol-3-on mit (6-Chlorpyrimidin-4-yl)-methanon-Derivaten:

Zu einer entsprechendne Menge eines (6-Chlorpyrimidin-4-yl)-methanon-Derivats (s. Tabelle) und 100 µL (0.58 mmol) DIPEA in 10 mL DMF wurden 100 mg (0.41 mmol) 5-Phenyl-2-piperidin-4-yl-2,4-dihydro-1,2,4-triazol-3-on zugegeben. Der Reaktionsansatz wurde für 2 h bei RT gerührt, dann eingeengt und der Rückstand mit 20 mL Wasser versetzt und 30 min bei RT gerührt. Das ausgefallene Produkt wurde abgesaugt, mit Methanol verrührt und abermals abgesaugt. Das Produkt wurde im ULTS bei 40°C getrocknet.

| Beispiel | Struktur | 6-Chlorpyrimidin-4-yl-Derivat [Menge 6-Chlor-pyrimidin-4-yl-Derivat] Ausbeute | Analytische Daten |
|---|---|---|---|
| Vergleichsbeispiel: | 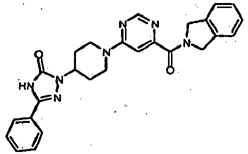 | (6-Chlorpyrimidin-4-yl)-(1,3-dihydro-isoindol-2-yl)-methanon | ESI-MS: m/z = 468 [M+H]⁺ |
| | 2-{1-[6-(1,3-Dihydro-isoindol-2-carbonyl)-pyrimidin-4-yl]-piperidin-4-yl}-5-phenyl-2,4-dihydro-1,2,4-triazol-3-on | 100 mg (0.39 mmol) | R_{f} = 0.57 Laufmittel A |
| | | 95 mg (53% d.Th.) | |
| Vergleichsbeispiel: | 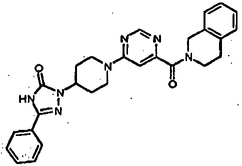 | (6-Chlorpyrimidin-4-yl)-(3,4-dihydro-1*H*-isochinolin-2-yl)-methanon 100 mg (0.37 mmol) | ESI-MS: m/z = 482 [M+H]⁺ |
| | 2-{1-[6-(3,4-Dihydro-1H-isochinolin-2-carbonyl)-pyrimidin-4-yl]-piperidin-4-yl}-5-phenyl-2,4-dihydro-1,2,4-triazol-3-on | | R_{f} = 0.58 Laufmittel A |
| | | 60 mg (34% d.Th.) | |

### Allgemeine Arbeitsvorschrift 4 (AAV4) zur Umsetzung von 3-Piperidin-4-yl-1,3-dihydro-imidazo[4,5-c]chinolin-2-on mit (6-Chlorpyrimidin-4yl)-methanon Derivaten:

Zu einer entsprechenden Menge eines (6-Chlorpyrimidin-4-yl)-methanon-Derivats (s. Tabelle) und 100 µL (0.58 mmol) DIPEAin 10 mL DMF wurden 100 mg (0.37 mmol) 3-Piperidin-4-yl-1,3-dihydro-imidazo[4,5-*c*]chinolin-2-on zugegeben. Der Reaktionsansatz wurde für 2 h bei RT gerührt, dann i.vac. eingeengt und der Rückstand mit 20 mL Wasser versetzt und 30 min bei RT gerührt. Das ausgefallene Produkt wurde abgesaugt, mit Methanol verrührt und abermals abgesaugt. Das Produkt wurde im ULTS bei 40°C getrocknet.

| Beispiel | Struktur | 6-Chlorpyrimidin-4-yl)-(Derivate [Menge 6-Chlor-pyrimidinDerivat] Ausbeute | Analytische Daten |
|---|---|---|---|
| Vergleichsbeispiel: | 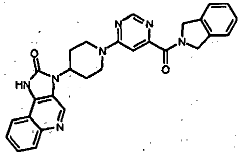 | (6-Chlorpyrimidin-4-yl)-(1,3-dihydro-isoindol-2-yl)-methanon | ESI-MS: m/z = 492 [M+H]⁺ |
| | 3-{1-[6-(1,3-Dihydro-isoindol-2-carbonyl)-pyrimidin-4-yl]-piperidin-4-yl}-1,3-dihydro-imidazo[4,5-*c*]chinolin-2-on | 95 mg (0.37 mmol) | R_{f} = 0.55 Laufmittel A |
| | | 130 mg (72% d.Th.) | |
| Vergleichsbeispiel: | 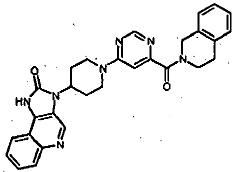 | (6-Chlorpyrimidin-4-yl)-(3,4-dihydro-1*H*-isochinolin-2-yl)-methanon | ESI-MS: m/z = 506 [M+H]⁺ |
| | 3-{1-[6-(3,4-Dihydro-1*H-*isochinolin-2-carbonyl)-pyrimidin-4-yl]-piperidin-4-yl}-1,3-dihydro-imidazo[4,5-*c*]chinolin-2-on | 100 mg (0.37 mmol) | R_{f} = 0.56 Laufmittel A |
| | | 120 mg (65% d.Th.) | |
| Vergleichsbeispiel: | 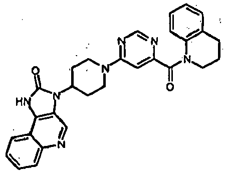 | (6-Chlorpyrimidin-4-yl)-(3,4-dihydro-2*H-*chinolin-1-yl)-methanon | ESI-MS: m/z = 506 [M+H]⁺ |
| | 3-{1-[6-(3,4-Dihydro-2*H-*chinolin-1-carbonyl)-pyrimidin-4-yl]-piperidin-4-yl}-1,3-dihydro-imidazo[4,5-*c*]chinolin-2-on | 100 mg (0.37 mmol) | R_{f} = 0.53 Laufmittel A |
| | | 55 mg (30% d.Th.) | |

### Beispiel 11:

### 3-{1-[6-(2,3-Dihydroindol-1-carbonyl)-pyrimidin-4-yl]-piperidin-4-yl}-7-methoxy-1,3,4,5-tetrahydro-1,3-benzodiazepin-2-on

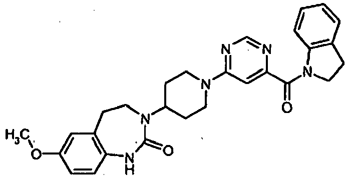

Zu 100 mg (0.39 mmol) (6-Chlorpyrimidin-4-yl)-(2,3-dihydroindol-1-yl)-methanon und 100 µL (0.58 mmol) DIPEA in 10 mL DMF wurden 110 mg (0.4 mmol) 7-Methoxy-3-piperidin-4-yl-1,3,4,5-tetrahydro-1,3-benzodiazepin-2-on zugegeben. Der Reaktionsansatz wurde 2 h bei RT gerührt und anschließend i.vac. eingeengt. Der Rückstand wurde mit 20 mL Wasser versetzt und 30 min bei RT gerührt. Das ausgefallene Produkt wurde abgesaugt, mit Methanol verrührt, nochmals abgesaugt und im ULTS bei 40°C getrocknet.

| | |
|---|---|
| Ausbeute: | 35 mg (18% d.Th.) |
| ESI-MS: | m/z = 499 (M+H)⁺ |
| R_{f}: | 0.70 (Kieselgel, Laufmittel A) |

### Beispiel 12:

### 7-Chlor-3-{1-[6-(2,3-dihydroindol-1-carbonyl)-pyrimidin-4-yl]-piperidin-4-yl}-1,3,4,5-tetrahydro-1,3-benzodiazepin-2-on

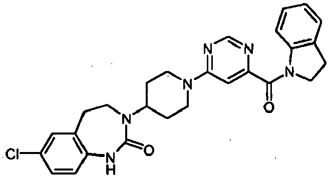

Zu 87.6 mg (0.34 mmol) (6-Chlorpyrimidin-4-yl)-(2,3-dihydro-indol-1-yl)-methanon und 63 µL (0.37 mmol) DIPEA in 2.5 mL DMF wurden 110 mg (0.36 mmol) 7-Chlor-3-piperidin-4-yl-1,3,4,5-tetrahydro-1,3-benzodiazepin-2-on zugegeben. Der Reaktionsansatz wurde über Nacht bei RT gerührt, dann am Rotationsverdampfer einrotiert und über präparative HPLC aufgereinigt. Die entsprechenden Produktfraktionen wurden vereinigt und am Rotationsverdampfer eingeengt. Der Rückstand wurde in DMF aufgenommen und mit Methanol versetzt. Die Substanz fiel aus, wurde abgesaugt, mit wenig Methanol gewaschen und getrocknet.

| | |
|---|---|
| Ausbeute: | 117 mg (69% d.Th.) |
| ESI-MS: | m/z = 503/505 (M+H)⁺ |
| Rₜ (HPLC-MS): | 3.87 min (Methode E) |

### Beispiel 13:

### 1-{1-[6-(5-Chlor-2,3-dihydroindol-1-carbonyl)-pyrimidin-4-yl]-piperidin-4-yl}-1,3-dihydro-imidazo[4,5-b]pyridin-2-on

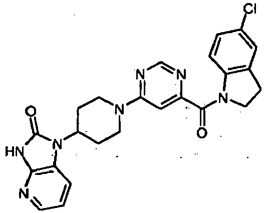

Zu 100 mg (0.34 mmol) (5-Chlor-2,3-dihydroindol-1-yl)-(6-chlorpyrimidin-4-yl)-methanon und 100 µL (0.58 mmol) DIPEA in 10 mL DMF wurden 80 mg (0.27 mmol) 1-Piperidin-4-yl-1,3-dihydro-imidazo[4,5-*b*]pyridin-2-on-Dihydrochlorid zugegeben. Der Reaktionsansatz wurde 22 h bei RT gerührt und anschließend i.vac. eingeengt. Der Rückstand wurde mit 20 mL Wasser versetzt und 30 min bei RT gerührt. Das ausgefallene Produkt wurde abgesaugt. Die Reinigung erfolgte über eine Kieselgel-Säule. Die Produktfraktionen wurden vereinigt und das Lösungsmittel am Rotationsverdampfer entfernt. Der Rückstand wurde mit Wasser verrührt, abgesaugt und getrocknet.

| | |
|---|---|
| Ausbeute: | 30 mg (19% d.Th.) |
| ESI-MS: | m/z = 476/478 (M+H)⁺ |
| R_{f}: | 0.44 (Kieselgel, Laufmittel A) |

### Allgemeine Arbeitsvorschrift 5 (AAV5) zur Umsetzung von (5-Chlor-2,3-dihydro-indol-1-yl)-(6-chlorpyrimidin-4-yl)-methanon mit Aminen:

Zu 100 mg (0.34 mmol) (5-Chlor-2,3-dihydro-indol-1-yl)-(6-chlorpyrimidin-4-yl)-methanon und 100 µL (0.58 mmol) DIPEA in 10 mL DMF wurde eine entsprechende Menge eines Amin-Derivats (s. Tabelle) zugegeben. Der Reaktionsansatz wurde 2 h bei RT gerührt, dann i.vac.eingeengt und der Rückstand mit 20 mL Wasser versetzt und 30 min bei RT gerührt. Das ausgefallene Produkt wurde abgesaugt, mit Methanol verrührt und abermals abgesaugt. Die Trocknung des Produkts erfolgte im ULTS bei 40°C. Die folgenden Beispiele wurden gemäß dieser allgemeinen Arbeitsvorschrift synthetisiert:

| Beispiel | Struktur | Amin [Menge Amin] Ausbeute | Analytische Daten |
|---|---|---|---|
| Beispiel 14: | 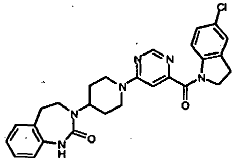 | 3-Piperidin-4-yl-1,3,4,5-tetrahydro-1,3-benzodiazepin-2-on | ESI-MS: m/z = 503/505 [M+H]⁺ |
| | 3-{1-[6-(5-Chlor-2,3-dihydro-indol-1-carbonyl)-pyrimidin-4-yl]-piperidin-4-yl}-1,3,4,5-tetrahydro-1,3-benzodiazepin-2-on | 90 mg (0.37 | |
| | | mmol) 145 mg (85% d.Th.) | R_{f} = 0.72 Laufmittel A |
| Beispiel 15: | 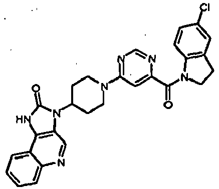 | 3-Piperidin-4-yl-1,3-dihydro-imidazo[4,5-*c*]chinolin-2-on | ESI-MS: m/z = 526/528 [M+H]⁺ |
| | 3-{1-[6-(5-Chlor-2,3-dihydro-indol-1-carbonyl)-pyrimidin-4-yl]-piperidin-4-yl}-1,3-dihydroimidazo[4,5-*c*]chinolin-2-on | 90 mg (0.34 mmol) | R_{f} = 0.50 Laufmittel A |
| | | 115 mg (64% d.Th.) | |
| Beispiel 16: | 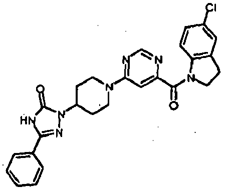 | 5-Phenyl-2-piperidin-4-yl-2,4-dihydro-1,2,4-triazol-3-on | ESI-MS: m/z = 502/504 [M+H]⁺ |
| | 2-{1-[6-(5-Chlor-2,3-dihydroindol-1-carbonyl)-pyrimidin-4-yl]-piperidin-4-yl}-5-phenyl-2,4-dihydro-1,2,4-triazol-3-on | 85 mg (0.35 mmol) | |
| | | 65 mg (38% d.Th.) | R_{f} = 0.54 Laufmittel A |
| Beispiel 17: | 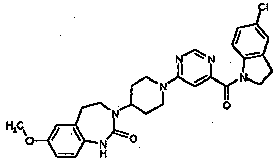 | 7-Methoxy-3-piperidin-4-yl-1,3,4,5-tetrahydro-1,3-benzodiazepin-2-on | ESI-MS: m/z = 533/535 [M+H]⁺ |
| | 3-{1-[6-(5-Chlor-2,3-dihydroindol-1-carbonyl)-pyrimidin-4-yl]-piperidin-4-yl}-7-methoxy-1,3,4,5-tetrahydro-1,3-benzodiazepin-2-on | 100 mg (0.36 mmol) | R_{f} = 0.68 Laufmittel A |
| | | 120 mg (66% d.Th.) | |

### Beispiel 18:

### 1-{1-[6-(5-Brom-2,3-dihydro-indol-1-carbonyl)-pyrimidin-4-yl]-piperidin-4-yl}-1,3-dihydro-imidazo[4,5-b]pyridin-2-on

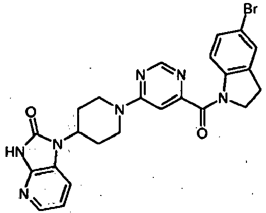

Zu 100 mg (0.30 mmol) (5-Brom-2,3-dihydroindol-1-yl)-(6-chlorpyrimidin-4-yl)-methanon und 100 µL (0.58 mmol) DIPEA in 10 mL DMF wurden 70 mg (0.24 mmol) 1-Piperidin-4-yl-1,3-dihydroimidazo[4,5-*b*]pyridin-2-on Dihydrochlorid zugegeben. Der Reaktionsansatz wurde 22 h bei RT gerührt und anschließend i.vac. eingeengt. Der Rückstand wurde mit 20 mL Wasser versetzt und 30 min bei RT gerührt. Das ausgefallene Produkt wurde abgesaugt. Die Reinigung erfolgte über eine Kieselgel-Säule. Die Produktfraktionen wurden vereinigt und das Lösungsmittel am Rotationsverdampfer entfernt. Der Rückstand wurde mit Wasser verrührt, abgesaugt und im ULTS bei 40°C getrocknet.

| | |
|---|---|
| Ausbeute: | 55 mg (36% d.Th.) |
| ESI-MS: | m/z = 520/522 (M+H)⁺ |
| R_{f}: | 0.44 (Kieselgel, Laufmittel A) |

### Allgemeine Arbeitsvorschrift 6 (AAV6) zur Umsetzung von (5-Brom-2,3-dihydro-indol-1-yl)-(6-chloripyrimidin-4-yl)-methanon mit Aminen:

Zu 100 mg (0.30 mmol) (5-Brom-2,3-dihydroindol-1-yl)-(6-chlorpyrimidin-4-yl)-methanon und 100 µL (0.58 mmol) DIPEA in 10 mL DMF wurden 0.30 mmol eines Aminderivats zugegeben. Der Reaktionsansatz wurde 2 h bei RT gerührt, dann i.vac. eingeengt und der Rückstand mit 20 mL Wasser versetzt und 30 min bei RT gerührt. Das ausgefallene Produkt wurde abgesaugt, mit Methanol verrührt und abermals abgesaugt. Die Trocknung des Produkts erfolgte im ULTS bei 40°C. Die folgenden Beispiele wurden gemäß dieser allgemeinen Arbeitsvorschrift synthetisiert:

| Beispiel | Struktur | Amin [Menge Amin] Ausbeute | Analytische Daten |
|---|---|---|---|
| Beispiel 19: | 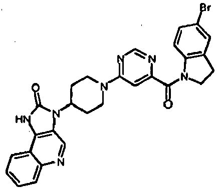 | 3-Piperidin-4-yl-1,3-dihydro-imidazo[4,5-c]chinolin-2-on | ESI-MS: m/z = 570/572 [M+H]⁺ |
| | -{1-[6-(5-Brom-2,3-dihydro-indol-1-carbonyl)-pyrimidin-4-yl]-piperidin-4-yl}-1,3-dihydro-imidazo[4,5-c]chinolin-2-on | 80 mg (0.30 mmol) | |
| | | 85 mg (51% d.Th.) | R_{f} = 0.43 Laufmittel A |
| Beispiel 20: | 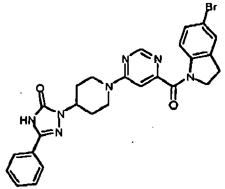 | 5-Phenyl-2-piperidin-4-yl-2,4-dihydro-1,2,4-triazol-3-on | ESI-MS: m/z = 546/548 [M+H]⁺ |
| | 2-{1-[6-(5-Brom-2,3-dihydroindol-1-carbonyl)-pyrimidin-4-yl]-piperidin-4-yl}-5-phenyl-2,4-dihydro-1,2,4-triazol-3-on | 75 mg (0.30 mmol) | R_{f} = 0.52 Laufmittel A |
| | | 30 mg (19% d.Th.) | |

### Beispiel 21:

### 1-{1-[6-(5-Fluor-2,3-dihydroindol-1-carbonyl)-pyrimidin-4-yl]-piperidin-4-yl}-1,3-dihydro-imidazo[4,5-b]pyridin-2-on

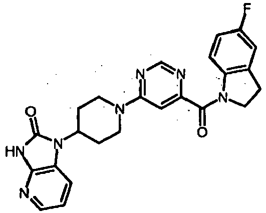

100 mg (0.294 mmol) 6-[4-(2-Oxo-2,3-dihydroimidazo[4,5-*b*]pyridin-1-yl)-piperidin-1-yl]-pyrimidin-4-carbonsäure, 42 mg (0.31 mmol) 5-Fluor-2,3-dihydro-1*H*-indol, 100 mg (0.311 mmol) TBTU und 45 µL (0.320 mmol) Triethylamin in 10 mL DMF wurden über Nacht bei RT gerührt. Der Reaktionsansatz wurde am Rotationsverdampfer eingeengt. Der Rückstand wurde in 3 mL DMF gelöst und mittels präparativer HPLC-MS aufgereinigt. Die Produktfraktionen wurden vereinigt und lyophilisiert.

| | |
|---|---|
| Ausbeute: | 50 mg (37% d.Th.) |
| ESI-MS: | m/z = 460 (M+H)⁺ |
| R_{f} : | 0.52 (Laufmittel A) |

### Beispiel 22:

### 1-{1-[6-(5-Fluor-3,3-dimethyl-2,3-dihydro-indol-1-carbonyl)-pyrimidin-4-yl]-piperidin-4-yl}-1,3-dihydroimidazo[4,5-b]pyridin-2-on

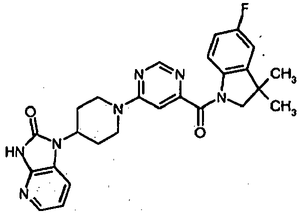

100 mg (0.294 mmol) 6-[4-(2-Oxo-2,3-dihydro-imidazo[4,5-*b*]pyridin-1-yl)-piperidin-1-yl]-pyrimidin-4-carbonsäure, 50 mg (0.300mmol) 5-Fluor-3,'3-dimethyl-2,3-dihydro-1*H*-indol, 106.1 mg (0.33 mmol) TBTU und 84 µL (0.60 mmol) Triethylamin in 2 mL DMF wurden über Nacht bei RT gerührt. Der Reaktionsansatz wurde mittels präparätiver HPLC-MS aufgereinigt. Die Produktfraktionen wurden vereinigt und lyophilisiert.

| | |
|---|---|
| Ausbeute: | 100 mg (70% d.Th.) |
| ESI-MS: | m/z = 488 (M+H)⁺ |
| Rₜ (HPLC): | 3.5 min (Methode C) |

### Beispiel 23:

### (5-Fluor-1-{6-[4-(2-oxo-2,3-dihydroimidazo[4,5-b]pyridin-1-yl)-piperidin-1-yl]-pyrimidin-4-carbonyl}-2,3-dihydro-1H-indol-3-yl)-essigsäuremethylester

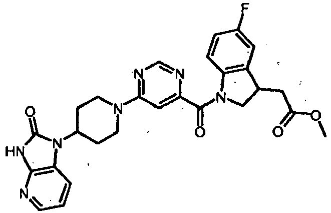

150 mg (0.441 mmol) 6-[4-(2-Oxo-2,3,-dihydroimidazo[4,5-*b*]pyridin-1-yl)-piperidin-1-yl]-pyrimidin-4-carbonsäure, 105 mg (0.500 mmol) (5-Fluor-2,3-dihydro-1*H*-indol-3-yl)-essigsäuremethylester, 148 mg (0.460 mmol) TBTU und 112 µL (0.80 mmol) Triethylamin in 2 mL DMF wurden 3 h bei RT gerührt. Ohne weitere Aufarbeitung wurde der Reaktionsansatz mittels präparativer HPLC-MS aufgereinigt. Die Produktfraktionen wurden vereinigt und lyophilisiert.

| | |
|---|---|
| Ausbeute: | 116mg (50%d.Th.) |
| ESI-MS: | m/z = 532 (M+H)⁺ |
| Rₜ (HPLC-MS): | 1.32 min (Methode C) |

### Beispiel 24:

### 1-{1-[6-(4,5-Difluor-2,3-dihydroindol-1-carbonyl)-pyrimidin-4-yl]-piperidin-4-yl}-1,3-dihydro-imidazo[4,5-b]pyridin-2-on

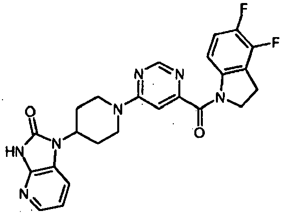

100 mg (0.294 mmol) 6-[4-(2-Oxo-2,3-dihydroimidazo[4,5-*b*]pyridin-1-yl)-piperidin-1-yl]-pyrimidin-4-carbonsäure, 60.0 mg (0.313 mmol) 4,5-Difluorindolin Hydrochlorid, 100 mg (0.311 mmol) TBTU und 45 µL (0.32 mmol) Triethylamin in 10 mL DMF wurden über Nacht bei RT gerührt. Der Reaktionsansatz wurde am Rotationsverdampfer unter Erwärmen und vermindertem Druck einrotiert. Der Rückstand wurde in 3 mL DMF gelöst und über eine präparative HPLC aufgereinigt. Dir Produktfraktionen wurden vereinigt und lyophilisiert.

| | |
|---|---|
| Ausbeute: | 85 mg (61 % d.Th.) |
| ESI-MS: | m/z = 478 (M+H)⁺ |
| R_{f} : | 0.52 (Laufmittel A) |

### Beispiel 25:

### 1-{1-[6-(3,3-Dimethyl-2,3-dihydroindol-1-carbonyl)-pyrimidin-4-yl]-piperidin-4-yl}-1,3-dihydro-imidazo[4,5-b]pyridin-2-on

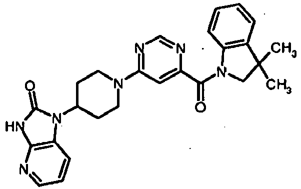

70 mg (0.21 mmol) 6-[4-(2-Oxo-2,3-dihydroimidazo[4,5-*b*]pyridin-1-yl)-piperidin-1-yl]-pyrimidin-4-carbonsäure, 30.33 mg (0.21 mmol) 3,3-Dimethyl-2,3-dihydro-1*H*-indol, 71 mg (0.22 mmol) TBTU und 56 µL (0.40 mmol) Triethylamin in 2 mL DMF wurden über Nacht bei RT gerührt. Der Ansatz wurde mittels präparativer HPLC getrennt. Die Produktfraktionen wurden vereinigt und lyophilisiert.

| | |
|---|---|
| Ausbeute: | 55 mg (57% d.Th.) |
| ESI-MS: | m/z = 470 (M+H)⁺ |
| Rₜ (HPLC-MS): | 3.5 min (Methode K) |

### Beispiel 26:

### 1-(1-{6-[3-(3-Pyrrolidin-1-yl-propyl)-2,3-dihydroindol-1-carbonyl]-pyrimidin-4-yl}-piperidin-4-yl)-1,3-dihydro-imidazo[4,5-b]pyridin-2-on

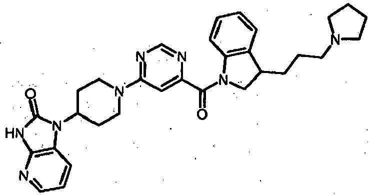

140 mg (0.41 mmol) 6-[4-(2-Oxo-2,3-dihydroimidazo[4,5-*b*]pyridin-1-yl)-piperidin-1-yl]-pyrimidin-4-carbonsäure, 94.4 mg (0.41 mmol) 3-(3-Pyrrolidin-1-yl-propyl)-2,3-dihydro-1*H-*indol, 138.2 mg (0.43 mmol) TBTU und 112 µL (0.8 mmol) Triethylamin in 4 mL DMF wurden über Nacht bei RT gerührt. Der Ansatz wurde mittels präparativer HPLC getrennt. Die Produktfraktionen wurden vereinigt und lyophilisiert.

| | |
|---|---|
| Ausbeute: | 55 mg (24% d.Th.) |
| ESI-MS: | m/z = 553 (M+H)⁺ |
| Rₜ (HPLC-MS): | 1.0 min (Methode C) |

### Beispiel 27:

### 1-{6-[4-(2-Oxo-2,3-dihydroimidazo[4,5-b]pyridin-1-yl)-piperidin-1-yl]-pyrimidin-4-carbonyl}-2,3-dihydro-1H-indol-2-carbonsäureethylester

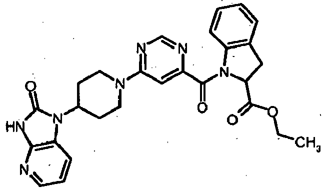

150 mg (0.44 mmol) 6-[4-(2-Oxo-2,3-dihydroimidazo[4,5-*b*]pyridin-1-yl)-piperidin-1-yl]-pyrimidin-4-carbonsäure, 110 mg (0.48 mmol) 2,3-Dihydro-1*H*-indol-2-carbonsäureethyl-ester-Hydrochlorid, 150.0 mg (0.47 mmol) TBTU und 150 µL (1.1 mmol) Triethylamin in 2 mL DMF wurden über Nacht bei RT gerührt. Der Ansatz wurde über einen Spritzenfilter filtriert und die Lösung mittels präparativer HPLC gereinigt. Die Produktfraktionen wurden vereinigt und lyophilisiert.

| | |
|---|---|
| Ausbeute: | 35 mg (16% d.Th.) |
| ESI-MS: | m/z = 514 (M+H)⁺ |
| R_{f} : | 0.55 (Kieselgel, Laufmittel A) |

### Beispiel 28:

### 1-(1-(6-(Spiro[cyclobutan-1,3'-indolin]-1'-ylcarbonyl)pyrimidin-4-yl)piperidin-4-yl)-1H-imidazo[4,5-b]pyridin-2(3H)-on

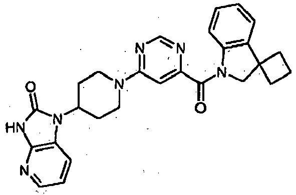

214 mg (0.63 mmol) 6-[4-(2-Oxo-2,3-dihydro-imidazo[4,5-*b*]pyridin-1-yl)-piperidin-1-yl]-pyrimidin-4-carbonsäure, 100 mg (0.628 mmol) Spiro[cyclobutan-1,3'-indolin], 212mg (0.66 mmol) TBTU und 168 µL (1.20 mmol) Triethylamin in 4 mL DMF wurden über Nacht bei RT gerührt. Der Ansatz wurde mittels präparativer HPLC getrennt. Die Produktfraktionen wurden vereinigt und das Acetonitril am Rotationsverdampfer abgezogen. Die ausgefallene Substanz wurde abgesaugt, mit 20 mL Wasser nachgewaschen und im ULTS bei 50°C getrocknet.

| | |
|---|---|
| Ausbeute: | 187 mg (62% d.Th.) |
| ESI-MS: | m/z = 482 (M+_{H})⁺ |
| Rₜ (HPLC-MS): | 1.37 min (Methode C) |

### Allgemeine Arbeitsvorschrift 7 (AAV7) zur Umsetzung von 6-[4-(2-Oxo-2,3-dihydro-imidazo[4,5-b]pyridin-1-yl)-piperidin-1-yl]-pyrimidin-4-carbönsäure mit Aminen:

Zu 100 mg (0.29 mmol) 6-[4-(2-Oxo-2,3-dihydro-imidazo[4,5-*b*]pyridin-1-yl)-piperidin-1-yl]-pyrimidin-4-carbonsäure, einer entsprechenden Menge eines Amins (s. Tabelle) und 45 µL Triethylamin in 10.0 mL DMF wurden 100 mg (0.31 mmol) TBTU hinzugefügt und über Nacht bei RT gerührt. Der Reaktionsansatz wurde am Rotationsverdampfer eingedampft. Der Rückstand wurde in 3 mL DMF gelöst und mittels einer präparativen HPLC aufgereinigt. Die Produktfraktionen wurden vereinigt und anschließend lyophilisiert.

| Beispiel | Struktur | [Menge Amin] Amin Ausbeute | Analytische Daten |
|---|---|---|---|
| Verqleichsbeispiel: | 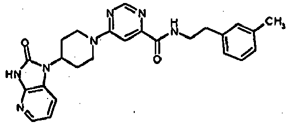 | 40 mg (0.30 mmol) | ESI-MS: m/z = 458 [M+H]⁺ |
| | 6-[4-(2-Oxo-2,3-dihydro-imidazo[4,5-*b*]pyridin-1-yl)-piperidin-1-yl]-pyrimidin-4-carbonsäure (2-*m*-tolylethyl-amid | 3-Methyl-phenethylamin | |
| | | 48 mg (36% d.Th.) | R_{f} = 0.57 Laufmittel A |
| Vergleichsbeispiel: | 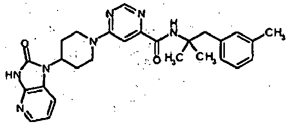 | 50 mg (0.31 mmol) | ESI-MS: m/z = 486 [M+H]⁺ |
| | 6-[4-(2-Oxo-2,3-dihydro-imidazo[4,5-*b*]pyridin-1-yl)-piperidin-1-yl]-pyrimidin-4-carbonsäure (1,1-dimethyl-2-*m*-tolyl-ethyl)-amid | 1,1-Dimethyl-2-*m*-tolylethylamin | |
| | | 72 mg (51% d.Th.) | R_{f} = 0.66 Laufmittel A |
| Beispiel 29: | | 40 µL (0.31 mmol) | |
| | 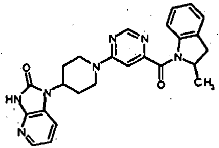 | 2-Methyl-2,3-dihydro-1*H-*indol | ESI-MS: m/z = 456 [M+H]⁺ |
| | 1-{1-[6-(2-Methyl-2,3-dihydro-indol-1-carbonyl)-pyrimidin-4-yl]-piperidin-4-yl}-1,3-dihydroimidazo[4,5-*b*]pyridin-2-on | 91 mg (68% d.Th.) | R_{f} = 0.59 Laufmittel A |
| | 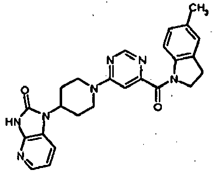 | 40 µL (0.31 mmol) | ESI-MS: m/z = 456 [M+H]⁺ |
| Beispiel 30: | 1-{1-[6-(5-Methyl-2,3-dihydro-indol-1-carbonyl)-pyrimidin-4-yl]-piperidin-4-yl}-1,3-dihydroimidazo[4,5-*b*]pyridin-2-on | 5-Methyl-2,3-dihydro-1*H-*indol | |
| | | 80 mg (60% d.Th.) | R_{f} = 0.40 Laufmittel A |

### Beispiel 31:

### 1-{6-[4-(2-Oxo-2,3-dihydroimidazo[4,5-b]pyridin-1-yl)-piperidin-1-yl]-pyrimidin-4-carbonyl} 2,3-dihydro-1H-indol-2-carbonsäure

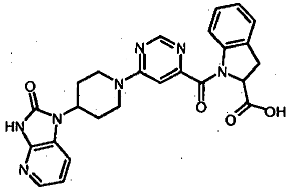

107 mg (0.31 mmol) 6-[4-(2-Oxo-2,3-dihydroimidazo[4,5-*b*]pyridin-1-yl)-piperidin-1-yl]-pyrimidin-4-carbonsäure, 65.0 mg (0.40 mmol) 2,3-Dihydro-1*H*-indol-2-carbonsäure, 107.0 mg (0.33 mmol) TBTU und 100 µL (0.71 mmol) Triethylamin in 10 mL DMF wurden über Nacht bei RT gerührt. Der Ansatz wurde i.vac. eingeengt und der Rückstand in 5 mL DMF gelöst. Die Reinigung erfolgte mittels präparativer HPLC. Die Produktfraktionen wurden vereinigt und lyophilisiert.

| | |
|---|---|
| Ausbeute: | 10 mg (7% d.Th.) |
| ESI-MS: | m/z = 486 (M+H)⁺ |
| R_{f} : | 0.07 (Kieselgel, Laufmittel A) |

### Allgemeine Arbeitsvorschrift 8 (AAV8) zur Umsetzung von 6-[4-(2-Oxo-2,3-dihydro-imidazo[4,5-b]pyridin-1-yl)-piperidin-1-yl]-pyrimidin-4-carbonsäure mit Aminen:

Zu 100 mg (0.29 mmol) 6-[4-(2-Oxo-2,3-dihydroimidazo[4,5-*b*]pyridin-1-yl)-piperidin-1-yl]-pyrimidin-4-carbonsäure, einer entsprechende Menge Amin (s. Tabelle) und 100 µL (0.71 mmol) Triethylamin in 1.5 mL DMF wurden 100 mg (0.31 mmol) TBTU hinzugefügt und über Nacht bei RT gerührt. Die Aufarbeitung erfolgte auf zwei verschiedene Arten:
[A] Der Reaktionsansatz wurde mit 5 mL Wasser verdünnt und mittels einer präparativen HPLC aufgereinigt. Die Produktfraktionen wurden vereinigt und anschließend lyophilisiert.
[B] Der Reaktionsansatz wurde über einen Spritzenfilter filtriert und mittels einer präparativen HPLC aufgereinigt. Die Produktfraktionen wurden vereinigt und anschließend lyophilisiert.

| Beispiel Methode | Struktur | [Menge Amin] Amin Ausbeute | Analytische Daten |
|---|---|---|---|
| Vergleichsbeispiel: AAV 8[A] | 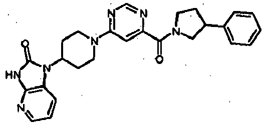 | 55 mg (0.3 mmol) | ESI-MS: m/z = 470 [M+H]⁺ |
| | 1-{1-[6-(3-Phenyl-pyrrolidin-1-carbonyl)-pyrimidin-4₋yl]-piperidin-4-yl}-1,3-dihydroimidazo[4,5-*b*]pyridin-2-on | 3-Phenyl-pyrrolidin-hydrochlorid | |
| | | 55 mg (40% d.Th.) | R_{f} = 0.59 Laufmittel) A |
| Vergleichsbeispiel: AAV 8[A] | 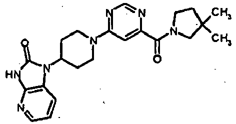 | 41 mg (0.3 mmol) | ESI-MS: m/z = 422 [M+H]⁺ |
| | 1-{1-[6-(3,3-Dimethylpyrrolidin-1-carbonyl)-pyrimidin-4-yl]-piperidin-4-yl}-1,3-dihydroimidazo[4,5-*b*]pyridin-2-on | 3,3-Dimethyl-pyrrolidin hydrochlorid | |
| | | 65 mg (53% d.Th.) | R_{f} = 0.57 Laufmittel A |
| Vergleichsbeispiel: AAV 8[A] | 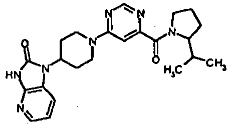 | 45 mg (0.30 mmol) | ESI-MS: m/z = 436 [M+H]⁺ |
| | | 2-(Methylethyl)-pyrrolidin-hydrochlorid | |
| | 1-{1-[6-(2-Isopropyl-pyrrolidin-1-carbonyl)-pyrimidin-4-yl]-piperidin-4-yl}-1,3-dihydro-imidazo[4,5-*b*]pyridin-2-on | | R_{f} = 0.57 Laufmittel A |
| | | 65 mg (51% d.Th.) | |
| Vergleichsbeispiel: AAV 8[B] | 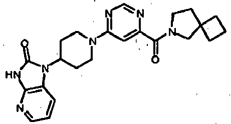 | 40 mg (0.36 mmol) | ESI-MS: m/z = 434 [M+H]⁺ |
| | 1-{1-[6-(6-Aza-spiro[3.4]octan-6-carbonyl)-pyrimidin-4-yl]-piperidin-4-yl}-1,3-dihydro-imidazo[4,5-*b*]pyridin-2-on | 6-Aza-spiro[3.4]octan | |
| | | 40 mg (31% d.Th.) | R_{f} = 0.61 Laufmittel A |
| Vergleichsbeispiel: AAV 8[B] | 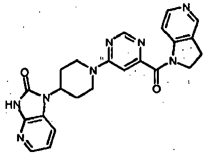 | 40 mg (0.33 mmol) | ESI-MS: m/z = 443 [M+H]⁺ |
| | | 2,3-Dihydro-1*H-*pyrrolo[3,2-*c*]pyridin | |
| | 1-{1-[6-(2,3-Dihydro-pyrrolo[3,2-*c*]pyridin-1-carbonyl)-pyrimidin-4-yl]-piperidin-4-yl}-1,3-dihydroimidazo[4,5-*b*]pyridin-2-on | | |
| | | 60 mg (46% d.Th.) | R_{f} = 0.50 Laufmittel A |

### Allgemeine Arbeitsvorschrift 9 (AAV9) zur Umsetzung von 6-[4-(2-Oxo-2,3-dihydro-imidazo[4,5-b]pyridin-1-yl)-piperidin-1-yl]-pyrimidin-4-carbonsäure mit Aminen:

Zu 60 mg (0.18 mmol) 6-[4-(2-Oxo-2,3-dihydroimidazo[4,5-*b*]pyridin-1-yl)-piperidin-1-yl]-pyrimidin-4-carbonsäure, 0.18 mmol Amin und 52 µL (0.37 mmol) Triethylamin in 1.5 mL DMF wurden 62 mg (0.19 mmol) TBTU hinzugefügt und 4 h bei RT gerührt. Der Reaktionsansatz wurde mittels präparativer HPLC aufgereinigt. Die Produktfraktionen wurden vereinigt und lyophilisiert.

| Beispiel | Struktur | [Menge Amin] Amin Ausbeute | Analytische Daten |
|---|---|---|---|
| Vergleichsbeispiel: | 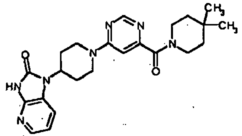 | 31 mg (0.18 mmol) | ESI-MS: m/z = 436 [M+H]⁺ |
| | 1-{1-[6-(4,4-Dimethyl-piperidin-1-carbonyl)-pyrimidin-4-yl]-piperidin-4-yl}-1,3-dihydroimidazo[4,5-*b*]pyridin-2-on | 4,4-Dimethyl-piperidin | |
| | | 16 mg (21% d.Th.) | Rₜ = 1.12 min Methode C |
| Vergleichsbeispiel: | 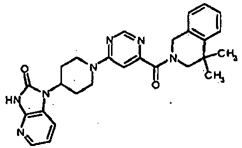 | 28 mg (0.18 mmol) | ESI-MS: m/z = 484 [M+H]⁺ |
| | | 4,4-Dimethyl-1,2,3,4-tetrahydroisochinolin | |
| | 1-{1-[6-(4,4-Dimethyl-3,4-dihydro-1*H*-isochinolin-2-carbonyl)-pyrimidin-4-yl]-piperidin-4-yl}-1,3-dihydroimidazo[4,5-*b*]pyridin-2-on | | Rₜ = 1.25 min Methode C |
| | | 35 mg (41% d.Th.) | |
| Vergleichsbeispiel: | 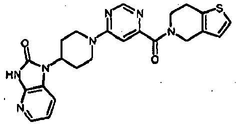 | 24.5 mg (0.18 mmol) | ESI-MS: m/z = 462 [M+H]⁺ |
| | 1-{1-[6-(6,7-Dihydro-4*H-*thieno[3,2-*c*]pyridin-5-carbonyl)-pyrimidin-4-yl]-piperidin-4-yl}-1,3-dihydroimidazo[4,5-*b*]pyridin-2-on | 4,5,6,7-Tetrahydro-thieno[3,2-*c*]pyridin | Rₜ = 1.13 min Methode C |
| | | 36 mg (44% d.Th.) | |
| Vergleichsbeispiel: | 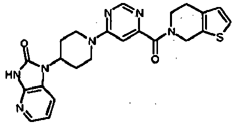 | 25 mg (0.18 mmol) | ESI-MS: m/z = 462 [M+H]⁺ |
| | 1-{1-[6-(4,7-Dihydro-5*H-*thieno[2,3-*c*]pyridin-6-carbonyl)-pyrimidin-4-yl]-piperidin-4-yl}-1,3-dihydro-imidazo[4,5-*b*]pyridin-2-on | 4,5,6,7-Tetrahydro-thieno[2,3-*c*]pyridin | |
| | | | Rₜ = 1.15 min Methode C |
| | | 32 mg (39% d.Th.) | |

### Beispiel 32:

### 1-{1-[6-(4-Amino-2,3-dihydroindol-1-carbonyl)-pyrimidin-4-yl]-piperidin-4-yl}-1,3-dihydro-imidazo[4,5-b]pyridin-2-on

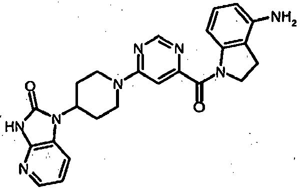

Unter einer Wasserstoff-Atmosphäre wurden 130 mg (0.27 mmol) 1-{1-[6-(4-Nitro-2,3-dihydroindol-1-carbonyl)-pyrimidin-4-yl]-piperidin-4-yl}-1,3-dihydroimidazo[4,5-*b*]pyridin-2-on und 25 mg Palladium auf Kohle (10%) in einer 1:1 Mischung Methanol:THF (je 15 mL) bei RT und 3 bar Wasserstoffdruck hydriert. Anschließend wurde der Katalysator abgesaugt und mit 50 mL DMF gewaschen. Das Filtrat wurde i.vac. eingeengt, der Rückstand mit Methanol verrührt und abgesaugt. Der Feststoff wurde bei 50°C im ULTS getrocknet.

| | |
|---|---|
| Ausbeute: | 40 mg (33% d.Th.) |
| ESI-MS: | m/z = 457 (M+H)⁺ |
| R_{f}: | 0.53 (Kieselgel, Laufmittel A) |

### Beispiel 33:

### 1-{1-[6-(5-Amino-2,3-dihydro-indol-1-carbonyl)-pyrimidin-4-yl]-piperidin-4-yl}-1,3-dihydro-imidazo[4,5-b]pyridin-2-on

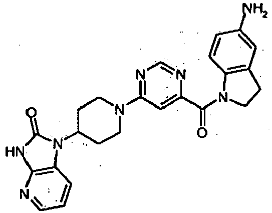

Unter einer Wasserstoff-Atmosphäre wurden 30 mg (0.06 mmol) 1-{1-[6-(5-Nitro-2,3-di-hydro-indol-1-carbonyl)-pyrimidin-4-yl]-piperidin-4-yl}-1,3-dihydro-imidazo[4,5-*b*]pyridin-2-on und 10 mg Palladium auf Kohle (10%) min einer 1:1 Mischung Methanol:THF (je 5 mL) bei RT und 3 bar Wasserstoffdruck hydriert. Anschließend wurde der Katalysator abgesaugt und das Filtrat i.vac. eingeengt. Der Rückstand wurde in 1 mL DMF gelöst, über einen Spritzenfilter filtriert und mittels präparativer HPLC aufgereinigt. Die Produktfraktionen wurden vereinigt und lyophilisiert.

| | |
|---|---|
| Ausbeute: | 1 mg (4% d.Th.) |
| ESI-MS: | m/z = 457 (M+H)⁺ |
| R_{f} : | 0.51 (Kieselgel, Laufmittel A) |

### Vergleichsbeispiel:

### 3-{1-[6-(7,8-Dihydro-5H-1,6-naphthyridin-6-carbonyl)-pyrimidin-4-yl]-piperidin-4-yl}-7-methoxy-1,3,4,5-tetrahydro-1,3-benzodiazepin-2-on

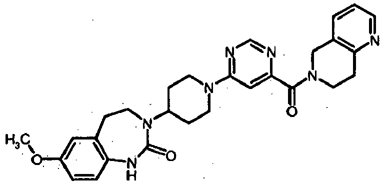

Unter einer Wasserstoff-Atmosphäre wurden 77 mg (0.12 mmol) 3-{1-[6-(3-Brom-7,8-di-hydro-5*H*-1,6-naphthyridin-6-carbonyl)-pyrimidin-4-yl]-piperidin-4-yl}-7-methoxy-1,3,4,5-tetrahydro-1,3-benzodiazepin-2-on und 40 mg Palladium auf Kohle (10%) in einer 10 mL Methanol bei 50°C und 50 psi Wasserstoffdruck hydriert. Anschließend wurde der Katalysator abgesaugt und das Filtrat i.vac. eingeengt. Der Rückstand wurde mittels präparativer HPLC aufgereinigt. Die Produktfraktionen wurden vereinigt und lyophilisiert.

| | |
|---|---|
| Ausbeute: | 27 mg (43% d.Th.) |
| ESI-MS: | m/z = 514 (M+H)⁺ |
| Rₜ (HPLC-MS): | 2.19 min (Methode E) |

### Vergleichsbeispiel:

### 6-[4-(2-Oxo-2, 3-dihydro-imidazo[4,5-b]pyridin-1-yl)-piperidin-1-yl]-pyrimidin-4-carbon-säure-indan-2-ylamid

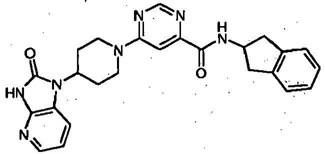

100 mg (0.29 mmol) 6-[4-(2-Oxo-2,3-dihydro-imidazo[4,5-*b*]pyridin-1-yl)-piperidin-1-yl]-pyrimidin-4-carbonsäure, 40.0 mg (0.30 mmol) Indan-2-ylamin, 100 mg (0.31 mmol) TBTU und 45 µL (0.32 mmol) Triethylamin in 10 mL DMF wurden über Nacht bei RT gerührt. Der Ansatz wurde i.vac. eingeengt, der Rückstand in 3 mL DMF gelöst und mittels präparativer HPLC aufgereinigt. Die Produktfraktionen wurden vereinigt und lyophilisiert.

| | |
|---|---|
| Ausbeute: | 62 mg (46% d.Th.) |
| ESI-MS: | m/z = 456 (M+H)⁺ |
| R_{f} : | 0.74 (Kieselgel, Laufmittel A) |

### Vergleichsbeispiel:

### 6-[4-(7-Methoxy-2-oxo-1,2,4,5-tetrahydro-1,3-benzodiazepin-3-yl)-piperidin-1-yl]-pyrimidin-4-carbonsäure-phenylamid

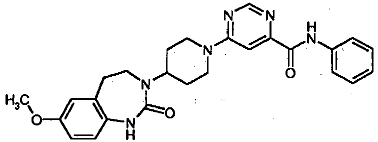

120.0 mg (0.30 mmol) 6-[4-(7-Methoxy-2-oxo-1,2,4,5-tetrahydro-1,3-benzodiazepin-3-yl)-piperidin-1-yl]-pyrimidin-4-carbonsäure, 30 µL (0.33 mmol) Anilin, 120.0 mg (0.37 mmol) TBTU und 100 µL (0.71 mmol) Triethylamin in 2 mL DMF wurden über Nacht bei RT gerührt. Der Ansatz wurde über einen Spritzenfilter filtriert und mittels präparativer HPLC gereinigt. Die Produktfraktionen wurden vereinigt und lyophilisiert.

| | |
|---|---|
| Ausbeute: | 65 mg (46% d.Th.) |
| ESI-MS: | m/z = 473 (M+H)⁺ |
| R_{f} : | 0.57 (Kieselgel, Laufmittel A) |

### Beispiel 34:

### 1-{1-[6-(Indol-1-carbonyl)-pyrimidin-4-yl]-piperidin-4-yl}-1,3-dihydro-imidazo[4,5-b]pyridin-2-on

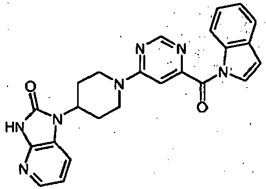

Zu 100 mg (0.39 mmol) (6-Chlorpyrimidin-4-yl)-indol-1-yl-methanon in 30 mL DMF wurden 100 mg (0.34 mmol) 1-Piperidin-4-yl-1,3-dihydro-imidazo[4,5-*b*]pyridin-2-on Dihydrochlorid und 200 mL (1.162 mmol) DIPEA zugegeben. Der Reaktionsansatz wurde 3 h bei RT gerührt. Der Reaktionsansatz wurde i.vac. eingeengt. Der Rückstand wurde mit 20 mL Wasser versetzt und 10 min nachgerührt. Der Feststoff wurde abgesaugt und mittels flash-Chromatographie aufgereinigt. Die Produktfraktionen wurden vereinigt und i.vac. eingeengt. Der Rückstand wurde mit Diisopropylether verrieben und abgesaugt. Der Feststoff wurde im ULTS bei 40°C getrocknet.

| | |
|---|---|
| Ausbeute: | 75 mg (44% d.Th.) |
| ESI-MS: | m/z = 440 (M+H)⁺ |
| R_{f} : | 0.55 (Kieselgel, Laufmittel A) |

### Beispiel 35:

### 1-{1-[6-(3-Methyl-indol-1-carbonyl)-pyrimidin-4-yl]-piperidin-4-yl}-1,3-dihydro-imidazo-[4,5-b]pyridin-2-on

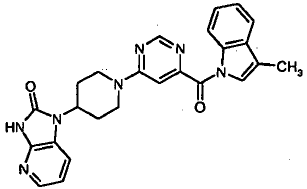

Zu 200 mg (0.74 mmol) (6-Chlorpyrimidin-4-yl)-(3-methyl-indol-1-yl)-methanon und 413 µL (2.40 mmol) DIPEA in 5 mL DMF wurden 227 mg (0.78 mmol) 1-Piperidin-4-yl-1,3-dihydro-imidazo[4,5-*b*]pyridin-2-on Dihydrochlorid zugegeben. Der Reaktionsansatz wurde über Nacht bei RT gerührt und anschließend mittels präparativer HPLC aufgereinigt. Die Produktfraktionen wurden vereinigt und am Roationsverdampfer einrotiert. Der Rückstand wurde in Methanol aufgenommen und über eine Kieselgel-Säule gereinigt. Die Produktfraktionen wurden vereinigt und am Roationsverdampfer einrotiert.

| | |
|---|---|
| Ausbeute: | 210 mg (63% d.Th.) |
| ESI-MS: | m/z = 454 (M+H)⁺ |
| Rₜ (HPLC-MS): | 1.46 min (Methode C) |

### Beispiel 36

### 1-{1-[6-(5-Fluorindol-1-carbonyl)-pyrimidin-4-yl]-piperidin-4-yl}-1,3-dihydro-imidazo[4,5-b]-pyridin-2-on

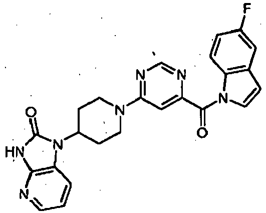

Zu 70 mg (0.25 mmol) (6-Chlorpyrimidin-4-yl)-(5-fluorindol-1-yl)-methanon in 5 mL DMF wurden 75.0 mg (0.26 mmol) 1-Piperidin-4-yl-1,3-dihydro-imidazo[4,5-*b*]pyridin-2-on Dihydrochlorid und 150 µL (0.87mmol) DIPEA zugegeben. Der Reaktionsansatz wurde über Nacht bei RT gerührt und anschließend i.vac. eingeengt. Der Rückstand wurde mit 20 mL Wasser aufgenommen und 10 min gerührt. Der Niederschlag wurde abgesaugt, in 2.5 mL DMF gelöst und mittels präparativer HPLC aufgereinigt. Die Produktfraktionen wurden vereinigt und am Roationsverdampfer einrotiert.

| | |
|---|---|
| Ausbeute: | 40 mg (34% d.Th.) |
| ESI-MS: | m/z = 458 (M+H)⁺ |
| R_{f} (Kieselgel): | 0.45 (Laufmittel A) |

### Beispiel 37

### 3-{1-[6-(4,5-Difluor-2,3-dihydro-indol-1-carbonyl)-pyrimidin-4-yl]-piperidin-4-yl}-7-methoxy-1,3,4,5-tetrahydro-1,3-benzodiazepin-2-on

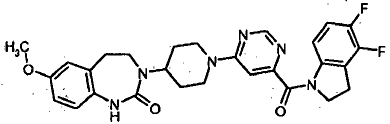

0.12 g (0.29 mmol) 6-[4-(7-Methoxy-2-oxo-1,2,4,5-tetrahydro-1,3-benzodiazepin-3-yl)-piperidin-1-yl]-pyrimidin-4-carbonsäure, 60 mg (0.31 mmol) 4,5-Difluorindolin-Hydrochlorid, 0.10 mL (0.71 mmol) TEA und 0.10 g (0.31 mmol) TBTU wurden in 10 mL DMF über Nacht bei RT gerührt. Das Reaktionsgemisch wurde zur Trockene einrotiert und dann mittels HPLC gereinigt. Die Produkt enthaltenden Fraktionen wurden vereinigt und gefriergetrocknet.

| | |
|---|---|
| Ausbeute: | 58 mg (38% d.Th.) |
| ESI-MS: | m/z = 535 (M+H)⁺ |
| R_{f} (Kieselgel): | 0.70 (DCM/Cyclohexan/MeOH/NH4OH = 70:15:15:2) |

Analog zu 3-{1-[6-(4,5-Difluor-2,3-dihydro-indol-1-carbonyl)-pyrimidin-4-yl]-piperidin-4-yl}-7-methoxy-1,3,4,5-tetrahydro-1,3-benzodiazepin-2-on wurden folgende Verbindungen aus jeweils 0.15 - 0.44 mmol 6-[4-(7-Methoxy-2-oxo-1,2,4,5-tetrahydro-1,3-benzodiazepin-3-yl)-piperidin-1-yl]-pyrimidin-4-carbonsäure, 1.0 bis 1.3 eq TBTU, 1.2 bis 4.2 eq TEA und 1 bis 1.5 eq des jeweiligen Amins in einer geeigneten Menge Lösungsmittel wie NMP oder DMF hergestellt:

| Bsp. | Struktur Name | [Menge Amin] Amin Ausbeute | Analytische Daten |
|---|---|---|---|
| 38 | 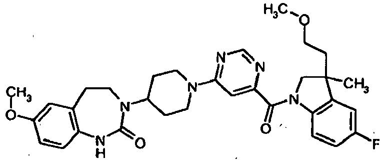 | 80 mg (0.38 mmol) | ESI-MS: m/z = 589 [M+H]⁺ |
| | 3-(1-{6-[5-Fluor-3-(2-methoxy-ethyl)-3-methyl-2,3-dihydro-indol-1-carbonyl]-pyrimidin-4-yl}-piperidin-4-yl)-7-methoxy-1,3,4,5-tetrahydro-1,3-benzodiazepin-2-on | 5-Fluor-3-(2-methoxy-ethyl)-3-methyl-2,3-dihydro-1H-indol_{.} | |
| | | 110 mg (49% d.Th.) | Rₜ = 1.54 min Methode C |
| 39 | 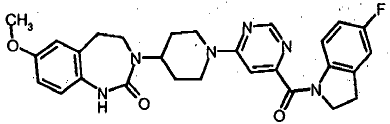 | 45 mg (0.33 mmol) | ESI-MS: m/z = 517 [M+H]⁺ |
| | 3-{1-[6-(5-Fluor-2,3-dihydro-indol-1-carbonyl)-pyrimidin-4-yl]-piperidin-4-yl}-7-methoxy-1,3,4,5-tetrahydro-1,3-benzodiazepin-2-on | 5-Fluor-2,3-dihydro-1 H-indol | |
| | | 94 mg (60% d.Th.) | R_{f} = 0.75 Kieselgel, (Laufmitte I A |
| 40 | 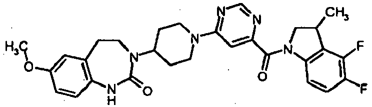 | 37 mg (0.22 mmol) | ESI-MS: m/z = 549 [M+H]⁺ |
| | 3-{1-[6-(4,5-Difluor-3-methyl-2,3-dihydro-indol-1-carbonyl)-pyrimidin-4-yl]-piperidin-4-yl}-7-methoxy-1,3,4,5-tetrahydro-1,3-benzodiazepin-2-on | 4,5-Difluor-3-methyl-2,3-dihydro-1H-indol | |
| | | 70 mg (63% d.Th.) | Rₜ = 4.2 min Methode K |
| 41 | 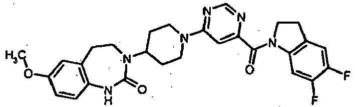 | 47 mg (0.30 mmol) | ESI-MS: m/z = 535 [M+H]⁺ |
| | 3-{1-[6-(5,6-Difluor-2,3-dihydro-indol-1-carbonyl)-pyrimidin-4-yl]-piperidin-4-yl}-7-methoxy-1,3,4,5-tetrahydro-benzodiazepin-2-on | 5,6-Difluor-2,3-dihydro-1H-indol | |
| | | 66 mg (49% d.Th.) | Rₜ =1.5 min Methode C |
| | | | |
| | 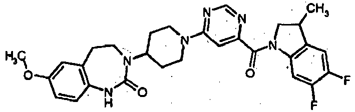 | 36 mg (0.21 mmol) | ESI-MS: m/z = 549 [M+H]⁺ |
| 42 | 3-{1-[6-(5,6-Difluor-3-methyl-2,3-dihydro-indol-1-carbonyl)-pyrimidin-4-yl]-piperidin-4-yl}-7-methoxy-1,3,4,5-tetrahydro-1,3-benzodiazepin-2-on | 5,6-difluor-3-methyl-2,3-dihydro-1H-indol | |
| | | 45 mg (41% d.Th.) | Rₜ = 4.4 min Methode K |
| Ver glei chs beis piel | 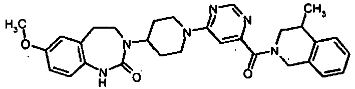 | 37 mg (0.20 mmol) | |
| | | 4-Methyl-1,2,3,4-tetrahydro-isochinolin Hydrochlorid | ESI-MS: m/z = 527 [M+H]⁺ |
| | 7-Methoxy-3-{1-{6-(4-methyl-3,4-dihydro-1H-isochinolin-2-carbonyl)-pyrimidin-4-yl]-piperidin-4-yl}-1,3,4,5-tetrahydro-1,3-benzodiazepin-2-on | | Rₜ = 1.38 min Methode C |
| | | 17 mg (18% d.Th.) | |
| Ver glei chs beis piel | 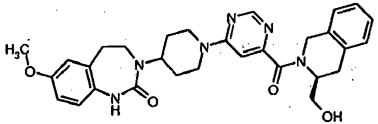 | 25 mg (0.15 mmol) | |
| | | S-(-)-(1,2,3,4-Tetrahydroisochinolin-3-yl)-methanol | ESI-MS: m/z = 543 [M+H]⁺ |
| | (*S*)-3-{1-[6-(3-Hydroxymethyl-3,4-dihydro-1H-isochinolin-2-carbonyl)-pyrimidin-4-yl]-piperidin-4-yl}-7-methoxy-1,3,4,5-tetrahydro-1,3₋benzodiazepin-2-on | | Rₜ = 1.27 min Methode C |
| | | 42 mg (51% d.Th.) | |
| Ver glei chs beis piel | 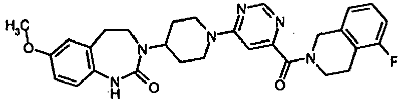 | 28 mg (0.15 mmol) | ESI-MS: m/z = 531 [M+H]⁺ |
| | | 5-Flubr-1,2,3,4-tetrahydro-isochinolin Hydrochlorid | |
| | 3-{1-[6-(5-Fluor-3,4-dihydro-1H-isochinolin-2-carbonyl)-pyrimidin-4-yl]-piperidin-4-yl}-7-methoxy-1,3,4,5-tetrahydro-1,3-benzodiazepin-2-on | | 1.37 min Rₜ = Methode C |
| | | 12 mg (15% d.Th.) | |
| Ver glei chs beis piel | 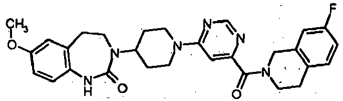 | 28 mg (0.15 mmol) | ESI-MS: m/z = 531 |
| | | 7-Fluor-1,2,3,4-tetrahydro-isochinolin Hydrochlorid | |
| | 3-{1-[6-(7-Fluor-3,4-dihydro-1H-isochinolin-2-carbonyl)-pyrimidin-4-yl]-piperidin-4-yl}-7-methoxy-1,3,4,5-tetrahydro-1,3-benzodiazepin-2-on | | [M+H]⁺ |
| | | 12 mg (15% d.Th.) | Rₜ = 1.36 min Methode C |
| 43 | 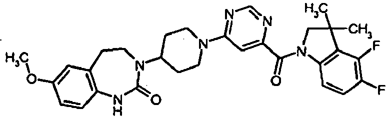 | 80 mg (0.44 mmol) | ESI-MS: m/z = 563 [M+H]⁺ |
| | | 4,5-Difluor-3,3-dimethyl-2,3-dihydro-1H-indol | |
| | 3-{1-[6-(4,5-Difluor-3,3-dimethyl-2,3-dihydro-indol-1-carbonyl)-pyrimidin-4-yl]-piperidin-4-yl}-7-methoxy-1,3,4,5-tetrahydro-1,3-benzodiazepin-2-on | 150 mg (61% d.Th.) | Rₜ = 1.63 min Methode C |
| Ver glei chs beis piel | 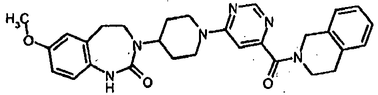 | 20 mg (0.15 mmol) | ESI-MS: m/z = 513 [M+H]⁺ |
| | 3-{1-[6-(3,4-Dihydro-1H-isochinolin-2-carbonyl)-pyrimidin-4-yl]-piperidin-4-yl}-7-methoxy-1,3,4,5-tetrahydro-1,3-benzodiazepin-2-on | 1,2,3,4-Tetrahydro-isochinolin | Rₜ = 1.36 min Methode C |
| | | 40 mg (52% d.Th.) | |
| Ver glei chs beis piel | 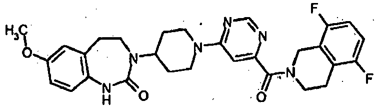 | 31 mg (0.15 mmol) | ESI-MS: m/z = 549 [M+H]⁺ |
| | 3-{1-[6-(5,8-Difluor-3,4-dihydro-1H-isochinolin-2-carbonyl)-pyrimidin-4-yl]-piperidin-4-yl}-7-methoxy-1,3,4,5-tetrahydro-1,3-benzodiazepin-2-on | 5,8-Difluor-1,2,3,4-tetrahydro-isochinolin | |
| | | 47 mg (57% d.Th.) | Rₜ = 1.43 min Methode C |
| Ver glei chs beis piel | 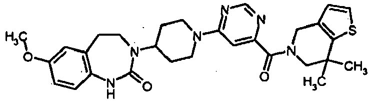 | 40 mg (0.21 mmol) | ESI-MS: m/z = 547 [M+H]⁺ |
| | 3-{1-[6-(7,7-Dimethyl-6,7-dihydro-4H-thieno[3,2-c]pyridin-5-carbonyl) -pyrimidin-4-yl]-piperidin-4-yl}-7-methoxy-1,3,4,5-tetrahydro-1,3-benzodiazepin-2-on | 7,7-Dimethyl-4,5,6,7-tetrahydro-thieno-[3,2-c]pyridine | |
| | | 29 mg (25% d.Th.) | Rₜ = 3.94 min Methode K |
| Ver glei chs beis piel | 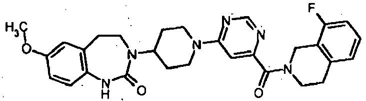 | 28 mg (0.15 mmol) | |
| | 3-{1-[6-(8-Fluor-3,4-dihydro-1H-iso-chinolin-2-carbonyl)-pyrimidin-4-yl]-piperidin-4-yl}-7-methoxy-1,3,4,5-tetrahydro-1,3-benzodiazepin-2-on | 8-Fluor-1,2,3,4-tetrahydroisochinolin Hydrochlorid | ESI-MS: m/z = 531 [M+H]⁺ |
| | | 22 mg (28% d.Th.) | Rₜ = 1.38 min Methode C |
| 44 | 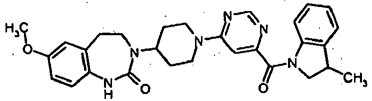 | 40 mg (0.30 mmol) | ESI-MS: m/z = 513 [M+H]⁺ |
| | 7-Methoxy-3-{1-[6-(3-methyl-2,3-dihydro-indol-1-carbonyl)-pyrimidin-4-yl]-piperidin-4-yl}-1,3,4,5-tetrahydro-1,3-benzodiazepin-2-on | 3-Methyl-2,3-dihydro-1H-indol | |
| | | 85 mg (66% d.Th.) | R_{f} = 0.77 Kieselgel, (Laufmittel A |
| Ver glei beis piel | 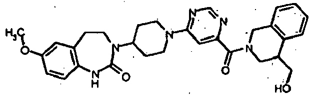 | 25 mg (0.15 mmol) | ESI-MS: m/z = 543 [M+H]⁺ |
| | 3-{1-[6-(4-hydroxymethyl-3,4-dihydro-1H-isochinolin-2-carbonyl)-pyrimidin-4-yl]-piperidin-4-yl}-7-methoxy-1,3,4,5-tetrahydro-1,3-benzodiazepin-2-on | (1,2,3,4-Tetrahydro-isochinolin-4-yl)-methanol 4 mg (5% d.Th.) | Rₜ = 1.27 min Methode C |
| 45 | 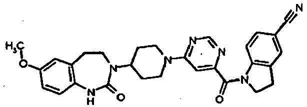 | 50 mg (0.35 mmol) | ESI-MS: m/z = 524 [M+H]⁺ |
| | 1-{6-[4-(7-Methoxy-2-oxo-1,2,4,5-tetrahydro-benzo[d][1,3]diazepin-3-yl)-piperidin-1-yl]-pyrimidin-4-carbonyl}-2,3-dihydro-1H-indol-5-carbonitril | 2,3-Dihydro-1H-indol-5-carbonitril | |
| | | 30mg (19% d.Th.) | R_{f} = 0.77 Kieselgel, (Laufmittel A |
| Ver glei chs beis piel | 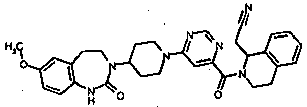 | 32 mg (0.15 mmol) | ESI-MS: m/z = 552 [M+H]⁺ |
| | | (1,2,3,4-Tetrahydroisochinolin-1-yl)-acetonitril Hydrochlorid | |
| | (2-{6-[4-(7-Methoxy-2-oxo-1,2,4,5-tetrahydro-1,3-benzodiazepin-3-yl)-piperidin-1-yl]-pyrimidin-4-carbonyl}-1,2,3,4-tetrahydro-isochinolin-1-yl)-acetonitril | | |
| | | | Rₜ = 1.39 min Methode C |
| | | 39 mg (47% d.Th.) | |

| Bsp. | Struktur Name | [Menge Amine] Amin Ausbeute | Analytische Daten |
|---|---|---|---|
| Ver glei chs beis piel | 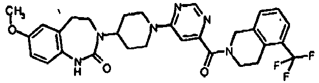 | 36 mg (0.15 mmol) | ESI-MS: m/z = 581 [M+H]⁺ |
| | 7-Methoxy-3-{1-[6-(5-trifluormethyl-3,4-dihydro-1H-isochinolin-2-carbonyl)-pyrimidin-4-yl]-piperidin-4-yl}-1,3,4,5-tetrahydro-1,3-benzodiazepin-2-on | 5-Trifluormethyl-1,2,3,4-tetrahydro-isochinolin Hydrochlorid | |
| | | | Rₜ = 147 min Methode C |
| | | 41 mg (47% d.Th.) | |
| Ver glei chs beis piel | 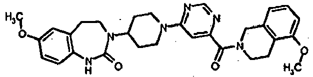 | 30 mg (0.15 mmol) | ESI-MS: m/z = 543 [M+H]⁺ |
| | | 5-Methoxy-1,2,3,4-tetrahydro-isochinolin Hydrochlorid | |
| | 7-Methoxy-3-{1-[6-(5-methoxy-3,4-dihydro- 1H-isochinolin-2-carbonyl)-pyrimidin-4-yl]-piperidin-4-yl}-1,3,4,5-tetrahydro-1,3-benzodiazepin-2-on | | Rₜ = 1.39 min Methode C |
| | | 42 mg (51% d.Th.) | |
| 46 | 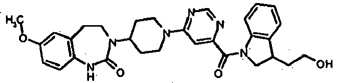 | 50 mg mmol) | ESI-MS: m/z = 543 [M+H]⁺ |
| | 3-(1-{6-[3-(2-Hydroxy-ethyl)-2,3-dihydroindol-1-carbonyl]-pyrimidin-4-yl}-piperidin-4-yl)-7-methoxy-1,3,4,5-tetrahydro-benzo[d][1,3]-diazepin-2-on | 2-(2,3-Dihydro-1H-indol-3-yl)-ethanol 90 mg (66% d.Th.) | R_{f} = 0.77 Kieselgel, Laufmittel A |
| 47 | 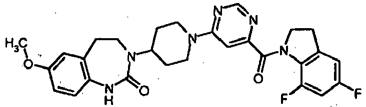 | 33 mg (0.21 mmol) | ESI-MS: m/z = 535 [M+H]⁺ |
| | 3-{1-[6-(5,7-Difluor-2,3-dihydro-indol-1-carbonyl)-pyrimidin-4-yl]-piperidin-4-yl}-7-methoxy-1,3,4,5-tetrahydro-benzo[d][1,3]diazepin-2-on | 5,7-Difluor-2,3-dihydro-1H-indol | |
| | | 35 mg (33% d.Th.) | Rₜ = 4.8 min Methode B |

| Bsp. | Struktur Name | [Menge Amin] Amin Ausbeute | Analytische Daten |
|---|---|---|---|
| 48 | 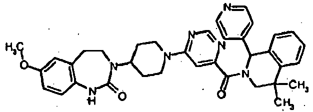 | 36 mg (0.15 mmol) | ESI-MS: m/z = 618 [M+H]⁺ |
| | 3-{1-[6-(4,4-Dimethyl-1-pyridin-4-yl-3,4-dihydro-1H-isochinoline-2-carbonyl)-pyrimidin-4-yl]-piperidin-4-yl}-7-methoxy-1,3,4,5-tetrahydro-benzo-[d][1,3]diazepin-2-on | 4,4-Dimethyl-1-pyridin-4-yl-1,2,3,4-tetrahydro-isochinolin | |
| | | | Rₜ = 1.51 min Methode C |
| | | 34 mg (37% d.Th.) | |
| Ver glei chs beis piel | 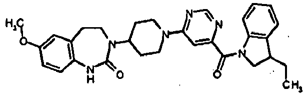 | 34 mg (0.23 mmol) | ESI-MS: m/z = 527 [M+H]⁺ |
| | 3-{1-[6-(3-Ethyl-2,3-dihydroindol-1-carbonyl)-pyrimidin-4-yl]-piperidin-4-yl}-7-methoxy-1,3,4,5-tetrahydro-benzo[d][1,3]diazepin-2-on | 3-Ethyl-2,3-dihydro-1H-indol | |
| | | 29 mg (26% d.Th.) | Rt = 1.52 min Methode C |
| Ver glei chs beis piel | 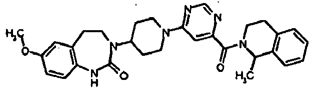 | 69 mg (0.15 mmol) | ESI-MS: m/z = 527 [M+H]⁺ |
| | 7-Methoxy-3-{1-[6-(1-methyl-3,4-dihydro-1 H-isochinolin-2-carbonyl)-pyrimidin-4-yl]-piperidin-4-yl}-1,3,4,5-tetrahydro-benzo[d][1,3]diazepin-2-on | 1-Methyl-1,2,3,4-tetrahydroisochinolin Hydrochlorid | |
| | | 10 mg (13% d.Th.) | Rₜ = 1.44 min Methode C |
| Ver glei chs beis piel | 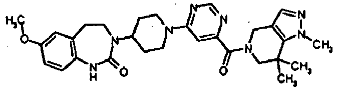 | 125 mg (0.34 mmol) | ESI-MS: m/z = 545 [M+H]⁺ |
| | 7-Methoxy-3-{1-[6-(1,7,7-trimethyl-1,4,6,7-tetrahydro-pyrazolo[4,3-c]-pyridin-5-carbonyl)-pyrimidin-4-yi]-piperidin-4-yl}-1,3,4,5-tetrahydro-benzo[d][1,3]diazepin-2-on | 1,7,7-Trimethyl-4,5,6,7-tetrahydro-1H-pyrazolo[4,3-c]-pyridinium Trifluoracetat | |
| | | | Rₜ = 2.75 min Methode E |
| | | 44 mg (29% d.Th.) | |
| Ver glei chs beis piel | 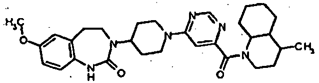 | 11 mg (0.08 mmol) | ESI-MS: m/z = 533 [M+H]⁺ |
| | 7-Methoxy-3-{1-[6-(4-methyl-octahydro-chinolin-1-carbonyl)-pyrimidin-4-yl]-piperidin-4-yl}-1,3,4,5-tetrahydro-benzo[d][1,3]diazepin-2-on | 4-Methyldecahydro-chinolin | |
| | | 12mg(15%d.Th.) | Rₜ = 1.45 min Methode C |
| Ver glei chs beis piel | 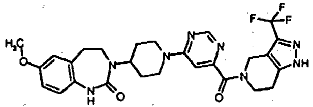 | 50 mg (0.26 mmol) | ESI-MS: m/z = 571 [M+H]⁺ |
| | | 3-Trifluormethyl-4,5,6,7-tetrahydro-1H-pyrazolo[4,3-c]pyridin | |
| | 7-Methoxy-3-{1-[6-(3-trifluormethyl-1,4,6,7-tetrahydro-pyrazolo[4,3-c]pyridin-5-carbonyl)-pyrimidin-4-yl]-piperidin-4-yl} 1,3,4,5-tetrahydro-benzo[d][1,3]diazepin-2-on | | Rₜ = 2.92 min Methode E |
| | | 65 mg (57% d.Th.) | |
| 49 | 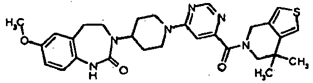 | 0.16 g (0.24 mmol) | ESI-MS: m/z = 547 [M+H]⁺ |
| | 3-{1-[6-(7,7-Dimethyl-6,7-dihydro-4H-thieno[3,4-c]pyridin-5-carbonyl) -pyrimidin-4-yl]-piperidin-4-yl}-7-methoxy-1,3,4,5-tetrahydro-benzo[d][1,3]diazepin-2-on | 7,7-Dimethyl-4,5,6,7-tetrahydro-thieno[3,4c]pyridin | Rₜ = 4.03 min |
| | | 7.4 mg (6% d.Th.) | Methode E |
| Ver glei chs beis piel | 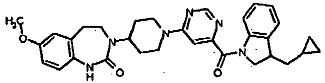 | 40 mg (0.23 mmol) | ESI-MS: m/z = 553 [M+H]⁺ |
| | 3-{1-[6-(3-Cyclopropylmethyl-2,3-dihydro-indol-1-carbonyl)-pyrimidin-4-yl]-piperidin-4-yl}-7-methoxy-1,3,4,5-tetrahydro-benzo[d][1,3]diazepin-2-on | 3-Cyclopropylmethyl -2,3-dihydro-1H-indol | |
| | | | Rₜ = 1.62 min Methode C |
| | | 76 mg (66%, d.Th.) | |
| Ver glei chs beis piel | 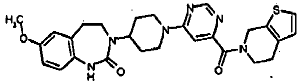 | 21 mg (0.15 mmol) | ESI-MS: m/z = 519 [M+H]⁺ |
| | 3-{1-[6-(4,7-Dihydro-5H-thieno[2,3-c]pyridin-6-carbonyl)-pyrimidin-4-yl]-piperidin-4-yl}-7-methoxy-1,3,4,5-tetrahydro-benzo[d][1,3]diazepin-2-on | 4,5,6,7-Tetrahydro-thieno[2,3-c]pyridin | |
| | | 38 mg (49% d.Th.) | Rₜ = 1.31 min Methode C |
| Ver glei chs beis piel | 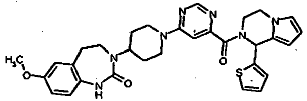 | 31 mg (0.15 mmol) | ESI-MS: m/z = 584 [M+H]⁺ |
| | 7-Methoxy-3-{1-[6-(1-thiophen-2-yl-3,4-dihydro-1H-pyrrolo[1,2-a]pyrazin-2-carbonyl)-pyrimidin-4-yl]-piperidin-4-yl}-1,3,4,5-tetrahydro-benzo[d]-[1,3]diazepin-2-on | 1-Thiophen-2-yl-1,2,3,4-tetrahydro-pyrrol-[1,2-a]pyrazin | |
| | | 47 mg (53% d.Th.) | Rₜ = 1.50 min Methode C |
| Ver glei chs beis piel | 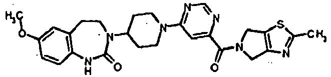 | 55 mg (0.25 mmol) | ESI-MS: m/z = 520 [M+H]⁺ |
| | | 2-Methyl-5,6-dihydro-4H-pyrrolo[3,4-d]thiazol . Hydrobromid | |
| | 7-Methoxy-3-{1-[6-(2-methyl-4,6-dihydro-pyrrolo[3,4-d]th iazol-5-carbonyl)-pyrimidin-4-yl]-piperidin-4-yl}-1,3,4,5-tetrahydro-benzo[d]-[1,3]diazepin-2-on | | Rₜ = 2.95 min Methode E |
| | | 62 mg (53% d.Th.) | |
| 50 | 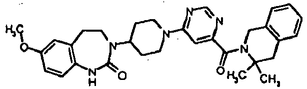 | 24 mg (0.15 mmol) | ESI-MS: m/z = 541 [M+H]⁺ |
| | 3-{1-[6-(3,3-Dimethyl-3,4-dihydro-1H-isochinolin-2-carbonyl)-pyrimidin-4-yl]-piperidin-4-yl}-7-methoxy-1,3,4,5-tetrahydro-benzo[d]-[1,3]diazepin-2-on | 3,3-Dimethyl-1,2,3,4-tetrahydro-isochinolin | |
| | | 35 mg (43% d.Th.) | Rₜ = 1.57 min Methode C |
| Ver glei chs beis piel | 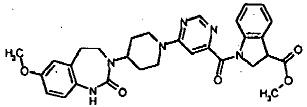 | 110 mg (0.52 mmol) | ESI-MS: m/z = 557 [M+H]⁺ |
| | | 2,3-Dihydro-1H-indol-3-carbonsäuremethyl-ester Hydrochlorid | |
| | 1-{6-[4-(7-Methoxy-2-oxo-1,2,4,5-tetrahydro-benzo[d][1,3]diazepin-3-yl)-piperidin-1-yl]-pyrimidine-4-carbonyl}-2,3-dihydro-1H-indol-3-carbonsäuremethylester | | R_{f} = 0.78 Kieselgel, Laufmittel A |
| | | 20 mg (7% d.Th.) | |
| Ver glei chs beispiel | 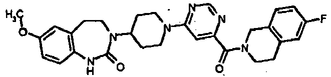 | 28 mg (0.15 mmol) | ESI-MS: m/z = 531 [M+H]⁺ |
| | 3-{1-[6-(6-Fluor-3,4-dihydro-1H-isochinolin-2-carbonyl)-pyrimidin-4-yl]-piperidin-4-yl}-7-methoxy-1,3,4,5-tetrahydro-benzo[d]-[1,3]diazepin-2-on | 6-Fluor-1,2,3,4-tetrahydro-isochinolin Hydrochlorid | Rₜ = 1.36 min Methode C. |
| | | 41 mg (51% d.Th.) | |
| Ver glei chs beis piel | 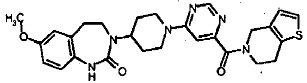 | 21 mg (0.15mmol) | ESI-MS: m/z=519 [M+H]⁺ |
| | 3-{1-[6-(6,7-Dihydro-4H-thieno[3,2-c]pyridin-5-cärbonyl)-pyrimidin-4-yl]-piperidin-4-yl}-7-methoxy-1,3,4,5-tetrahydro-benzo[d]-[1,3]diazepin-2-on | 4,5,6,7-Tetrahydro-thieno[3,2-c]pyridin | Rₜ = 1,31 min Methode C |
| | | 40 mg (51% d.Th.) | |
| Ver glei chs beis piel | 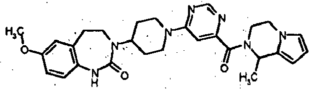 | 21 mg (0.15 mmol) | ESI-MS: m/z = 516 [M+H]⁺ |
| | 7-Methoxy-3-{1-[6-(1-methyl-3,4-dihydro-1 H-,pyrrolö[1,2 .-a]pyrazin-2-carbonyl)-pyrimidin-4-yl]-piperidin-4 -yl}-1,3,4,5-tetra-hydro-benzo[d]-[1,3]diazepin-2-on | 1-Methyl-1,2,3,4-tetrahydro-pyrrolo-[1,2-a]pyrazin | Rₜ = 1.30 min Methode C |
| | | 40mg(51% d.Th.) | |
| Ver glei chs beis piel | 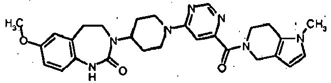 | 21 mg (0.15 mmol) | ESI-MS: m/z=516 [M+H]⁺ |
| | 7-Methoxy-3-{1-[6-(1 -methyl-1,4,6,7-tetrahydro-pyrrolo[3,2-c]pyridin-5-carbonyl)-pyrimidin-4-yl]-piperidin-4-yl}-1,3,4,5-tetrahydro-benzo[d]-[1,3]diazepin-2-on | 1-Methyl-4,5,6,7-tetrahydro-1H-pyrrolo[3,2-c]pyridin | Rₜ = 1.28 min Methode C |
| | | 22 mg (28% d.Th.) | |
| Ver glei chs beis piel | 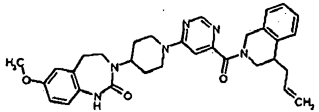 | 26 mg (0.15 mmol) | ESI-MS: m/z = 553 [M+H]⁺ |
| | 3-{1-[6-(4-Allyl-3,4-dihydro-1H-iso-chinolin-2-carbonyl)-pyrimidin-4-yl]-piperidin-4-yl}-7-methoxy-1,3,4,5-tetrahydro-benzo[d]-[1,3]diazepin-2-on | 4-Allyl-1,2,3,4-tetrahydro-isochinolin | Rₜ = 1.63 min Methode C |
| | | 18 mg (22% d.Th.) | |
| 51 | 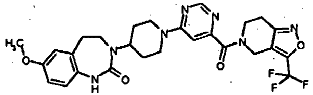 | 45 mg (0.23 mmol) | ESI-MS: m/z = 572 [M+H]⁺ |
| | 7-Methoxy-3-{1-[6-(3-trifluormethyl-6,7-dihydro-4H-isoxazolo[4,3-c]pyridin-5-carbonyl)-pyrimidin-4-yl]-piperidin-4-yl}-1,3,4,5-tetrahydro-benzo[d]-[1,3]diazepin-2-on | 3-Trifluormethyl-4,5,6,7-tetrahydro-isoxazolo-[4,3-c]pyridin | |
| | | 85 mg (74% d.Th.) | Rₜ = 3.45 minMethode E |
| Ver glei chs beis piel | 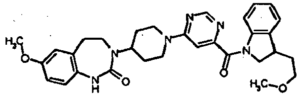 | 41 mg (0.23 mmol) | ESI-MS: m/z = 557 [M+H]⁺ |
| | 7-Methoxy-,3-(1-{6-[3-(2-methoxy-ethyl)-2,3-dihydro-indol-1-carbonyl]-pyrimidin-4-yl}-piperidin-4-yl)-1,3,4,5-tetrahydro-benzo[d]-[1,3]diazepin-2-on | 3-(2-Methoxy-ethyl)-2,3-dihydro-1H-Indol | Rₜ = 3.94 min Methode E |
| | | 83 mg (71% d.Th.) | |
| Ver glei chs beis piel | 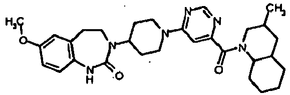 | 29 mg (0.15 mmol) | ESI-MS: m/z = 533 [M+H]⁺ |
| | 7-Methoxy-3-{1-[6-(3-methyl-octahydro-chinolin-1-carbonyl)-pyrimidin-4-yl]-piperidin-4-yl}-1,3,4,5-tetrahydro-benzo[d]-[1,3]diazepin-2-on | 3-Methyl-decahydro-chinolin Hydrochlorid | Rₜ = 1.48 min Methode C |
| | | 13 mg (16% d.Th.) . | |
| Vergleichs beis piel | 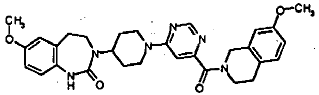 | 30 mg (0.15 mmol) | ESI-MS: m/z = 543 [M+H]⁺ |
| | 7-Methoxy-3-{1-[6-(7-methoxy-3,4-dihydro-1H-isochinolin-2-carbonyl)-pyrimidin-4-yl]-piperidin-4-yl}-1,3,4,5-tetrahydro-benzo[d]-[1,3]diazepin-2-on | 7-Methoxy-1,2,3,4-tetrahydro-iso-chinolin Hydrochlorid | Rₜ = 1.36 min Methode C |
| | | 42 mg (51% d.Th.) | |
| Ver glei chs beis piel | 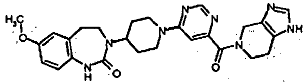 | 60 mg (0.31 mmol) 4,5,6,7-Tetrahydro-1H-imidazo[4,5-c]pyridinDihydro-chlorid | ESI-MS: . m/z = 503 [M+H]⁺ |
| | 7-Methoxy-3-{1-[6-(1,4,6,7-tetrahydro-imidazo[4,5-c]pyridin-5-carbonyl)-pyrimidin-4-yl]-piperidin-4-yl}-1,3,4,5-tetrahydro-benzo[d]-[1,3]diazepin-2-on | | Rₜ = 2.05 min Methode E |
| | | 32 mg (32% d.Th.) | |
| Ver glei chs beis piel | 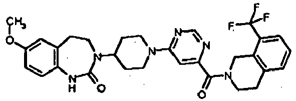 | 36 mg (0.15mmol) | ESI-'MS: m/z = 581 [M+H]⁺ |
| | 7-Methoxy-3-{1-[6-(8-trifluormethyl-3,4-dihydro-1H-isochinolin-2-carbonyl)-pyrimidin-4-yl]-piperidin-4-yl}-1,3,4,5-tetrahydro-benzo[d]-[1,3]diazepin-2-on | 8-Trifluormethyl-1,2,3,4-tetrahydro-isochinolin Hydrochlorid | |
| | | 42 mg (48% d.Th.) | Rₜ = 1.45 min Methode C |
| Ver glei chs beis piel | 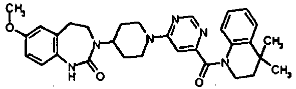 | 69 mg (0.43 mmol) | ESI-MS: m/z = 541 [M+H]⁺ |
| | 3-{1-[6-(4,4-Dimethyl-3,4-dihydro-2H-chinolin-1-carbonyl)-pyrimidin-4 -yl]-piperidin-4-yl}-7-methoxy-1,3,4,5-tetrahydro-benzo[d]-[1,3]diazepin-2-on | 4,4-Dimethyl-1,2,3,4-tetrahydrochinolin | Rₜ = 3.75 min Methode K |
| | | 0.12 g (59% d.Th.) | |
| Ver glei chs beis piel | | 75 mg (0.31 mmol) | ESI-MS: m/z = 503 [M+H]⁺ |
| | 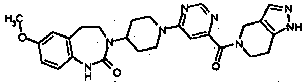 | 4,5,6,7-Tetrahydro-1H-pyrazolo[4,3-, c]pyridin Dihydrochlorid Dimethylether | |
| | 7-Methoxy-3-{1-[6-(1,4,6,7-tetrahydro-pyrazolo[4,3-c]pyridin-5₋carbonyl)-pyrimidin-4-yl]-piperidin-4-yl}-1,3,4,5-tetrahydro-benzo[d]-[1,3]diazepin-2-on | | Rₜ = 2.24 min Methode E |
| | | 40 mg (40% d.Th.) | |
| Ver glei chs beis piel | | 50 mg (0.28 mmol) | ESI-MS: m/z = 489 [M+H]⁺ |
| | 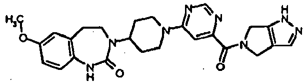 | 1,4,5,6-Tetrahydro-pyrrolo[3,4-c]pyrazol Dihydrochlorid | |
| | 3-{1-[6-(4,6-Dihydro-1H-pyrrolo[3,4c]-pyrazol-5-carbonyl)-pyrimidin-4-yl]-pi-peridin-4-yl}-7-methoxy-1,3,4,5-tetra-hydrobenzo[d]-[1,3]diazepin-2-on | | Rₜ = 2.4 min Methode E |
| | | 63 mg (64% d.Th.) | |
| Ver glei chs beis piel | 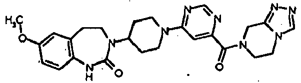 | 60 mg (0.37 mmol) | ESI-MS: m/z = 504 [M+H]⁺ |
| | 3-{1-[6-(5,6-Dihydro-8H-[1,2,4]-triazolo[4,3-a]pyrazin-7-carbonyl)-pyrimidin-4-yl]₋piperidin-4-yl}-7-methoxy-1,3,4,5-tetra-hydrobenzo[d]-[1,3]diazepin-2-on | 5,6,7,8-Tetrahydro-[1,2,4]-triazolo[4,3a]-pyrazin Hydrochlorid | Rₜ = 2.3 min Methode E |
| | | 23 mg (23% d.Th.) | |
| 52 | | 26 mg (0.15 mmol) | ESI-MS: m/z = 517 [M+H]⁺ |
| | 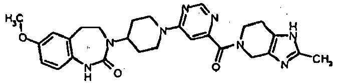 | 2-Methyl-4,5,6,7-tetra-hydro-1H. imidazo[4,5-c]pyridin Hydrochlorid | |
| | 7-Methoxy-3-{1 -[6-(2-methyl-1,4,6,7-tetrahydro-imidazo[4,5-c]pyridin-5-carbönyl)-pyrimidin-4-yl]-piperidin-4-yl}-1,3,4,5-tetra-hydrobenzo[d]-[1,3]diazepin-2-on | | Rₜ = 1.19 min Methode C |
| | | 27 mg (35% d.Th.) | |

### Vergleichsbeispiel:

### 4'-(4,5-Difluor-2,3-dihydroindol-1-carbonyl)-4-(7-methoxy-2-oxo-1,2,4,5-tetrahydro-benzo[d]-[1,3]diazepin-3-yl)-3,4,5,6-tetrahydro-2H-[1,2']bipyridinyl-5'-carbonitril

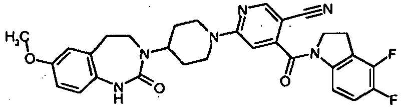

0.10 g (0.24 mmol) 5'-Cyano-4-(7-methoxy-2-oxo-1,2,4,5-tetrahydro-1,3-benzodiazepin-3-yl)-3,4,5,6-tetrahydro-2H-[1,4']bipyridinyl-2'-carbonsäure, 45 mg (0.24 mmol) 4,5-Difluor-2,3-dihydro-1 H-indol Hydrochlorid und 0.103 mL (0.74 mmol) TEA wurden in 2 mL DMF vorgelegt. 83.8 mg (0.26 mmol) TBTU wurden zugegeben, das Reaktionsgemisch wurde über Nacht bei RT gerührt und dann mittels HPLC gereinigt. Die das Produkt enthaltenden Fraktionen wurden vereinigt und gefriergetrocknet.

| | |
|---|---|
| Ausbeute: | 24 mg (18% d.Th.) |
| ESI-MS: | m/z = 559 (M+H)⁺ |
| Rₜ (HPLC-MS): | 3.23 min (Methode O) |

### Beispiel 53:

### 4'-(7,7-Dimethyl-1,4,6,7-tetrahydro-pyrazolo[4,3-c]pyridin-5-carbonyl)-4-(7-methoxy-2-oxo-1,2,4,5-tetrahydro-1,3-benzodiazepin-3-yl)-3,4,5,6-tetrahydro-2H-[1,2']bipyridinyl-5'-carbo-nitril

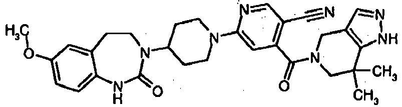

100 mg (0.24 mmol) 5'-Cyano-4-(7-methoxy-2-oxo-1,2,4,5-tetrahydro-1,3-benzodiazepin-3-yl)-3,4,5,6-tetrahydro-2H-[1 ,2']bipyridinyl-4'-carbonsäure, 53.2 mg (0.24 mmol) 7,7-Di-methyl-4,5,6,7-tetrahydro-1H-pyrazolo[4,3-c]pyridin Dihydrochlorid, 137 µl (0.97 mmol) TEA und 83.8 mg (0.26 mmol) TBTU wurden in 2 mL DMF über Nacht bei RT gerührt. Das Reaktionsgemisch wurde mittels HPLC gereingt. Die Produkt enthaltenden Fraktionen wurden vereinigt und gefriergetrocknet.

| | |
|---|---|
| Ausbeute: | 19 mg (14% d.Th.) |
| ESI-MS: | m/z = 555 (M+H)⁺ |
| Rₜ (HPLC-MS): | 1.29 min (Methode C) |

### Beispiel 54:

### 3-[4'-(5-Fluor-2,3-dihydro-indol-1-carbonyl)-6'-methoxy-3,4,5,6-tetrahydro-2H-[1,2']bi-pyridinyl-4-yl]-7-methoxy-1,3,4,5-tetrahydro-benzo[d][1,3]diazepin-2-on

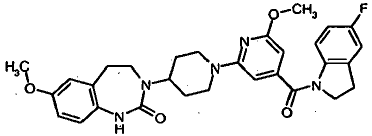

633 mg (2.3 mmol) 7-Methoxy-3-piperidin-4-yl-1,3,4,5-tetrahydro-1,3-benzodiazepin-2-on, 700 mg (2.28 mmol) (2-Chlor-6-methoxy-pyridin-4-yl)-(5-fluor-2,3-dihydro-indol-1-yl)-methanon und 953.6 mg (6.9 mmol) Kaliumcarbonat wurden in 5 mL NMP 8 h bei 130°C gerührt. Der ungelöste Feststoff wurde abgefiltert und das Filtrat wurde mittels HPLC gereinigt. Die Produkt enthaltenden Fraktionen wurden vereinigt und zur Trockene einrotiert. Zur weiteren Reinigung wurde der Rückstand mit DMF versetzt und der ungelöste Feststoff wurde abgesaugt und getrocknet.

| | |
|---|---|
| Ausbeute: | 490 mg (39% d.Th.) |
| ESI-MS: | m/z = 547 (M+H)⁺ |
| Rₜ (HPLC-MS): | 1.5 min (Methode C) |

### Vergleichsbeispiel:

### 3-[2'-(4,5-Difluor-2,3-dihydro-indol-1-carbonyl)-3,4,5,6-tetrahydro-2H-[1,4']bipyridinyl-4-yl]-7-methoxy-1,3,4,5-tetrahydro-1,3-benzodiazepin-2-on

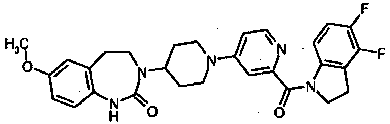

0.18 g (0.67 mmol) 7-Methoxy-3-piperidin-4-yl-1,3,4,5-tetrahydro-1,3-benzodiazepin-2-on, 0.20 g (0.68 mmol) (4-Chlor-pyridin-2-yl)-(4,5-difluor-2,3-dihydro-indol-1-yl)-methanon und 97 mg (0.70 mmol) Kaliumcarbonat wurden in 3 mL NMP 4 h bei 130°C und über Nacht bei RT gerührt. Der Reaktionsansatz wurde mittels HPLC aufgereinigt. Die Produkt enthaltenden Fraktionen wurden vereinigt und gefriergetrocknet.

| | |
|---|---|
| Ausbeute: | 55 mg (15% d.Th.) |
| ESI-MS: | m/z = 534 (M+H)⁺ |
| Rₜ (H PLC-MS): | 1.40 min (Methode C) |

### Vergleichsbeispiel:

### 7-Methoxy-3-{1-[6-(octahydro-indol1-carbonyl)-pyrimidin-4-yl]-piperidin-4-yl}-1,3,4,5-tetra-hydro-1,3-benzodiazepin-2-on

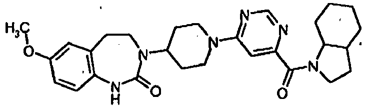

705 mg (2.56 mmol) 7-Methoxy-3-piperidin-4-yl-1,3,4,5-tetrahydro-1,3-benzodiazepin-2-on, 680 mg (2.56 mmol) (6-Chlor-pyrimidin-4-yl)-(octahydro-indol-1-yl)-methanon und 0.871 mL (5 mmol) DIPEA wurden in 10 mL DMF 2 h bei RT gerührt. Das Reaktionsgemisch wurde mittels HPLC gereinigt. Die Produkt enthaltenden Fraktionen wurden vereinigt und das ACN wurde abrotiert. Die wässrige Phase wurde mit 4M NaOH alkalisch gestellt und mit EtOAc extrahiert. Die organische Phase wurde getrocknet, filtriert und das Filtrat zur Trockene einrotiert. Der Rückstand wurde aus einem Gemisch aus MeOH und Diethylether kristallisiert und der Feststoff abgesaugt und getrocknet.

| | |
|---|---|
| Ausbeute: | 520 mg (40% d.Th.) |
| ESI-MS: | m/z = 505 (M+H)⁺ |
| Rₜ (HPLC-MS): | 3.28 min (Methode E) |

### Beispiel 55:

### 3-{1-[4-(7,7-Dimethyl-1,4,6,7-tetrahydro-pyrazolo[4,3-c]pyridin-5-carbonyl)-[1,3,5]triazin-2-yl]-piperidin-4-yl}-7-methoxy-1,3,4,5-tetrahydro-1,3-benzodiazepin-2-on

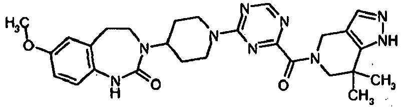

0.11 g (0.26 mmol) 4-[4-(7-Methoxy-2-oxo-1,2,4,5-tetrahydro-1,3-benzodiazepin-3-yl)-piperidin-1-yl]-[1,3,5]triazin-2-carbonsäure, 59 mg (0.26 mmol) 7,7-Dimethyl-4,5,6,7-tetrahydro-1H-pyrazolo[4,3-c]pyridin Dihydrochlorid und 0.15 mL (1.1 mmol) TEA wurden in 1.5 mL DMF vorgelegt. 93 mg (0.29 mmol) TBTU wurden zugegeben und das Reaktionsgemisch drei Tage bei RT gerührt. Die Substanz wurde mittels HPLC-MS gereinigt. Die Produkt enthaltenden Fraktionen wurden vereinigt und gefriergetrocknet.

| | |
|---|---|
| Ausbeute: | 8 mg (6% d.Th.) |
| ESI-MS: | m/z = 532 (M+H)⁺ |
| Rₜ (HPLC-MS): | 3.11 min (Methode E) |

### Beispiel 56:

### 3-{1-[6-(5-Fluor-2,3-dihydro-indol-1-carbonyl)-5-methyl-pyrimidin-4-yl]-piperidin-4-yl}-7-methoxy-1,3,4,5-tetrahydro-1,3-benzodiazepin-2-on

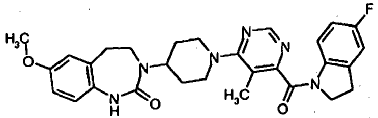

70 mg (0.17 mmol) 6-[4-(7-Methoxy-2-oxo-1,2,4,5-tetrahydro-1,3-benzodiazepin-3-yl)-piperidin-1-yl]-5-methyl-pyrimidin-4-carbonsäure, 26.1 mg (0.19 mmol) 5-Fluorindolin, 61 mg (0.19 mmol) TBTU und 0.027 mL(0.19 mmol) TEA wurden in 1 mL DMF 3 h bei RT gerührt. Das Reaktionsgemsich wurde mittels HPLC-MS gereinigt. Die Produkt enthaltenden Fraktionen wurden,vereinigt und gefriergetrocknet.

| | |
|---|---|
| Ausbeute: | 58 mg (6% d.Th.) |
| ESI-MS: | m/z = 531 (M+H)⁺ |
| Rₜ (HPLC-MS): | 1.45 min (Methode C) |

### Beispiel 57:

### 4'-(2,3-Dihydro-indol-1 -carbonyl)-4-(7-methoxy-2-oxo-1,2,4,5-tetrahydro-1,3-benzo-diazepin-3-yl)-3,4,5,6-tetrahydro-2H-[1,2']bipyridinyl-5'-carbonitril

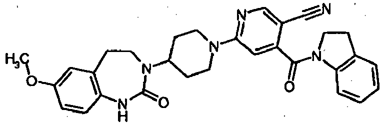

0.02 g (0.48 mmol) 5'-Cyano-4-(7-methoxy-2-oxo-1,2,4,5-tetrahydro-1,3-benzodiazepin-3-yl)-3,4,5,6-tetrahydro-2H-[1,2']bipyridinyl-4'-carbonsäure, 57 mg (0.48-mmol) 2,3-Dihydro-1H-indol und 0.21 mL (1.5 mmol) TEA wurden in 3 mL DMF vorgelegt. 0.17 g (0.52 mmol) TBTU wurden zugegeben. Das Reaktionsgemisch wurde über Nacht bei RT gerührt und dann mittels HPLC gereinigt. Die Produkt enthaltenden Fraktionen wurden vereinigt und gefriergetrocknet.

| | |
|---|---|
| Ausbeute: | 38 mg (15% d.Th.) |
| ESI-MS: | m/z = 523 (M+H)⁺ |
| R, (HPLC-MS): | 1.53 min (Methode C) |

### Vergleichsbeispiel:

### 3-[4'-(4,5-Difluor-2,3-dihydro-indol-1-carbonyl)-3,4,5,6-tetrahydro-2H-[1,2']bipyridinyl-4-yl]-7-methoxy-1,3,4,5-tetrahydro-1,3-benzodiazepin-2-on

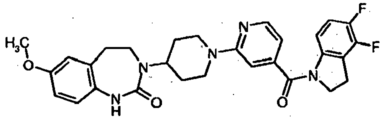

0.18 g (0.67 mmol) 7-Methoxy-3-piperidin-4-yl-1,3,4,5-tetrahydro-1,3-benzodiazepin-2-on, 0.020 g (0.68 mmol) (2-Chlor-pyridin-4-yl)-(4,5-difluor-2,3-dihydro-indol-1-yl)-methanon und 97 mg (0.70 mmol) Kaliumcarbonat wurden in 3 mL NMP 4 h bei 130°C gerührt, dann über Nacht bei RT. Das Reaktionsgemisch wurde mittels HPLC gereinigt. Die Produkt enthaltenden Fraktionen wurden vereinigt und gefriergetrocknet.

| | |
|---|---|
| Ausbeute: | 42 mg (12% d.Th.) |
| ESI-MS: | m/z = 534 (M+H)⁺ |
| Rₜ (HPLC-MS): | 1.4 min (Methode C) |

### Vergleichsbeispiel:

### 3-{1-[6-(Hexahydro-cyclopenta[c]pyrrol-2-carbonyl)-pyrimidin-4-yl]-piperidin-4-yl}-7-methoxy-1,3,4,5-tetrahydro-1,3-benzodiazepin-2-on

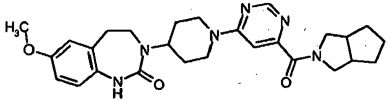

80 mg (0.20 mmol) 6-[4-(7-Methoxy-2-oxo- 1,2,4,5-tetrahydro-1,3-benzodiazepin-3-yl)-piperidin-1-yl]-pyrimidin-4-carbonsäure, 40 mg (0.27 mmol) Octahydrocyclopenta[c]pyrrol-Hydrochlorid0.080 mL (0.57 mmol) TEA und 80 mg (0.25 mmol) TBTU wurden in 0.9 mL DMF über Nacht bei RT gerührt. Das Reaktionsgemisch wurde mit gesättigter Natriumhydrogencarbonat-Lösung und Eiswasser versetzt, und der ausgefallene Feststoff wurde abgesaugt und getrocknet.

| | |
|---|---|
| Ausbeute: | 74 mg (75% d.Th.) |
| ESI-MS: | m/z = 491 (M+H)⁺ |
| Rₜ (HPLC-MS): | 3.14 min (Methode E) |

### Vergleichsbeispiel:

### 3-{1-[6-(4,4-Dimethyl-4,7-dihydro-5H-thieno[2,3-c]pyridin-6-carbonyl)-pyrimidin-4-yl]-piperidin-4-yl}-7-methoxy-1,3,4,5-tetrahydro-1,3-benzodiazepin-2-on

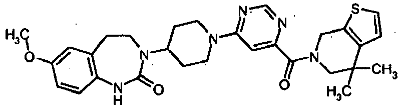

84 mg (0.21 mmol) 6-[4-(7-Methoxy-2-oxo-1,2,4,5-tetrahydro-1,3-benzodiazepin-3-yl)-piperidin-1-yl]-pyrimidin-4-carbonsäure, 40 mg (0.24 mmol) 4,4-Dimethyl-4,5,6,7-tetra-hydro-thieno[2,3-c]pyridin, 34 µL (0.24 mmol) TEA und 77 mg (0.24 mmol) TBTU wurden in 1 mL DMF über Nacht bei RT gerührt. Das Reaktionsgemisch wurde mittels HPLC gereinigt. Die Produkt enthaltenden Fraktionen wurden vereinigt und gefriergetrocknet.

| | |
|---|---|
| Ausbeute: | 62 mg (54% d.Th.) |
| ESI-MS: | m/z = 547 (M+H)⁺ |
| Rₜ (HRLC-MS): | 3.57 min (Methode E) |

### Beispiel 58:

### 3-{1-[6-(6-Fluor-4,4-dimethyl-3,4-dihydro-1H-isochinolin-2-carbonyl)-pyrimidin-4-yl]-piperidin-4-yl}-7-methoxy-1,3,4,5-tetrahydro-1,3-benzodiazepin-2-on

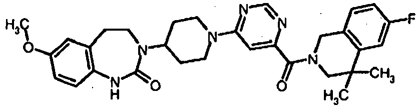

60 mg (0.15 mmol) 6-[4-(7-Methoxy-2-oxo-1,2,4,5-tetrahydro-1,3-benzodiazepin-3-yl)-piperidin-1-yl]-pyrimidin-4-carbonsäure, 33 mg (0.15 mmol) 6-Fluor-4,4-dimethyl-1,2,3,4-tetrahydro-isochinolin Hydrochlorid, 66 µL (0.47 mmol) TEA und 54 mg (0.17 mmol) TBTU wurden in 1.5 mL DMF über Nacht bei RT gerührt. Das Reaktionsgemisch wurde mittels HPLC gereinigt. Die Produkt enthaltenden Fraktionen wurden vereinigt und gefriergetrocknet.

| | |
|---|---|
| Ausbeute: | 22 mg (26% d.Th.) |
| ESI-MS: | m/z = 559 (M+H)⁺ |
| Rₜ (HPLC-MS): | 1.49 min (Methode C) |

### Vergleichsbeispiel:

### 3-{1-[6-(5-Fluor-3-methyl-2,3-dihydro-indol-1-carbonyl)-pyrimidin-4-yl]-piperidin-4-yl}-7-methoxy-1,3,4,5-tetrahydro-1,3-benzodiazepin-2-on

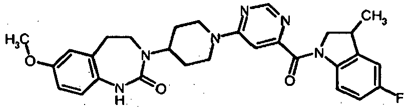

80 mg (0.21 mmol) 6-[4-(7-Methoxy-2-oxo-1,2,4,5-tetrahydro-1,3-benzodiazepin-3-yl)-piperidin-1-yl]-pyrimidin-4-carbonsäure, 32 mg (0.21 mmol) 5-Fluor-3-methyl-2,3-dihydro-1H-indol, 70 µL (0.50 mmol) TEA und 74 mg (0.23 mmol) TBTU wurden in 1.8 mL DMF über Nacht bei RT gerührt. Das Reaktionsgemisch wurde mittels HPLC gereinigt. Die Produkt enthaltenden Fraktionen wurden vereinigt und gefriergetrocknet.

| | |
|---|---|
| Ausbeute: | 64 mg (60% d.Th.) |
| ESI-MS: | m/z = 531 (M+H)⁺ |
| Rₜ (HPLC-MS): | 3.8 min (Methode K) |

### Vergleichsbeispiel:

### 3-{1-[6-(4,4-Dimethyl-3,4-dihydro-1H-isochinolin-2-carbonyl)-pyrimidin-4-yl]-piperidin-4-yl}-7-methoxy-1,3,4,5-tetrahydro-1,3-benzodiazepin-2-on

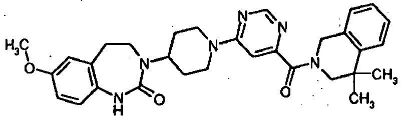

0.10 g mg (0.25 mmol) 6-[4-(7-Methoxy-2-oxo-1,2,4,5-tetrahydro-1,3-benzodiazepin-3-yl)-piperidin-1-yl]-pyrimidin-4-carbonsäure, 57 mg (0.35 mmol) 4,4-Dimethyl-1,2,3,4-tetra-hydro-isochinolin, 75 µL (0.53 mmol) TEA und 0.11 (0.30 mmol) TBTU wurden in 1.1 mL DMF über Nacht bei RT gerührt. Das Reaktionsgemisch wurde mittels HPLC gereinigt. Die Produkt enthaltenden Fraktionen wurden vereinigt und gefriergetrocknet.

| | |
|---|---|
| Ausbeute: | 81 mg (60% d.Th.) |
| ESI-MS: | m/z = 541 (M+H)⁺ |
| Rₜ (HPLC-MS): | 3.59 min (Methode E) |

### Beispiel 59:

### 3-(1-(6-(2',3'-Dihydro-1'H-spiro[cyclopropan-1,4'-isochinolin]-2'-ylcarbonyl)pyrimidin-4-yl)-piperidin-4-yl)-7-methoxy-4,5-dihydro-1 H-benzo[d][1,3]diazepin-2(3H)-on

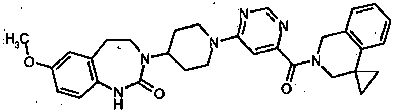

50 mg (0.13 mmol) 6-[4-(7-Methoxy-2-oxo-1,2,4,5-tetrahydro-1,3-benzodiazepin-3-yl)-piperidin-1-yl]-pyrimidin-4-carbonsäure, 20 mg (0.13 mmol) 2',3'-Dihydro-1'H-spiro-[cyclo-propan-1,4'-isochinolin], 37 µL (0.26 mmol) TEA und 44.3 mg (0.14 mmol) TBTU wurden in 1.5 mL DMF über Nacht bei RT gerührt. Das Reaktionsgemisch wurde mittels HPLC gereinigt. Die Produkt enthaltenden Fraktionen wurden vereinigt und gefriergetrocknet.

| | |
|---|---|
| Ausbeute: | 5 mg (7% d.Th.) |
| ESI-MS: | m/z = 539 (M+H)⁺ |
| Rₜ (HPLC-MS): | 3.98 min (Methode E) |

### Beispiel 60:

### 1-{1-[6-(4,5-Difluor-3-methyl-2,3-dihydro-indol-1-carbonyl)-pyrimidin-4-yl]-piperidin-4-yl}-1,3-dihydro-imidazo[4,5-b]pyridin-2-on

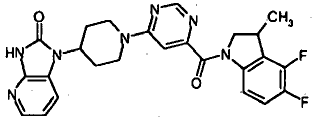

80 mg (0.24 mmol) 6-[4-(2-Oxo-2,3-dihydroimidazo[4,5-b]pyridin-1-yl)-piperidin-1-yl]-pyrimidin-4-carbonsäure, 43 mg (0.25 mmol) 4,5-Difluor-3-methyl-2,3-dihydro-1H-indol, 70 µL (0.50 mmol) TEA und 90 mg (0.28 mmol) TBTU wurden in 1.8 mL DMF über Nacht bei RT gerührt. Das Reaktionsgemisch wurde mittels HPLC gereinigt. Die Produkt enthaltenden Fraktionen wurden vereinigt und gefriergetrocknet.

| | |
|---|---|
| Ausbeute: | 76 mg (66% d.Th.) |
| ESI-MS: | m/z = 492 (M+H)⁺ |
| Rt (HPLC-MS): | 3.6 min (Methode K) |

Analog zu 1-{1-[6-(4,5-Difluor-3-methyl-2,3-dihydro-indol-1-carbonyl)-pyrimidin-4-yl]-piperidin-4-yl)-1,3-dihydro-imidazo[4,5-b]pyridin-2-on wurden folgende Verbindungen aus jeweils 0.24 - 0.43 mmol -[4-(2-Oxo-2,3-dihydroimidazo[4,5-b]pyridin-1-yl)-piperidin-1-yl]-pyrimidin-4-carbonsäure, 1 bis 1.5 eq des jeweiligen Amins, 1.0 bis 1.2 eq TBTU und 1.1 bis 4.1 eq Triethylamin in einer geeigneten Menge DMF hergestellt:

| Bsp. | Struktur Name | [Menge Amin] Amin Ausbeute | Analytische Daten |
|---|---|---|---|
| 61 | 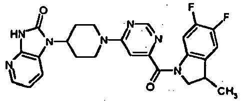 | 40 mg (0.24 mmol) | ESI-MS: m/z = 492 [M+H]⁺ |
| | 1-{1-[6-(5,6-Difluor-3-methyl-2,3-dihydro-indol-1-carbonyl)-pyri-midin-4-yl]-piperidin-4-yl}-1,3-dihydroimidazo[4,5-b]pyridin-2-on | 5,6-Difluor-3-methyl-2,3-dihydro-1H-indol | Rₜ = 3.8 min Methode K |
| | | 70 mg (61% d.Th.) | |
| 62 | 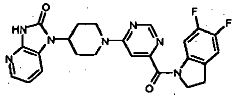 | 62 mg (0.40 mmol) | ESI-MS: m/z = 478 |
| | 1-{1-[6-(5,6-Difluor-2,3-dihydro-indol-1-carbonyl)-pyrimidin-4-yl]-piperidin-4-yl}-1,3-dihydro-imidazo[4,5-b]pyridin-2-on | 5,6-Difluor-2,3-dihydro-1H-indol | [M+H]⁺ |
| | | 72 mg (51% d.Th.) | 1.4 min Methode C |
| 63 | 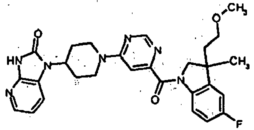 | 80 mg (0.38 mmol) | ESI-MS: |
| | 1-(1-{6-[5-Fluor-3-(2-methoxy-ethyl)-3-methyl-2,3-dihydro-indol-1-carbonyl]-pyrimidin-4-yl}-piperidin-4-yl)-1,3-dihydro-imidazo[4,5-b]pyridin-2-on | 5-Fluor-3-(2-methoxy-ethyl)-3-methyl-2,3-dihydro-1 H-indol | m/z = 530 [M+H]⁺ |
| | | 83 mg (41% d.Th.) | 1.4 min Methode C |
| 64 | 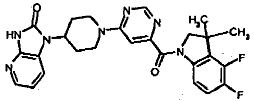 | 80 mg (0.44 mmol) | ESI-MS: m/z = 506 [M+H]⁺ |
| | 1-{1-[6-(4,5-Difluor-3,3-dimethyl-2,3-dihydro-indol-1-carbonyl)-pyrimidin-4-yl]-piperidin-4-yl}-1,3-dihydroimidazo[4,5-b]pyridin-2-on | 4,5-Difluor-3,3-dimethyl-2,3-dihydro-1H-indol | |
| | | 0.10 g (45% d.Th.) | 1.4 min Methode C |
| Verqleichsbeispiel | 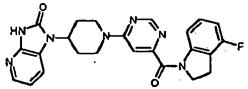 | 45 mg (0.33 mmol) | ESI-MS: m/z = 460 [M+H]⁺ |
| | 1-{1-[6-(4-Fluor-2,3-dihydroindol-1-carbonyl)-pyrimidin-4-yl]-piperidin-4-yl}-1,3-dihydro-imidazo[4,5-b]pyridin-2-on | 4-Fluor-2,3-dihydro-1H-indol | R_{f} = 0.63 Kieselgel, Laufmittel A) |
| | | 95 mg (70% d.Th.) | |
| Vergleichsbeispiel | 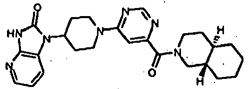 | 50 mg (0.36 mmol) | ESI-MS: m/z = 462 [M+H]⁺ |
| | 1-{1-[6-(Octahydro-isochinolin-2-carbonyl)-pyrimidin-4-yl]-piperidin-4-yl}-1,3-dihydro-imidazo[4,5-b]pyridin-2-on | Decahydro-isöchinolin | 3.03 min Methode E |
| | | 33 mg (23% d.Th.) | |
| 65 | 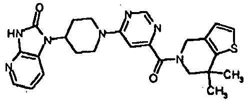 | 40 mg (0.24 mmol) | ESI-MS: m/z = 490 [M+H]⁺ |
| | 1-{1-[6-(7,7-Dimethyl-6,7-dihydro-4H-thieno[3,2-c]pyridin-5-carbonyl)-pyrimidin-4-yl]-piperidin-4-yl}-1,3-dihydro-imidazo[4,5-b]pyridin-2-on | 7,7-Dimethyl-4,5,6,7-tetrahydro-thieno[3,2-c]pyridin | |
| | | 12 mg (12% d.Th.) | 3.42 min Methode K |
| 66 | 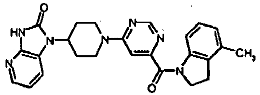 | 50 mg (0.30 mmol) | ESI-MS: m/z = 456 [M+H]⁺ |
| | 1-{1-[6-(4-Methyl-2,3-dihydro-indol-1-carbonyl)-pyrimidin-4-yl]-piperidin-4-yl}-1,3-dihydro-imidazo[4,5-b]pyridin-2-on | 4-Methyl-2,3-dihydro-1H-indol Hydrochlorid | |
| | | 95 mg (71% d.Th.) | R_{f} = 0.67 Kieselgel, Laufmittel A |
| Vergleichsbeispiel | 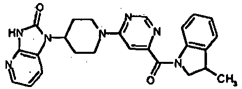 | 40 mg (0.30 mmol) | ESI-MS: m/z = 456 [M+H]⁺ |
| | 1-{1-[6-(3-Methyl-2,3-dihydro-indol-1-carbonyl)-pyrimidin-4-yl]-piperid in-4-yl}-1,3-dihydro-imidazo[4,5-b]pyridin-2-on | 3-Methyl-2,3-dihydro-1H-indol | R_{f} = 0.62 Kieselgel, Laufmittel A) |
| | | 65 mg (54% d.Th.) | |
| 67. | 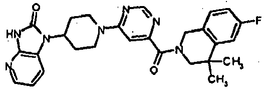 | 38 mg (0.18 mmol) | ESI-MS: |
| | 1-{1-[6-(6-Fluor-4,4-dimethyl-3,4-dihydro-1H-isochinolin-2-carbonyl)-pyrimidin-4-yl]-piperidin-4-yl}-1,3-dihydro-imidazo[4,5-b]pyridin-2-on | 6-Fluor-4,4-dimethyl-1,2,3,4-tetrahydro-isochinolin Hydrochlorid | m/z = 502 [M+H]⁺ |
| | | 5 mg (6% d.Th.) | 1.34 min Methode C |
| 68 | 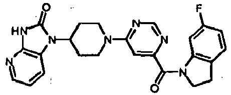 | 45 mg (0.33 mmol) | ESI-MS: m/z = 460 [M +H]⁺ |
| | 1-{1-[6-(6-Fluor-2,3-dihydro-indol-1-carbonyl)-pyrimidin-4-yl]-piperidin-4-yl}-1,3-dihydro-imidazo[4,5-b]pyridin-2-on | 6-Fluor-2,3-dihydro-1H-indol | |
| | | 0.10 g (74% d.Th.) | R_{f} = 0.71 Kieselgel, Laufmittel A |
| Vergleichsbeispiel | 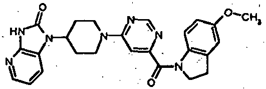 | 60 mg (0.32 mmol) | ESI-MS: m/z = 470 [M+H]⁺ |
| | 1-{1-[6-(5-Methoxy-2,3-dihydro-indol-1-carbonyl)-pyrimidin-4-yl]-piperidin-4-yl}-1,3-dihydro-imidazo[4,5-b]pyridin-2-on | 5-Methoxy-2,3-dihydro-1H-indol Hydrochlorid | R_{f}=0.64 |
| | | 95 mg (69% d.Th.) | Kieselgel, Laufmittel A |
| Vergleichsbeispiel | 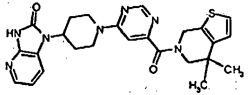 | 40 mg (0.24 mmol) | ESI-MS: m/z = 490 [M+H]⁺ |
| | 1-{1-[6-(4,4-Dimethyl-4,7-dihydro-5H-thieno[2,3-c]pyridin-6-carbonyl)-pyrimidin-4-yl]-piperidin-4-yl}-1,3-dihydro-imidazo[4,5-b]pyridin-2-on | 4,4-Dimethyl-4,5,6,7-tetrahydro-thieno[2,3-c]pyridin | |
| | | 31 mg (30% d.Th.) | 3.03 min Methode E |
| 69 | 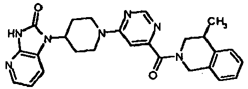 | 42 mg (0.23 mmol) | ESI-MS: m/z = 478 [M+H]⁺ |
| | 1-{1-[6-(4-Methyl-3,4-dihydro-1 H-isochinolin-2-carbonyl)-pyrimidin-4-yl]-piperidin-4-yl}-1,3-dihydro-imidazo[4,5-b]pyridin-2-on | 4-Methyl-1,2,3,4-tetrahydroisochinolin Hydrochlorid | |
| | | 25 mg (26% d.Th.) | 3.9 min Methode B |
| 70 | 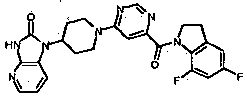 | 37 mg (0.24 mmol) | ESI-MS: m/z = 470 [M+H]⁺ |
| | 1-{1-[6-(5, 7-Difluor-2,3-dihydro-indol-1-carbonyl)-pyrimidin-4-yl]-piperidin-4-yl}-1,3-dihydro-imidazo[4,5-b]pyridin-2-on | 4-Methyl-1,2,3,4-tetrahydro-isochinolin | |
| | | 48 mg (43% d.Th.) | 1.2 min Methode C |
| 71 | 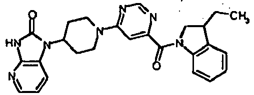 | 34 mg (0.23 mmol) | ESI-MS: m/z = 470 [M+H]⁺ |
| | 1-{1-[6-(3-Ethyl-2,3-dihydro-indol-1-carbonyl)-pyrimidin-4-yl]-piperidin-4-yl}-1,3-dihydro-imidazo[4,5-b]pyridin-2-on | 3-Ethyl-2,3-dihydro-1H-indol | |
| | | 22 mg (23% d.Th.) | 1.36 min Methode C |
| 72 | 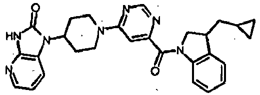 | 40 mg (0.23 mmol) | ESI-MS: m/z = 496 [M+H]⁺ |
| | 1-{1-[6-(3-Cyclopropylmethyl-2,3-dihydro-indol-1-carbonyl)-pyrimidin-4-yl]-piperidin-4-yl}-1,3-dihydro-imidazo[4,5-b]pyridin-2-on | 3-Cyclo-propylmethyl-2,3-dihydro-1H-indol | |
| | | 45 mg (44% d.Th.) | 1.47 min (Methode C) |
| 73 | 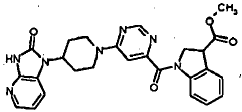 | 130 mg (0.61 mmol) | ESI-MS: m/z = 500 [M+H]⁺ |
| | 1-{6-[4-(2-Oxo-2,3-dihydro-imidazo[4,5-b]pyridin-1-yl)-piperidin-1-yl]-pyrimidin-4-carbonyl}-2,3-dihydro-1H-indol-3-carbonsäuremethylester | 2,3-Dihydro-1 H-indol-3-carbonsäuremethylester Hydrochlorid | |
| | | 160 mg (55% d.Th.) | R_{f} = 0.64 Kieselgel, Laufmittel A |
| 74 | 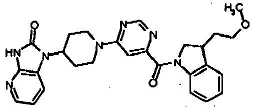 | 41 mg (0.23 mmol) | ESI-MS: m/z = 500 [M+H]⁺ |
| | 1-(1-{6-[3-(2-Methoxy-ethyl)-2,3-dihydro-indol-1-carbonyl]-pyrimidin-4-yl}-piperidin-4-yl}-1,3-dihydro-imidazo[4,5-b]pyridin-2-on | 3-(2-Methoxy-ethyl)-2,3-dihydro-1 H-indol | 3.33 min |
| | | 72 mg (70% d.Th.) | Methode E |
| Vergleichsbeispiel | 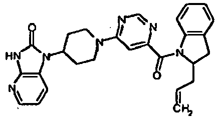 | 60 mg (0.31 mmol) | ESI-MS: m/z = 482 [M+H]⁺ |
| | 1-{1-[6-(2-Allyl-2,3-dihydro-indol-1-carbonyl)-pyrimidin-4-yl]-piperidin-4-yl}-1,3-dihydro-imidazo[4,5-b]pyridin-2-on | 2-Allyl-2,3-dihydro-1H-indol Hydrochlorid | |
| | | 72 mg (51% d.Th.) | R_{f} = 0.62 Kieselgel, Laufmittel A |
| 75 | 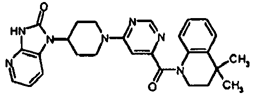 | 81 mg (0.5 mmol) | ESI-MS: m/z = 484 [M+H]⁺ |
| | 1-{1-[6-(4,4-Dimethyl-3,4-dihydro-2H-chinolin-1-carbonyl)-pyrimidin-4-yl]-piperidin-4-yl}-1,3-dihydro-im idazo[4,5-b]pyridin-2-on | 4,4-Dimethyl-1,2,3,4-tetrahydro-chinolin | |
| | | 118 mg (55% d.Th.) | 3.17 min (Methode K) |
| 76 | 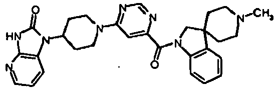 | 61 mg (0.3 mmol) | ESI-MS: m/z = 525 [M+H]⁺ |
| | 1-(1-(6-(1'-Methylspiro[indolin-3,4'- , piperidin]-1-ylcarbonyl)pyrimidin-4-yl)piperidin-4-yl)-1H-imidazo[4,5-b]pyridin-2(3H)-on | 1'-Methylspiro[indolin-3,4'-piperidin] | |
| | | 118 mg (77% d.Th.) | 2.2 min (Methode K) |
| Vergleichsbeispiel | 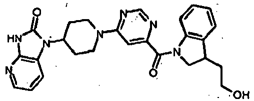 | 50 mg (0.31 mmol) | ESI-MS: m/z = 486 [M+H]⁺ |
| | 1-(1-{6-[3-(2-Hydroxy-ethyl)-2,3-dihydro-indol-1-carbonyl]-pyrimidin-4-yl}-piperidin-4-yl)-1,3-dihydro-imidazo[4,5-b]pyridin-2-on | 2-(2,3-Dihydro-1H-indol-3-yl)-ethanol | |
| | | 85 mg (66% d.Th.) | R_{f} = 0.59 Kieselgel, Laufmittel A |
| Vergleichsbeispiel | 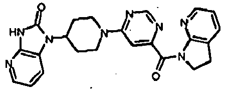 | 45 mg (0.32 mmol) | ESI-MS: m/z =443 [M+H]⁺ |
| | 1-{1-[6-(2,3-Dihydro-pyrrolo[2,3-b]pyridin-1-carbonyl)-pyrimidin-4-yl]-piperidin-4-yl}-1,3-dihydro-imidazo[4,5-b]pyridin-2-on | 2,3-Dihydro-1H-pyrrolo[2,3-b]pyridin | |
| | | 50 mg (39% d.Th.) | R_{f} = 0.54 Kieselgel, Laufmittel A |
| Vergleichsbeispiel | 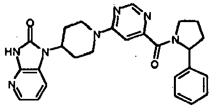 | 45 mg (0.31 mmol) | ESI-MS: m/z =470 [M+H]⁺ |
| | 1-{1-[6-(2-Phenyl-pyrrolidin-1-carbonyl)-pyrimidin-4-yl]-piperidin-4-yl}-1,3-dihydro-imidazo[4,5-b]pyridin-2-on | 2-Phenylpyrrolidin | |
| | | 65 mg (47% d.Th.) | R_{f}=0.61 Kieselgel, Laufmittel A |
| Vergleichsbeispiel | 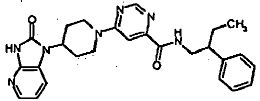 | 46 mg (0.31 mmol) | ESI-MS: m/z =472 [M+H]⁺ |
| | 6-[4-(2-Oxo-2,3-dihydro-imidazo[4,5-b]pyridin-1-yl)-piperidin-1-yl]-pyrimidin-4-carbonsäure (2-phenyl-butyl)-amid | 2-Phenylbutan-1-amin | |
| | | 80 mg (58% d.Th.) | R_{f} = 0.56 Kieselgel, Laufmittel A |
| Vergleichsbeispiel | 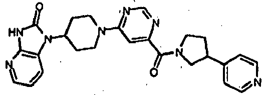 | 45 mg (0.3 mmol) | ESI-MS: m/z =471 [M+H]⁺ |
| | 1-{1-[6-(3-Pyridin-4-yl-Pyrrolidin-1-carbonyl)-pyrimidin-4-yl]-piperidin-4-yl}-1,3-dihydro-imidazo[4,5-b ]pyridin-2-on | 4-Pyrrolinin-3-ylpyridin | R_{f} = 0.55 Kieselgel, Laufmittel A |
| | | 20 mg (15% d.Th.) | |
| Vergleichsbeispiel | 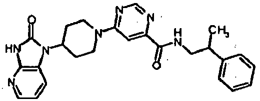 6-[4-(2-Oxo-2,3-dihydro-imidazo[4,5-b]pyridin-1-yl)-piperidin-1-yl]-pyrimidin-4-carbonsäure (2-phenyl-propyl)-amid | 40 mg (0.3 mmol) | ESI-MS: m/z =458 [M+H]⁺ |
| | | Beta-methyl-phenethylamin | R_{f} = 0.62 Kieselgel, Laufmittel A |
| | | 72 mg (54% d.Th.). | |

### Beispiel 77:

### 1-{1-[2-(7,7-Dimethyl-1,4,6,7-tetrahydro-pyrazolo[4,3-c]pyridin-5-carbonyl)-pyrimidin-4-yl]-piperidin-4-yl}-1,3-dihydro-imidazo[4,5-b]pyridin-2-on

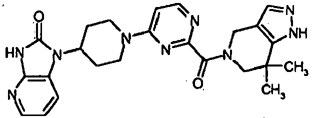

60 mg (0.18 mmol) 4-[4-(2-Oxo-2,3-dihydro-imidazo[4,5-b]pyridin-1-yl)piperidin-1-yl]-pyrimidin-2-carbonsäure, 40 mg (0.18 mmol) 7,7-Dimethyl-4,5,6,7-tetrahydro-1H-pyrazolo-[4,3-c]pyridin Dihydrochlorid, 0.10 (0.72 mmol) Triethylamin und 62 (0.19 mmol) TBTU in 1.5 mL DMF wurden über Nacht bei RT gerührt. Der Reaktionsansatz wurde mittels HPLC gereinigt. Die Produkt enthaltenden Fraktionen wurden vereinigt und gefriergetrocknet.

| | |
|---|---|
| Ausbeute: | 25 mg (30% d.Th.) |
| ESI-MS: | m/z = 474 (M+H)⁺ |
| Rₜ (HPLC-MS): | 0.91 min (Methode C) |

### Beispiel 78:

### 1-[4'-(4,5-Difluor-2,3-dihydro-indol-1-carbonyl)-3,4,5,6-tetrahydro-2H-[1,2']bipyridinyl-4-yl]-1,3-dihydro-imidazo[4,5-b]pyridin-2-on

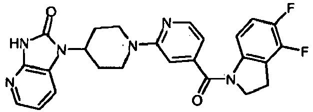

0.59 g (2.7 mmol) 1-Piperidin-4-yl-1,3-dihydroimidazo[4,5-b]pyridin-2-on, 0.80 g (2.7 mmol) (2-Chlor-pyridin-4-yl)-(4,5-difluor-2,3-dihydro-indol-1-yl)-methanon und 0.39 g (2.8 mmol) Kaliumcarbonat wurden in 3 ml NMP 4 h bei 130°C gerührt. Das Reaktionsgemisch wurde mittels HPLC gereinigt. Die Produkt enthaltenden Fraktionen wurden vereinigt und gefriergetrocknet.

| | |
|---|---|
| Ausbeute: | 74 mg (6% d.Th.) |
| ESI-MS: | m/z = 477 (M+H)⁺ |
| Rₜ (HPLC-MS): | 1.26 min (Methode C) |

### Beispiel 79:

### 1-[4'-(5,6-Difluor-2,3-dihydro-indol-1-carbonyl)-3,4,5,6-tetrahydro-2H-[1,2']bipyridinyl-4-yl]-1,3-dihydro-imidazo[4,5-b]pyridin-2-on

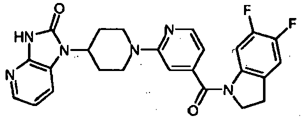

1.0 g (4.6 mmol) 1-Piperidin-4-yl-1,3-dihydroimidazo[4,5-b]pyridin-2-on, 0.45 g (1.5 mmol) (2-Chlor-pyridin-4-yl)-(5,6-difluor-2,3-dihydro-indol-1-yl)-methanon wurden in 4 mL NMP 4 h bei 130°C gerührt. Das Reaktionsgemisch wurde mittels HPLC gereinigt. Die Produkt enthaltenden Fraktionen wurden vereinigt und gefriergetrocknet.

| | |
|---|---|
| Ausbeute: | 46 mg (2% d.Th.) |
| ESI-MS: | m/z = 477 (M+H)⁺ |
| Rₜ (HPLC-MS): | 3.4 min (Methode E) |

Gemäß der folgenden allgemeinen Arbeitsvorschrift wurden folgende Verbindungen synthetisiert:
0.4 bis 1 mmol 1-Piperidin-4-yl-1,3-dihydroimidazo[4,5-b]pyridin-2-on Dihydrochlorid, 1 eq des jeweiligen Chlorpyridins und 3 eq Kaliumcarbonat in 1 bis 3 mL NMP wurden für 4 bis 12 h bei 130°C gerührt. Die Aufreinigung erfolgte mittel HPLC. Wurde anstelle des 1-Piperidin-4-yl-1,3-dihydroimidazo[4,5-b]pyridin-2-on-Diydrochlorids die freie Base verwendet, so wurde nur 1 eq Kaliumcarbonat eingesetzt:

| Beispiel | Struktur Name | [Menge Pyridin] Amin Ausbeute | Analytische Daten |
|---|---|---|---|
| 80 | 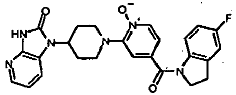 | 140 mg (0.48 mmol) | ESI-MS: m/z = 475[M+H]⁺ |
| | 1-[4'-(5-Fluor-2,3-dihydro-indol-1-carbonyl)-1'-oxy-3,4,5,6-tetrahydro-2H-[1,2']bipyridinyl-4-yl]-1,3-dihydro-imidazo[4_{;}5-b]pyridin-2-on | (2-Chlor-1-oxy-pyridin-4-yl)-(5-fl uor-2,3-dihydro-indöl-1-yl)-methanon | |
| | | | 3.84 min (Methode K) |
| | | 70 mg (30% d.Th.) | |
| 81 | 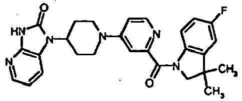 | 120 mg (0.39 mmol) | ESI-MS: m/z = 487 [M+H]⁺ |
| | 1-[2'-(5-Fluor-3,3-dimethyl-2,3-dihydro-indol-1-carbonyl)-3,4,5,6-tetrahydro-2H-[1,4']bipyridinyl-4-yl]-1,3-dihydro-imidazo[4,5-b]pyridin-2-on | (4-Chlor-pyridin-2-yl)-(5-fluor-3,3-dimethyl-2,3-dihydro-indol-1-yl)-methanon | |
| | | | 1.29 min (Methode C) |
| | | 80 mg (41 % d.Th.) | |
| 82 | 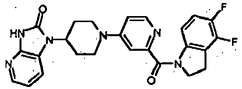 | 200 mg (0.68 mmol) | ESI-MS: m/z = 475 [M-H]⁻ |
| | 1-[2'-(4,5-Difluor-2,3-dihydro-indol-1-carbonyl)-3,4,5,6-tetrahydro-2H-[1,4']bipyridinyl-4-yl]-1,3-dihydro-imidazo[4,5-b]pyridin-2-on | (4-Chlor-pyridin-2-yl)-(4,5-difluoro-2,3-dihydro-indol-1-yl)-methanon | |
| | | | 1.23 min (Methode C) |
| | | 111 mg (35% d.Th.) | |
| 83 | 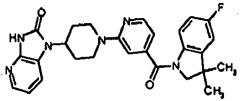 | 120 mg (0.4mmol) | ESI-MS: m/z = 487 [M+H]⁺ |
| | 1-[4'-(5-Fluor-3,3-dimethyl-2,3-dihydro-indol-1-carbonyl)-3,4,5,6-tetrahydro-2H-[1,2']bipyridinyl-4-yl]-1,3-dihydro-imidazo[4,5-b]pyridin-2-on | (2-Chlor-pyridin-4-yl)-(5-fluor-3,3-dimethyl-2,3-dihydro-indol-1-yl)-methanon | |
| | | | 1.28 min (Methode C) |
| | | 42 mg (22% d.Th.) | |
| 84 | 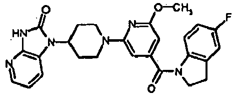 | 300 mg (0.98 mmol) | ESI-MS: m/z = 489 [M+H]⁺ |
| | 1-[4'-(5-Fluor-2,3-dihydro-indol-1-carbonyl)-6'-methoxy-3,4,5,6-tetrahydro-2H-[1,2']bipyridinyl-4-yl]-1,3-dihydro-imidazo[4,5-b]pyridin-2-on | (2-Chlor-6-methoxy-pyridin-4-yl)-(5-fluor-2,3-dihydro-indol-1-yl)-methanon | |
| | | | 1.5 min (Methode C) |
| | | 33 mg (7% d.Th.) | |
| 85 | 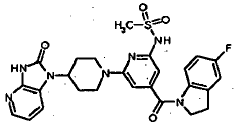 | 340 mg (0.92 mmol) | ESI-MS: m/z = 552 [M+H]⁺ |
| | N-[4'-(5-Fluor-2,3-dihydro-indol-1-carbonyl)-4-(2-oxo-2,3-dihydro-imidazo[4,5-b]pyridin-1-yl)-3,4,5,6-tetrahydro-2H-[1,2']bipyridinyl-6'-yl]-methansulfonamid | N-[6-Chlor-4-(5-fluor-2,3-dihydro -indol-1-carbonyl)-pyridin-2-yl]- 1.22 min methansulfonamid | |
| | | | (Methode C) |
| | | 30 mg (6% d.Th.) | |

### Vergleichsbeispiel:

### 1-{1-[6-(5-Fluor-2,3-dihydro-indol-1-carbonyl)-pyrazin-2-yl]-piperidin-4-yl}-1,3-dihydro-imidazo[4,5-b]pyridin-2-on

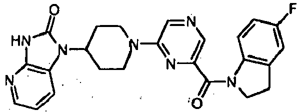

0.10 g (0.34 mmol) 1-Piperidin-4-yl-1,3-dihydroimidazo[4,5-*b*]pyridin-2-on Dihydrochlorid, 0.11 g (0.34 mmol) (6-Chlor-pyrazin-2-yl)-(5-fluor-2,3-dihydro-indol-1-yl)-methanon und 0.20 mL (1.1 mmol) DIPEA wurden in 1.0 mL DMF über Nacht bei 80°C gerührt. Zur Reakionslösung wurden 10 mL Wasser gegeben, der entstandene Niederschlag wurde abgesaugt. Der Niederschlag wurde in Methanol verrührt, abgesaugt und getrocknet.

| | |
|---|---|
| Ausbeute: | 74 mg (6% d.Th.) |
| ESI-MS: | m/z = 477 (M+H)⁺ |
| Rₜ (HPLC-MS): | 1.26 min (Methode C) |

### Vergleichsbeispiel:

### 6'-(7,7-Dimethyl-1,4,6,7-tetrahydro-pyrazolo[4,3-c)pyridin-5-carbonyl)-4-(2-oxo-2,3-dihydro-imidazo[4,5-b]pyridin-1-yl)-3,4,5,6-tetrahydro-2H-[1,4']bipyridinyl-3'-carbonitril

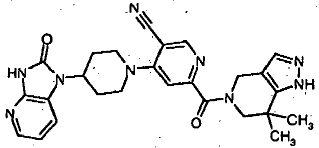

0.11 mg (0.29 mmol) eines Isomerengemischs aus 5'-Cyano-4-(2-oxo-2,3-dihydro-imidazo[4,5-b]pyridin-1-yl)-3,4,5,6-tetrahydro-2H-[1,2']bipyridinyl-4'-carbonsäure und 5'-Cyano-4-(2-oxo-2,3-dihydro-imidazo[4,5-b]pyridin-1-yl)-3,4,5,6-tetrahydro-2H-[1,4']bi-pyridinyl-2'-carbonsäure, 65 mg (0.29 mmol) 7,7-Dimethyl-4,5,6,7-tetrahydro-1H-pyrazolo-[4,3-c]pyridin Dihydrochlorid und 0.17 mL (1.2 mmol) TEA wurden in 1.5 mL DMF vorgelegt. 0.10 g (0.32 mmol) TBTU wurden zugegeben. Das Reaktionsgemisch wurde über das Wochenende bei RT gerührt. Die Aufreinigung und Auftrennung der Isomere erfolgte mittels HPLC-MS. Die Produkt enthaltenden Fraktionen wurden vereinigt und gefriergetrocknet.

| | |
|---|---|
| Ausbeute: | 23 mg (16% d.Th.) |
| ESI-MS: | m/z = 498 (M+H)⁺ |
| Rₜ (HPLC-MS): | 3.33 min (Methode B) |

### Als zweites Produkt erhielt man:

### Beispiel 86:

### 4'-(7,7-Dimethyl-1,4,6,7-tetrahydro-pyrazolo[4,3-c]pyridin-5-carbonyl)-4-(2-oxo-2,3-dihydro-imidazo[4,5-b]pyridin-1-yl)-3,4,5,6-tetrahydro-2H-[1,2']bipyridinyl-5'-carbonitril

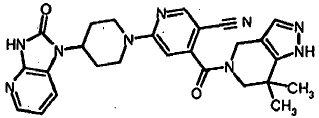

Die Verbindung wurde wie bei 6'-(7,7-Dimethyl-1,4,6,7-tetrahydro-pyrazolo[4,3-c]pyridin-5-carbonyl-4-(2-oxo-2,3-dihydro-imidazo[4,5-b]pyridin-1-yl)-3,4,5,6-tetrahydro-2H-[1,4']bi-pyridinyl-3'-carbonitril beschrieben erhalten.

| | |
|---|---|
| Ausbeute: | 16 mg (11% d.Th.) |
| ESI-MS: | m/z = 498 (M+H)⁺ |
| Rₜ (HPLC-MS): | 3.67 min (Methode B) |

### Beispiel 87:

### 6'-(2,3-Dihydro-indol-1-carbonyl)-4-(2-oxo-2,3-dihydro-imidazo[4,5-b]pyridin-1-yl)-3,4,5,6-tetrahydro-2H-[1,4']bipyridinyl-3'-carbonitril

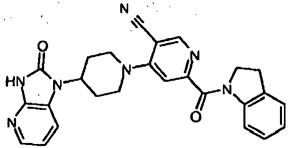

0.11 mg (0.29 mmol) eines Isomerengemischs aus 5'-Cyano-4-(2-oxo-2,3-dihydro-imidazo[4,5-b]pyridin-1-yl)-3,4,5,6-tetrahydro-2H-[1,2']bipyridinyl-4'-carbonsäure und 5'-Cyano-4-(2-oxo-2,3-dihydro-imidazo[4,5-b]pyridin-1-yl)-3,4,5,6-tetrahydro-2H-[1,4']bipyridinyl-2'-carbonsäure, 34 mg (0.29 mmol) 2,3-Dihydro-1H-indol und 90 μL (0.61 mmol) TEA wurden in 1.5 mL DMF vorgelegt. 0.10 g (0.32 mmol) TBTU wurden zugegeben. Das Reaktionsgemisch wurde über das Wochenende bei RT gerührt. Die Aufreinigung und Auftrennung der Isomere erfolgte mittels HPLC-MS. Die Produkt enthaltenden Fraktionen wurden vereinigt und gefriergetrocknet.

| | |
|---|---|
| Ausbeute: | 18 mg (13% d.Th.) |
| ESI-MS: | m/z = 466 (M+H)⁺ |
| Rₜ (HPLC-MS): | 3.67 min (Methode E) |

### Als zweites Produkt erhielt man:

### Beispiel 88:

### 4'-(2,3-Dihydro-indol-1-carbonyl)-4-(2-oxo-2,3-dihydro-imidazo[4,5-b]-pyridin-1-yl)-3,4,5,6-tetrahydro-2H-[1,2']bipyridinyl-5'-carbonitril

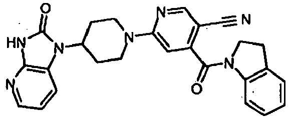

Diese Verbindung wurde wie bei 6'-(2,3-Dihydro-indol-1-carbonyl)-4-(2-oxo-2,3-dihydro-imidazo[4,5-b]pyridin-1-yl)-3,4,5,6-tetrahydro-2H-[1,4']bipyridinyl-3'-carbonitril beschrieben erhalten.

| | |
|---|---|
| Ausbeute: | 5 mg (4% d.Th.) |
| ESI-MS: | m/z = 466 (M+H)⁺ |
| Rₜ (HPLC-MS): | 3.77 min (Methode E) |

### Beispiel 89:

### 1-{4'-[3-(2-Methoxy-ethyl)-2,3-dihydro-indol-1-carbonyl]-3,4,5,6-tetrahydro-2H-[1,2']bipyridinyl-4-yl}-1,3-dihydro-imidazo[4,5-b]pyridin-2-on

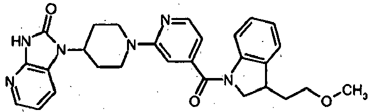

60 mg (0.18 mmol) 4-(2-Oxo-2,3-dihydro-imidazo[4,5-b]pyridin-1-yl)-3,4,5,6-tetrahydro-2H-[1,2']bipyridinyl-4'-carbonsäure, 36 mg (0.2 mmol) 3-(2-Methoxy-ethyl)-2,3-dihydro-1H-indol und 28 μL (0.2 mmol) TEA wurden in 1 mL DMF vorgelegt. 64 mg (0.2 mmol) TBTU wurden zugegeben und das Reaktionsgemisch wurde 3 h bei RT gerührt. Das Reaktionsgemisch wurde mittels HPLC gereinigt. Die Produkt enthaltenden Fraktionen wurden vereinigt und gefriergetrocknet.

| | |
|---|---|
| Ausbeute: | 35 mg (40% d.Th.) |
| ESI-MS: | m/z = 499 (M+H)⁺ |
| Rₜ (HPLC-MS): | 1.24 min (Methode C) |

Analog zu 1-{4'-[3-(2-Methoxy-ethyl)-2,3-dihydro-indol-1-carbonyl]-3,4,5,6-tetrahydro-2H-[1,2']bipyridinyl-4-yl}-1,3-dihydro-imidazo[4,5-b]pyridin-2-on wurden folgende Verbindungen aus jeweils 0.18 mmol 4-(2-Oxo-2,3-dihydro-imidazo[4,5-b]pyridin-1-yl)-3,4,5,6-tetrahydro-2H-[1,2']bipyridinyl-4'-carbonsäure, 1.1 eq Triethylamin, 1.1 eq TBTU und 1 Äquivalent des jeweiligen Amins in 1 mL DMF hergestellt:

| Bsp. | Struktur Name | [Menge Amin] Amin Ausbeute | Analytische Daten |
|---|---|---|---|
| 90 | 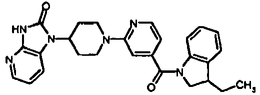 | 29 mg (0.2 mmol) | ESI-MS: m/z = 469 [M+H]⁺ |
| | 1-[4'-(3-Ethyl-2,3-dihydro-indol-1-carbonyl)-3,4,5,6-tetrahydro-2H-[1,2']bipyridinyl-4-yl]-1,3-dihydro-imidazo[4,5-b]pyridin-2-on | 3-Ethyl-2,3-dihydro-1H-indol | |
| | | 6 mg (7% d.Th.) | Rₜ = 1.34 min (Methode C) |
| 91 | 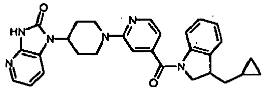 | 35 mg (0.2 mmol) | ESI-MS:m/z = 495 [M+H]⁺ |
| | 1-[4'-(3-Cyclopropylmethyl-2,3-dihydro-indol-1-carbonyl)-3,4,5,6-tetrahydro-2H-[1,2']bipyridinyl-4-yl]-1,3-dihydro-imidazo-[4,5b]pyridin-2-on | 3-Cyclopropyl-methyl-2,3-dihydro-1 H-indol | |
| | | 40 mg (46% d.Th.) | Rₜ = 1.43 min (Methode C) |

### Beispiel 92:

### 2-{1-[6-(2,3-Dihydro-indol-1-carbonyl)-pyrimidin-4-yl]-piperidin-4-yl}-1,4-dihydro-2H-isochinolin-3-on

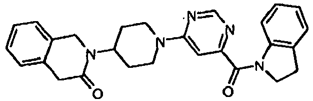

0.10 g (0.39 mmol) (6-Chlor-pyrimidin-4-yl)-(2,3-dihydro-indol-1-yl)-methanon und 100 μL (0.58 mmol) DIPEA wurden in 10 mL DMF vorgelegt. 95 mg (0.41 mmol) 2-Piperidin-4-yl-1,4-dihydro-2H-isochinolin-3-on wurden zugegeben. Der Reaktionsansatz wurde 2 h bei RT gerührt. Anschließend wurde der Reaktionsansatz mit Wasser versetzt und weiter gerührt. Der ausgefallene Feststoff wurde abgesaugt, mit Methanol gewaschen und getrocknet.

| | |
|---|---|
| Ausbeute: | 125 mg (72% d.Th.) |
| ESI-MS: | m/z = 454 (M+H)⁺ |
| R_{f}: | 0.67 (Kieselgel, DCM/Cyclohexan/MeOH/NH₄OH 70:15:15:2) |

### Beispiel 93:

### 3-{1-[6-(5-Fluor-2,3-dihydro-indol-1-carbonyl)-pyrimidin-4-yl]-piperidin-4-yl}-1H-chinolin-2-on

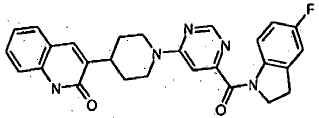

80 mg (0.23 mmol) 6-[4-(2-Oxo-1,2-dihydrochinolin-3-yl)-piperidin-1-yl]-pyrimidin-4-carbonsäure, 35 mg (0.26 mmol) 5-Fluor-2,2-Dihydro-(1H)-indol, 70 μL (0.5 mmol) und 90 mg (0.28 mmol) TBTU wurden in 3 mL DMF über Nacht bei RT gerührt. Der Reaktionsansatz wurde mittels HPLC gereingt. Die Produkt enthaltenden Fraktionen wurden vereinigt und gefriergetrocknet.

| | |
|---|---|
| Ausbeute: | 30 mg (28% d.Th.) |
| ESI-MS: | m/z = 470 (M+H)⁺ |
| Rₜ (HPLC-MS): | 1.49 min (Methode C) |

Analog zu 3-{1-[6-(5-Fluor-2,3-dihydro-indol-1-carbonyl)-pyrimidin-4-yl]-piperidin-4-yl}-1H-chinolin-2-on wurden folgende Verbindungen aus jeweils 0.23 mmol 6-[4-(2-Oxo-1,2-dihydro-chinolin-3-yl)-piperidin-1-yl]-pyrimidin-4-carbonsäure, 2.2 bis 3.1 eq Triethylamin, 1.2 eq TBTU und 1.1 Äquivalente des jeweiligen Amins in 3 mL DMF hergestellt:

| Beispiel | Struktur Name | [Menge Amin] Amin Ausbeute | Analytische Daten |
|---|---|---|---|
| 94 | 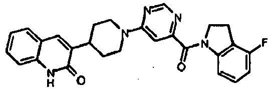 | 35 mg (0.26 mmol) | ESI-MS: m/z = 470 [M+H]⁺ |
| | 3-{1-[6-(4-Fluor-2,3-dihydro-indol-1-carbonyl)-pyrimidin-4-yl]-piperidin-4-yl}-1H-chinolin-2-on | 4-Fluor-2,3-dihydro-1H-indol | |
| | | 15 mg (14% d.Th.) | Rₜ = 1.5 min (Methode C) |
| Vergleichsbeispiel | 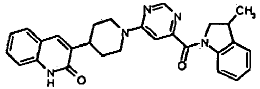 | 35 mg (0.26 mmol) | ESI-MS: m/z = 466 [M+H]⁺ |
| | 3-{1-[6-(3-Methyl-2,3-dihydro-indol-1-carbonyl)-pyrimidin-4-yl]-piperidin-4-yl}-1H-chinolin-2-on | 3-Methyl-2,3-dihydroindol | |
| | | 35 mg (33% d.Th.) | Rₜ = 1.51 min (Methode C) |
| 95 | 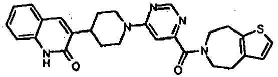 | 60 mg (0.26 mmol) | ESI-MS: m/z = 486 [M+H]⁺ |
| | 3-{1-[6-(4,5,7,8-Tetrahydro-thieno[2,3-d]azepin-6-carbonyl)-pyrimidin-4-yl]-piperidin-4-yl}-1H-chinolin-2-on | 5,6,7,8-Tetrahydro-4H-thieno[2,3-d]-azepin Hydrobromid | |
| | | 5 mg (5% d.Th.) | Rₜ = 1.42 min (Methode C) |
| 96 | 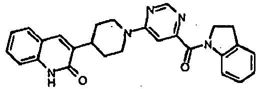 | 28 μL (0.25 mmol) | ESI-MS: m/z = 452 [M+H]⁺ |
| | 3-{1-[6-(2,3-Dihydro-indol-1-carbonyl)-pyrimidin-4-yl]-piperidin-4-yl}-1H-chinolin-2-on | Indolin | |
| | | 35 mg (34% d.Th.) | Rₜ = 1.45 min (Methode C) |
| Vergleichsbeispiel | 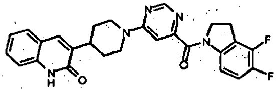 | 50 mg (0.26mmol) | ESI-MS: m/z = 488 [M+H]⁺ |
| | 3-{1-[6-(4,5-Difluor-2,3-dihydro-indol-1-carbonyl)-pyrimidin-4-yl]-piperidin-4-yl}-1H-chinolin-2-on | 4,5-Difluorindolin Hydrochlorid | |
| | | | |
| | | | Rₜ = 1.54 min |
| | | 13 mg (12% d.Th.) | (Methode C) |
| Vergleichsbeispiel | 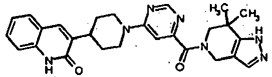 | 55 mg (0.25 mmol) | ESI-MS: m/z = 484 [M+H]⁺ |
| | 3-{1-[6-(7,7-Dimethyl-1,4,6,7-tetrahydro-pyrazolo[4,3-c]pyridin-5-carbonyl)-pyrimidin-4-yl]-piperidin-4-yl}-1H-chinolin-2-on | 7,7-Dimethyl-4,5,6,7-tetrahydro-1H-pyrazolo[4,3-c]-pyridin Dihydrochlorid | |
| | | 2 mg (2% d.Th.) | Rₜ = 1.16 min (Methode C) |
| 97 | 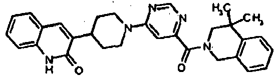 | 50 mg (0.25 mmol) | ESI-MS: m/z = 494 [M+H]⁺ |
| | 3-{1-[6-(4,4-Dimethyl-3,4-dihydro-1H-isochinolin-2-carbonyl)-pyrimidin-4-yl]-piperidin-4-yl}-1H-chinolin-2-on | 4,4-Dimethyl-1,2,3,4-tetrahydro-isochinolin Hydrochlorid | |
| | | 23 mg (20% d.Th.) | Rₜ = 1.5 min (Methode C) |

### Vergleichsbeispiel:

### 1-{1-(6-(5-Fluor-3-methyl-2,3-dihydro-indol-1-carbonyl)-pyrimidin-4-yl]-piperidin-4-yl}-1,3-dihydro-imidazo[4,5-b]pyridin-2-on

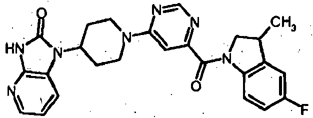

80 mg (0.24 mmol) 6-[4-(2-Oxo-2,3-dihydro-imidazo[4,5-b]pyridin-1-yl)-piperidin-1-yl]-pyrimidin-4-carbonsäure, 36 mg (0.24 mmol) 5-Fluor-3-methyl-2,3-dihydro-1H-indol, 70 μL (0.5 mmol) TEA und 90 mg (0.28 mmol) TBTU wurden in 1.8 mL DMF über Nacht bei RT gerührt. Der Reaktionsansatz wurde mittels HPLC gereingt. Die Produkt enthaltenden Fraktionen wurden vereinigt und gefriergetrocknet.

| | |
|---|---|
| Ausbeute: | 75 mg (67% d.Th.) |
| ESI-MS: | m/z = 474(M+H)⁺ |
| Rₜ (HPLC-MS): | 3.2 min (Methode K) |

### Beispiel 98:

### 6-[4-(2-Oxo-2,3-dihydro-imidazo[4,5-b]pyridin-1-yl)-piperidin-1-yl]-pyrimidin-4-carbon-säure-[2-(2-fluor-phenyl)-ethyl]-amid

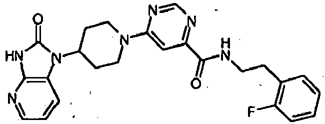

0.10 mg (0.29 mmol) 6-[4-(2-Oxo-2,3-dihydro-imidazo[4,5-b]pyridin-1-yl)-piperidin-1-yl]-pyrimidin-4-carbonsäure, 40 µL (0.31 mmol) 2-Fluorphenethylamin, 45 μL (0.32 mmol) TEA und 100 mg (3.1 mmol) TBTU wurden in 10 mL DMF über Nacht bei RT gerührt. Der Reaktionsansatz wurde mittels HPLC aufgereingt. Die Produkt enthaltenden Fraktionen wurden vereinigt und gefriergetrocknet.

| | |
|---|---|
| Ausbeute: | 70 mg (52% d.Th.) |
| ESI-MS: | m/z = 462 (M+H)⁺ |
| R_{f}: | 0.73 (Kieselgel, DCM/Cyclohexan/MeOH/NH₄OH 70:15:15:2) |

### Beispiel 99:

### 1-[4'-(5-Fluor-3,3-dimethyl-2,3-dihydro-indol-1-carbonyl)-6'-methoxy-3,4,5,6-tetrahydro-2H-[1,2']bipyridinyl-4-yl]-1,3-dihydro-imidazo[4,5-b]pyridin-2-on

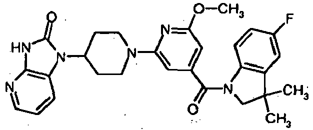

0.26 g (0.9 mmol) 1-Piperidin-4-yl-1,3-dihydroimidazo[4,5-*b*]pyridin-2-on Dihydrochlorid, 0.30 g (0.9 mmol) (2-Chlor-6-methoxy-pyridin-4-yl)-(5-fluor-3,3-dimethyl-2,3-dihydro-indol-1-yl)-methanon und 0.37 g (2.7 mmol) Kaliumcarbonat wurden in 3 mL NMP 12 h bei 130°C gerührt. Der Reaktionsansatz wurde mittels HPLC gereinigt. Die Produkt enthaltenden Fraktionen wurden vereinigt und gefriergetrocknet.

| | |
|---|---|
| Ausbeute: | 40 mg (9% d.Th.) |
| ESI-MS: | m/z = 517 (M+H)⁺ |
| Rₜ (HPLC-MS): | 1.62 min (Methode C) |

### Beispiel 100:

### 1-[6'-(5-Fluor-2,3-dihydro-indol-1-carbonyl)-2'-methoxy-3,4,5,6-tetrahydro-2H-[1,4']bi-pyridinyl-4-yl]-1,3-dihydro-imidazo[4,5-b]pyridin-2-on

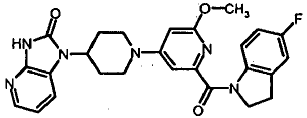

436 mg (2.00 mmol) 1-Piperidin-4-yl-1,3-dihydro-imidazo[4,5-b]pyridin-2-on und 200 mg (0.652 mmol) (4-Chlor-6-methoxy-pyridin-2-yl)-(5-fluor-2,3-dihydro-indol-1-yl)-methanon in 2 mL NMP würden über Nacht bei 120°C gerührt. Der Reaktionsansatz wurde mittel präparativer HPLC aufgereinigt. Die produktenthaltenden Fraktionen wurden vereinigt und am Rotationsverdampfer eingeengt.

| | |
|---|---|
| Ausbeute: | 62 mg (20% d.Th.) |
| ESI-MS: | m/z = 487 (M-H)⁻ |
| Rₜ(HPLC): | 1.7 min (Methode C) |

### Beispiel 101:

### N-[4'-(5-Fluor-2,3-dihydro-indol-1-carbonyl)-4-(2-oxo-2,3-dihydro-imidazo[4,5-b]pyridin-1-yl)-3,4,5,6-tetrahydro-2H-[1,2']bipyridinyl-6'-yl]-N-methyl-methansulfonamid

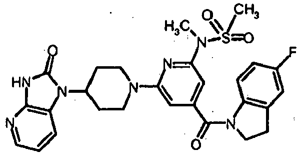

0.25 g (0.89 mmol) 1-Piperidin-4-yl-1,3-dihydroimidazo[4,5-*b*]pyridin-2-on Dihydrochlorid, 0.34 g (0.89 mmol) N-[6-Chlor-4-(5-fluor-2,3-dihydro-indol-1-carbonyl)-pyridin-2-yl]-N-methyl-methansulfonamid und 0.37 g (2.7 mmol) Kaliumcarbonat wurden in 3 mL NMP 4 h bei 130°C gerührt. Der Reaktionsansatz wurde mittels HPLC aufgereinigt. Die Produkt enthaltenden Fraktionen wurden vereinigt und Acetonitril wurde abrotiert. Die ausgefallene Substanz wurde abgesaugt und getrocknet.

| | |
|---|---|
| Ausbeute: | 160 mg (32% d.Th.) |
| ESI-MS: | m/z = 566(M+H)⁺ |
| Rₜ (HPLC-MS): | 1.4 min (Methode C) |

### Vengleichsbeispiel:

### 1-[4'-(4,5-Difluor-2,3-dihydro-indol-1-carbonyl)-6'-methoxy-3,4,5,6-tetrahydro-2H-[1,2']bipyridinyl-4-yl]-1,3-dihydro-imidazo[4,5-b]pyridin-2-on

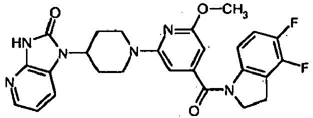

0.98 g (4.5 mmol) 1-Piperidin-4-yl-1,3-dihydroimidazo[4,5-*b*]pyridin-2-on und 0.50 g (1.5 mmol) (2-Chlor-6-methoxy-pyridin-4-yl)-(4,5-difluor-2,3-dihydro-indol-1-yl)-methanon wurden in 3 mL NMP 12 h bei 130°C gerührt. Der Reaktionsansatz wurde mittels HPLC gereinigt. Die Produkt enthaltenden Fraktionen wurden vereinigt und gefriergetrocknet.

| | |
|---|---|
| Ausbeute: | 0.25 g (32% d.Th.) |
| ESI-MS: | m/z = 507(M+H)⁺ |
| Rₜ (HPLC-MS): | 1.59 min (Methode C) |

### Vergleichsbeispiel:

### 3-(1-(6-((4aR,8aS)-Decahydroisochinolin-2-carbonyl)pyrimidin-4-yl)piperidin-4-yl)-7-methoxy-4,5-dihydro-1H-benzo[d][1,3]diazepin-2(3H)-on

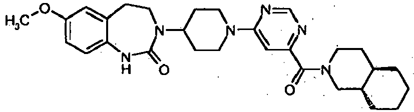

1.7 mg (12 µmol) (4aR,8aS)-Decahydroisochinolin und 1.6 mg (12 µmol) DIPEA wurden in 350 μL DMF vorgelegt. 4.0 mg (10 µmol) 6-[4-(7-Methoxy-2-oxo-1,2,4,5-tetrahydro-1,3-benzodiazepin-3-yl)-piperidin-1-yl]-pyrimidin-4-carbonsäure und 10 μmol) DIPEA in 100 μL DMF wurden mit 3.5 mg (11 μmol) TBTU in 50 μL DMF aktiviert und anschließend zugetropft. Das Reaktionsgemisch wurde über Nacht gerührt und im Folgenden mit 15 μL einer wässrigen 2M Kaliumcarbonat-Lösung versetzt. Nach 1h Rühren ließ man den ausgefallenen Niederschlag absetzen. Die überstehende Lösung wurde abpipettiert und zur Trockene einrotiert.

| | |
|---|---|
| Ausbeute: | 5.1 mg |
| ESI-MS: | m/z = 519 (M+H)⁺ |

Analog zu Decahydroisochinolin-2-carbonyl)pyrimidin-4-yl)piperidin-4-yl)-7-methoxy-4,5-dihydro-1H-benzo[d][1,3]diazepin-2(3H)-on wurden folgende Verbindungen aus 4.0 mg (10 μmo)6-[4-(7-Methoxy-2-oxo-1,2,4,5-tetrahydro-1,3-benzodiazepin-3-yl)-piperidin-1-yl]-pyrimidin-4-carbonsäure, 3.6 mg (11 μmol) TBTU, 12 μmol des entsprechenden Amins und 3.9 μL DIPEA in 350 μL DMF hergestellt:

| Beispiel | Struktur | Amin Ausbeute | Analytische Daten |
|---|---|---|---|
| Vergleichsbeispiel | 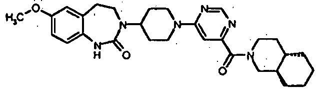 | (4aS,8aS)-Decahydro-isochinolin | ESI-MS: m/z = 519 [M+H]⁺ |
| | 3-(1-(6-((4aS,8aS)-Decahydro-isochinolin-2-carbonyl)-pyrimidin-4-yl)-piperidin-4-yl)-7-methoxy-4,5-dihydro-1H-benzo[d][1,3]-diazepin-2(3H)-on | 5 mg | |
| Vergleichsbeispiel | 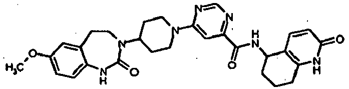 | 5-Amino-5,6,7,8-tetrahydro-1H-chinolin-2-on-Dihydrochlorid | ESI-MS: m/z = 544 [M+H]⁺ |
| | 6-[4-(7-Methoxy-2-oxo-1,2,4,5-tetrahydro-1,3-benzodiazepin-3-yl)-piperidin-1-yl]-pyrimidin-4-carbonsäure (2-oxo-1,2,5,6,7,8-hexahydro-chinolin-5-yl)-amid | 1.3 mg | |
| Verqleichsbeispiel | 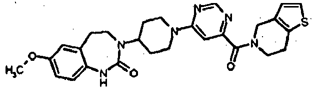 | 4,5,6,7-Tetrahydrothieno[3,2-c]pyridin | ESI-MS: m/z =519 [M+H]⁺ |
| | 3-{1-[6-(6,7-Dihydro-4H-thieno-[3,2-c]pyridin-5-carbonyl)-pyrimidin-4-yl]-piperidin-4-yl}-7-methoxy-1,3,4,5-tetrahydro-1,3-benzodiazepin-2-on | | |
| | | 5.2 mg | |
| Vergleichsbeispiel | 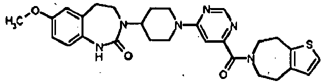 | 5,6,7,8-Tetrahydro-4H-thieno[2,3-d] azepin | ESI-MS: m/z = 533 [M+H]⁺ |
| | 7-Methoxy-3-{1-[6-(4,5,7,8-tetrahydro-thieno[2,3-d]azepin-6-carbonyl)-pyrimidin-4-yl]-piperidin-4-yl}-1,3,4,5-tetrahydro-benzo[d][1,3]diazepin-2-on | 6 mg | |
| Vergleichsbeispiel | 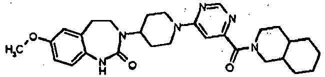 | Decahydro-isochinolin | ESI-MS: m/z = 519 [M+H]⁺ |
| | 7-Methoxy-3-{1-[6-(octahydro-isochinolin-2-carbonyl)-pyrimidin-4-yl]-piperidin-4-yl}-1,3,4,5-tetrahydro-1,3-benzodiazepin-2-on | 5 mg | |
| Verqleichsbeispiel | 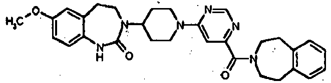 | 2,3,4,5-Tetrahydro-1H-benzo[d]azepin | ESI-MS: m/z = 527 [M+H]⁺ |
| | 7-Methoxy-3-{1-[6-(1,2,4,5-tetra-hydro-benzo[d]azepin-3-carbonyl)-pyrimidin-4-yl]-piperidin-4-yl}-1,3,4,5-tetrahydro-1,3-benzodiazepin-2-on | 3.1mg | |

### Vergleichsbeispiel:

### 6-[4-(7-Methoxy-2-oxo-1,2,4,5-tetrahydro-1,3-benzodiazepin-3-yl)-piperidin-1-yl]-pyrimidin-4-carbonsäure methyl-(4-oxo-3,4-dihydro-chinazolin-2-ylmethyl)-amid

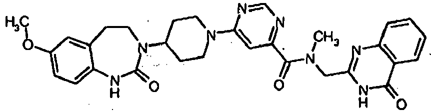

2.3 mg (12 µmol) 2-Methylaminomethyl-3H-chinazolin-4-on und 1.6 mg (12 μmol) DIPEA wurden in 350 μL DMF vorgelegt. 4.0 mg (10 μmol) 6-[4-(7-Methoxy-2-oxo-1,2,4,5-tetrahydro-1,3-benzodiazepin-3-yl)-piperidin-1-yl]-pyrimidin-4-carbonsäure und 1.3 mg (10 µmol) DIPEA in 100 uL DMF wurden mit 3.5 mg (11 μmol) TBTU in 50 μL DMF aktiviert und anschließend zugetropft. Der Reaktionsansatz wurde über Nacht gerührt. Es wurden 15 μL einer wässrigen 2M Kaliumcarbonat-Lösung zugesetzt und 1 h weitergerührt. Den ausgefallenen Niederschlag ließ man absetzen und die überstehende Lösung wurde abpipettiert und zur Trockene einrotiert.

| | |
|---|---|
| Ausbeute: | 2.2 mg |
| ESI-MS: | m/z = 569 (M+H)⁺ |

### Beispiel 102:

### 3-{1-[6-(7,7-Dimethyl-3-trifluormethyl-1,4,6,7-tetrahydro-pyrazolo[4,3-c]pyridin-5-carbonyl)-pyrimidin-4-yl]-piperidin-4-yl}-7-methoxy-1,3,4,5-tetrahydro-benzo[d][1,3]diazepin-2-on

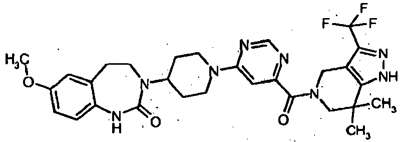

Zu 0.10 g (0.25 mmol) 6-[4-(7-Methoxy-2-oxo-1,2,4,5-tetrahydro-1,3-benzodiazepin-3-yl)-piperidin-1-yl]-pyrimidin-4-carbonsäure, 90 mg (0.32 mmol) 7,7-Dimethyl-3-trifluormethyl-4,5,6,7-tetrahydro-1H-pyrazolo[4,3-c]pyridin Hydrochlorid und 0.12 mL TEA in 1.0 mL DMF wurden 0.11 g (0.28 mmol) TBTU zugefügt und 4 h bei RT gerührt. Anschließend wurde der Reaktionsansatz mittels präparativer HPLC-MS gereinigt. Die das Produkt enthaltenden Fraktionen wurden vereinigt und gefriergetrocknet.

| | |
|---|---|
| Ausbeute: | 40 mg (25% d.Th.) |
| ESI-MS: | m/z = 599 (M+H)⁺ |
| Rₜ (HPLC-MS): | 3.11 min (Methode E) |

### Beispiel 103:

### 3-(5-Fluorindolin-1-carbonyl)-5-(4-(7-methoxy-2-oxo-4,5-dihydro-1H-benzo[d][1,3]-diazepin-3(2H)-yl)piperidin-1-yl)pyridin-1-oxid

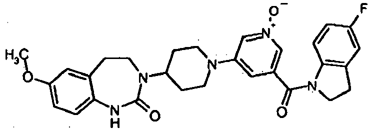

Unter einer Argonatmosphäre wurden zu 69 mg (0.25 mmol) 7-Methoxy-3-piperidin-4-yl-1,3,4,5-tetrahydro-1,3-benzodiazepin-2-on, 84 mg (0.25 mmol) 3-Brom-5-(5-fluorindolin-1-carbonyl)-pyridin1-oxid und 0.12 g (0.38 mmol) Cäsiumcarbonat in 4.0 mL Dioxan 5.0 mg (0.02 mmol) Palladium(II)acetat und 14 mg (20 µmol) BINAP zugefügt und über Nacht bei 120°C gerührt. Der Reaktionsansatz wurde anschließend eingeengt und der Rückstand in DMF/MeOH gelöst und mittels präparativer HPLC-MS gereinigt. Die Produkt enthaltenden Fraktionen wurden vereinigt, das organische Lösungsmittel wurde eingeengt und der Rückstand wurde mit einer 1 N wässrigen Natronlauge Lösung alkalisch gestellt. Der ausgefallene Niederschlag wurde abgesaugt, mit Wasser nachgewaschen und im HV getrocknet.

| | |
|---|---|
| Ausbeute: | 40 mg (30% d.Th.) |
| ESI-MS: | m/z = 532 (M+H)⁺ |
| R, (HPLC-MS): | 1.44 min (Methode C) |

### Beispiel 104:

### 1-[2'-(5-Fluor-2,3-dihydro-indol-1-carbonyl)-5'-(3-hydroxy-prop-1-ynyl)-3,4,5,6-tetrahydro-2H-[1,4']bipyridinyl-4-yl]-1,3-dihydro-imidazo[4,5-b]pyridin-2-on

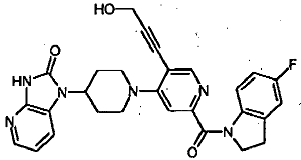

Unter einer Argonatmosphäre wurden zu 0.10 g (0.17 mmol) 1-[2'-(5-Fluor-2,3-dihydro-indol-1-carbonyl)-5'-iod-3,4,5,6-tetrahydro-2H-[1,4']bipyridinyl-4-yl]-1,3-dihydro-imidazo-[4,5-b]pyridin-2-on in 4.0 mL 1,4-Dioxan 44 mg (0.34 mmol) 3-Trimethylsilanyl-prop-2-yn-1-ol, 50 μL TEA, 13 mg 1,1'-Bis(diphenylphosphin)ferrocendichlorpalladiüm (II) und 3 mg (20 μmol) Kupfer(I)jodid zugefügt. Anschließend wurden 0.36 mL (0.36 mmol) einer 1 molaren Tetrabutylammoniumfluorid-Lösung in THF zugetropft und im Folgenden 4 h bei 80°C gerührt. Der Reaktionsansatz wurde mit Wasser versetzt und der entstandene Niederschlag abgesaugt. Der Niederschlag wurde in 6 mL DMF verrührt und abgesaugt. Das Filtrat wurde mittels präparative HPLC-MS gereinigt. Die Produkt enthaltenden Fraktionen wurden vereinigt und gefriergetrocknet.

| | |
|---|---|
| Ausbeute: | 0.4 mg (0.5% d.Th.) |
| ESI-MS: | m/z = 513 (M+H)⁺ |
| Rₜ (HPLC-MS): | 1.44 min (Methode C) |

### Beispiel 105:

### 6'-(5-Fluor-2,3-dihydro-indol-1-carbonyl)-4-(7-methoxy-2-oxo-1,2,4,5-tetrahydro-benzo-[d][1,3]diazepin-3-yl)-3,4,5,6-tetrahydro-2H-[1,4']bipyridinyl-3'-carbonitril

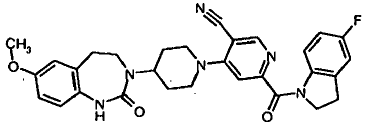

150 mg (0.50 mmol) 4-Chlor-6-(5-fluor-2,3-dihydro-indol-1-carbonyl)-nicotinonitril, 137 mg (0.50 mmol) 7-Methoxy-3-piperidin-4-yl-1,3,4,5-tetrahydro-1,3-benzodiazepin-2-on und 103 mg (0.75 mmol) Kaliumcarbonat in 2 mL DMF wurden 10 h bei 130°C gerührt. Anschließend wurde der Reaktionsansatz mit 4 mL DMF verdünnt und der entstandene Niederschlag abgesaugt. Das Filtrat wurde mittels präparativer HPLC-MS gereinigt. Die Produkt enthaltenden Fraktionen wurden vereinigt und gefriergetrocknet.

| | |
|---|---|
| Ausbeute: | 10 mg (4% d.Th.) |
| ESI-MS: | m/z = 541 (M+H)⁺ |
| Rₜ (HPLC-MS): | 1.68 min (Methode C) |

### Beispiel 106:

### 3-{1-[6-(3-Hydroxymethyl-2,3-dihydro-indol-1-carbonyl)-pyrimidin-4-yl]-piperidin-4-yl}-7-methoxy-1,3,4,5-tetrahydro-benzo[d][1,3]diazepin-2-on

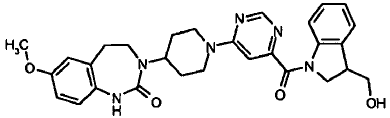

100 mg (0.25 mmol) 6-[4-(7-Methoxy-2-oxo-1,2,4,5-tetrahydro-1,3-benzodiazepin-3-yl)-piperidin-1-yl]-pyrimidin-4-carbonsäure und 50 mg (0.32 mmol) (2,3-Dihydro-1H-indol-3-yl)-methanol in 50 μL (0.27 mmol) TEA und 0.90 mL DMF wurden mit 85 mg (0.27 mmol) TBTU versetzt und 5 h bei RT gerührt. Anschließend wurde der Reaktionsansatz mittels präparativer HPLC-MS gereinigt. Die Produkt enthaltenden Fraktionen wurden vereinigt und auf ca. die Hälfte eingeengt. Der ausgefallene Feststoff wurde abgesaugt und getrocknet.

| | |
|---|---|
| Ausbeute: | 69 mg (52% d.Th.) |
| ESI-MS: | m/z = 529 (M+H)⁺ |
| Rₜ (HPLC-MS): | 2.87 min (Methode E) |

### Beispiel 107:

### 3-[6'-(5-Fluor-2,3-dihydro-indol-1-carbonyl-2'-methyl-3,4,5,6-tetrahydro-2H-[1,4']bipyridiny)-4-yl]-7-methoxy-1,3,4,5-tetrahydro-benzo[d][1,3]diazepin-2-on

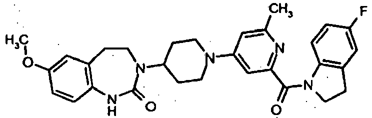

125 mg (0.43 mmol) (4-Chlor-6-methyl-pyridin-2-yl)-(5-fluor-2,3-dihydro-indol-1-yl)-methanon, 118 mg (0.43 mmol) 7-Methoxy-3-piperidin-4-yl-1,3,4,5-tetrahydro-1,3-benzodiazepin-2-on und 89 mg (0.65 mmol) Kaliumcarbonat in 2.0 mL NMP wurden 10 h bei 130°C gerührt. Anschließend wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 33 mg (14% d.Th.) |
| ESI-MS: | m/z = 530 (M+H)⁺ |
| Rₜ (HPLC-MS): | 3.07 min (Methode B) |

### Beispiel 108:

### 3-{1-[6-(5-Fluor-2,3-dihydro-indol-1-carbonyl)-pyrimidin-4-yl]-piperidin-4-yl}-6-methoxy-1H-chinolin-2-on

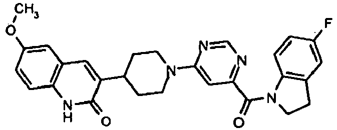

25 mg (70 µmol) 6-Methoxy-3-piperidin-4-yl-1 H-chinolin-2-on, 20 mg (70 µmol) (6-Chlor-pyrimidin-4-yl)-(5-fluor-2,3-dihydro-indol-1-yl)-methanon und 50 μL (0.29 mmol) DIPEA in 2 mL DMF wurden über Nacht bei RT gerührt. Anschließend wurde der Reaktionsansatz mit DMF verdünnt und mittels präparativer HPLC-MS gereinigt. Die Produkt enthaltenden Fraktionen wurden vereinigt und gefriergetrocknet.

| | |
|---|---|
| Ausbeute: | 4 mg (11% d.Th.) |
| ESI-MS: | m/z = 500 (M+H)⁺ |
| Rₜ (HPLC-MS): | 1.56 min (Methode C) |

### Beispiel 109:

### 3-{1-[6-(6-Fluor-2,3-dihydro-indol-1-carbonyl)-pyrimidin-4-yl]-piperidin-4-yl}-7-methoxy-1,3,4,5-tetrahydro-benzo[d][1,3]diazepin-2-on

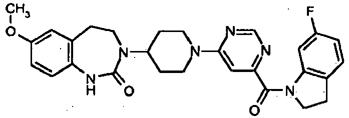

100 mg (0.25 mmol) 6-[4-(7-Methoxy-2-oxo-1,2,4,5-tetrahydro-1,3-benzodiazepin-3-yl)-piperidin-1-yl]-pyrimidin-4-carbonsäure und 35 mg (0.26 mmol) 6-Fluor-2,3-dihydro-1 H-indol in 0.10 mL (0.71 mmol) TEA und 1.5 mL DMF wurden mit 90 mg (0.28 mmol) TBTU versetzt und 1 h bei RT gerührt. Anschließend wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 58 mg (44% d.Th.) |
| ESI-MS: | m/z = 517 (M+H)⁺ |
| Rₜ (HPLC-MS): | 1.56 min (Methode C) |

### Beispiel 110:

### 3-{1-[6-(5-Fluor-2,3-dihydro-indol-1-carbonyl)-pyrimidin-4-yl]-piperidin-4-yl}-3,4-dihydro-1H-pyrido[4,3-d]pyrimidin-2-on

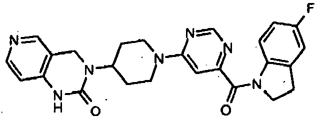

70 mg (0.25 mmol) (6-Chlor-pyrimidin-4-yl)-(5-fluor-2,3-dihydro-indol-1-yl)-methanon, 60 mg (0.26 mmol) 3-Piperidin-4-yl-3,4-dihydro-1H-pyrido[4,3-d]pyrimidin-2-on und 50 uL (0.30 mmol) DIPEA in 5.0 mL DMF wurden 2 h bei RT geschüttelt. Anschließend wurde der Reaktionsansatz auf Wasser gegossen, nachgerührt und der entstandene Niederschlag wurde abgesaugt. Dieser wurde mit Diisopropylether gewaschen und getrocknet.

| | |
|---|---|
| Ausbeute: | 26 mg (22% d.Th.) |
| ESI-MS: | m/z = 474 (M+H)⁺ |
| Rₜ (H PLC-MS): | 1.20 min (Methode C) |

### Beispiel 111:

### 3-[5'-Ethynyl-2'-(5-fluor-2,3-dihydro-indol-1-carbonyl)-3,4,5,6-tetrahydro-2H-[1,4']-bipyridinyl-4-yl]-7-methoxy-1,3,4,5-tetrahydro-benzo[d][1,3]diazepin-2-on

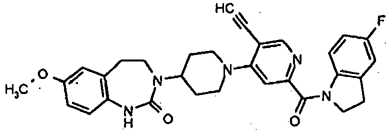

Zu 35 mg (57 µmol) 3-[2'-(5-Fluor-2,3-dihydro-indol-1-carbonyl)-5'-trimethylsilanyl-ethynyl-3,4,5,6-tetrahydro-2H-[1,4']bipyridinyl-4-yl]-7-methoxy-1,3,4,5-tetrahydro-benzo[d][1,3]-diazepin-2-on in 1.5 mL THF wurden 86 µL (86 μmol) einer 1 N Tetrabutylammoniumfluorid-Lösung in THF zugegeben und 3 h bei RT gerührt. Anschließend wurde der Reaktionsansatz mit einer 0.1 molaren wässrigen Salzsäure-Lösung versetzt und der entstandene Niederschlag abgesaugt.

| | |
|---|---|
| Ausbeute: | 8 mg (26% d.Th.) |
| ESI-MS: | m/z = 540 (M+H)⁺ |
| Rₜ (HPLC-MS): | min (Methode C) |

### Beispiel 112:

### 1-[6'-Chlor-4'-(5-fluor-2,3-dihydro-indol-1-carbonyl)-3)-4,5,6-tetrahydro-2H-[1,2']bipyridinyl-4-yl]-1,3-dihydro-imidazo[4,5-b]pyridin-2-on

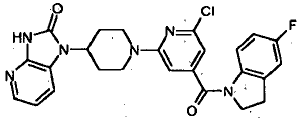

0.74 g (3.4 mmol) 1-Piperidin-4-yl-1,3-dihydroimidazo[4,5-*b*]pyridin-2-on, 1.0 g (3.2 mmol) (2,6-Dichlor-pyridin-4-yl)-(5-fluor-2,3-dihydro-indol-1-yl)-methanon und 0.65 mL (3.8 mmol) DIPEA in 10 mL DMF wurden 2 h bei RT gerührt. Anschließend wurde der Reaktionsansatz eingeengt, der Rückstand mit Wasser versetzt und 30 min bei RT nachgerührt. Der ausgefallene Feststoff wurde abgesaugt, mit Diisopropylether und Isopropanol verrührt und nochmals abgesaugt. Nach dem Trocknen wurde das Rohprodukt mittels präparativer HPLC-MS gereinigt. Die Produkt enthaltenden Fraktionen wurden vereinigt und gefriergetrocknet.

| | |
|---|---|
| Ausbeute: | 350 mg (22% d.Th.) |
| ESI-MS: | m/z = 493 (M+H)⁺ |
| Rₜ (HPLC-MS): | 1.73 min (Methode O) |

### Beispiel 113:

### 3-{1-[6-(5-Fluor-2,3-dihydro-indol-1-carbonyl)-pyrimidin-4-yl]-piperidin-4-yl}-3,4-dihydro-1H-pyrido[2,3-d]pyrimidin-2-on

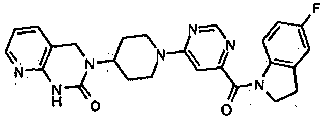

70 mg (0.25 mmol) (6-Chlor-pyrimidin-4-yl)-(5-fluor-2,3-dihydro-indol-1-yl)-methanon, 60 mg (0.26 mmol) 3-Piperidin-4-yl-3,4-dihydro-1H-pyrido[2,3-d]pyrimidin-2-on und 52 µL (0.30 mmol) DIPEA in 5.0 mL DMF wurden 2 h bei RT geschüttelt. Dann wurde der Reaktionsansatz auf Wasser gegossen, nachgerührt und der entstandene Niederschlag wurde abgesaugt. Dieser wurde mit Diisopropylether gewaschen und getrocknet.

| | |
|---|---|
| Ausbeute: | 99 mg (83% d.Th.) |
| ESI-MS: | m/z = 474 (M+H)⁺ |
| Rₜ (HPLC-MS): | 1.43 min (Methode C) |

### Vergleichsbeispiel:

### 1-[4-(7-Methoxy-2-oxo-1,2,4,5-tetrahydro-benzo[d][1,3]diazepin-3-yl)-3,4,5,6-tetrahydro-2H-[1,4']bipyridinyl-2'-carbonyl]-1,2,2a,5-tetrahydro-3H-pyrrolo[4,3,2-de]chinolin-on

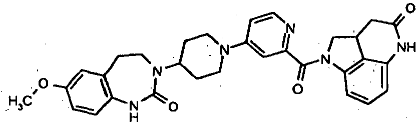

67 mg (0.17 mmol) 4-(7-Methoxy-2-oxo-1,2,4,5-tetrahydro-benzo[d][1,3]diazepin-3-yl)-3,4,5,6-tetrahydro-2H-[1,4']bipyridinyl-2'-carbonsäure und 30 mg (0.17 mmol) 1,2,2a,5-Tetrahydro-3H-pyrrolo[4,3,2-de]chinolin-4-on in 56 μL (0.40 mmol) TEA und 1.8 mL DMF wurden mit 58 mg (0.18 mmol) TBTU versetzt und über Nacht bei RT gerührt. Anschließend wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 70 mg (75% d.Th.) |
| ESI-MS: | m/z = 553 (M+H)⁺ |
| Rₜ (H PLC-MS): | 2.84 min (Methode E) |

### Beispiel 114:

### 1-[4-(7-Methoxy-2-oxo-1,2,4,5-tetrahydro-benzo[d][1,3]diazepin-3-yl)-3,4,5,6-tetrahydro-2H-[1,2']bipyridinyl-4'-carbonyl]-1,2,2a,5-tetrahydro-3H-pyrrolo[4,3,2-de]chinolin-4-on

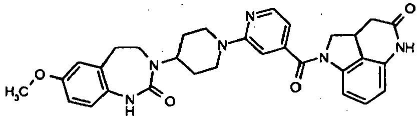

67 mg (0.17 mmol) 4-(7-Methoxy-2-oxo-1,2,4,5-tetrahydro-benzo[d][1,3]diazepin-3-yl)-3,4,5,6-tetrahydro-2H-[1,2']bipyridinyl-4'-carbonsäure und 30 mg (0.17 mmol) 1,2,2a,5-Tetrahydro-3H-pyrrolo[4,3,2-de]chinolin-4-on in 56 µL (0.40 mmol) TEA und 1.8 mL DMF wurden mit 58 mg (0.18 mmol) TBTU versetzt und über Nacht bei RT gerührt. Anschließend wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 44 mg (47% d.Th.) |
| ESI-MS: | m/z = 553 (M+H)⁺ |
| R, (HPLC-MS): | 3.00 min (Methode E) |

### Beispiel 115:

### 3-[6'-Chlor-4'-(5-fluor-2,3-dihydro-indol-1-carbonyl)-3,4,5,6-tetrahydro-2H-[1,2']bipyridinyl-4-yl]-7-methoxy-1,3,4,5-tetrahydro-benzo[d][1,3]diazepin-2-on

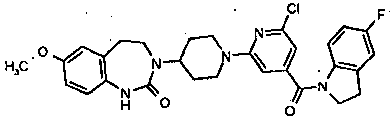

1.0 g (3.2 mmol) 7-Methoxy-3-piperidin-4-yl-1,3,4,5-tetrahydro-1,3-benzodiazepin-2-on, 0.94 g (3.4 mmol) (2,6-Dichlor-pyridin-4-yl)-(5-fluor-2,3-dihydro-indol-1-yl)-methanon und 0.65 mL (3.8 mmol) DIPEA in 10 mL DMF wurden 2 h bei RT gerührt. Anschließend wurde der Reaktionsansatz eingeengt, der Rückstand mit Wasser versetzt und 30 min bei RT nachgerührt. Der ausgefallene Feststoff wurde abgesaugt, mit Diisopropylether und Isopropanol verrührt und nochmals abgesaugt. Nach dem Trocknen wurde das Rohprodukt mittels präparativer HPLC-MS gereinigt. Die Produkt enthaltenden Fraktionen wurden vereinigt und gefriergetrocknet.

| | |
|---|---|
| Ausbeute: | 750 mg (42% d.Th.) |
| ESI-MS: | m/z = 550 (M+H)⁺ |
| Rₜ (HPLC-MS): | 1.96 min (Methode O) |

### Vergleichsbeispiel:

### 3-{1-[6-(5-Hydroxymethyl-4,7-dihydro-5H-thieno[2,3-c]pyridin-6-carbonyl)-pyrimidin-4-yl]-piperidin-4-yl}-7-methoxy-1,3,4,5-tetrahydro-benzo[d][1,3]diazepin-2-on

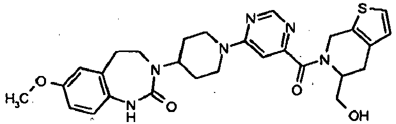

100 mg (0.25 mmol) 4-(7-Methoxy-2-oxo-1,2,4,5-tetrahydro-benzo[d][1,3]diazepin-3-yl)-3,4,5,6-tetrahydro-2H-[1,2']bipyridinyl-4'-carbonsäure und 50 mg (0.28 mmol) (4,5,6,7-Tetrahydro-thieno[2,3-c]pyridin-5-yl)-methanol in 50 µL (0.36 mmol) TEA und 0.90 mL DMF wurden mit 85 mg (0.27 mmol) TBTU versetzt und 5 h bei RT gerührt. Anschließend wurde der Reaktionsansatz mittels präparativer HPLC-MS gereinigt. Die das Produkt enthaltenden Fraktionen wurden vereinigt und auf ca. Die Hälfte eingeengt. Der ausgefallene Niederschlag wurde abgesaugt und getrocknet.

| | |
|---|---|
| Ausbeute: | 40 mg (29% d.Th.) |
| ESI-MS: | m/z = 549 (M+H)⁺ |
| Rₜ (HPLC-MS): | 2.89 min (Methode E) |

### Beispiel 116:

### 4-(5-Fluor-2,3-dihydro-indol-1-carbonyl)-2-[4-(2-oxo-2,3-dihydro-imidazo[4,5-b]pyridin-1-yl)-piperidin-1-yl]-benzonitril

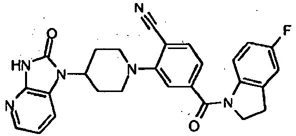

70 mg (0.19 mmol) 4-Cyano-3-[4-(2-oxo-2,3-dihydro-imidazo[4,5-b]pyridin-1-yl)-piperidin-1-yl]-benzoesäure und 30 mg (0.21 mmol) 5-Fluor-2,3-Dihydro-(1H)-indol in 0.10 mL (0.72 mmol) TEA und 1.5 mL DMF wurden mit 65 mg (0.20 mmol) TBTU versetzt und 1 h bei RT gerührt. Anschließend wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 4 mg (4% d.Th.) |
| ESI-MS: | m/z = 483 (M+H)⁺ |
| Rₜ (HPLC-MS): | 1.64 min (Methode C) |

### Vergleichsbeispiel:

### 1-{1-[6-(3-Hydroxymethyl-2,3-dihydro-indol-1-carbonyl)-pyrimidin-4-yl]-piperidin-4-yl}-1,3-dihydro-imidazo[4,5-b]pyridin-2-on

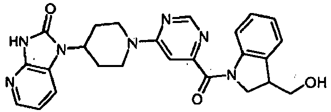

85 mg (0.25 mmol) 6-[4-(2-Oxo-2,3-dihydroimidazo[4,5-*b*]pyridin-1-yl)-piperidin-1-yl]-pyrimidin-4-carbonsäure und 50 mg (0.32 mmol) (2,3-Dihydro-1H-indol-3-yl)-methanol in 50 μL (0.36 mmol) TEA und 0.90 mL DMF wurden mit 85 mg (0.27 mmol) TBTU versetzt und 5 h bei RT gerührt. Anschließend wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 57 mg (46% d.Th.) |
| ESI-MS: | m/z = 472 (M+H)⁺ |
| Rₜ (HPLC-MS): | 1.11 min (Methode C) |

### Vergleichsbeispiel:

### 6-[4-(7-Methoxy-2-oxo-1,2,4,5-tetrahydro-benzo[d][1,3]diazepin-3-yl)-piperidin-1-yl]-pyrimidin-4-carbonsäure(1H-indazol-4-yl)-amid

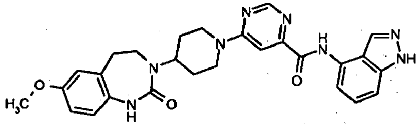

100 mg (0.25 mmol) 4-(7-Methoxy-2-oxo-1,2,4,5-tetrahydro-benzo[d][1,3]diazepin-3-yl)-3,4,5,6-tetrahydro-2H-[1,2']bipyridinyl-4'-carbonsäure und 34 mg (0.25 mmol) 4-Amino-indazol in 74 mμL (0.53 mmol) TEA und 1.5 mL DMF wurden mit 89 mg (0.28 mmol) TBTU versetzt und über Nacht bei RT gerührt. Anschließend wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 24 mg (19% d.Th.) |
| ESI-MS: | m/z = 513 (M+H)⁺ |
| Rₜ (HPLC-MS): | 1.35 min (Methode C) |

### Beispiel 117:

### 3-{1-[3-(5-Fluor-2,3-dihydro-indol-1-carbonyl)-phenyl]-piperidin-4-yl}-7-methoxy-1,3,4,5-tetrahydro-benzo[d][1,3]diazepin-2-on

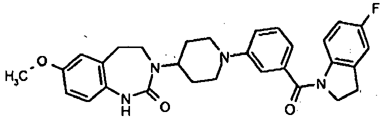

Unter einer Argonatmosphäre wurden zu 86 mg (0.31 mmol) 7-Methoxy-3-piperidin-4-yl-1,3,4,5-tetrahydro-1,3-benzodiazepin-2-on, 0.10 g (0.31 mmol) (3-Brom-phenyl)-(5-fluor-2,3-dihydro-indol-1-yl)-methanon und 0.16 g (0.48 mmol) Cäsiumcärbonat in 4.0 mL Dioxan, 7 mg (31 μmol) Palladium(II)acetat und 20 mg (32 μmol) BINAP hinzugefügt und über Nacht bei 120°C gerührt. Der Reaktionsansatz wurde anschließend eingeengt und der Rückstand in DMF/MeOH gelöst und mittels präparativer HPLC-MS gereinigt. Die Produkt enthaltenden Fraktionen wurden vereinigt und gefriergetrocknet.

| | |
|---|---|
| Ausbeute: | 10 mg (5% d.Th.) |
| ESI-MS: | m/z = 515 (M+H)⁺ |
| Rₜ (HPLC-MS): | 1.69 min (Methode C) |

### Vergleichsbeispiel:

### 7-Methoxy-3-{1-[6-(4,5,7,8-tetrahydro-thiazolo[4,5-d]azepin-6-carbonyl)-pyrimidin-4-yl]-piperidin-4-yl}-1,3,4,5-tetrahydro-benzo[d][1,3]diazepin-2-on

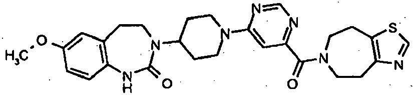

83 mg (0.21 mmol) 4-(7-Methoxy-2-oxo-1,2,4,5-tetrahydro-benzo[d][1,3]diazepin-3-yl)-3,4,5,6-tetrahydro-2H-[1,2']bipyridinyl-4'-carbonsäure und 37 mg (0.24 mmol) 5,6,7,8-Tetrahydro-4H-thiazolo[4,5-d]azepin in 0.13 mL (0.72 mmol) DIPEA und 1.0 mL DMF wurden mit 77 mg (0.24 mmol) TBTU versetzt und über Nacht bei RT gerührt. Anschließend wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 14 mg (12% d.Th.) |
| ESI-MS: | m/z = 534 (M+H)⁺ |
| Rₜ (HPLC-MS): | 1.30 min (Methode C) |

### Vergleichsbeispiel:

### 1-{6-[4-(2-Oxo-1,2,4,5-tetrahydro-benzo[d][1,3]diazepin-3-yl)-piperidin-1-yl]-pyrimidin-4-carbonyl}-1,2,2a,5-tetrahydro-3H-pyrrolo[4,3,2-de]chinolin-4-on

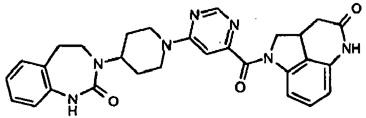

63 mg (0.17 mmol) 6-[4-(2-Oxo-1,2,4,5-tetrahydro-1,3-benzodiazepin-3-yl)-piperidin-1-yl]-pyrimidin-4-carbonsäure und 30 mg (0.17 mmol) 1,2,2a,5-Tetrahydro-3H-pyrrolo-[4,3,2-de]chinolin-4-on in 56 μL (0.40 mmol) TEA und 1.8 mL DMF wurden mit 58 mg (0.18 mmol) TBTU versetzt und über Nacht bei RT gerührt. Anschließend wurde der Reaktionsansatz mittels präparativer HPLC-MS gereinigt. Die das Produkt enthaltenden Fraktionen wurden vereinigt und gefriergetrocknet.

| | |
|---|---|
| Ausbeute: | 60 mg (67% d.Th.) |
| ESI-MS: | m/z = 524 (M+H)⁺ |
| Rₜ (HPLC-MS): | 3.20 min (Methode E) |

### Vergleichsbeispiel:

### 7-Methoxy-3-(1-(6-(1'-methyl-2',3'-dihydro-1'H-spiro[cyclopentan-1,4'-isochinolin]-2'-yl-carbonyl)-pyrimidin-4-yl)piperidin-4-yl)-4,5-dihydro-1H-benzo[d][1,3]diazepin-2(3H)-on

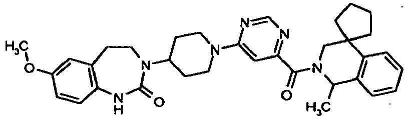

42 mg (0.11 mmol) 6-[4-(7-Methoxy-2-oxo-1,2,4,5-tetrahydro-1,3-benzodiazepin-3-yl)-piperidin-1-yl]-pyrimidin-4-carbonsäure und 25 mg (0.11 mmol) 1'-Methyl-2',3'-dihydro-1'H-spiro[cyclopentan-1,4'-isochinolin Hydrochlorid in 63 μL (0.36 mmol) DIPEA und 1.0 mL DMF wurden mit 39 mg (0.12 mmol) TBTU versetzt und über Nacht bei RT gerührt. Anschließend wurde der Reaktionsansatz mittels präparativer HPLC-MS gereinigt. Die Produkt enthaltenden Fraktionen wurden vereinigt und teilweise eingeengt. Der entstandene Niederschlag wurde abgesaugt und getrocknet. Das Filtrat wurde gefriergetrocknet. Der zurückgebliebene Rückstand war identisch mit dem Niederschlag.

| | |
|---|---|
| Ausbeute: | 33 mg (54% d.Th.) |
| ESI-MS: | m/z = 581 (M+H)⁺ |
| Rₜ (HPLC-MS): | 1.74 min (Methode C) |

### Beispiel 118:

### 3-{1-[6-(2-Hydroxymethyl-2,3-dihydro-indol-1-carbonyl)-pyrimidin-4-yl]-piperidin-4-yl}-7-methoxy-1,3,4,5-tetrahydro-benzo[d][1,3]diazepin-2-on

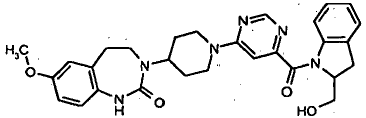

100 mg (0.25 mmol) 6-[4-(7-Methoxy-2-oxo-1,2,4,5-tetrahydro-1,3-benzodiazepin-3-yl)-piperidin-1-yl]-pyrimidin-4-carbonsäure und 45 mg (0.30 mmol) (2,3-Dihydro-1H-indol-2-yl)-methanol in 50 µL (0.36 mmol) TEA und 0.90 mL DMF wurden mit 85 mg (0.27 mmol) TBTU versetzt und über Nacht bei RT gerührt. Anschließend wurde der Reaktionsansatz mittels präparativer HPLC-MS gereinigt. Die Produkt enthaltenden Fraktionen wurden vereinigt und teilweise eingeengt. Der entstandene Niederschlag wurde abgesaugt und getrocknet.

| | |
|---|---|
| Ausbeute: | 45 mg (30% d.Th.) |
| ESI-MS: | m/z = 529 (M+H)⁺ |
| Rₜ (HPLC-MS): | 2.97 min (Methode E) |

### Vergleichsbeispiel:

### 3-{1-[6-(3,4-Dihydro-1H-pyrrolo[1,2-a]pyrazin-2-carbonyl)-pyrimidin-4-yl]-piperidin-4-yl}-7-methoxy-1,3,4,5-tetrahydro-benzo[d][1,3]diazepin-2-on

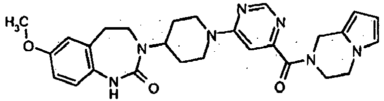

83 mg (0.21 mmol) 6-[4-(7-Methoxy-2-oxo-1,2,4,5-tetrahydro-1,3-benzodiazepin-3-yl)-piperidin-1-yl]-pyrimidin-4-carbonsäure und 37 mg (0.24 mmol) 1,2,3,4-Tetrahydropyrrolo[1,2-a]pyrazin in 0.10 mL (0.72 mmol) TEA und 2.0 mL DMF wurden mit 77 mg (0.24 mmol) TBTU versetzt und über Nacht bei RT gerührt. Dann wurde der Reaktionsansatz mittels präparativer HPLC-MS gereinigt. Die Produkt enthaltenden Fraktionen wurden vereinigt, das Acetonitril wurde eingeengt und der Rückstand getrocknet.

| | |
|---|---|
| Ausbeute: | 43 mg (41% d.Th.) |
| ESI-MS: | m/z = 502 (M+H)⁺ |
| Rₜ (HPLC-MS): | 1.32 min (Methode C) |

### Beispiel 119:

### 1-{1-[6-(5-Hydroxymethyl-4,7-dihydro-5H-thieno[2,3-c]pyridin-6-carbonyl)-pyrimidin-4-yl]-piperidin-4-yl}-1,3-dihydro-imidazo[4,5-b]pyridin-2-on

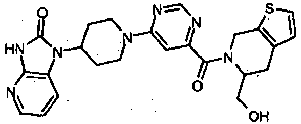

85 mg (0.25 mmol) 6-[4-(2-Oxo-2,3-dihydroimidazo[4,5-*b*]pyridin-1-yl)-piperidin-1-yl]-pyrimidin-4-carbonsäure und 50 mg (0.32 mmol) (4,5,6,7-Tetrahydro-thieno[2,3-c]pyridin-5-yl)-methanol in 50 µL (0.36 mmol) TEA und 0.90 mL DMF wurden mit 80 mg (0.25 mmol) TBTU versetzt und 5 h bei RT gerührt. Anschließend wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 25 mg (20% d.Th.) |
| ESI-MS: | m/z = 492 (M+H)⁺ |
| Rₜ (H PLC-MS): | 1.12 min (Methode C) |

### Vergleichsbeispiel:

### 6-[4-(2-Oxo-2,3-dihydro-imidazo[4,5-b]pyridin-1-yl)-piperidin-1-yl]-pyrimidin-4-carbonsäure(1 H-indazol-4-yl)-amid

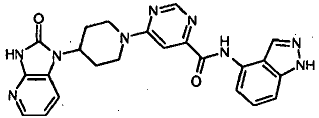

100 mg (0.29 mmol) 6-[4-(2-Oxo-2,3-dihydroimidazo[4,5-*b*]pyridin-1-yl)-piperidin-1-yl]-pyrimidin-4-carbonsäure und 39 mg (0.29 mmol) 1H-Indazol-4-ylamin in 87 mL (0.62 mmol) TEA und 1.5 mL DMF wurden mit 104 mg (0.32 mmol) TBTU versetzt und über Nacht bei RT gerührt. Anschließend wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 6 mg (5% d.Th.) |
| ESI-MS: | m/z = 456 (M+H)⁺ |
| Rₜ (HPLC-MS): | 1.10 min (Methode C) |

### Beispiel 120:

### 1-{1-[6-(2-Hydroxymethyl-2,3-dihydro-indol-1-carbonyl)-pyrimidin-4-yl]-piperidin-4-yl}-1,3-dihydro-imidazo[4,5-b]pyridin-2-on

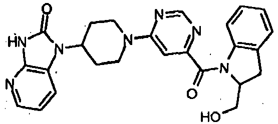

85 mg (0.25 mmol) 6-[4-(2-Oxo-2,3-dihydroimidazo[4,5-*b*]pyridin-1-yl)-piperidin-1-yl]-pyrimidin-4-carbonsäure und 45 mg (0.30 mmol) (2,3-Dihydro-1H-indol-2-yl)-methanol in 50 μL (0.36 mmol) TEA und 0.90 mL DMF wurden mit 85 mg (0.27 mmol) TBTU versetzt und über Nacht bei RT gerührt. Anschließend wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 36 mg (29% d.Th.) |
| ESI-MS: | m/z = 472 (M+H)⁺ |
| Rₜ (HPLC-MS): | 2.44 min (Methode E) |

### Vergleichsbeispiel:

### 3-(1-{6-[2-(3,5-Difluor-phenyl)-5,5-dimethyl-piperidin-1-carbonyl]-pyrimidin-4-yl}-piperidin-4-yl)-7-methoxy-1,3,4,5-tetrahydro-benzo[d][1,3]diazepin-2-on

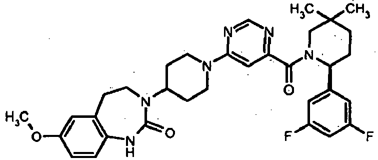

65 mg (0.16 mmol) 6-[4-(7-Methoxy-2-oxo-1,2,4,5-tetrahydro-1,3-benzodiazepin-3-yl)-piperidin-1-yl]-pyrimidin-4-carbonsäure und 70 mg (0.16 mmol) (S)-2-(3,5-difluorphenyl)-5,5-dimethylpiperidin in 0.04 mL (0.72 mmol) DIPEA und 0.80 mL DMF wurden mit 65 mg (0.17 mmol) HATU versetzt und über Nacht bei RT gerührt. Dann wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 40 mg (43% d.Th.) |
| ESI-MS: | m/z = 605 (M+H)⁺ |

### Vergleichsbeispiel:

### 3-(1-(6-(1,4,5,6,7,8-Hexahydropyrazolo[4,3-d]azepin-6-carbonyl)pyrimidin-4-yl)piperidin-4-yl)-7-methoxy-4,5-dihydro-1H-benzo[d][1,3]diazepin-2(3H)-on

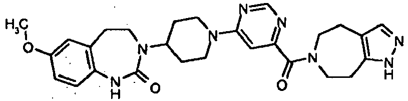

65 mg (0.16 mmol) 6-[4-(7-Methoxy-2-oxo-1,2,4,5-tetrahydro-1,3-benzodiazepin-3-yl)-piperidin-1-yl]-pyrimidin-4-carbonsäure und 35 mg (0.20 mmol) 1,4,5,6,7,8-Hexahydropyrazolo[4,3-d]azepin Hydrochlorid in 80 μL (0.57 mmol) TEA und 0.80 mL DMF wurden mit 68 mg (0.18 mmol) HATU versetzt und über Nacht bei RT gerührt. Anschließend wurde der Reaktionsansatz mittels präparative HPLC-MS zwei Mal gereinigt. Die Produkt enthaltenden Fraktionen wurden vereinigt und gefriergetrocknet.

| | |
|---|---|
| Ausbeute: | 21 mg (25% d.Th.) |
| ESI-MS: | m/z = 517 (M+H)⁺ |
| Rₜ (HPLC-MS): | 3.29 min (Methode B) |

### Vergleichsbeispiel:

### 7-Methoxy-3-{1-[6-(6-methoxy-2,3-dihydro-indol-1-carbonyl)-pyrimidin-4-yl]-piperidin-4-yl}-1,3,4,5-tetrahydro-benzo[d][1,3]diazepin-2-on

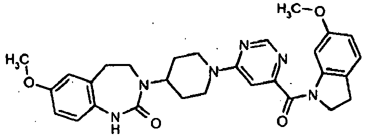

80 mg (0.20 mmol) 6-[4-(7-Methoxy-2-oxo-1,2,4,5-tetrahydro-1,3-benzodiazepin-3-yl)-piperidin-1-yl]-pyrimidin-4-carbonsäure und 30 mg (0.20 mmol) 6-Methoxy-2,3-dihydro-1 H-indol in 50 μL (0.36 mmol) TEA und 1.5 mL DMF wurden mit 70 mg (0.22 mmol) TBTU versetzt und 1 h bei RT gerührt. Anschließend wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 35 mg (33% d.Th.) |
| ESI-MS: | m/z = 529 (M+H)⁺ |
| Rₜ (HPLC-MS): | 1.55 min (Methode C) |

### Vergleichsbeispiel:

### 6-[4-(2-Oxo-2,3-dihydro-imidazo[4,5-b]pyridin-1-yl}-piperidin-1-yl]-pyrimidin-4-carbon-säure(1H-indazol-5-yl)-amid

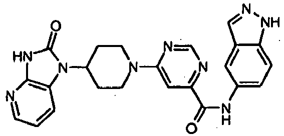

100 mg (0.29 mmol) 6-[4-(2-Oxo-2,3-dihydroimidazo[4,5-*b*]pyridin-1-yl)-piperidin-1-yl]-pyrimidin-4-carbonsäure und 39 mg (0.29 mmol) 5-Aminobenzopyrrazol in 87 µL (0.62 mmol) TEA und 1.5 mL DMF wurden mit 104 mg (0.32 mmol) TBTU versetzt und über Nacht bei RT gerührt. Anschließend wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 45 mg (34% d.Th.) |
| ESI-MS: | m/z = 456 (M+H)⁺ |
| Rₜ (HPLC-MS): | 1.18 min (Methode ?) |

### Vergleichsbeispiel:

### 3-{1-[6-(2-Ethyl-2,3-dihydro-indol-1-carbonyl)-pyrimidin-4-yl]-piperidin-4-yl}-7-methoxy-1,3,4,5-tetrahydro-benzo[d][1,3]diazepin-2-on

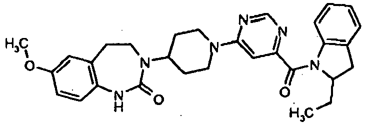

110 mg (0.40 mmol) 7-Methoxy-3-piperidin-4-yl-1,3,4,5-tetrahydro-1,3-benzodiazepin-2-on, 103 mg (0.36 mmol) (6-Chlor-pyrimidin-4-yl)-(2-ethyl-2,3-dihydro-indol-1-yl)-methanon und 0.14 mL (0.80 mmol) DIPEA in 3.0 mL DMF wurden über Nacht bei RT gerührt. Anschließend wurde der Reaktionsansatz mittels präparativer HPLC-MS gereinigt. Die Produkt enthaltenden Fraktionen wurden vereinigt, das organische Lösungsmittel wurde eingeengt und der Rückstand wurde mit 1 N wässriger Natronlauge neutralisiert. Der entstandene Niederschlag wurde abgesaugt, mit Wasser nachgewaschen und im HV getrocknet.

| | |
|---|---|
| Ausbeute: | 110 mg (52% d.Th.) |
| ESI-MS: | m/z = 527 (M+H)⁺ |
| Rₜ (H PLC-MS): | 1.62 min (Methode C) |

### Vergleichsbeispiel:

### 6-[4-(7-Methoxy-2-oxo-1,2,4,5-tetrahydro-benzo[d][1,3]diazepin-3-yl)-piperidin-1-yl]-pyrimidin-4-carbonsäure(7-methyl-1H-indazol-5-yl)-amid

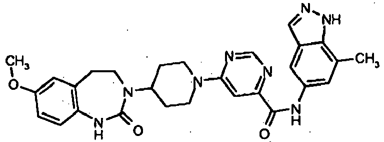

100 mg (0.25 mmol) 6-[4-(7-Methoxy-2-oxo-1,2,4,5-tetrahydro-1,3-benzo,diazepin-3-yl)-piperidin-1-yl]-pyrimidin-4-carbönsäure und 37 mg (0.25 mmol) 7-Methyl-1H-indazol-5-amin in 74 µL (0.53 mmol) TEA und 1.5 mL DMF wurden mit 89 mg (0.28 mmol) TBTU versetzt und über Nacht bei RT gerührt. Dann wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 7 mg (5% d.Th.) |
| ESI-MS: | m/z = 527 (M+H)⁺ |
| Rₜ (HPLC-MS): | 2.07 min (Methode S) |

### Vergleichsbeispiel:

### 7-Methoxy-3-{1-[6-(5-oxo-octahydro-pyrrolo[3,2-b]pyridin-1-carbonyl)-pyrimidin-4-yl]-piperidin-4-yl}-1,3,4,5-tetrahydro-benzo[d][1,3]diazepin-2-on

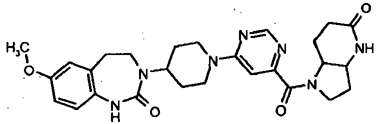

0.17 g (0.28 mmol) 3-{1-[6-(5-Benzyloxy-pyrrolo[3,2-b]pyridin-1-carbonyl)-pyrimidin-4-yl]-piperidin-4-yl}-7-methoxy-1,3,4,5-tetrahydro-benzo[d][1,3]diazepin-2-on und 50 mg Palladium (Pd/C 5%) in 20 ml EtOH wurden mehrere Stunden bei RT in einer Wasserstoffatmosphäre hydriert. Da keine Umsetzung stattfand wurden zusätzlich noch 10 ml THF zugegeben und bei 50°C weiter hydriert. Anschließend wurde der Katalysator abgesaugt und das Filtrat eingeengt. Der Rückstand wurde mittels präparativer HPLC-MS gereinigt. Die Produkt enthaltenden Fraktionen wurden vereinigt und gefriergetrocknet.

| | |
|---|---|
| Ausbeute: | 16 mg (11% d.Th.) |
| ESI-MS: | m/z = 520 (M+H)⁺ |
| Rₜ (H PLC-MS): | 2.17 min (Methode E) |

Die nachfolgenden Beispiele beschreiben die Herstellung pharmazeutischer Anwendungsformen, die als Wirkstoff eine beliebige Verbindung der allgemeinen Formel I enthalten:

### Beispiel I

Kapseln zur Pulverinhalation mit 1 mg Wirkstoff

### Zusammensetzung:

| 1 Kapsel zur Pulverinhalation enthält: | |
|---|---|
| Wirkstoff | 1.0 mg |
| Milchzucker | 20.0 mg |
| Hartgelatinekapseln | 50.0 mg |
| | 71.0 mg |

### Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

### Beispiel II

Inhalationslösung für Respimat^{®} mit 1 mg Wirkstoff

### Zusammensetzung:

| 1 Hub enthält: | |
|---|---|
| Wirkstoff | 1.0 mg |
| Benzalkoniumchlorid | 0.002 mg |
| Dinatriumedetat | 0.0075 mg |
| Wasser gereinigt ad | 15.0 µl |

### Herstellungsverfahren:

Der Wirkstoff und Benzalkoniumchlorid werden in Wasser gelöst und in Respimat^{®}-Kartuschen abgefüllt.

### Beispiel III

Inhalationslösung für Vernebler mit 1 mg Wirkstoff

### Zusammensetzung:

| 1 Fläschchen enthält: | |
|---|---|
| Wirkstoff | 0.1 g |
| Natriumchlorid | 0.18 g |
| Benzalkoniumchlorid | 0.002 g |
| Wasser gereinigt ad | 20.0 ml |

### Herstellungsverfahren:

Wirkstoff, Natriumchlorid und Benzalkoniumchlorid werden in Wasser gelöst.

### Beispiel IV

Treibgas-Dosieraerosol mit 1 mg Wirkstoff

### Zusammensetzung:

| 1 Hub enthält: | |
|---|---|
| Wirkstoff | 1.0 mg |
| Lecithin | 0.1 % |
| Treibgas ad | 50.0 µl |

### Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

### Beispiel V

Nasalspray mit 1 mg Wirkstoff

| | |
|---|---|
| Zusammensetzung: | |
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

### Herstellungsverfahren:

Der Wirkstoff und die Hilfsstoffe werden in Wasser gelöst und in ein entsprechendes Behältnis abgefüllt.

### Beispiel VI

Injektionslösung mit 5 mg Wirksubstanz pro 5 ml

### Zusammensetzung:

| | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

### Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

### Beispiel VII

Injektionslösung mit 100 mg Wirksubstanz pro 20 ml

### Zusammensetzung:

| | |
|---|---|
| Wirksubstanz | 100 mg |
| Monokaliumdihydrogenphosphat = KH₂PO₄ | 12 mg |
| Dinatriumhydrogehphosphat = Na₂HPOₐ*2H₂O | 2 mg |
| Natriumchlorid | 180 kg |
| Human-Serum-Albumin | 50 mg |
| Polysorbat 80 | 20 mg |
| Wasser für Injektionszwecke ad | 10 ml |

### Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen.

### Beispiel VIII

Lyophilisat mit 10 mg Wirksubstanz

### Zusammensetzung:

| | |
|---|---|
| Wirksubstanz | 10 mg |
| Mannit | 300 mg |
| Human-Serum-Albumin | 20 mg |
| Wasser für Injektionszwecke ad | 2 ml |

### Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.

### Lösungsmittel für Lyophilisat:

| | |
|---|---|
| Polysorbat 80 = Tween 80 | 20 mg |
| Mannit | 200 mg |
| Wasser für Injektionszwecke ad | 10 ml |

### Herstellung:

Polysorbat 80 und Mannit in Wasser für Injektionszwecke (Wfl) auflösen; in Ampullen abfüllen.

### Beispiel IX

Tabletten mit 20 mg Wirksubstanz

### Zusammensetzung:

| | |
|---|---|
| Wirksubstanz | 20 mg |
| Lactose | 120 mg |
| Maisstärke | 40 mg |
| Magnesiumstearat | 2 mg |
| Povidon K 25 | 18 mg |

### Herstellung:

Wirksubstanz, Lactose und Maisstärke homogen mischen; mit einer wässrigen Lösung von Povidon granulieren; mit Magnesiumstearat mischen; auf einer Tablettenpresse abpressen; Tablettengewicht 200 mg.

### Beispiel X

Kapseln mit 20 mg Wirksubstanz

### Zusammensetzung:

| | |
|---|---|
| Wirksubstanz | 20 mg |
| Maisstärke | 80 mg |
| Kieselsäure, hochdispers | 5 mg |
| Magnesiumstearat | 2.5 mg |

### Herstellung:

Wirksubstanz, Maisstärke und Kieselsäure homogen mischen; mit Magnesiumstearat mischen; Mischung auf einer Kapselfüllmaschine in Hartgelatine-Kapseln Größe 3 abfüllen.

### Beispiel XI

Zäpfchen mit 50 mg Wirksubstanz

### Zusammensetzung:

| | |
|---|---|
| Wirksubstanz | 50 mg |
| Hartfett (Adeps solidus) q.s. ad | 1700 mg |

### Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgießen.

### Beispiel XII

Injektionslösung mit 10 mg Wirksubstanz pro 1 mL

### Zusammensetzung:

| | |
|---|---|
| Wirksubstanz | 10 mg |
| Mannitol | 50 mg |
| Human-Serum-Albumin | 10 mg |
| Wasser für Injektionszwecke ad | 1 ml |

### Herstellung:

Mannitol in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

## Patentansprüche

1. Verbindungen der allgemeinen Formel I 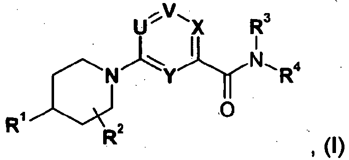 in der
**R¹** eine Gruppe ausgewählt aus 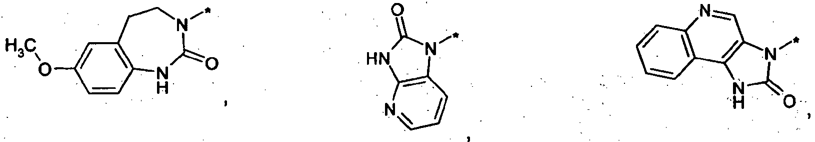 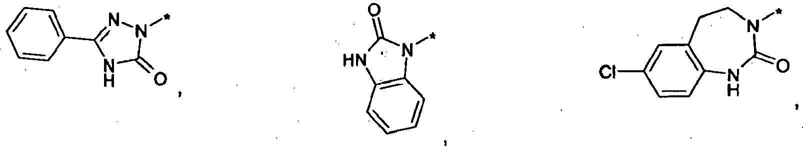 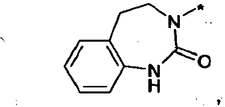
**R³** und **R⁴** zusammen mit dem Stickstoffatom, an das sie gebunden sind, eine Gruppe ausgewählt aus 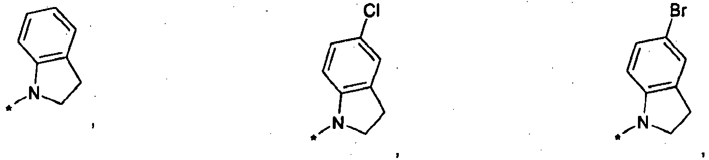 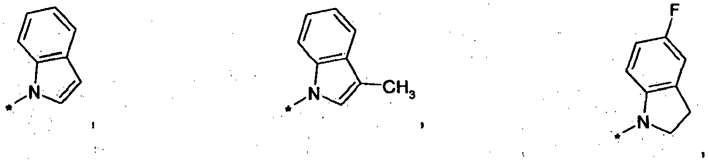 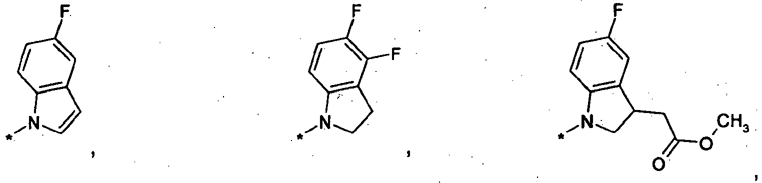 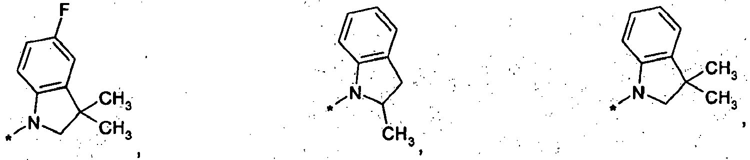 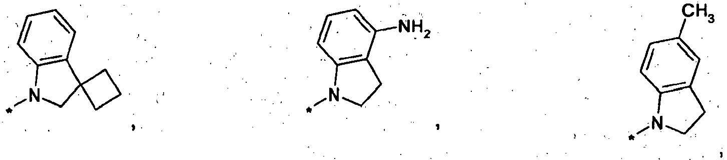 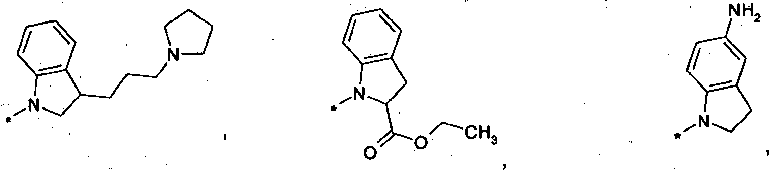 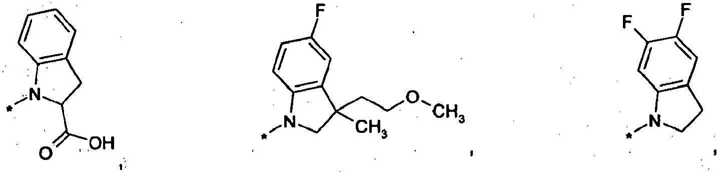 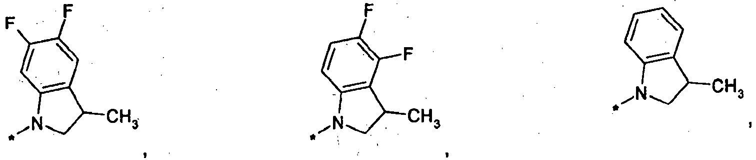 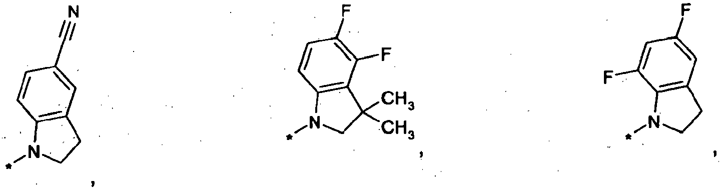 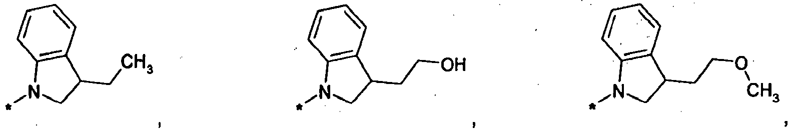 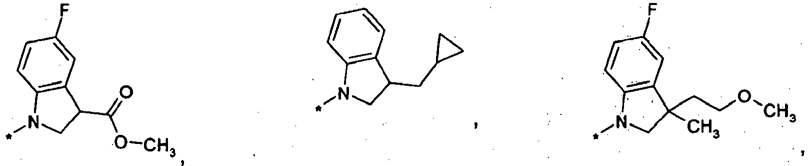 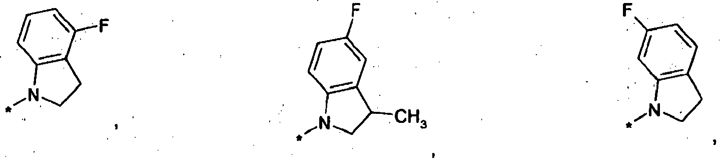 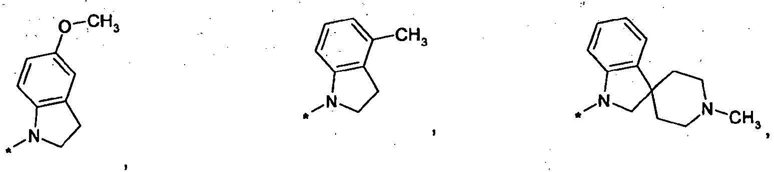 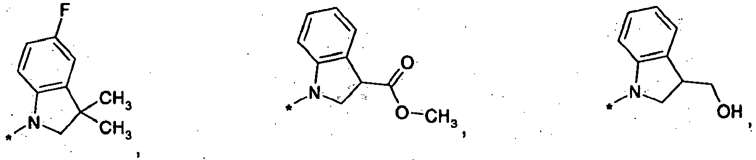 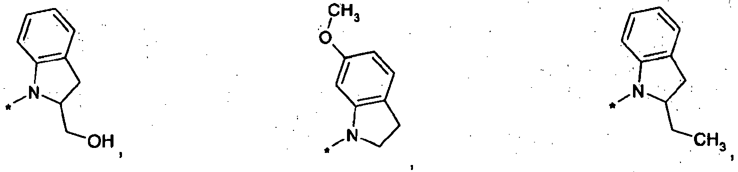 und der Ring 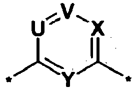 eine Gruppe ausgewählt aus 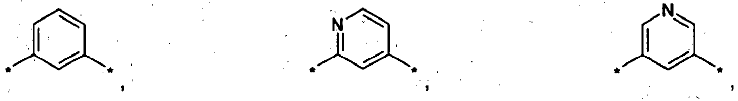 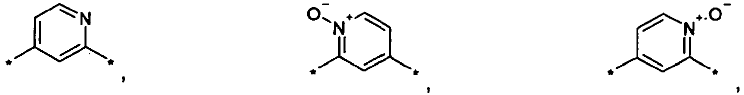 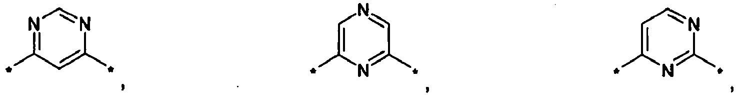 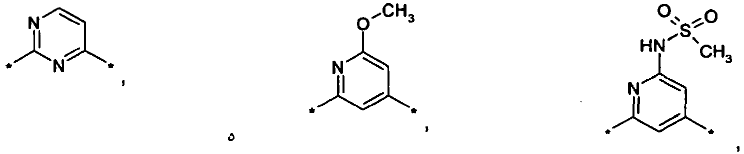 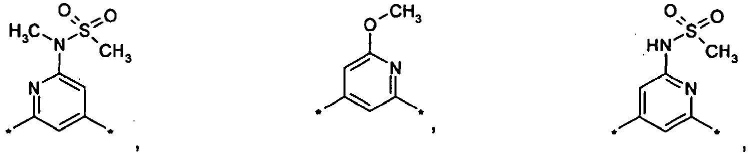 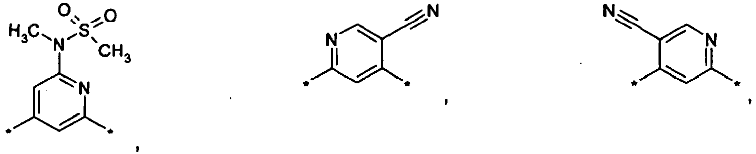 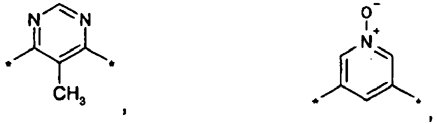 bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

2. Folgende Verbindungen der allgemeinen Formel I gemäß Anspruch 1:
| **Nr.** | **Struktur** |
|---|---|
| (1) | 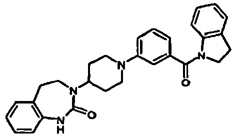 |
| (2) | 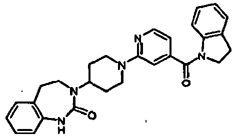 |
| (3) | 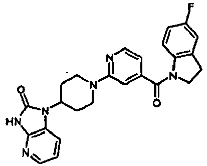 |
| (4) | 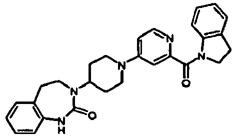 |
| (5) | 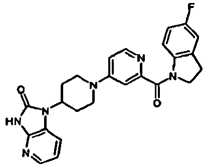 |
| (6) | 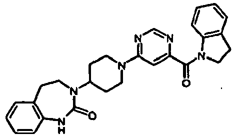 |
| (7) | 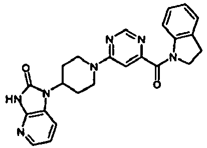 |
| (8) | 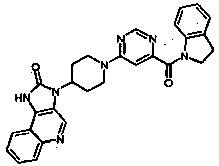 |
| (9) | 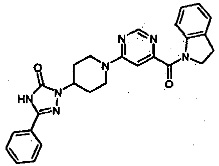 |
| (10) | 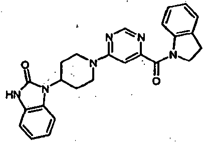 |
| (11) | 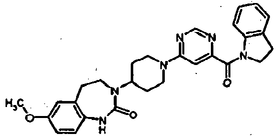 |
| (12) | 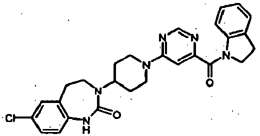 |
| (13) | 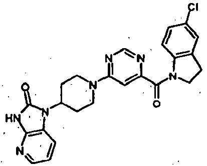 |
| (14) | 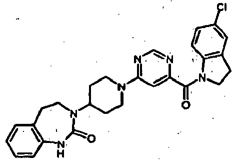 |
| (15) | 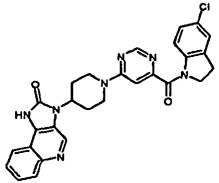 |
| (16) | 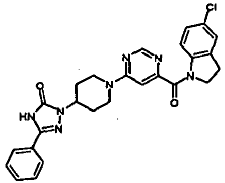 |
| (17) | 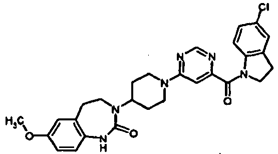 |
| (18) | 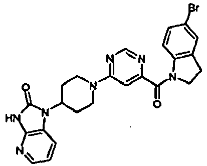 |
| (19) | 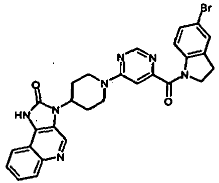 |
| (20) | 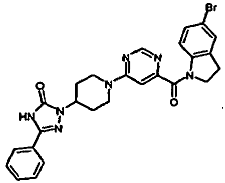 |
| (21) | 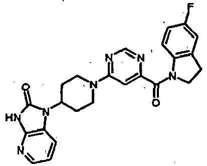 |
| (22) | 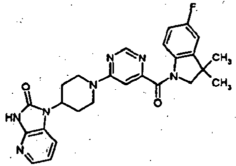 |
| (23) | 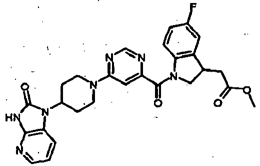 |
| (24) | 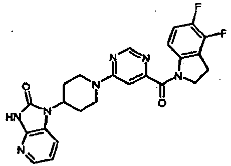 |
| (25) | 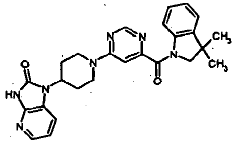 |
| (26) | 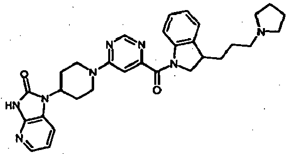 |
| (27) | 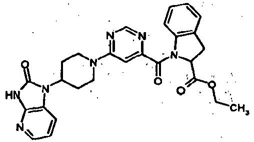 |
| (28) | 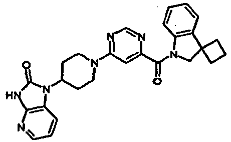 |
| (29) | 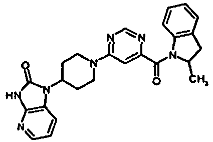 |
| (30) | 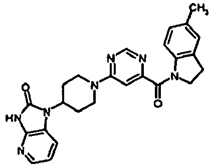 |
| (31) | 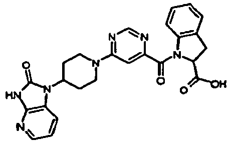 |
| (32) | 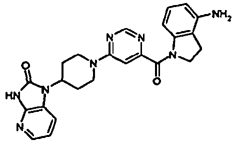 |
| (33) | 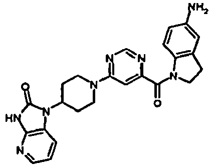 |
| (34) | 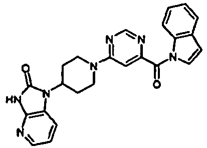 |
| (35) | 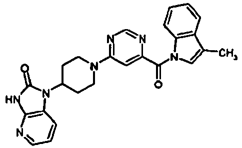 |
| (36) | 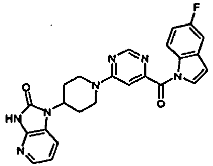 |
| (37) | 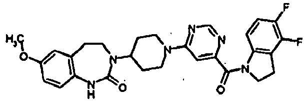 |
| (38) | 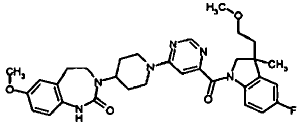 |
| (39) | 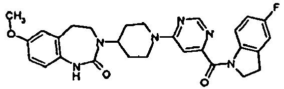 |
| (40) | 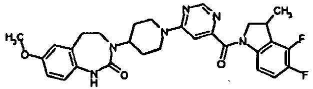 |
| (41) | 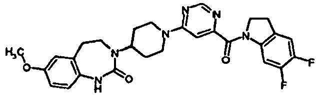 |
| (42) | 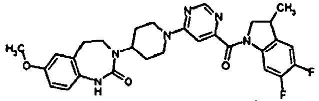 |
| (43) | 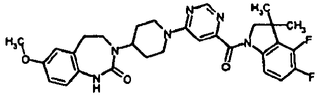 |
| (44) | 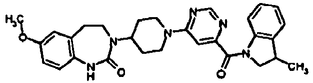 |
| (45) | 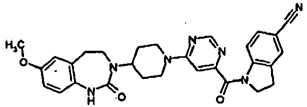 |
| (46) | 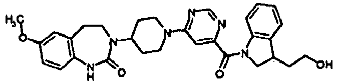 |
| (47) | 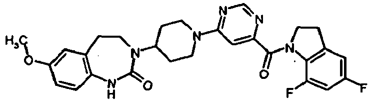 |
| (48) | 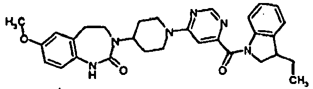 |
| (49) | 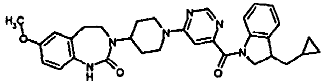 |
| (50) | 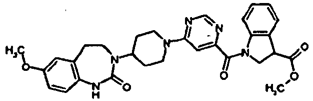 |
| (51) | 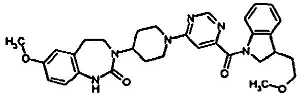 |
| (52) | 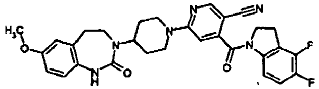 |
| (53) | 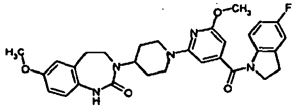 |
| (54) | 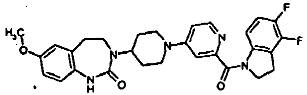 |
| (55) | 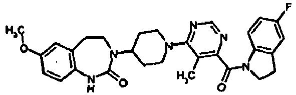 |
| (56) | 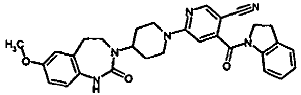 |
| (57) | 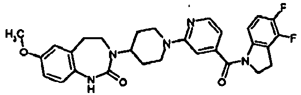 |
| (58) | 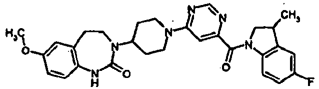 |
| (59) | 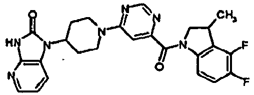 |
| (60) | 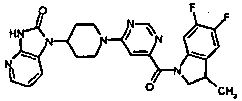 |
| (61) | 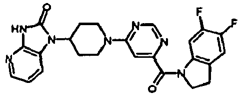 |
| (62) | 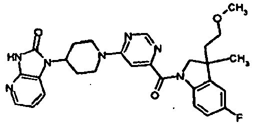 |
| (63) | 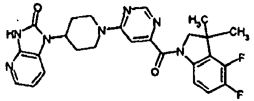 |
| (64) | 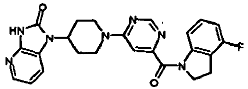 |
| (65) | 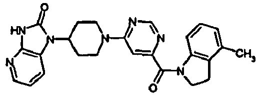 |
| (66) | 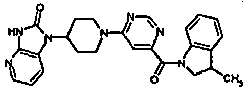 |
| (67) | 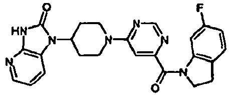 |
| (68) | 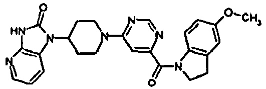 |
| (69) | 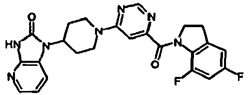 |
| (70) | 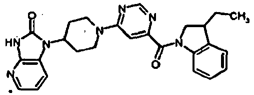 |
| (71) | 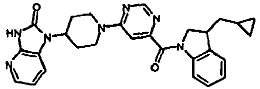 |
| (72) | 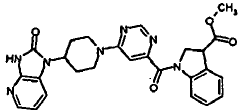 |
| (73) | 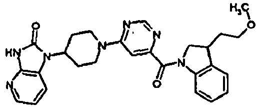 |
| (74) | 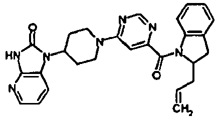 |
| (75) | 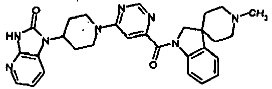 |
| (76) | 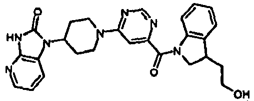 |
| (77) | 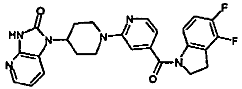 |
| (78) | 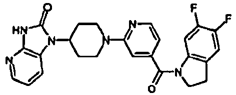 |
| (79) | 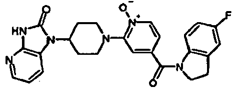 |
| (80) | 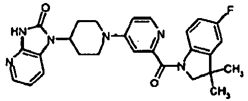 |
| (81) | 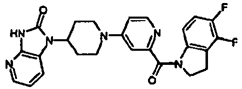 |
| (82) | 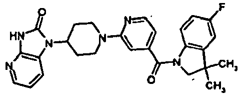 |
| (83) | 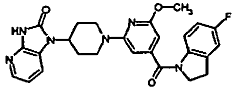 |
| (84) | 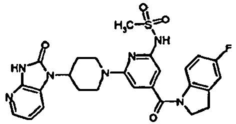 |
| (85) | 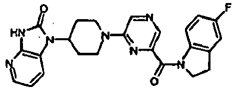 |
| (86) | 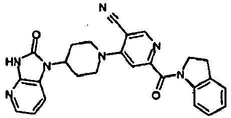 |
| (87) | 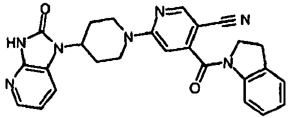 |
| (88) | 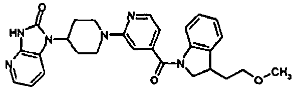 |
| (89) | 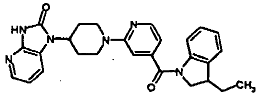 |
| (90) | 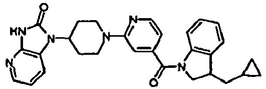 |
| (91) | 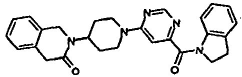 |
| (92) | 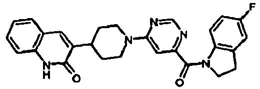 |
| (93) | 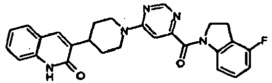 |
| (94) | 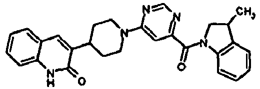 |
| (95) | 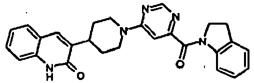 |
| (96) | 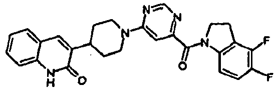 |
| (97) | 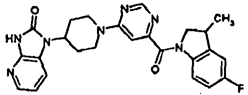 |
| (98) | 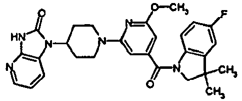 |
| (99) | 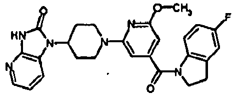 |
| (100) | 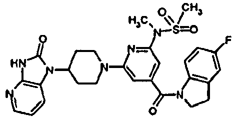 |
| (101) | 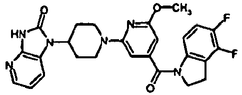 |
| (102) | 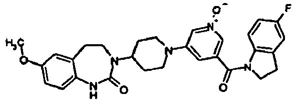 |
| (103) | 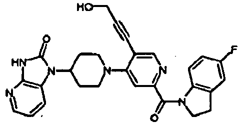 |
| (104) | 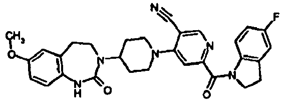 |
| (105) | 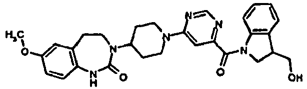 |
| (106) | 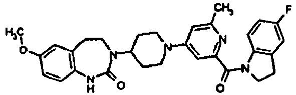 |
| (107) | 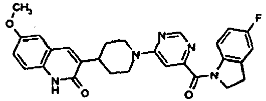 |
| (108) | 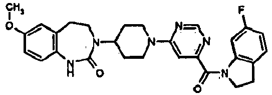 |
| (109) | 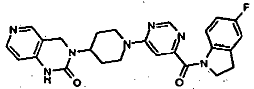 |
| (110) | 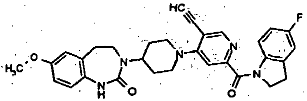 |
| (111) | 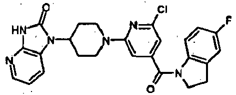 |
| (112) | 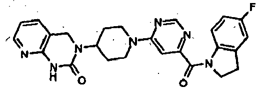 |
| (113) | 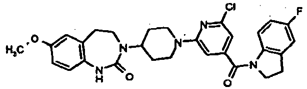 |
| (114) | 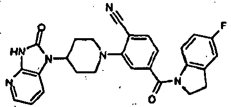 |
| (115) | 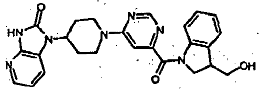 |
| (116) | 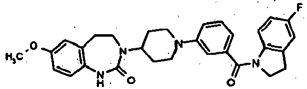 |
| (117) | 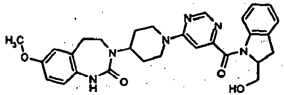 |
| (118) | 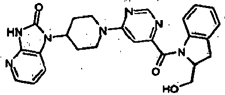 |
| (119) | 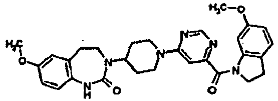 |
| (120) | 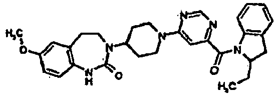 |
deren Enantiomere, deren Diastereomere, deren Hydrate, deren Gemische und deren Salze sowie die Hydrate der Salze, insbesondere deren physiologisch verträgliche Salze mit anorganischen oder organischen Säuren oder Basen.

3. Physiologisch verträgliche Salze der Verbindungen nach einem der Ansprüche 1 bis 2 mit anorganischen oder organischen Säuren oder Basen.

4. Arzneimittel, enthaltend eine Verbindung nach einem der Ansprüche 1 bis 2 oder ein physiologisch verträgliches Salz gemäß Anspruch 3 neben gegebenenfalls einem oder mehreren inerten Trägerstoffen und/oder Verdünnungsmitteln.

5. Verwendung einer Verbindung nach einem der Ansprüche 1 bis 3 zur Herstellung eines Arzneimittels zur akuten und prophylaktischen Behandlung von Kopfschmerzen, insbesondere Migräne- und Cluster-Kopfschmerz sowie Spannungskopfschmerzen.

6. Verwendung einer Verbindung nach einem der Ansprüche 1 bis 3 zur Herstellung eines Arzneimittels zur Behandlung von nicht-insulinabhängigem Diabetes mellitus ("NIDDM"), cardiovaskulärer Erkrankungen, Morphintoleranz, Clostridiumtoxin-bedingter Durchfallerkrankungen, Erkrankungen der Haut, insbesondere thermischer und strahlenbedingter Hautschäden inklusive Sonnenbrand, Lichen, Prurigo, pruriginöse Toxidermien sowie schwerer Juckreiz, entzündlicher Erkrankungen, z.B. entzündlicher Gelenkerkrankungen (Osteoarthritis, rheumatoide Arthritis, neurogene Arthritis), generalisierter Weichteilrheumatismus (Fibromyalgie), neurogener Entzündungen der oralen Mucosa, entzündlicher Lungenerkrankungen, allergischer Rhinitis, Asthma, COPD, Erkrankungen, die mit einer überschießenden Gefäßerweiterung und dadurch bedingter verringerter Gewebedurchblutung einhergehen, z.B. Schock und Sepsis, chronischer Schmerzerkrankungen, wie z.B. diabetische Neuropathien, durch Chemotherapien induzierter Neuropathien, HIV-induzierter Neuropathien, postherpetischer Neuropathien durch Gewebetrauma induzierter Neuropathien, trigeminaler Neuralgien, temporomandibulärer Dysfunktionen, CRPS (complex regional pain syndrome), Rückenschmerzen, und viszeraler Erkrankungen, wie z.B. irritable bowel syndrome (IBS), inflammatory bowel syndrome, zur lindernden Wirkung auf Schmerzzustände im allgemeinen oder zur präventiven oder akut-therapeutischen Behandlung der Symptomatik menopausaler, durch Gefäßerweiterung und erhöhten Blutfluss verursachter Hitzewallungen östrogendefizienter Frauen sowie hormonbehandelter Prostatakarzinompatienten und Kastraten.

7. Verwendung einer Verbindung nach einem der Ansprüche 1 bis 3 zur Herstellung eines Arzneimittels zur akuten und prophylaktischen Behandlung von Migräne- und Cluster-Kopfschmerz, zur Behandlung des irritable bowel syndroms (IBS) und zur präventiven und akut-therapeutischen Behandlung von Hitzewallungen östrogendefizienter Frauen.

8. Verfahren zur Herstellung eines Arzneimittels gemäß Anspruch 4, **dadurch gekennzeichnet, dass** auf nichtchemischem Weg eine Verbindung nach einem der Ansprüche 1 bis 3 in einen oder mehrere inerte Trägerstoffe und/oder Verdünnungsmittel eingearbeitet wird.

## Claims

1. Compounds of general formula I 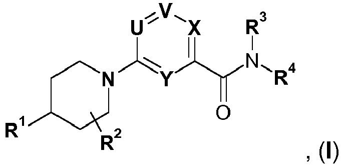 wherein
**R¹** denotes a group selected from 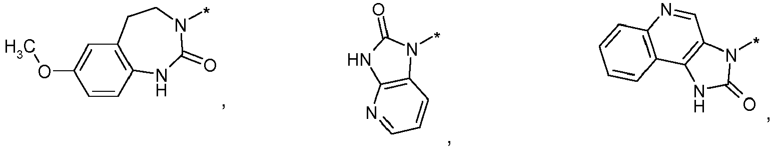 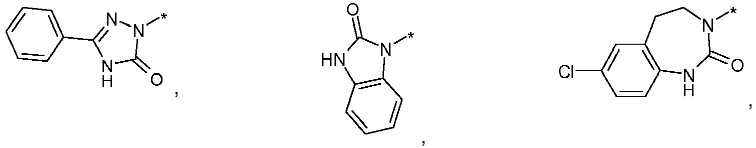 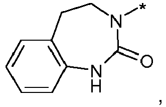
**R³** and **R⁴** together with the nitrogen atom to which they are attached denote a group selected from 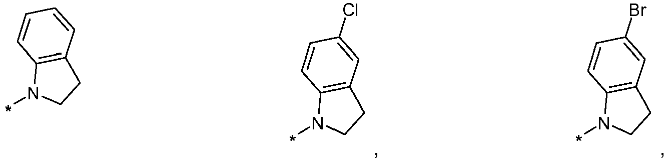 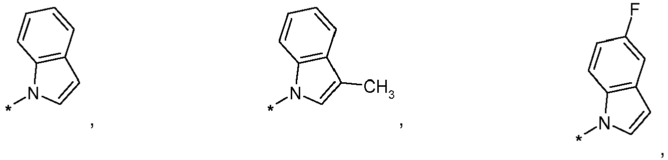 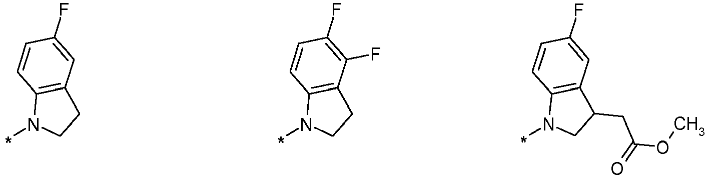 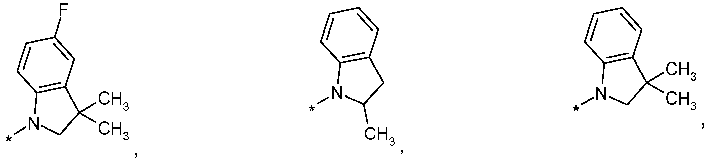 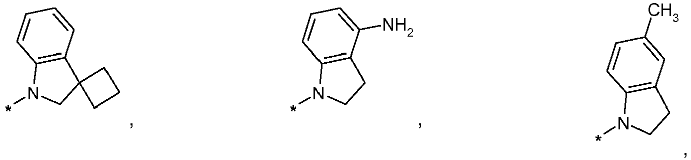 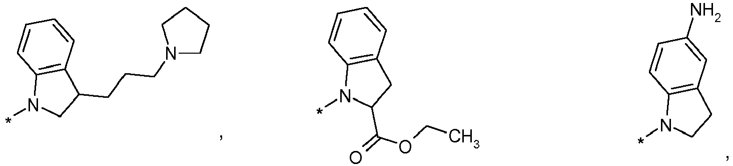 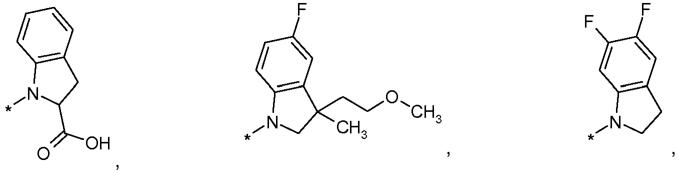 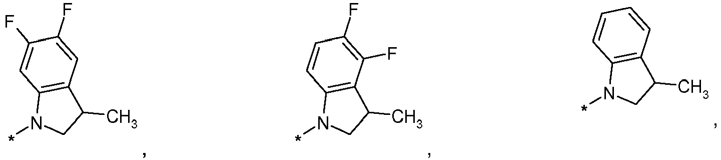 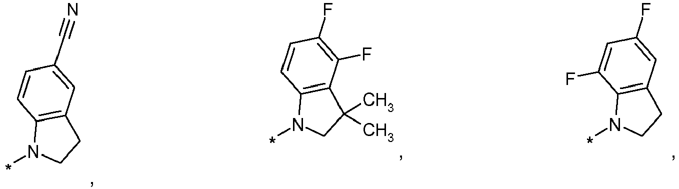 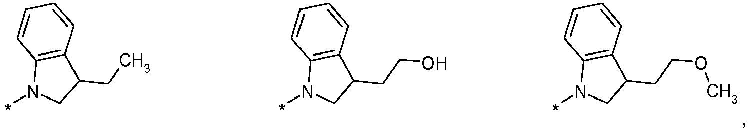 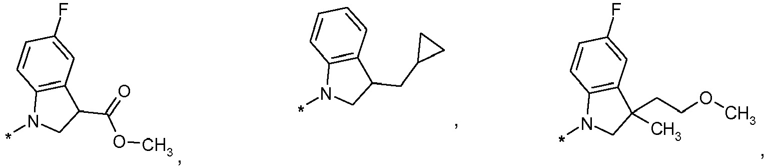 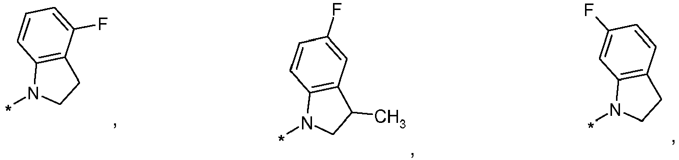 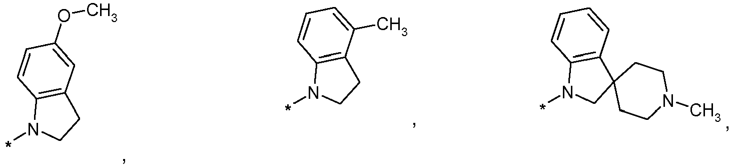 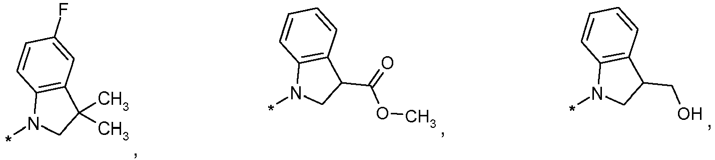 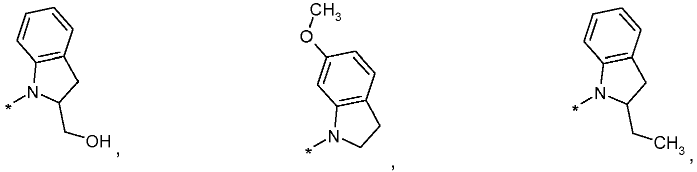 and the ring 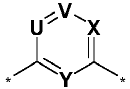 denotes a group selected from 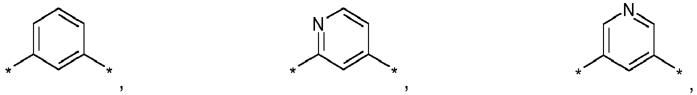 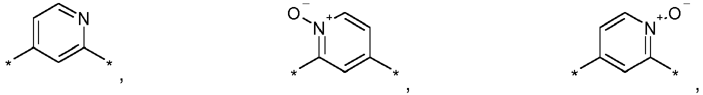 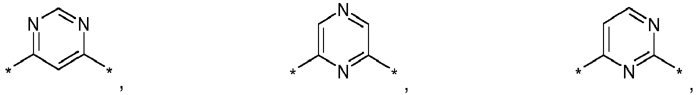 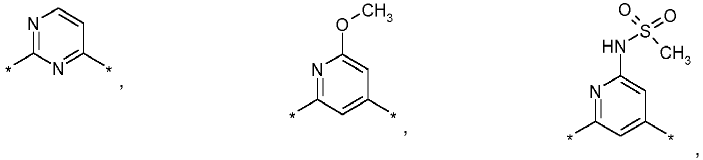 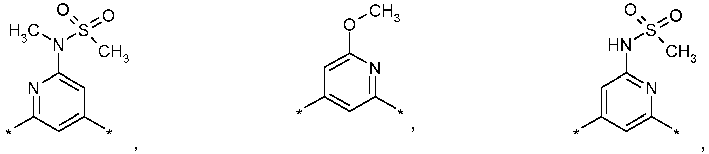 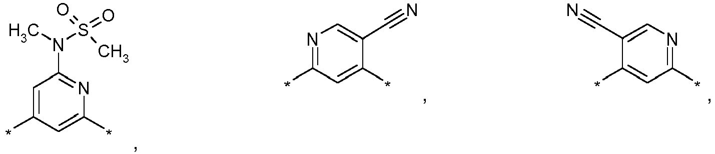 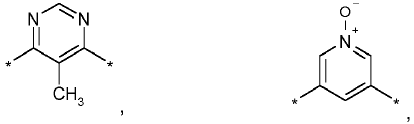 the tautomers, the diastereomers, the enantiomers, the hydrates, the mixtures thereof and the salts thereof as well as the hydrates of the salts, particularly the physiologically acceptable salts thereof with inorganic or organic acids or bases.

2. The following compounds of general formula **I** according to claim 1:
| No. | Structure |
|---|---|
| (1) | 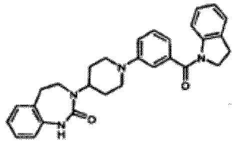 |
| (2) | 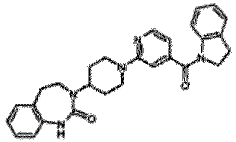 |
| (3) | 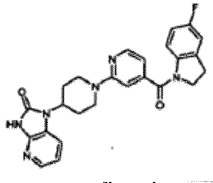 |
| (4) | 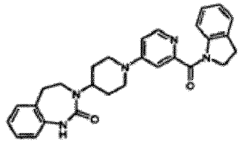 |
| (5) | 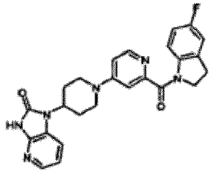 |
| (6) | 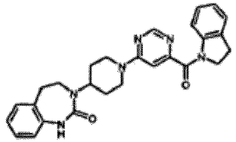 |
| (7) | 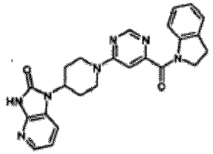 |
| (8) | 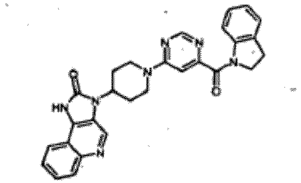 |
| (9) | 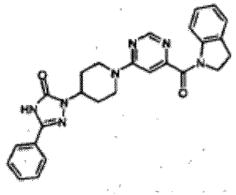 |
| (10) | 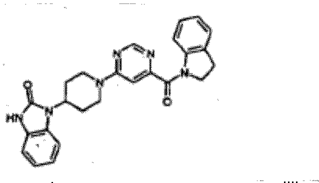 |
| (11) | 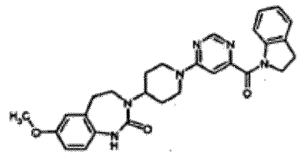 |
| (12) | 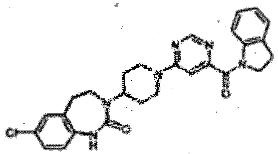 |
| (13) | 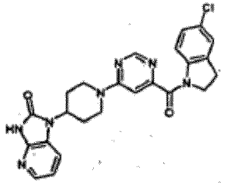 |
| (14) | 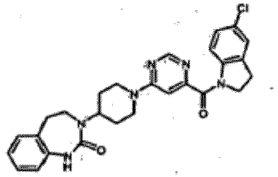 |
| (15) | 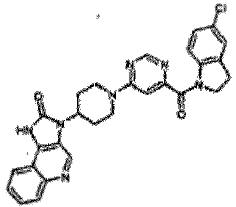 |
| (16) | 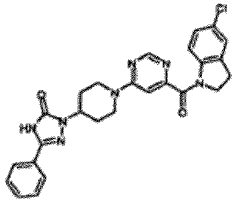 |
| (17) | 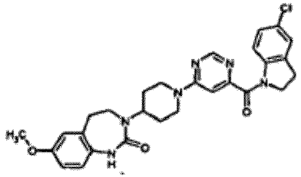 |
| (18) | 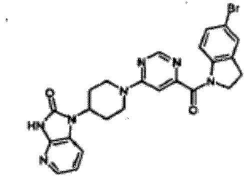 |
| (19) | 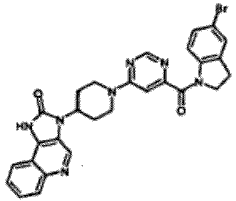 |
| (20) | 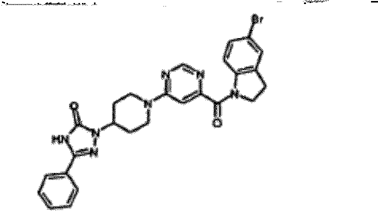 |
| (21) | 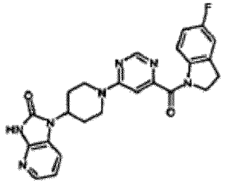 |
| (22) | 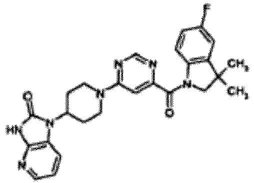 |
| (23) | 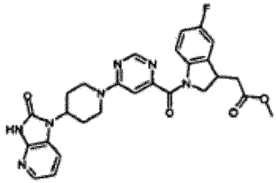 |
| (24) | 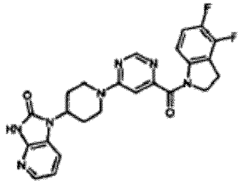 |
| (25) | 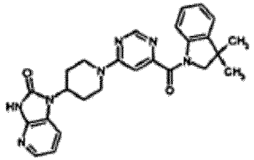 |
| (26) | 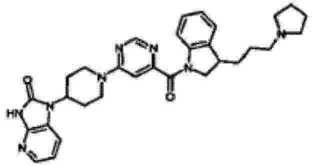 |
| (27) | 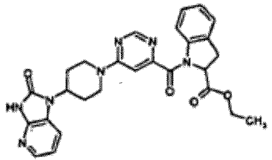 |
| (28) | 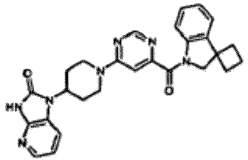 |
| (29) | 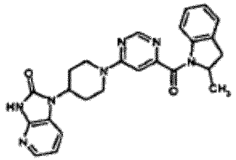 |
| (30) | 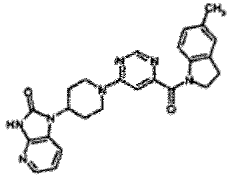 |
| (31) | 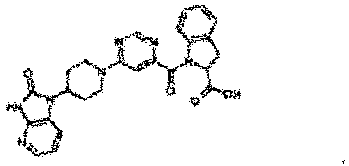 |
| (32) | 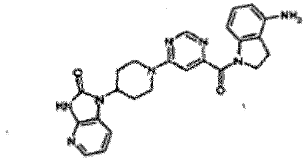 |
| (33) | 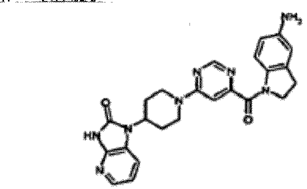 |
| (34) | 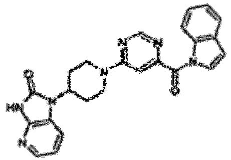 |
| (35) | 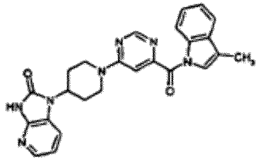 |
| (36) | 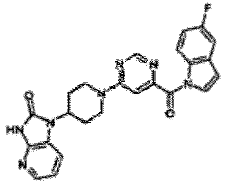 |
| (37) | 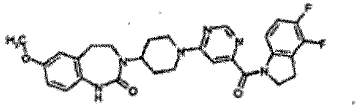 |
| (38) | 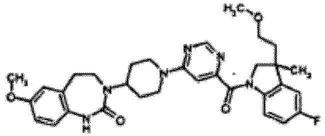 |
| (39) | 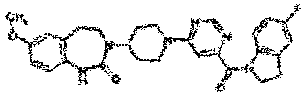 |
| (40) | 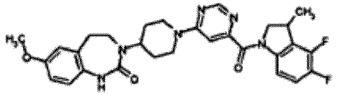 |
| (41) | 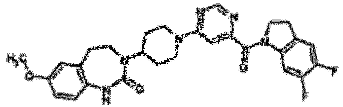 |
| (42) | 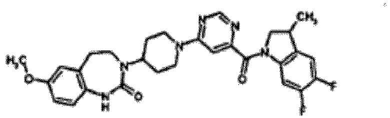 |
| (43) | 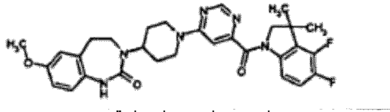 |
| (44) | 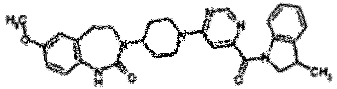 |
| (45) | 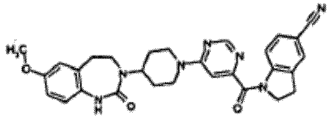 |
| (46) | 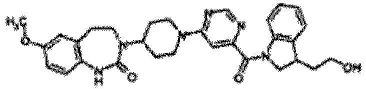 |
| (47) | 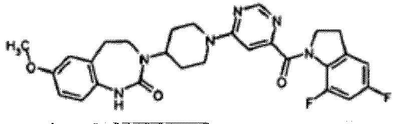 |
| (48) | 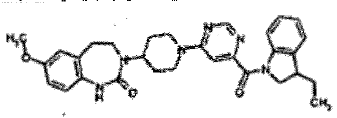 |
| (49) | 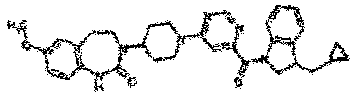 |
| (50) | 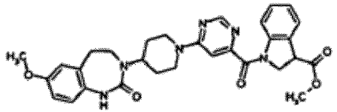 |
| (51) | 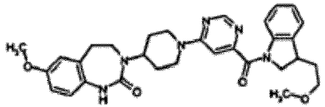 |
| (52) | 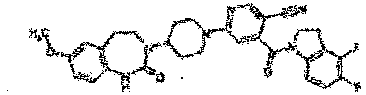 |
| (53) | 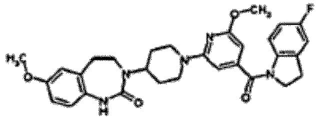 |
| (54) | 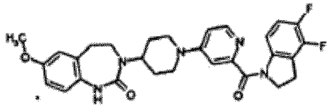 |
| (55) | 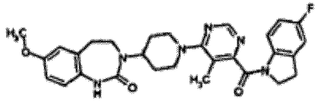 |
| (56) | 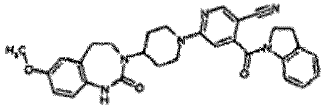 |
| (57) | 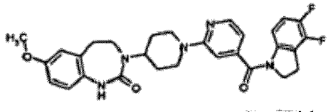 |
| (58) | 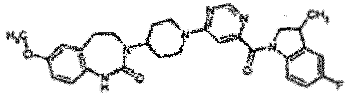 |
| (59) | 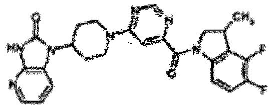 |
| (60) | 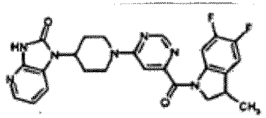 |
| (61) | 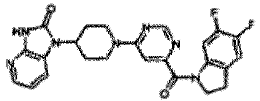 |
| (62) | 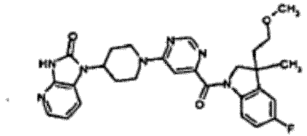 |
| (63) | 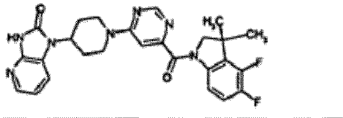 |
| (64) | 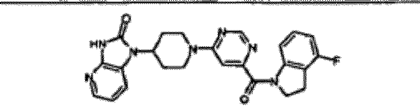 |
| (65) | 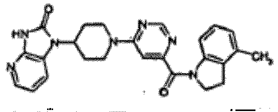 |
| (66) | 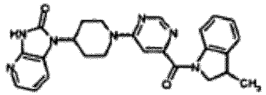 |
| (67) | 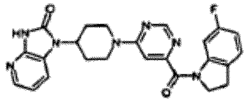 |
| (68) | 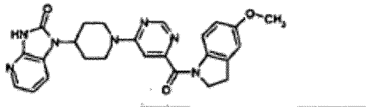 |
| (69) | 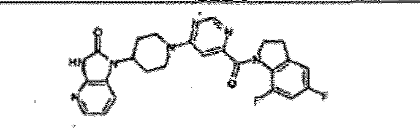 |
| (70) | 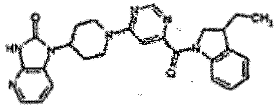 |
| (71) | 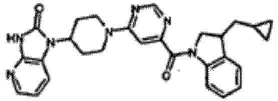 |
| (72) | 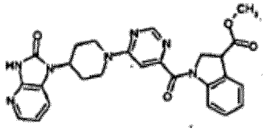 |
| (73) | 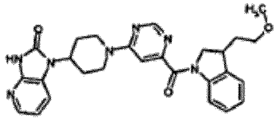 |
| (74) | 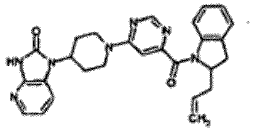 |
| (75) | 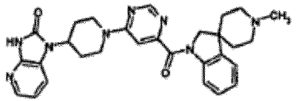 |
| (76) | 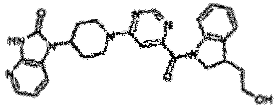 |
| (77) | 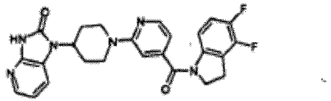 |
| (78) | 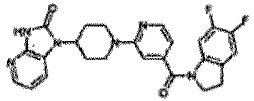 |
| (79) | 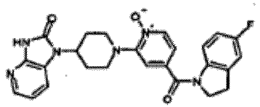 |
| (80) | 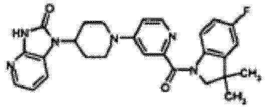 |
| (81) | 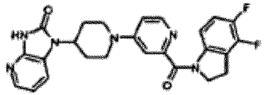 |
| (82) | 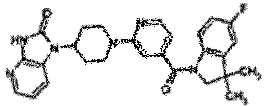 |
| (83) | 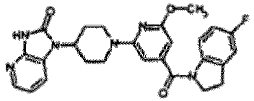 |
| (84) | 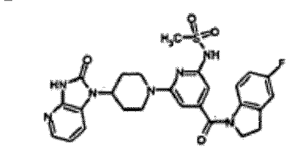 |
| (85) | 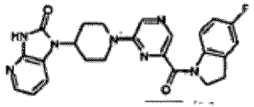 |
| (86) | 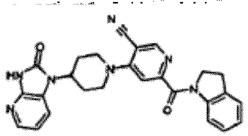 |
| (87) | 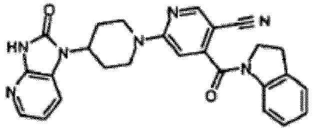 |
| (88) | 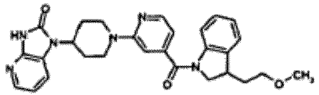 |
| (89) | 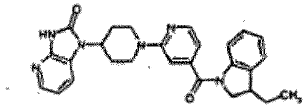 |
| (90) | 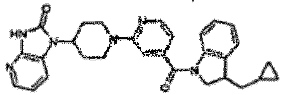 |
| (91) | 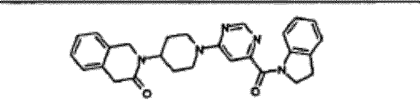 |
| (92) | 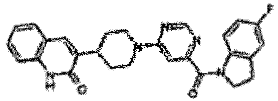 |
| (93) | 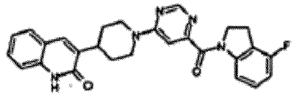 |
| (94) | 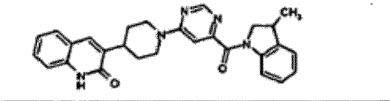 |
| (95) | 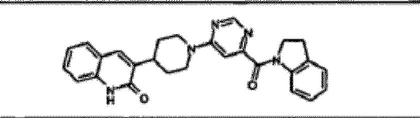 |
| (96) | 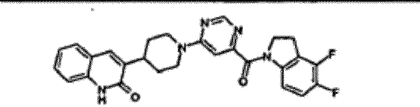 |
| (97) | 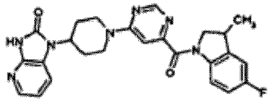 |
| (98) | 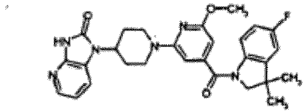 |
| (99) | 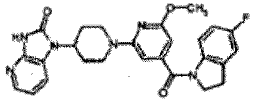 |
| (100) | 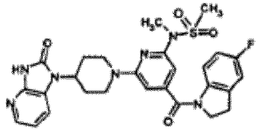 |
| (101) | 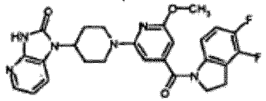 |
| (102) | 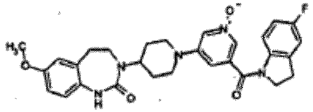 |
| (103) | 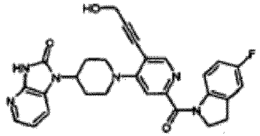 |
| (104) | 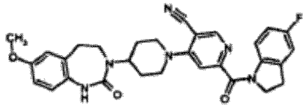 |
| (105) | 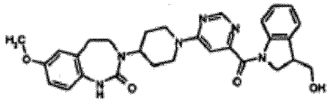 |
| (106) | 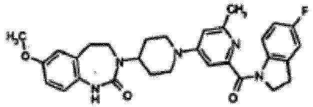 |
| (107) | 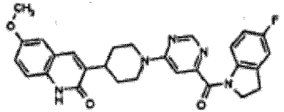 |
| (108) | 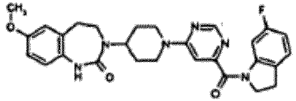 |
| (109) | 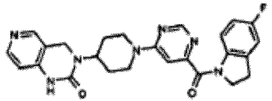 |
| (110) | 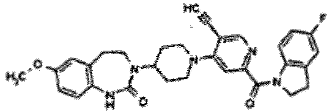 |
| (111) | 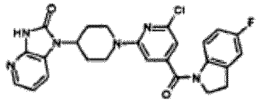 |
| (112) | 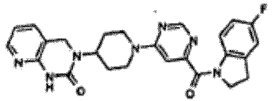 |
| (113) | 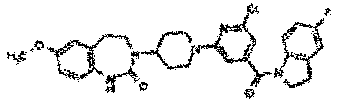 |
| (114) | 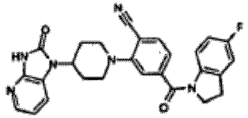 |
| (115) | 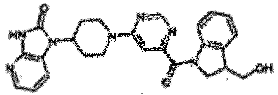 |
| (116) | 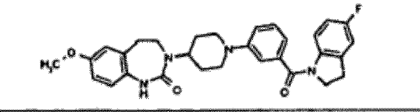 |
| (117) | 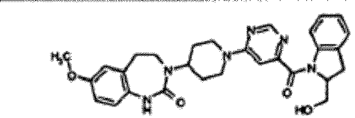 |
| (118) | 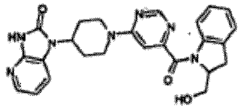 |
| (119) | 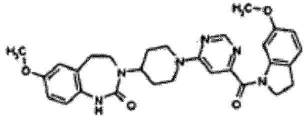 |
| (120) | 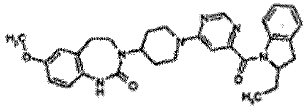 |
the enantiomers, the diastereomers, the hydrates, the mixtures thereof and the salts thereof as well as the hydrates of the salts, particularly the physiologically acceptable salts thereof with inorganic or organic acids or bases.

3. Physiologically acceptable salts of the compounds according to one of claims 1 to 2 with inorganic or organic acids or bases.

4. Medicaments, containing a compound according to one of claims 1 to 2 or a physiologically acceptable salt according to claim 3 optionally together with one or more inert carriers and/or diluents.

5. Use of a compound according to one of claims 1 to 3 for preparing a medicament for the acute and prophylactic treatment of headaches, particularly migraine or cluster headaches as well as tension headaches.

6. Use of a compound according to one of claims 1 to 3 for preparing a medicament for treating non-insulin-dependent diabetes mellitus ("NIDDM"), cardiovascular diseases, morphine tolerance, diarrhoea caused by clostridium toxin, skin diseases, particularly thermal and radiation-induced skin damage including sunburn, lichen, pruritis, pruritic taxidermies and severe itching, inflammatory diseases, e.g. inflammatory diseases of the joints (osteoarthritis, rheumatoid arthritis, neurogenic arthritis), generalised soft-tissue rheumatism (fibromyalgia), neurogenic inflammation of the oral mucosa, inflammatory lung diseases, allergic rhinitis, asthma, COPD, diseases accompanied by excessive vasodilatation and resultant reduced blood supply to the tissues, e.g. shock and sepsis, chronic pain, e.g. diabetic neuropathies, neuropathies induced by chemotherapy, HIV-induced neuropathies, post-herpetic neuropathies, neuropathies induced by tissue trauma, trigeminal neuralgias, temporomandibular dysfunctions, CRPS (complex regional pain syndrome), back pain, and visceral complaints, such as e.g. irritable bowel syndrome (IBS), inflammatory bowel syndrome, for relieving pain in general, or for preventive or acute therapeutic treatment of the symptoms of menopausal hot flushes caused by vasodilatation and increased blood flow in oestrogen-deficient women and hormone-treated patients with prostate carcinoma and castrated men.

7. Use of a compound according to one of claims 1 to 3 for preparing a medicament for the acute and prophylactic treatment of migraine and cluster headaches, for the treatment of irritable bowel syndrome (IBS) and for the preventive and acute therapeutic treatment of hot flushes in oestrogen-deficient women.

8. Method of preparing a medicament according to claim 4, **characterised in that** a compound according to one of claims 1 to 3 is incorporated in one or more inert carriers and/or diluents by a non-chemical method.

## Revendications

1. Composés de formule générale I 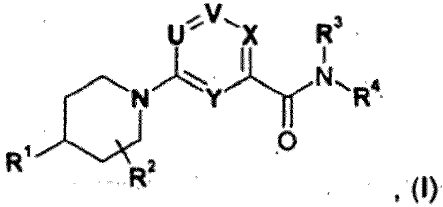 dans laquelle
**R¹** représente un groupe choisi parmi 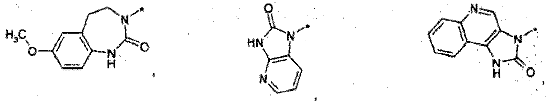 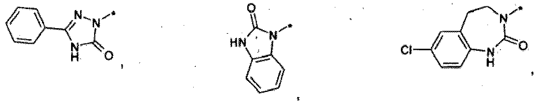 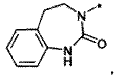
**R³** et **R⁴** conjointement avec l'atome d'azote auquel ils sont liés, représentent un groupe choisi parmi 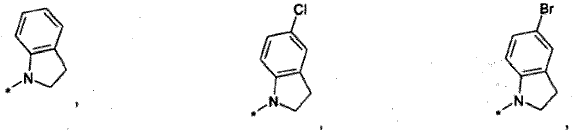 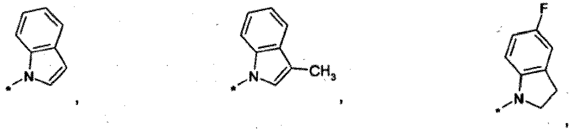 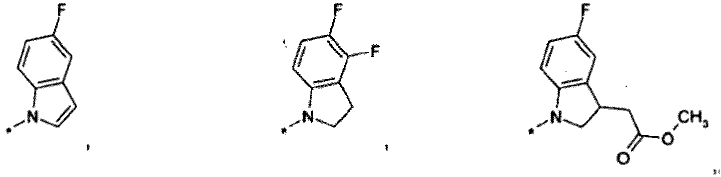 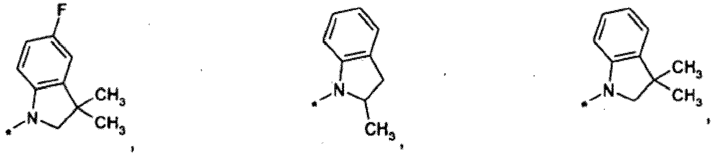 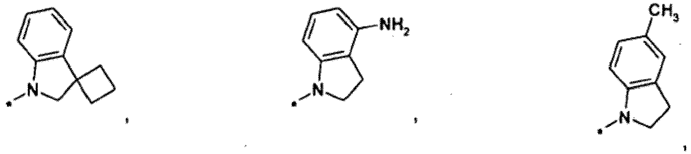 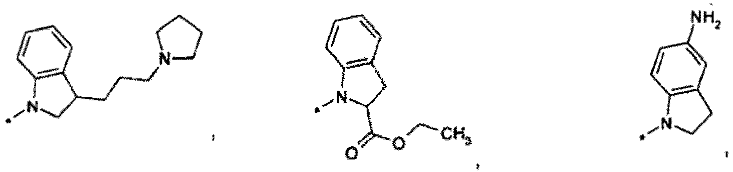 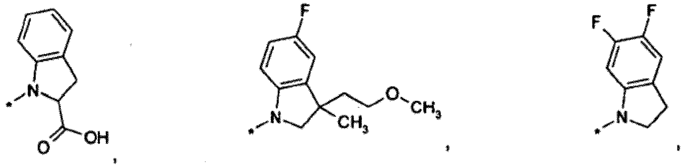 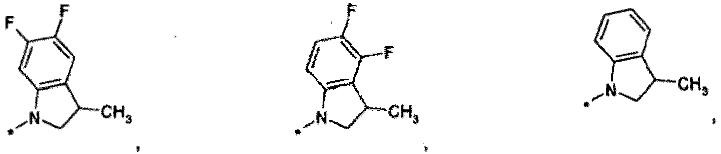 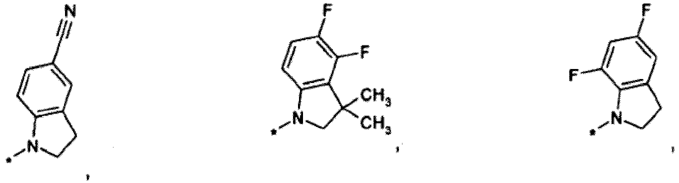 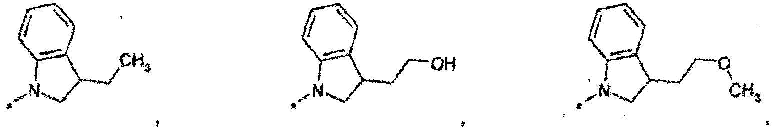 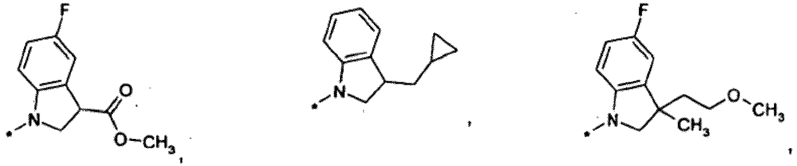 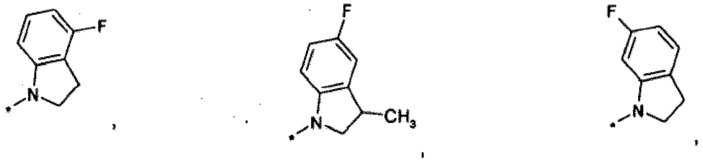 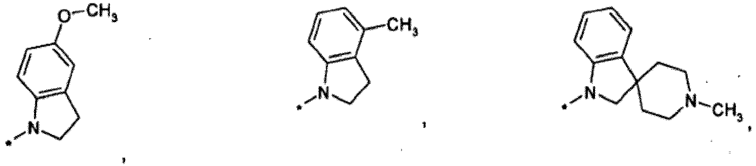 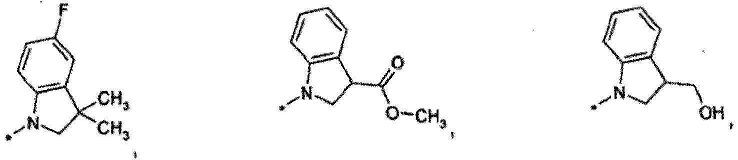 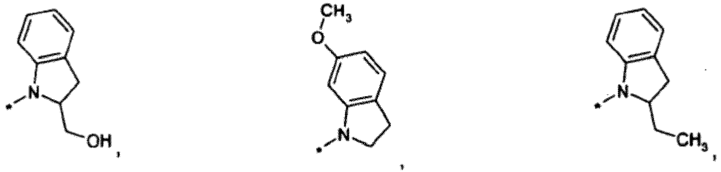 et le cycle 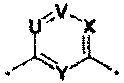 représente un groupe choisi parmi 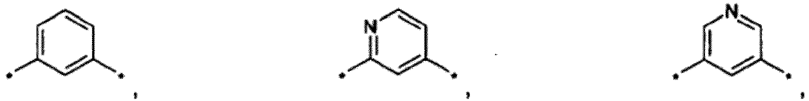 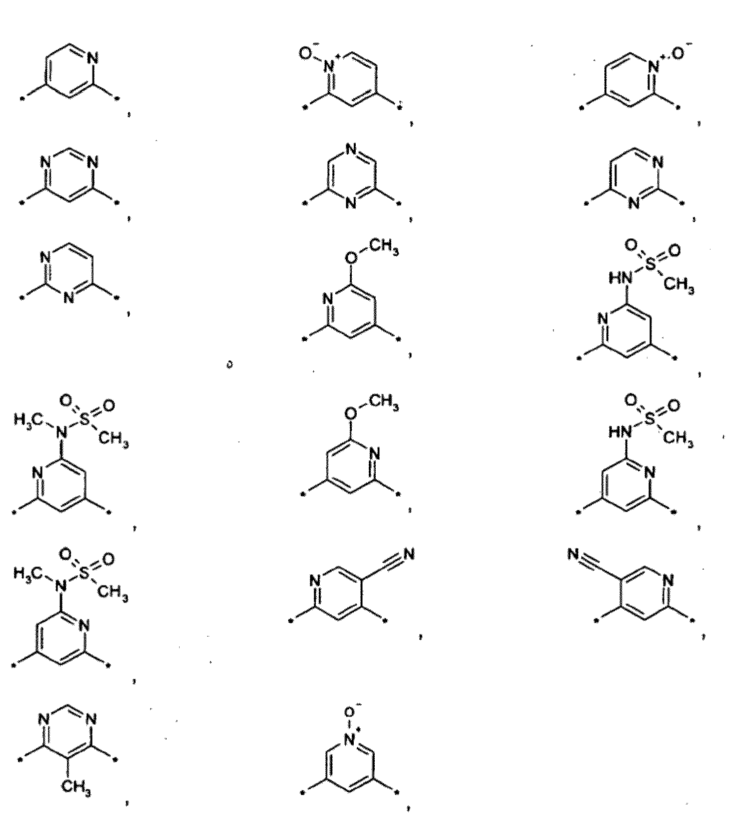 leurs tautomères, leurs diastéréomères, leurs énantiomères, leurs hydrates, leurs mélanges et leurs sels ainsi que les hydrates des sels, en particulier leurs sels physiologiquement acceptables avec des acides ou des bases inorganiques ou organiques.

2. Composés de formule générale I suivants, selon la revendication 1 :
| **N°** | **Structure** |
|---|---|
| (1) | 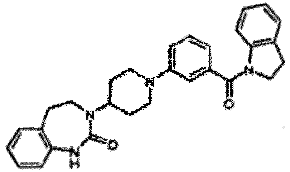 |
| (2) | 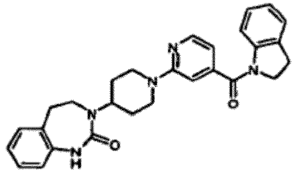 |
| (3) | 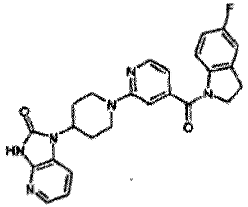 |
| (4) | 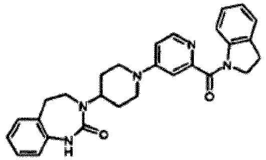 |
| (5) | 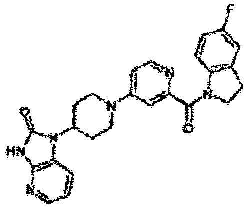 |
| (6) | 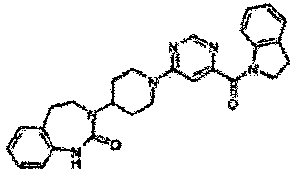 |
| (7) | 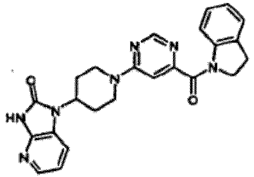 |
| (8) | 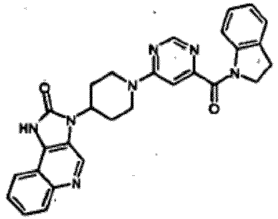 |
| (9) | 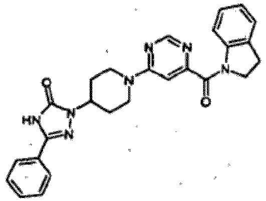 |
| (10) | 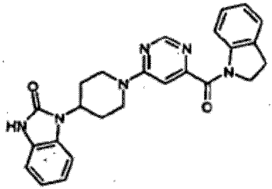 |
| (11) | 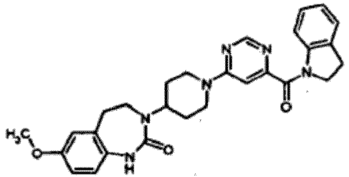 |
| (12) | 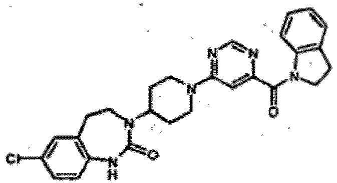 |
| (13) | 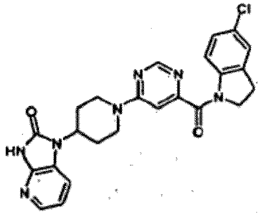 |
| (14) | 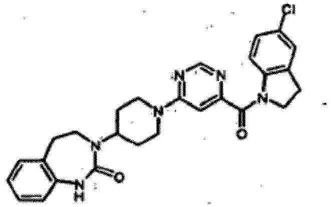 |
| (15) | 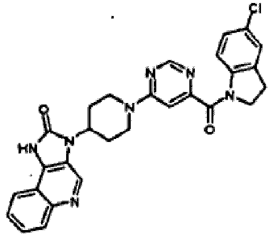 |
| (16) | 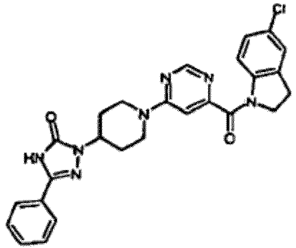 |
| (17) | 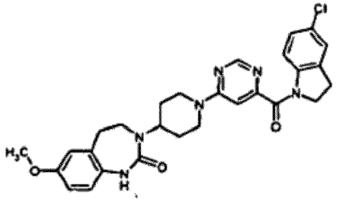 |
| (18) | 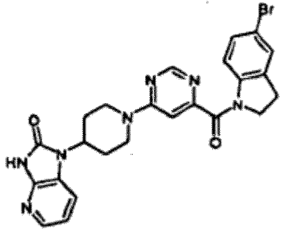 |
| (19) | 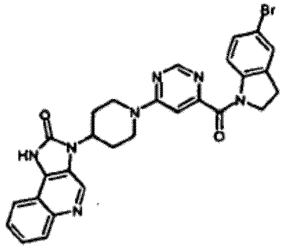 |
| (20) | 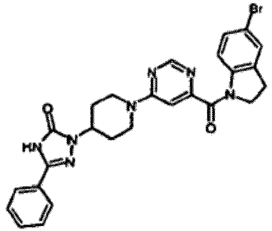 |
| (21) | 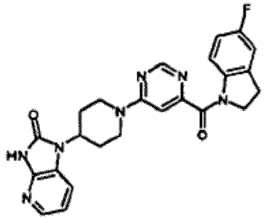 |
| (22) | 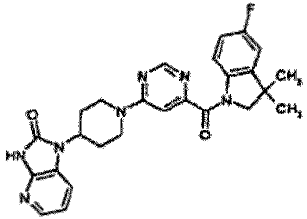 |
| (23) | 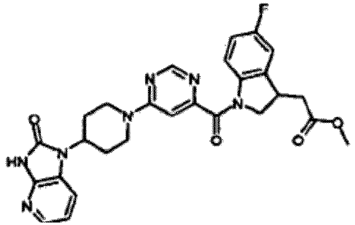 |
| (24) | 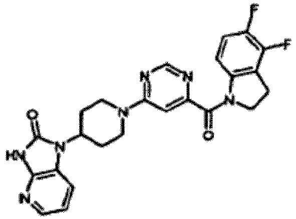 |
| (25) | 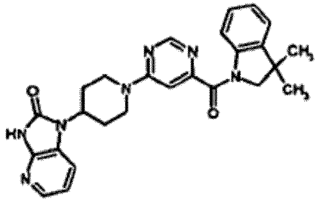 |
| (26) | 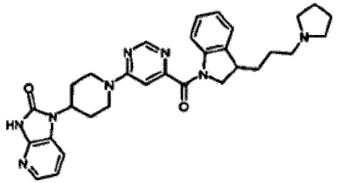 |
| (27) | 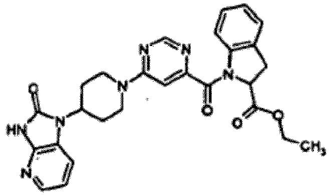 |
| (28) | 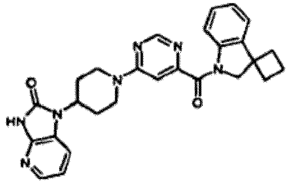 |
| (29) | 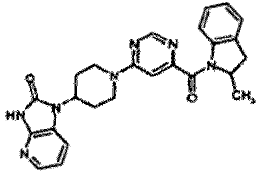 |
| (30) | 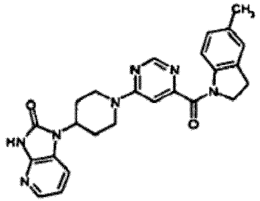 |
| (31) | 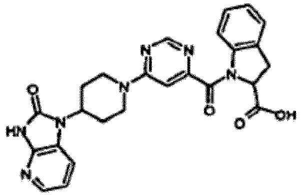 |
| (32) | 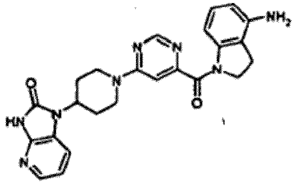 |
| (33) | 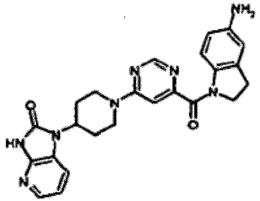 |
| (34) | 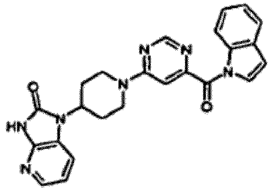 |
| (35) | 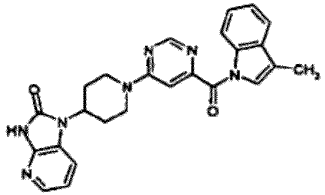 |
| (36) | 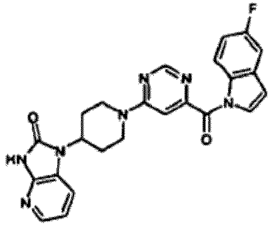 |
| (37) | 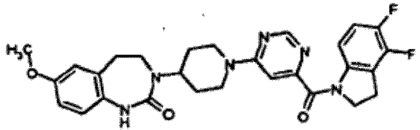 |
| (38) | 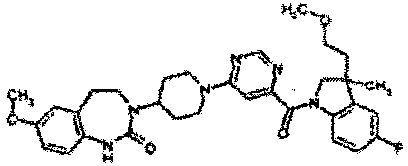 |
| (39) | 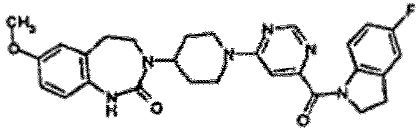 |
| (40) | 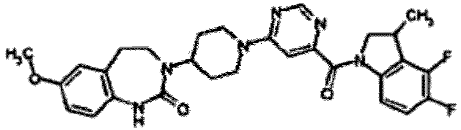 |
| (41) | 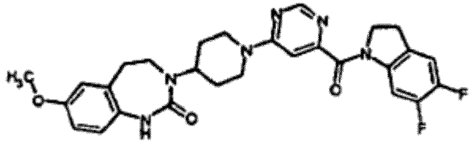 |
| (42) | 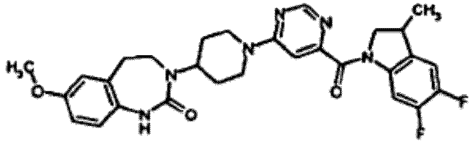 |
| (43) | 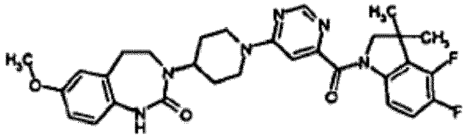 |
| (44) | 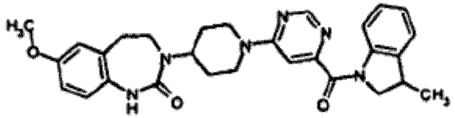 |
| (45) | 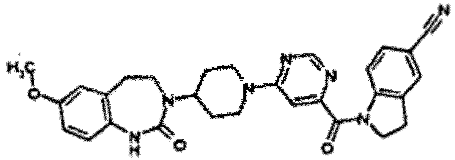 |
| (46) | 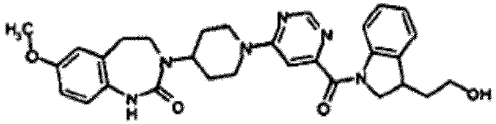 |
| (47) | 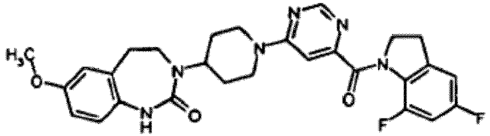 |
| (48) | 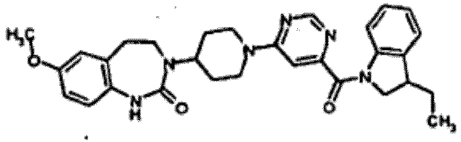 |
| (49) | 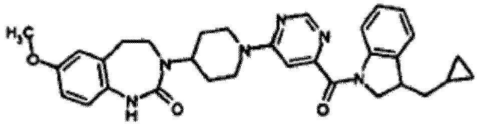 |
| (50) | 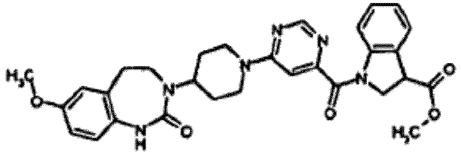 |
| (51) | 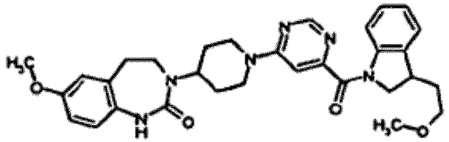 |
| (52) | 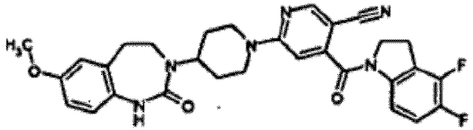 |
| (53) | 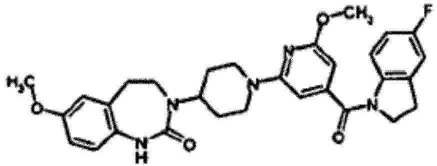 |
| (54) | 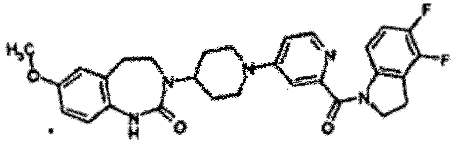 |
| (55) | 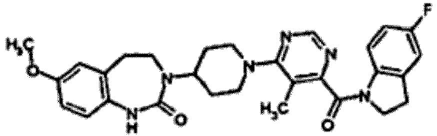 |
| (56) | 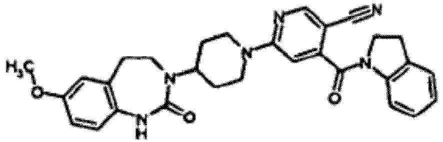 |
| (57) | 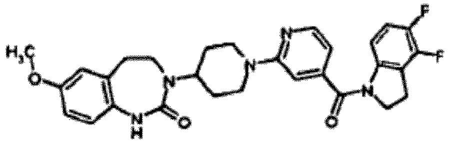 |
| (58) | 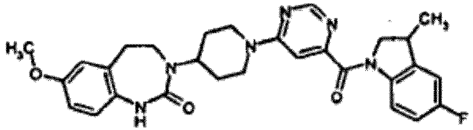 |
| (59) | 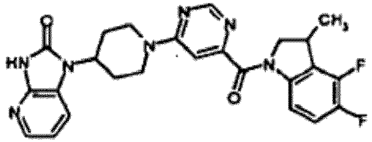 |
| (60) | 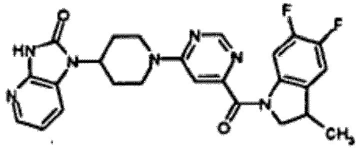 |
| (61) | 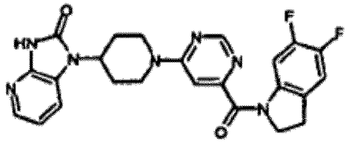 |
| (62) | 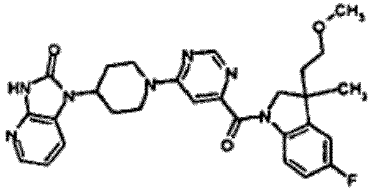 |
| (63) | 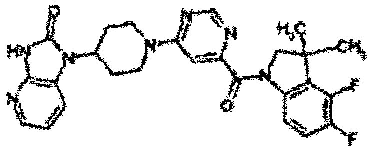 |
| (64) | 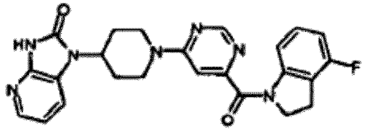 |
| (65) | 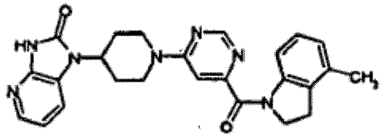 |
| (66) | 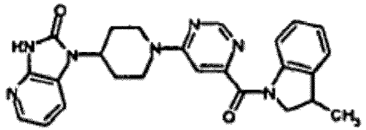 |
| (67) | 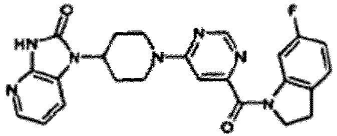 |
| (68) | 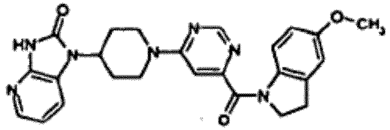 |
| (69) | 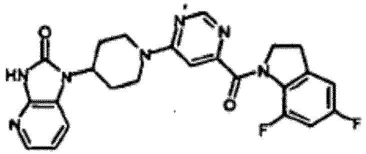 |
| (70) | 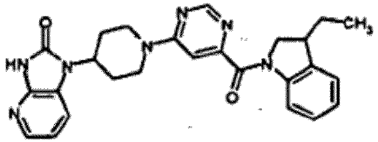 |
| (71) | 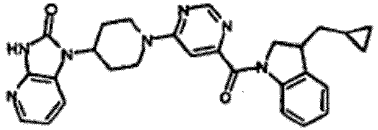 |
| (72) | 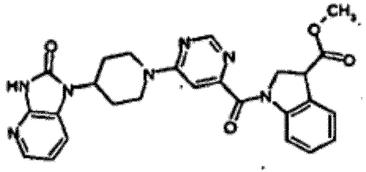 |
| (73) | 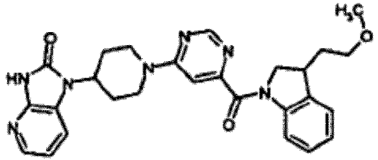 |
| (74) | 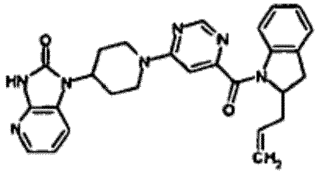 |
| (75) | 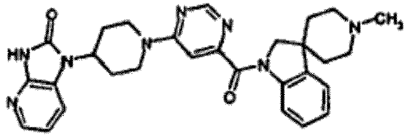 |
| (76) | 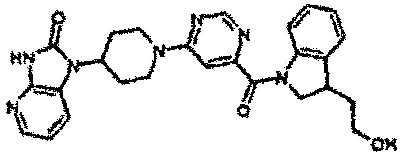 |
| (77) | 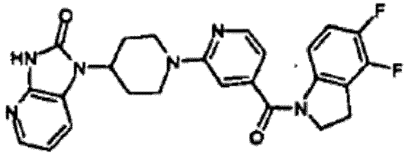 |
| (78) | 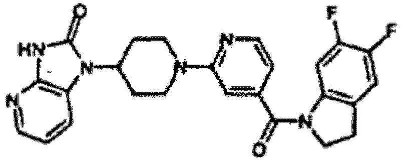 |
| (79) | 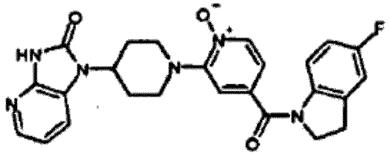 |
| (80) | 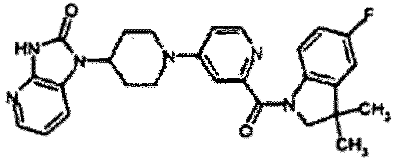 |
| (81) | 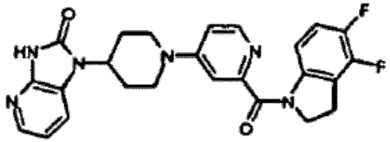 |
| (82) | 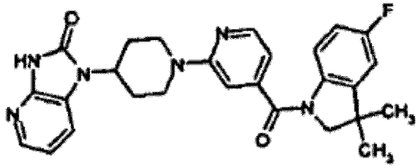 |
| (83) | 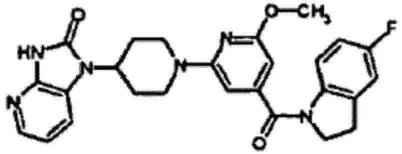 |
| (84) | 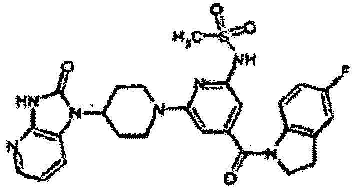 |
| (85) | 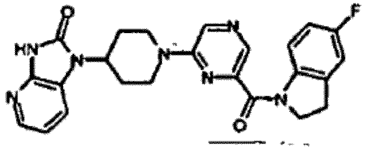 |
| (86) | 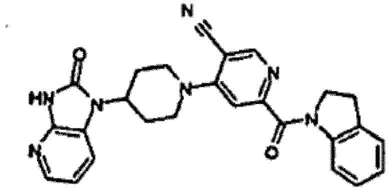 |
| (87) | 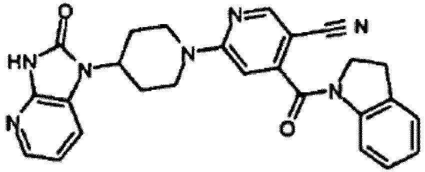 |
| (88) | 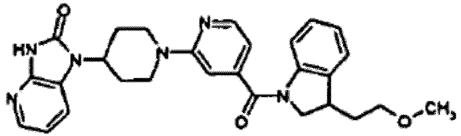 |
| (89) | 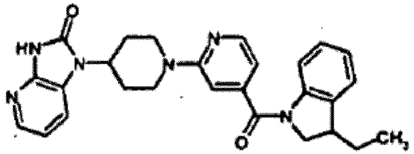 |
| (90) | 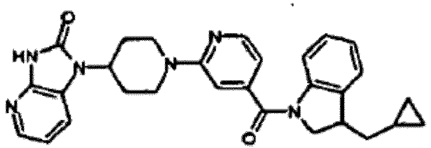 |
| (91) | 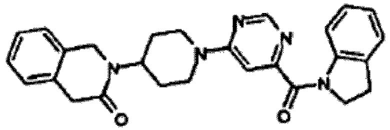 |
| (92) | 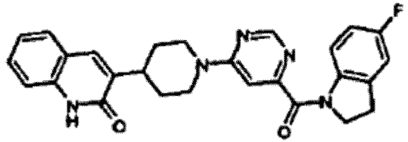 |
| (93) | 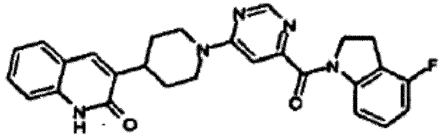 |
| (94) | 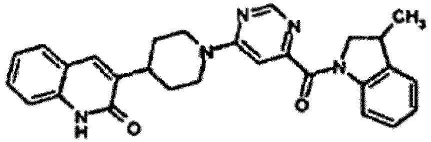 |
| (95) | 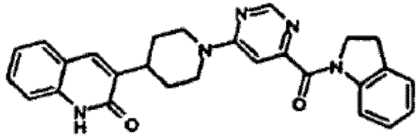 |
| (96) | 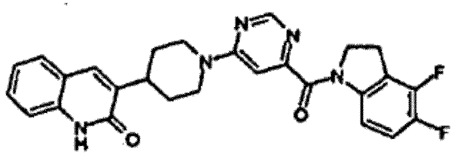 |
| (97) | 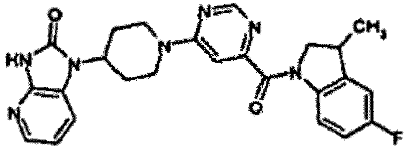 |
| (98) | 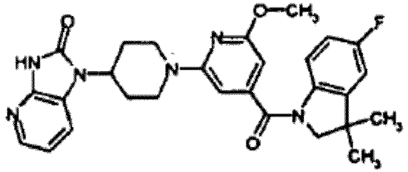 |
| (99) | 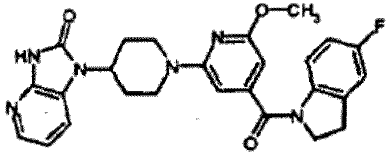 |
| (100) | 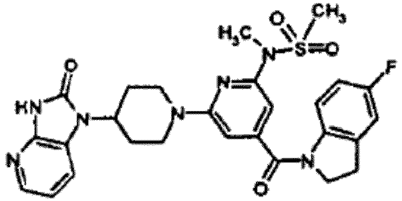 |
| (101) | 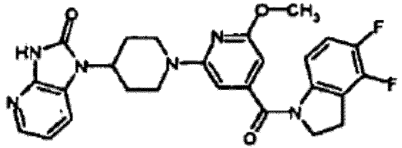 |
| (102) | 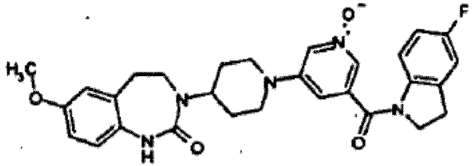 |
| (103) | 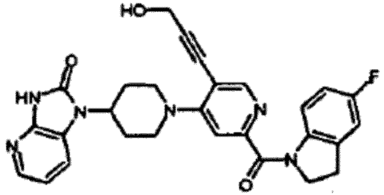 |
| (104) | 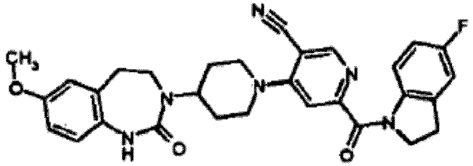 |
| (105) | 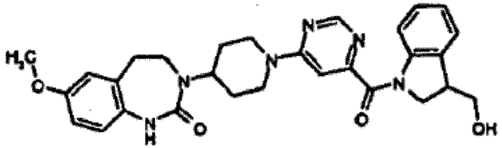 |
| (106) | 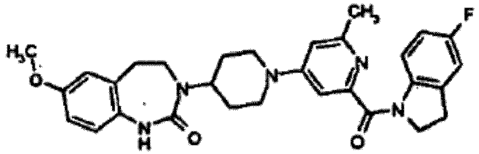 |
| (107) | 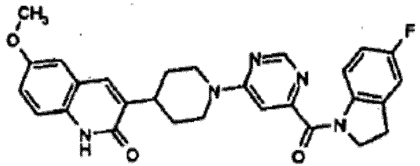 |
| (108) | 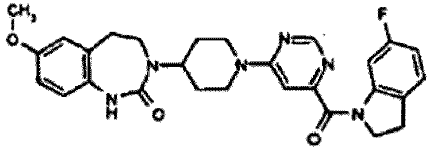 |
| (109) | 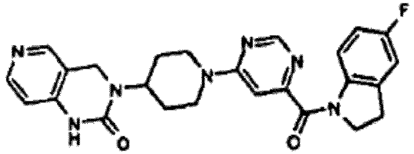 |
| (110) | 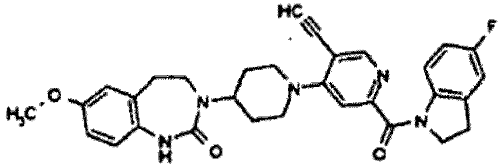 |
| (111) | 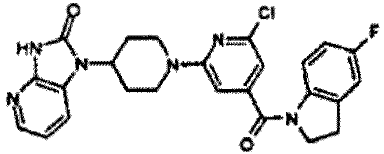 |
| (112) | 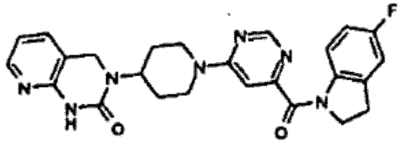 |
| (113) | 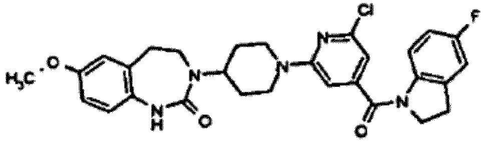 |
| (114) | 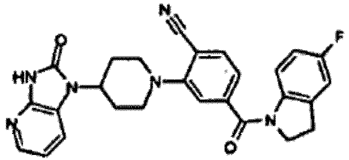 |
| (115) | 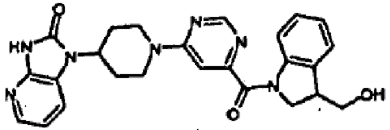 |
| (116) | 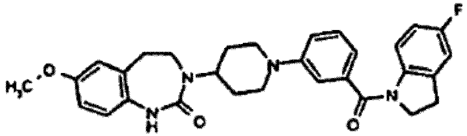 |
| (117) | 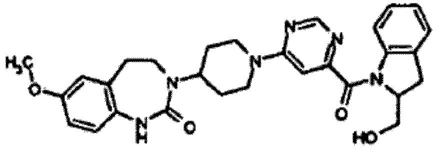 |
| (118) | 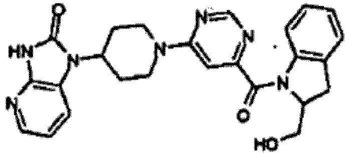 |
| (119) | 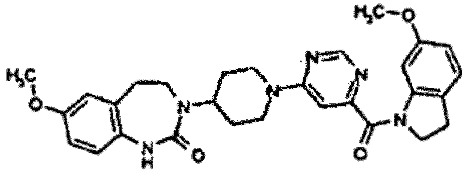 |
| (120) | 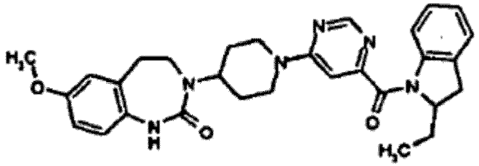 |
leurs énantiomères, leurs diastéréomères, leurs hydrates, leurs mélanges et leurs sels ainsi que les hydrates des sels, en particulier leurs sels physiologiquement acceptables avec des acides ou des bases inorganiques ou organiques.

3. Sels physiologiquement acceptables des composés selon l'une des revendications 1 à 2, avec des acides ou des bases inorganiques ou organiques.

4. Médicaments contenant un composé selon l'une des revendications 1 à 2 ou un sel physiologiquement acceptable selon la revendication 3, outre éventuellement un ou plusieurs véhicules et/ou diluants inertes.

5. Utilisation d'un composé selon l'une des revendications 1 à 3 pour la production d'un médicament pour le traitement aigu et prophylactique des céphalées, en particulier de la migraine et de l'algie vasculaire de la face ainsi que des céphalées de tension.

6. Utilisation d'un composé selon l'une des revendications 1 à 3, pour la production d'un médicament pour le traitement du diabète sucré non insulino-dépendant (« DNID »), des affections cardiovasculaires, de la tolérance à la morphine, des diarrhées dues à la toxine Clostridium, des affections cutanées, en particulier des dommages cutanés thermiques et dus à un rayonnement comprenant les coups de soleil, le lichen, le prurigo, les toxidermies prurigineuses ainsi que des prurits sévères, des affections inflammatoires, par exemple, des affections articulaires inflammatoires (arthrose, polyarthrite rhumatoïde, arthrite neurogène), du rhumatisme généralisé des parties molles (fibromyalgie), des inflammations neurogènes de la muqueuse buccale, des affections pulmonaires inflammatoires, de la rhinite allergique, de l'asthme, de la BPCO, des affections s'accompagnant d'une vasodilatation excessive et de la réduction de l'irrigation vasculaire qui en découle, par exemple, choc septique et sepsis, des affections douloureuses chroniques, comme par exemple, des neuropathies diabétiques, des neuropathies induites par chimiothérapies, des neuropathies induites par le VIH, des neuropathies post-herpétiques, des neuropathies induites par un traumatisme tissulaire, des névralgies du trijumeau, des dysfonctionnements temporo-mandibulaires, du CRPS (syndrome douloureux régional complexe), des douleurs dorsales et des affections viscérales, comme par exemple, le syndrome du côlon irritable (SCI), le syndrome intestinal inflammatoire, pour l'atténuation des états douloureux en général ou pour le traitement préventif ou thérapeutique aigu des symptômes de la ménopause, des bouffées de chaleurs dues à une vasodilatation et une augmentation du débit sanguin chez les femmes présentant un déficit en estrogènes ainsi que des patients atteints d'un cancer de la prostate soumis à un traitement hormonal et des sujets soumis à une castration.

7. Utilisation d'un composé selon l'une des revendications 1 à 3, pour la production d'un médicament pour le traitement aigu et prophylactique de la migraine et de l'algie vasculaire de la face, pour le traitement du syndrome du côlon irritable (SCI) et pour le traitement préventif et thérapeutique aigu des bouffées de chaleur chez les femmes présentant un déficit en oestrogène.

8. Procédé de production d'un médicament selon la revendication 4, **caractérisé en ce que**, de façon non chimique, un composé selon l'une des revendications 1 à 3 est incorporé dans un ou plusieurs véhicules et/ou diluants inertes.
